# EUROPEAN PATENT APPLICATION

(11) **EP 4 389 762 A1**
(43) Date of publication of application: **26.06.2024**
(21) Application number: 22216472.5
(22) Date of filing: 23.12.2022
(51) Int. Cl.: C07K 14/47, A61K 38/00, A61P 21/00, A61P 35/02, C12N 15/70, C12N 15/85, C12N 15/86

(54) **INHIBITORS OF DUX4 ACTIVITY AND THEIR USE IN THERAPY.**

(71) Applicant: Ospedale San Raffaele S.r.l., 20132 Milano (IT); Fondazione Centro San Raffaele, 20132 Milano (IT)
(72) Inventor: Gabellini, Davide, 20132 Milan (MI) (IT); Runfola, Valeria, 20132 Milan (MI) (IT); Ghezzi, Paola, 20132 Milan (MI) (IT); Salomè, Mara, 20132 Milan (MI) (IT); Musco, Giovanna, 20132 Milan (MI) (IT); Berardi, Andrea, 20132 Milan (MI) (IT)
(74) Representative: Ghirardi, Valeria

(57) **Abstract**

The present invention refers to a peptide or variants thereof wherein the peptide essentially consists of:
- an amino acid sequence comprised in the sequence from aa. 1 to aa. 288 of matrin 3 (MATR3) (SEQ ID NO:1), or
- a variant of an amino acid sequence comprised in the sequence from aa. 1 to aa. 288 of matrin 3 (MATR3),
with the proviso that the peptide does not consist of the amino acid sequence from aa. 1 to aa. 287 of matrin 3 (SEQ ID NO: 17),
and preferably wherein amino acid sequence is comprised in the sequence from aa. 1 to aa. 287 or from aa 2 to aa. 288 (SEQ ID NO: 18) or from aa. 2 to aa. 287 (SEQ ID NO: 19)

## Description

The present invention refers to a peptide or variants thereof wherein the peptide essentially consists of:
- an amino acid sequence comprised in the sequence from aa. 1 to aa. 288 of matrin 3 (MATR3) (SEQ ID NO:1), or
- a variant of an amino acid sequence comprised in the sequence from aa. 1 to aa. 288 of matrin 3 (MATR3),
with the proviso that the peptide does not consist of the amino acid sequence from aa. 1 to aa. 287 of matrin 3 (SEQ ID NO: 17),
and preferably wherein amino acid sequence is comprised in the sequence from aa. 1 to aa. 287 or from aa 2 to aa. 288 (SEQ ID NO: 18) or from aa. 2 to aa. 287 (SEQ ID NO: 19)

The present invention also refers to medical uses of such peptides, in particular for the treatment of Acute lymphoblastic leukemia and Facioscapulohumeral muscular dystrophy.

### BACKGROUND ART

Double homeobox 4 (DUX4) encodes for a transcription factor with increasingly important roles in normal physiology and in disease. Expression of DUX4 is normally confined to the cleavage stage of embryonic development, where it regulates genes important for pre- and post-implantation development. At the 8-cell stage DUX4 expression is repressed and remains normally silenced for the rest of our life. DUX4 presents a DNA-binding domain (dbd) located at its N-terminus and composed of two closely spaced 60-aminoacid homeodomains, HD1 and HD2, which are responsible for sequence-specific DNA binding. At the C-terminal region is located the DUX4 transcription activation domain (TAD) responsible for target gene activation. DUX4 aberrant reactivation or gene rearrangements are associated with several diseases, including facioscapulohumeral muscular dystrophy (FSHD) and acute lymphoblastic leukemia.

### Facioscapulohumeral muscular dystrophy

FSHD is one of the most common neuromuscular diseases, affecting 1:8000 individuals, with close to 1 million patients worldwide. The disease is caused by aberrant reactivation of DUX4 expression in skeletal muscle, leading to activation of pathways toxic to muscle tissue including inhibition of myogenic differentiation and cell apoptosis.

### Acute lymphoblastic leukemia

DUX4 translocation to the immunoglobulin heavy chain locus and consequent overexpression of rearranged DUX4 (DUX4-r) in B cell precursors causes 10% of acute lymphoblastic leukemia (ALL) cases, the most common cancer in children and the most frequent cause of cancer death at young age. DUX4-r always maintain the dbd of wild-type DUX4, while it presents variable size deletions and substitutions affecting the TAD. DUX4-r blocks pro-B cell differentiation and stimulate proliferation inducing leukemia. Based on the above information, blocking the activity of DUX4 and DUX4-r represents a plausible therapeutic option for FSHD and ALL, respectively. Previous analyses from the present inventors (see WO2020152367, herein incorporated by reference) identified MATRIN 3 (MATR3) as the first endogenous protein able to inhibit both wt DUX4 and DUX4-r activity. Inventors found that full-length MATR3 (MATR3₁₋₈₄₇) interacts with both wt DUX4 and DUX4-r. Moreover, MATR3₁₋₈₄₇ reduces the expression of wt DUX4 targets rescuing DUX4 toxicity in primary muscle cells from FSHD patients.

### Status of FSHD therapeutics

Currently, the therapeutic approaches under development for the treatment of FSHD are aimed at blocking DUX4 expression or treating downstream pathogenic signs of the disease like inflammation or loss of muscle mass (J Pers Med. 2022 May 25; 12(6):865). While intriguing proof of principle studies concerning the possibility to inhibit DUX4 transcription or to degrade its RNA have been published, their major limitation is poor delivery. The inappropriate expression of DUX4 in FSHD can possibly be suppressed by knockdown with antisense oligonucleotides (AON). In vitro, effective knockdown of DUX4 mRNA using AONs has been reported (PLoS One. 2011;6(10):e26820; Hum Mol Genet. 2015 Sep 1;24(17):4817-28; Hum Mol Genet. 2016 Apr 15;25(8):1468-78; Mol Ther. 2016 Aug;24(8):1405-11). One of the challenges in building a therapeutic strategy around AONs is their relatively poor delivery to skeletal muscles. This can be addressed with the use of carriers or conjugates, but the potential immunogenicity/toxicity of these would have to be assessed at efficacious doses. Moreover, to achieve and maintain therapeutic efficacy, AONs may need to be administered in large and repeated doses, which could result in harmful effects (Reactions Weekly 2018;1714:3; Mov Disord. 2021 Jan;36(1):263-264; Adv Drug Deliv Rev. 2015 Jun 29;87:104-7; https://www.science.org/content/article/tailored-genetic-drug-causes-fatal-brain-swelling). An alternative to AON approaches could be the use of nuclease-dead versions of Cas9 fused to effector domains to silence or edit the FSHD locus. While interesting proof of concept have been reported (Mol Ther. 2016 Mar;24(3):527-35; J Med Genet. 2019 Dec;56(12):828-837; Mol Ther Methods Clin Dev. 2020 Dec 10;20:298-311), significant concern remains regarding the immunogenicity related to CRISPR components and the risks of off-targets and genome instability effects. Finally, several compounds causing DUX4 downregulation have been reported. Notably, they are not "on target approaches" because none of them targets directly DUX4. Instead, they target various kinases affecting DUX4 indirectly through an unknown mechanism. Moreover, kinase inhibitors are notorious for their pleiotropic effects and the high frequency of acquired resistance. To date, only one putative small-molecule inhibitor of DUX4 expression has reached clinical trials. The p38 inhibitor losmapimod is currently undergoing a Phase III clinical trial for FSHD (ClinicalTrials.gov Identifier: NCT04264442), but the results thus far are not encouraging with only nominal benefit at 48 weeks, and the primary endpoint (reduction of DUX4 target gene expression) not met. Hence, any short-term functional improvement thus reported could possibly be DUX4-independent and, in the absence of DUX4 repression to halt new muscle pathology, the long-term benefit is questionable. Moreover, since p38 and its targets regulate many stages of myogenesis, there is concern over potential long-term negative effects. In addition, kinase pathways are notoriously compensatory raising additional concern for long-term inhibition efficacy (J Pers Med. 2022 May 25;12(6):865). DUX4 activates a variety of pathways and is currently unclear which one of them is most important for the FSHD pathogenesis. Hence, it is currently unknown whether targeting single pathways downstream of DUX4 could provide any therapeutic benefit. The molecular mechanism through which DUX4 activity is regulated is poorly understood, severely limiting the development of rational therapeutic approaches to counteract DUX4-induced toxicity in FSHD.

### Status of DUX4-r leukemia therapeutics

Currently, the DUX4-r leukemia is treated non-specifically with chemotherapy. Treatment toxicities combined with regimens that span years mean that childhood cancer treatment is a life-altering experience for those who survive. Most survivors experience chronic side effects, including cardiac, pulmonary, endocrine, and neurological sequelae. None of this is specific to childhood leukemia survivors either, with most people who survive leukemia at any age experiencing long-term side effects of their treatments. Moreover, non-responding/relapsing patients face a dismal prognosis. Hence, the need to find better therapeutic strategies tailored to the specific leukemic lesion.

### SUMMARY OF THE INVENTION

The inventors have found that the first 288 amino acids of MATR3 and portions of the first 288 amino acids (herein also defined as MATR3 fragments or peptides), in particular a region of 46 amino acids, are sufficient to bind DUX4 and inhibits its activity. Inventors herein shown that MATR3 fragments are natural inhibitors of wt DUX4 in FSHD and rearranged DUX4-r in leukemia. MATR3 fragments indeed bind directly to DUX4 DNA-binding domain and blocks activation of DUX4 or DUX4-r targets. In so doing, MATR3 fragments block toxicity of wt DUX4 in FSHD and leukemogenic activity of DUX4-r in ALL. Inventors have now identified MATR3 peptides that are sufficient to block activation of DUX4 targets and induction of apoptosis in the FSHD setting and activation of DUX4-r targets and leukemogenic activity in the ALL setting. This finding opens the possibility to inhibit DUX4 and DUX4-r activity for treating FSHD and ALL, a strategy that has been successful for others transcription factors (Nature. 2009 Nov 12;462(7270):182-8; Proc Natl Acad Sci USA. 2013 Sep 3;110(36):E3445-54; Cell Commun Signal. 2016 Mar 2;14:8). Building on the ability of MATR3 to bind both wt DUX4 and DUX4-r and block their activity, one development of the present findings is a MATR3-based DUX4 targeted protein degradation (TPD), e.g. proteolysis targeting chimaera (PROTAC), combining the ability to block both DUX4/DUX4-r expression and activity within a single inhibitory molecule. Such TPD may be used as therapeutics for e.g. for FSHD or ALL. Compared to other known approaches, peptides are highly selective and efficacious and, at the same time, relatively safe and well-tolerated. Moreover, TPDs have a catalytic mechanism of action, leading to increased durability of response and lower susceptibility to resistance mutations.

Inventors found that the present on target, double edged sword approach allows to combine strong efficacy with minimal side effects.

It is an object of the invention a peptide or variants thereof wherein the peptide essentially consists of:
- an amino acid sequence comprised in the sequence from aa. 1 to aa. 288 of matrin 3 (MATR3) (SEQ ID NO: 1), or
- a variant of an amino acid sequence comprised in the sequence from aa. 1 to aa. 288 of matrin 3 (MATR3),
preferably wherein the amino acid sequence is comprised in the sequence from aa. 1 to aa. 287 (SEQ ID NO:17) or from aa. 2 to aa. 288 (SEQ ID NO:18) or from aa. 2 to aa. 287 (SEQ ID NO: 19). It is therefore an object of the present invention a peptide or variants thereof wherein the peptide essentially consists of:
- an amino acid sequence comprised in the sequence from aa. 1 to aa. 288 of matrin 3 (MATR3) (SEQ ID NO: 1), or
- a variant of an amino acid sequence comprised in the sequence from aa. 1 to aa. 288 of matrin 3 (MATR3),
   with the proviso that the peptide does not consist of the amino acid sequence from aa.1 to aa. 287 of matrin 3 (SEQ ID NO: 17),
   and preferably wherein the amino acid sequence is comprised in the sequence from aa. 1 to aa. 287 or from aa. 2 to aa. 288 (SEQ ID NO:18) or from aa. 2 to aa. 287 (SEQ ID NO:19).

Preferably, said amino acid sequence is long at least about 45, 50, 55, 60 or 61 amino acids, preferably 46 amino acids.

Preferably the amino acid sequence essentially consists of, comprises, or is comprised in the sequence:
- from aa. 210 to aa. 255 of MATR3 (SEQ ID NO:5), or
- from aa. 188 to aa. 248 of MATR3 (SEQ ID NO:3), or
- from aa. 228 to aa. 287 of MATR3 (SEQ ID NO:4), or
- from aa. 209 to aa. 288 of MATR3 (SEQ ID NO:7), or
- from aa. 210 to aa. 288 of MATR3 (SEQ ID NO:8), or
- from aa. 209 to aa. 287 of MATR3 (SEQ ID NO:12), or
- from aa. 210 to aa. 287 of MATR3 (SEQ ID NO:13), or

Preferably the amino acid sequence essentially consists of, comprises, or is comprised in the sequence from aa. 188 to aa. 288 of MATR3 (SEQ ID NO:6) or from aa. 151 to aa. 287 of MATR3 (SEQ ID NO:11) or from 188 to aa. 287 of MATR3 (SEQ ID NO:14) or from aa. 149 to aa. 287 of MATR3 (SEQ ID NO:15) or from aa. 151 to aa. 288 of MATR3 (SEQ ID NO:16).

Preferably the peptide essentially consists of the amino acid sequence:
- from aa. 210 to aa. 255 of MATR3 (SEQ ID NO:5), or
- from aa. 188 to aa. 248 of MATR3 (SEQ ID NO:3), or
- from aa. 228 to aa. 287 of MATR3 (SEQ ID NO:4), or
- from aa. 151 to aa. 287 of MATR3 (SEQ ID NO:11), or
- from aa. 1 to aa. 288 of MATR3 (SEQ ID NO:1) or
- from aa. 2 to aa. 288 of MATR3 (SEQ ID NO:18).

Preferably the peptide essentially consists of the amino acid sequence from aa. 151 to aa. 288 of MATR3 (SEQ ID NO:16).

Preferably, the peptide or variants thereof directly binds to DUX4 DNA-binding domain and/or inhibits DUX4 and rearranged DUX4 (DUX4-r) and/or blocks toxicity of wt DUX4 and leukemogenic activity of DUX4-r and/or competes for DUX4 binding.

A further object of the invention is a targeted protein degradation/degrader (TPD) compound or a nanoparticle comprising at least one peptide or variant thereof as defined herein or a peptide essentially consisting of or comprising the sequence from aa. 1 to aa. 287 of MATR3 (SEQ ID NO:17).

Another object of the invention is an isolated nucleic acid encoding the peptide or variant thereof as defined above or herein or the TPD compound as defined above or herein.

A further object of the invention is a vector or expression vector comprising an isolated nucleic acid as defined above or herein, preferably the vector or expression vector comprises said nucleic acid and a promoter operatively linked thereto or wherein said nucleic acid is operably linked to regulatory sequences capable of directing expression of said nucleic acid encoding said peptide in a host, preferably wherein said expression vector is a viral vector, preferably an AVV vector or a lentivirus vector, and/or wherein the promoter is a muscle-specific and/or a B-cell specific promoter.

A further object of the invention is an host cell comprising and/or expressing a peptide or variant thereof as defined above or herein, or a TPD as defined above or herein, or an isolated nucleic acid as defined above or herein, or a vector or expression vector as defined above or herein, preferably the cell is transformed and/or is an eukaryotic cell selected from the group consisting of a mammalian cell, an insect cell, a plant cell, a yeast cell and a protozoa cell, preferably the cell is a bacterial cell.

Another object of the invention is a cell transduced with the vector as defined above or herein, preferably said cell is an HSPC.

A further object of the invention is a pharmaceutical composition comprising the peptide or variant thereof as defined above or herein, or a peptide essentially consisting of or comprising the sequence from aa. 1 to aa. 287 of MATR3, or the TPD compound or the nanoparticle as defined above or herein, or the isolated nucleic acid as defined above or herein, or the vector or expression vector as defined above or herein, or the cell as defined above or herein and at least one pharmaceutically acceptable carrier, preferably further comprises a therapeutic agent.

Another object of the invention is the peptide or variants thereof as described above or herein, or a peptide essentially consisting of or comprising the sequence from aa. 1 to aa. 287 of MATR3 (SEQ ID NO:17), the TPD or the nanoparticle as described above or herein, the nucleic acid as described above or herein, the vector or expression vector as described above or herein, the cell as above or described herein, the transduced cell as defined above or herein or the pharmaceutical composition as defined above or herein for medical use,
preferably for use in the treatment of a condition associated with an aberrant expression and/or function of at least one DUX4 protein and/or of at least one rearranged DUX4 protein, preferably the condition associated with aberrant expression and/or function of at least one DUX4 protein and/or of at least one rearranged DUX4 protein is selected from the group consisting of: muscular dystrophy or cancer,
more preferably the cancer is acute lymphoblastic leukemia, or the muscular dystrophy is facioscapulohumeral muscular dystrophy (FSHD).

A further object of the invention is the peptide or variants thereof as described above or herein, or a peptide essentially consisting of or comprising the sequence from aa. 1 to aa. 287 of MATR3 (SEQ ID NO:17), the TPD or the nanoparticle as described above or herein, the nucleic acid as described above or herein, the vector or expression vector as described above or herein, the cell as described above or herein, the transduced cell as described above or herein, or the pharmaceutical composition as described above or herein for use in the treatment of acute lymphoblastic leukemia or facioscapulohumeral muscular dystrophy (FSHD).

Another object of the invention is the transduced cell as described above or herein for use in the treatment of acute lymphoblastic leukemia or the viral vector as described above or herein for use in the treatment of facioscapulohumeral muscular dystrophy (FSHD).

It is a further object of the invention a method of producing the peptide herein disclosed comprising the steps of transforming a host cell with a vector or an expression vector encoding said peptide, culturing said host cell under conditions enabling expression of said peptide, and optionally recovering and purifying said peptide.

It is also an object of the invention a peptide or variants thereof wherein the peptide essentially consists of:
- an amino acid sequence comprised in the sequence from aa. 151 to aa. 287 of MATR3 (SEQ ID NO:11) or from 188 to aa. 287 of MATR3 (SEQ ID NO:14) or from aa. 149 to aa. 287 of MATR3 (SEQ ID NO:15) or from aa. 151 to aa. 288 of MATR3 (SEQ ID NO:16),
- a variant of the amino acid sequence comprised in the sequence from aa. 151 to aa. 287 of MATR3 (SEQ ID NO:11) or from 188 to aa. 287 of MATR3 (SEQ ID NO:14) or from aa. 149 to aa. 287 of MATR3 (SEQ ID NO:15) or from aa. 151 to aa. 288 of MATR3 (SEQ ID NO:16).

It is also objects of the present invention a peptide comprising or essentially consisting of, or consisting of SEQ ID NO:1, 2, 3, 4, 5, 6, 7, 8,11,12,13,14,15,16,17,18,19
or variants thereof.

Preferably the peptide of the invention does not consists of SEQ ID NO:2.

In a preferred embodiment the peptide of the invention comprises or consists of SEQ ID No. 3 (aa. 188-248 of MATR3) (5'-SFDDRGPSLNPVLDYDHGSRSQESGYYDRMDYEDDRLRDGERCRDDSFFGETSHNYHK FDS-3')
or comprises or consists of SEQ ID No. 4 (aa. 228-287 of MATR3) (5'-ERCRDDSFFGETSHNYHKFDSEYERMGRGPGPLQERSLFEKKRGAPPSSNIEDFHGLLPK -3).

### DETAILED DESCRIPTION OF THE INVENTION

The peptides of the invention may present a biotin at the 5' and/or a -NH₂ at the 3'.

The peptides or fragments of the invention can further comprise further contiguous amino acids (at 5' and/or 3') of the Matrin3 protein.

In the present invention any muscle-specific promoter known to skilled man may be used, in particular the muscle-specific promoter may be selected from the group consisting of:
- SPc5-12 synthetic promoter (Nat. Biotechnol. 17, 241-245 (1999)
- SP-301 synthetic promoter (Plasmid. 2019;106:102441)
- MH hybrid promoter (Mol. Ther. Methods Clin. Dev. 2019;15:157-169)
- Sk-CRM4/Des chimeric promoter (Nat Commun. 2019 Jan 30;10(1):492)
- CK8e chimeric promoter (Acta Naturae. 2021 Jan-Mar; 13(1): 47-58).

In the context of the present invention, the herein disclosed peptides may also be defined as MATR3 fragments. Preferably the MATRIN-3 (MATR3) fragment is a cell-penetrating peptide (CPP)-MATRIN-3 (MATR3) fusion protein or a CPP- targeted protein degradation (TPD) - MATRIN-3 (MATR3) fusion protein. Preferably the CPP is a muscle-targeting cell-penetrating peptide (MCPP). Still preferably the MATRIN-3 (MATR3) fragment is a fatty acid-MATRIN-3 (MATR3) conjugate or a PEG-MATRIN-3 (MATR3) conjugate. The peptides of the invention also include variants, mutants, fusions, fragments or conjugates thereof. Preferably such variants, mutants, fusions, fragments or conjugates thereof are functional. Preferably the pharmaceutical composition further comprises a therapeutic agent. The therapeutic agent is for example anti-inflammatory and/or anti-oxidant drugs for FSHD, and anti-cancer drugs (chemotherapy), radiation therapy and/or immune checkpoint blockade therapies for ALL. For example, the anti-inflammatory drug may be aspirin, celecoxib, diclofenac, diflunisal, etodolac, ibuprofen, indomethacin, ketoprofen, ketorolac, nabumetone, naproxen, oxaprozin, piroxicam, salsalate, sulindac, tolmetin or as known in the art. The anti-oxidant drug may be vitamin E, vitamin C, zinc, selenium or as known in the art. The anti-cancer drug may be Bleomycin Sulfate, Cisplatin, Cosmegen (Dactinomycin), Dactinomycin, Etopophos (Etoposide Phosphate), Etoposide, Etoposide Phosphate, Ifex (Ifosfamide), Ifosfamide, Vinblastine Sulfate, Keytruda (Pembrolizumab), Lenvatinib Mesylate, Lenvima (Lenvatinib Mesylate), Megestrol Acetate, Pembrolizumab or as known in the art. The Immune checkpoint blockade therapy may be Ipilimumab, Nivolumab, Pembrolizumab, Atezolizumab, Avelumab, Durvalumab, Cemiplima, Spartalizumab or as known in the art. The radiation therapy may be X-rays, protons or as known in the art.

The present invention also provides a method of treating a condition associated with aberrant expression and/or function of at least one DUX4 protein and/or of at least one rearranged DUX4 protein comprising administering a therapeutically effective amount of a nucleic acid construct encoding the peptide herein disclosed.

The present invention further provides a method of treating a condition associated with aberrant expression and/or function of at least one DUX4 protein and/or of at least one rearranged DUX4 protein comprising administering a therapeutically effective amount of a vector or an expression vector comprising the nucleic acid construct as defined above, preferably the expression vector comprises the nucleic acid construct as defined above, and a promoter operatively linked thereto. Preferably the promoter drives the expression of MATRIN-3 peptide in the muscle or in the tumor or in the infected cell.

In a preferred embodiment the vector or expression vector is a lentivirus or an AVV vector. Preferably the promoter is a muscle-specific promoter or a B-cell specific promoter.

The present invention also provides a method of treating a condition associated with aberrant expression and/or function of DUX4 protein and/or of rearranged DUX4 protein comprising administering a therapeutically effective amount of a transformed cell comprising the vector as defined above, preferably the cell is a eukaryotic cell selected from the group consisting of a mammalian cell, an insect cell, a plant cell, a yeast cell, and a protozoa cell. Still preferably the cell is a human cell or a bacterial cell.

Preferably the condition associated with aberrant expression and/or function of DUX4 protein and/or of rearranged DUX4 protein is selected from the group consisting of: muscular dystrophy or cancer.

Preferably the cancer is selected from the group consisting of: acute lymphoblastic leukemia, rhabdomyosarcoma, breast, testis, kidney, stomach, lung, thymus, liver, uterus, larynx, esophagus, tongue, heart, connective, mouth, colon, mesothelioma, bladder, ovary, brain, tonsil, pancreas, peritoneum, prostatic or thyroid cancer (Cell Stem Cell. 2018 Dec 6;23(6):794-805.e4 incorporated by reference).

Preferably the cancer is acute lymphoblastic leukemia.

Preferably the muscular dystrophy is FSHD.

A condition associated with an aberrant expression and/or function of DUX4 protein and/or of rearranged DUX4 protein means a condition in which the expression of the protein DUX4 itself or its rearranged forms (at the level of RNA or protein) is altered in comparison with a healthy subject, i.e. a subject not affected by such a condition.

DUX4 or its rearranged forms are normally not expressed in somatic tissues. As a result of genomic alterations, the expression of DUX4 or of its rearranged forms is aberrantly activated and/or the activity of DUX4 or of its rearranged form is altered in several conditions, specifically, muscular dystrophy or cancer such as FSHD and ALL.

In these conditions, DUX4 or its rearranged forms are overexpressed. In ALL aberrant expression and activity of rearranged DUX4 forms is observed.

Rearranged forms of DUX4 include the immunoglobulin heavy locus (DUX4-IGH) fusion transcripts.

With the exception of 4-cell embryonic stage, testis, and thymus, DUX4 is normally not expressed. Aberrant expression of DUX4 or its rearranged form is intended to be when expression (at the level of RNA or protein) is observed while it is not normally observed or overexpressed in respect to a proper control.

In the diseases or conditions of the invention, expression of DUX4 (RNA and protein) or its rearranged forms is observed and/or measured in tissues and cell types that normally do not express DUX4.

Due to genomic translocations, some cancers such as ALL display expression of rearranged proteins in which DUX4 is truncated at the C-terminus and can be fused to amino acids encoded by the immunoglobulin heavy locus (DUX4-IGH in ALL). These rearranged DUX4 versions display a different (aberrant) activity compared to normal DUX4, including the ability to regulate a different set of genes and to promote cell proliferation instead of cell death.

In the contest of the present invention "rearranged DUX4" or "rearranged forms of DUX4" includes DUX4 which maintain the dbd of wild-type DUX4, while it could present variable size deletions and substitutions affecting the TAD, and it might also include DUX4 which is truncated at the C-terminus and it's fused to amino acids encoded by the immunoglobulin heavy locus (DUX4-IGH in ALL). Therefore the "rearranged DUX4" also includes the fused form of DUX4.

Aberrant DUX4 or its rearranged forms expression can be evaluated by quantitative RT-PCR, RNA sequencing, RNA-FISH, immunoblotting and immunofluorescence or any known method in the art.

Aberrant function of DUX4 or its rearranged forms can be evaluated by monitoring the expression of DUX4 / DUX4-r (DUX4-IGH) target genes like for example MDB3L2, TRIM43, ERGalt (Hum Mol Genet. 2014 Oct 15;23(20):5342-52; Leukemia. 2018 Jun;32(6):1466-1476; Cancers (Basel). 2020 Sep 30;12(10):2815, EMBO Mol Med. 2021 Aug 9;13(8):e13695; Cells. 2021 Nov 26;10(12):3322) and by evaluating cell proliferation, cell differentiation, cell transformation, cell apoptosis, oxidative damage, tumor formation and tumor growth, according to any known method in the art.

MATR3 is an DNA- and RNA-binding component of the nuclear matrix involved in diverse processes, including the response to DNA damage (Cell Cycle 2010 9:1568-1576), mRNA stability (PLoS One 2011 6:e23882), RNA splicing (EMBO J 2015 34:653-668), nuclear retention of hyperedited RNA (Cell 2001 106:465-475) and restriction/latency of retroviruses (Retrovirology 2005 12:57; Mbio. 2018 Nov 13;9(6). Pii: e02158-18. Doi: 10.1128/mBio.02158-18). MATR3 is 847 amino acids long and contains four known functional domains: Zinc finger 1, RNA recognition motif 1, RNA recognition motif 2 and Zinc finger 2.

The peptides or variants thereof of the present invention are preferably functional.

For "functional" it is e.g. intended that they maintain the same activity of the full-length matrin3 and/or bind to DUX4 DNA-binding domain and/or inhibit DUX4 and rearranged DUX4 (DUX4-r) and/or block toxicity of wt DUX4 and leukemogenic activity of DUX4-r and/or compete for DUX4 binding.

For "functional" it is e.g. intended that it:
- inhibits DUX4-induced toxicity in particular in HEK293 cells,
- blocks induction of DUX4 targets, in particular in HEK293 cells,
- interacts with the DNA-binding domain of DUX4,
- inhibits DUX4 directly by blocking its ability to bind DNA,
- inhibits the expression of DUX4 and DUX4 targets in particular in FSHD muscle cells,
- rescues viability and myogenic differentiation in particular of FSHD muscle cells,
- inhibits the expression of DUX4-r and DUX4-r targets in particular in leukemia cells,
- blocks proliferation and cell adhesion in particular of ALL cells,
- inhibits DUX4-r leukemogenic activity,
- the ability to treat, prevent, or ameliorate condition associated with an aberrant expression and/or function of at least one DUX4 protein and/or of at least one rearranged DUX4 protein, such as muscular dystrophy, or cancer such as ALL.

In the present invention the functional fragments of MATRIN3 herein disclosed, peptides of the invention or variants thereof exhibits at least one of the following activities:
- inhibits DUX4-induced toxicity in particular in HEK293 cells,
- blocks induction of DUX4 targets, in particular in HEK293 cells,
- interacts with the DNA-binding domain of DUX4,
- inhibits DUX4 directly by blocking its ability to bind DNA,
- inhibits the expression of DUX4 and DUX4 targets in particular in FSHD muscle cells,
- rescues viability and myogenic differentiation in particular of FSHD muscle cells,
- inhibits the expression of DUX4-r and DUX4-r targets in particular in leukemia cells,
- blocks proliferation and cell adhesion in particular of ALL cells,
- inhibits DUX4-r leukemogenic activity,
- the ability to treat, prevent, or ameliorate condition associated with an aberrant expression and/or function of at least one DUX4 protein and/or of at least one rearranged DUX4 protein, such as muscular dystrophy, or cancer such as ALL.

These activities may be measured as described herein or using known methods in the art.

MATR3 fragments or peptides may be delivered in vivo to skeletal muscle by using recombinant adeno-associated viruses (rAAV), which are highly prevalent in musculoskeletal gene therapy due to their non-pathogenic nature, versatility, high transduction efficiency, natural muscle tropism and vector genome persistence for years (Curr Opin Pharmacol. 2017 Jun;34:56-63). To this aim, the nucleotide coding sequence for MATR3 fragments may be inserted in rAAV containing a muscle specific promoter such the CK8 regulatory cassette (Nat Commun. 2017 Feb 14;8:14454). MATR3 fragments may be delivered ex-vivo to hematopoietic stem and progenitors cells (HSPCs) by using lentiviruses, which are highly prevalent in ex-vivo gene therapy (EMBO Mol Med. 2019 Mar;11(3):e9958). To this aim, the nucleotide coding sequence for MATR3 fragments may be inserted in a lentivirus containing a muscle specific promoter such the B29 regulatory cassette (Blood. 1996, 87(2):666-73).

Hematopoietic stem cells (HSCs) are multipotent stem cells that may be found, for example, in peripheral blood, bone marrow and umbilical cord blood. HSCs are capable of self-renewal and differentiation into any blood cell lineage. They are capable of recolonizing the entire immune system, and the erythroid and myeloid lineages in all the hematopoietic tissues (such as bone marrow, spleen, and thymus). They provide for life-long production of all lineages of hematopoietic cells.

Hematopoietic progenitor cells have the capacity to differentiate into a specific type of cell.

In contrast to stem cells however, they are already far more specific: they are pushed to differentiate into their "target" cell. A difference between stem cells and progenitor cells is that stem cells can replicate indefinitely, whereas progenitor cells can only divide a limited number of times. Hematopoietic progenitor cells can be rigorously distinguished from HSCs only by functional in vivo assay (i.e. transplantation and demonstration of whether they can give rise to all blood lineages over prolonged time periods).

A population of hematopoietic stem and/or progenitor cells (HSPCs) may be obtained from a tissue sample.

For example, a population of hematopoietic stem and/or progenitor cells may be obtained from peripheral blood (e.g. adult and fetal peripheral blood), umbilical cord blood, bone marrow, liver, or spleen. Preferably, these cells are obtained from peripheral blood or bone marrow. They may be obtained after mobilization of the cells in vivo by means of growth factor treatment. Mobilization may be carried out using, for example, G-CSF, plerixaphor or combinations thereof. Other agents, such as NSAIDs and dipeptidyl peptidase inhibitors, may also be useful as mobilizing agents.

With the availability of the stem cell growth factors GM-CSF and G-CSF, most hematopoietic stem cell transplantation procedures are now performed using stem cells collected from the peripheral blood, rather than from the bone marrow. Collecting peripheral blood stem cells provides a bigger graft, does not require that the donor be subjected to general anesthesia to collect the graft, results in a shorter time to engraftment and may provide for a lower long-term relapse rate.

Bone marrow may be collected by standard aspiration methods (either steady-state or after mobilization), or by using next-generation harvesting tools (e.g. Marrow Miner).

In addition, hematopoietic stem and progenitor cells may also be derived from induced pluripotent stem cells.

Any suitable population of HSPCs may be administered. The population of HSPCs may be autologous HSPCs and/or allogenic HSPCs. In some embodiments, the population of HSPCs are autologous HSPCs. In some embodiments, the population of HSPCs is cultured ex vivo prior to administration. In the present invention the HSPC population may thus be genetically engineered to express the peptides of the present invention prior to administering to the patient.

In the present invention, the cells or a population of cells may be transduced in vitro or ex vivo with a viral vector encoding and/or expressing the peptide of the invention. The transduced cells or population may then be administered to a subject. In one embodiment, the transduced cells are administered to a subject as part of an autologous (stem) cell transplant procedure. In another embodiment, the transduced cells are administered to a subject as part of an allogeneic (stem) cell transplant procedure.

An alternative to the gene therapy-like approach for the delivery of MATR3 fragment can be the use of recombinant peptides. Peptides are highly selective and efficacious and, at the same time, relatively safe and well-tolerated. A particularly exciting application of peptides is the inhibition of protein-DNA interactions, which remain challenging targets for small molecules. Peptides are generally impermeable to the cell membrane. To allow cellular entry and drive selective uptake from skeletal muscle, MATR3-based peptides could be fused to cell-penetrating peptides (CPP) like B-MSP (Hum Mol Genet. 2009 Nov 15;18(22):4405-14), Pip6 (Mol Ther Nucleic Acids. 2012 Aug 14;1:e38), M12 (Mol Ther. 2014 Jul;22(7):1333-1341) or CyPep10 (Mol Ther. 2018 Jan 3;26(1): 132-147). To increase potency of the MATR3-based fusion peptides, they could further be modified by appending a ligand for an E3 ubiquitin ligase, which are factors driving attachment of ubiquitin molecules to a lysine on the target protein and triggering degradation of a protein of interest by the proteolytic activity mediated by the proteasome, a protein degradation "machine" within the cell that can digest a variety of proteins into short polypeptides and amino acids. Pharmacologic protein degradation is a powerful approach with therapeutic relevance. This approach uses bifunctional small molecules (targeted protein degradation/degrader, TPD) that engage both a target protein and an E3 ubiquitin ligase, like for example cereblon (CRBN) or Von-Hippel Lindau (VHL), which are expressed in skeletal muscle and have several already known and tested E3 ligase ligands, for instance thalidomide, lenalidomide, and pomalidomide (Science 2015 348, 1376-1381; Molecular Cell 2017 67, 5-18). This allows potent and selective degradation of target proteins by enforcing proximity of the targeted protein and the E3 ligase, leading to ubiquitination and proteasomal degradation. After binding to DUX4/DUX4-r, the CPP-degrader-MATR3-based peptide will lead to the ubiquitination of DUX4/DUX4-r at lysine residues and proteasomal degradation.

Moreover, cell permeability and metabolic stability of the MATR3-based fusion peptides could be increased by reversible bicyclization (Angew Chem Int Ed Engl. 2017 Feb 1;56(6): 1525-1529, incorporated by reference).

In the context of the present invention MATRIN-3 (MATR3) full length protein, is preferably characterized by the sequence disclosed with NCBI reference sequence number NP_954659.1, and/or encoded by the polynucleotide sequence which has NCBI reference sequence number NM_199189.2. By way of non-limiting example, in some embodiments, the invention comprises smaller fragments, domains, and/or regions of MATRIN-3 (MATR3) 1-288 region.

In some embodiments, the invention comprises variants or mutations of the peptides herein disclosed, e.g., biologically active variants, and can include truncated versions of the peptides (in which residues from the C- and/or N- terminal regions have been eliminated, thereby shortening/truncating the protein), as well as variants with one or more point substitutions, deletions, and/or site-specific incorporation of amino acids at positions of interest (e.g., with conservative amino acid residues, with non-conservative residues, or with non-natural amino acid residues such as pyrrolysine). The terms "variant" and "mutant" are used interchangeably and are further defined herein.

Preferably the variants or mutations or fragments or portions are functional.

In the context of the present invention, a variant may: (a) have one or more amino acid substitutions, insertions, or deletions in the amino acid sequence relative to the Matrin3 sequence or to the sequences herein disclosed (e.g. relative to one of SEQ ID Nos: 1-8 or 11-19), and/or (b) have at least 75%, 80%, 85%, 90%, 95%, 96%, 97%, 98%, 99% sequence identity over the matrin3 sequence or the sequences herein disclosed (e.g. over one of SEQ ID Nos: 1-8 or 11-19). Preferably the substitution is conservative, in particular relative to SEQ ID NO: 5.

The peptide of the invention or variants thereof may comprise one or more modifications, e.g. N-terminal acetylation, C-terminal amidation, PEGylation, cyclization, a C14-C18 fatty acid, caprylic acid, biotinylation, fusion with vitamin B12, and a combination of two or more thereof. The peptide or the amino acid sequence or the sequence of the invention is preferably long at least 10 amino acids. The peptide or the amino acid sequence or the sequence may be long e.g. 45, 50, 55, 46, 62,61 or 60 amino acids. The peptide or the amino acid sequence or the sequence of the invention is preferably long at most 40, 50, 60, 70, 80, 90 or 100 amino acids.

In some embodiments, the invention comprises fusion protein sequences, such as Fc fusions, or serum albumin (SA) fusion or fusion with cell-penetrating peptides, fusions with bifunctional small molecule degraders, or reversible bicyclization. The terms "fusion protein", "fusion polypeptide," and "fusions" are used interchangeably and are further defined herein. In still other embodiments, the invention comprises conjugations of the peptides and fatty acids. Said conjugates and fusions may be intended to extend the half-life of the MATRIN-3 (MATR3) moiety or fragments, in addition to serving as therapeutic agents for the conditions listed herein. In some embodiments, the conjugates and fusions of the invention comprise wild type MATRIN-3 (MATR3) fragments; in other embodiments, the conjugates and fusions comprise variant sequences relative to the wild-type full-length or mature protein or fragments.

Said fusions can comprise wild type MATRIN-3 (MATR3) fragments or variants thereof. In some embodiments, the invention comprises polypeptides which can be fused to a heterologous amino acid sequence, optionally via a linker as disclosed WO2020/152367. The heterologous amino acid sequence can be an IgG constant domain or fragment thereof (e.g., the Fc region), Human Serum Albumin (HSA), or albumin-binding polypeptides. In some embodiments, the heterologous amino acid sequence is derived from the human IgG4 Fc region because of its reduced ability to bind Fey receptors and complement factors compared to other IgG sub-types. The heterologous amino acid sequence can be a muscle or B-cell targeting antibody and/or cell-penetrating peptide, a bifunctional small molecule degrader, or a reversible bicyclization. The present invention also includes multimers of said fusion polypeptides. In some embodiments, the present invention comprises fusion proteins in which the heterologous amino acid sequence (e.g., CPP, TPD, etc.) is fused to the amino-terminal of the peptides or variants as described herein; in other embodiments, the fusion occurs at the carboxyl-terminal of the MATRIN-3 (MATR3) peptides or variants.

In some embodiments, the invention comprises MATRIN-3 (MATR3) conjugates, such as MATRIN-3 (MATR3) fatty acid (FA) conjugates, e.g., MATRIN-3 (MATR3) wild type protein fragments or truncations thereof or variant fragments or truncations thereof covalently attached to a fatty acid moiety via a linker.

In some embodiments, the invention comprises MATRIN-3 (MATR3) (peptide) fusion proteins or conjugates which are covalently linked to one or more polymers, such as polyethylene glycol (PEG) or polysialic acid. The PEG group is attached in such a way so as enhance, and/or not to interfere with, the biological function of the constituent portions of the fusion proteins or conjugates of the invention.

The invention also provides methods of treatment with a pharmaceutical composition comprising the MATRIN-3 (MATR3) (peptide) fusion proteins or MATRIN-3 (MATR3) (peptide) conjugates disclosed herein and a pharmaceutically acceptable formulation agent. Such pharmaceutical compositions can be used in a method for treating one or more of condition associated with an aberrant expression and/or function of DUX4 protein and/or of rearranged DUX4 protein and the methods comprise administering to a human patient in need thereof a pharmaceutical composition of the invention.

The invention also provides methods of treatment with a pharmaceutical composition comprising the MATRIN-3 (MATR3) fusion proteins or MATRIN-3 (MATR3) conjugates disclosed herein and a pharmaceutically acceptable formulation agent. Such pharmaceutical compositions can be used in a method for treating one or more of condition associated with an aberrant expression and/or function of DUX4 protein and/or of rearranged DUX4 protein and the methods comprise administering to a human patient in need thereof a pharmaceutical composition of the invention. The invention also provides MATRIN-3 (MATR3) fusion proteins or MATRIN-3 (MATR3) conjugates disclosed herein for the treatment of one or more condition associated with aberrant expression and/or function of DUX4 protein and/or of rearranged DUX4 protein, such as muscular dystrophy or cancer, in particular FSHD or ALL. The invention also provides pharmaceutical compositions comprising MATRIN-3 (MATR3) (peptide) fusion proteins or MATRIN-3 (MATR3) (peptide) conjugates disclosed herein for the treatment of one or more condition associated with aberrant expression and/or function of DUX4 protein and/or of rearranged DUX4 protein, such as muscular dystrophy or cancer, in particular FSHD or ALL.

In one embodiment, the methods of the invention comprise MATRIN-3 (MATR3) fusion proteins as described herein, e.g., serum albumin, the cell penetrating peptide fusions, etc. In some embodiments, said fusions can contain any suitable serum albumin, cell penetrating peptide (CPP) moiety, antibody moieties, any suitable MATRIN-3 (MATR3) moiety or fragment or peptides, and if desired, any suitable linker. Generally, the CPP moiety, MATRIN-3 (MATR3) moiety or fragment and, if present, linker, are selected to provide a fusion polypeptide that would be predicted to have therapeutic efficacy in a condition associated with aberrant expression and/or function of DUX4 protein and/or of rearranged DUX4 protein, such as muscular dystrophy or cancer, in particular FSHD or ALL or other disorders described herein, and to be immunologically compatible with the species to which it is intended to be administered. For example, when the fusion polypeptide is intended to be administered to humans the CPP and functional variants thereof and MATRIN-3 (MATR3) and functional variants thereof that are derived from other species (e.g., pet or livestock animals) can be used when the fusion protein is intended for use in such species.

Preferably, the MATRIN-3 (MATR3) peptide or fragment of the invention is a human MATRIN-3 (MATR3) peptide or fragment or a functional variant thereof.

Functional variants can include one or more amino acid deletions, additions, or replacements in any desired combination. The amount of amino acid sequence variation (e.g., through amino acid deletions, additions, or replacements) is limited to preserve weight loss activity of the mature MATRIN-3 (MATR3) peptide. In some embodiments, the functional variant of a mature MATRIN-3 (MATR3) peptide has from 1 to about 20, 1 to about 18, 1 to about 17, 1 to about 16, 1 to about 15, 1 to about 14, 1 to about 13, 1 to about 12, 1 to about 11, 1 to about 10, 1 to about 9, 1 to about 8, 1 to about 7, 1 to about 6, or 1 to about 5 amino acid deletions, additions or replacements, in any desired combination, relative to the sequences herein disclosed. Alternatively, or in addition, the functional variant can have an amino acid sequence that has at least about 80%, at least about 85%, at least about 90%, or at least about 95%, 96%, 97%, 98%, or 99% amino acid sequence identity with the sequences herein disclosed, preferably when measured over the full length of the sequences herein disclosed. In a specific embodiment, a MATRIN-3 (MATR3) functional peptide variant can have an amino acid sequence that has at least 90%, at least 95%, or at least 98% amino acid sequence identity with the sequences herein disclosed, preferably when measured over the full length of the sequences.

Without wishing to be bound by any particular theory, it may be that MATRIN-3 (MATR3) peptides of the invention present a therapeutic efficacy in a condition associated with aberrant expression and/or function of DUX4, such as FSHD or ALL, and related conditions mediated either through cellular signaling initiated by the binding of MATRIN-3 (MATR3) fragments (and the fusion proteins and variants described herein) to DUX4 and/or co-factors, or by regulation of pathways utilized by other factors via direct competition or allosteric modulation. Amino acid substitutions, deletions, or additions are preferably at positions that are not involved in maintaining overall protein conformation.

### Serum Albumin (SA) Moiety

The SA moiety is any suitable serum albumin (e.g., human serum albumin (HSA), or serum albumin from another species) or a functional variant thereof. Preferably, the SA moiety is an HSA or a functional variant thereof. The SA moiety prolongs the serum half-life of the fusion polypeptides to which it is added, in comparison to wild type MATRIN-3 (MATR3). Methods for pharmacokinetic analysis and determination of serum half-life will be familiar to those skilled in the art. Details may be found in Kenneth, A et al: Chemical Stability of Pharmaceuticals: A Handbook for Pharmacists and in Peters et al, Pharmacokinetic analysis: A Practical Approach (1996). Reference is also made to "Pharmacokinetics," M Gibaldi & D Perron, published by Marcel Dekker, 2nd Rev. ex edition (1982), which describes pharmacokinetic parameters such as t alpha and t beta half-lives and area under the curve (AUC).

Human Serum Albumin (HSA) is a plasma protein of about 66,500 KDa and is comprised of 585 amino acids, including at least 17 disulfide bridges. (Peters, T., Jr. (1996), All about Albumin: Biochemistry, Genetics and Medical, Applications, pp 10, Academic Press, Inc., Orlando (ISBN 0-12-552110-3). HSA has a long half-life and is cleared very slowly by the liver. The plasma half-life of HSA is reported to be approximately 19 days (Peters, T., Jr. (1985) Adv. Protein Chem. 37, 161-245; Peters, T., Jr. (1996) All about Albumin, Academic Press, Inc., San Diego, CA. (page 245-246)); Benotti P, Blackburn GL: Crit. Care Med (1979) 7:520-525).

HSA has been used to produce fusion proteins that have improved shelf and half-life. For example, PCT Publications WOO 1/79271 A and WO03/59934 A disclose (i) albumin fusion proteins comprising a variety of therapeutic protein (e.g., growth factors, scFvs); and (ii) HSAs that are reported to have longer shelf and half-lives than their therapeutic proteins alone.

HSA may comprise the full-length sequence of 585 amino acids of mature naturally occurring HSA (following processing and removal of the signal and pro-peptides) or naturally occurring variants thereof, including allelic variants. Naturally occurring HSA and variants thereof are well-known in the art. (See, e.g., Meloun, et al, FEBS Letters 5S: 136 (1975); Behrens, et al., Fed. Proc. 34:591 (1975); Lawn, et al., Nucleic Acids Research 9:6102-6114 (1981); Minghetti, et al, J. Biol. Chem. 261:6747 (1986)); and Weitkamp, et al, Ann. Hum. Genet. 37:219 (1973).)
Full length HSA (SEQ ID No:9)
Mature HSA (25-609) (SEQ ID No:10)

Fusion proteins that contain a human serum albumin moiety generally contain the 585 amino acid HSA (amino acids 25-609 of SEQ ID NO:9, SEQ ID NO:10) or a functional variant thereof. The functional variant can include one or more amino acid deletions, additions, or replacement in any desired combination, and includes functional fragments of HSA. The amount of amino acid sequence variation (e.g., through amino acid deletions, additions, or replacements) is limited to preserve the serum half-life extending properties of HSA.

In some embodiments, the functional variant of HSA for use in the fusion proteins disclosed herein can have an amino acid sequence that has at least about 80%, at least about 85%, at least about 90%, or at least about 95% amino acid sequence identity with SEQ ID NO: 9 or 10, preferably when measured over the full length sequence of SEQ ID NO: 9 or 10. Alternatively or in addition, the functional variant of HSA can have from 1 to about 20, 1 to about 18, 1 to about 17, 1 to about 16, 1 to about 15, 1 to about 14, 1 to about 13, 1 to about 12, 1 to about 11, 1 to about 10, 1 to about 9, 1 to about 8, 1 to about 7, 1 to about 6, or 1 to about 5 amino acid deletions, additions or replacement, in any desired combination.

Some functional variants of HSA for use in the fusion proteins disclosed herein may be at least 100 amino acids long, or at least 150 amino acids long, and may contain or consist of all or part of a domain of HSA, for example domain I , II, or III. If desired, a functional variant of HSA may consist of or alternatively comprise any desired HSA domain combination, such as, domains I + II, domains II + III or domains I + III. As is well-known in the art, each domain of HSA is made up of two homologous subdomains, with flexible inter-subdomain linker regions. In certain embodiments, the SA moiety of the fusions proteins of the present invention contains at least one subdomain or domain of HSA.

Functional fragments of HSA suitable for use in the fusion proteins disclosed herein will contain at least about 5 or more contiguous amino acids of HSA, preferably at least about 10, at least about 15, at least about 20, at least about 25, at least about 30, at least about 50, or more contiguous amino acids of HSA sequence or may include part or all of specific domains of HSA.

In some embodiments, the functional variant (e.g., fragment) of HSA for use in the fusion proteins disclosed herein includes an N-terminal deletion, a C-terminal deletion or a combination of N-terminal and C-terminal deletions. Such variants are conveniently referred to using the amino acid number of the first and last amino acid in the sequence of the functional variant.

Examples of HSA and HSA variants (including fragments) that are suitable for use in the MATRIN-3 (MATR3) fusion polypeptides described herein are known in the art. Suitable HSA and HSA variants include, for example full length mature HSA (SEQ ID NO:10) and fragments. Such HSA polypeptides and functional variants are disclosed in PCT Publication WO 2005/077042A2, which is incorporated herein by reference in its entirety. Further variants of HSA are disclosed in European Published Application EP322094A1, and in European Published Application EP399666A1.

### Cell penetrating peptide (CPP) moiety

Cell-penetrating peptides (CPPs) are short peptides that facilitate cellular intake/uptake of various cargo molecules (for example proteins or nucleic acids). CPPs typically have an amino acid composition that either contains a high relative abundance of positively charged amino acids such as lysine or arginine, has sequences that contain an alternating pattern of polar/charged amino acids and non-polar hydrophobic amino acids, or only apolar or hydrophobic amino acid groups. The cargo is associated with the CPP either through chemical linkage via covalent bonds or through non-covalent interactions.

One limitation of CPP use is the lack of cell specificity in CPP-mediated cargo delivery. Nevertheless, by mutagenesis or functional assays CPP variants with increased muscle-targeting have been discovered including B-MSP, Pip6, M12 or CyPep10 (Hum Mol Genet. 2009 Nov 15;18(22):4405-14; Mol Ther Nucleic Acids. 2012 Aug 14;1:e38; Mol Ther. 2014 Jul;22(7):1333-1341; Mol Ther. 2018 Jan 3;26(1):132-147).

### Antibody moiety

In the present invention it may also be used antibodies or antigen-binding fragments (Fabs) engineered to bind to a receptor which is highly expressed on muscle cells expressing DUX4 (such for example SLC34A2) (Exp Neurol. 2019 Oct;320:113011) or on leukemia cells expressing DUX4-r (such for example CD371) (Haematologica. 2019 Aug; 104(8): e352-e355) to enable targeted delivery to FSHD skeletal muscle or DUX4-r positive leukemia cells.

### Targeted protein degradation/degrader (TPD)

Targeted protein degradation/degrader (TPD) is a recent approach, utilizing the cellular protein degradation machinery to deplete specific proteins.

Proteolysis targeting chimera (PROTAC) is a TPD strategy that utilizes the ubiquitin-proteasome system to target a specific protein and induce its degradation in the cell. Physiologically, the ubiquitin-proteasome system is responsible for clearing denatured, mutated, or harmful proteins in cells. PROTAC takes advantage of this protein destruction mechanism to remove specifically targeted proteins from cells. This technology takes advantage of bifunctional small molecules (degrader) in which a moiety targets the protein of interest and a moiety recognizing a E3 ubiquitin ligase like for example cereblon (CRBN) or Von-Hippel Lindau (VHL) (Science 2015 348, 1376-1381; Molecular Cell 2017 67, 5-18). This allows potent and selective degradation of target proteins by enforcing proximity of the targeted protein and the E3 ligase, leading to ubiquitination and proteasomal degradation.

The TPD chaperone-mediated Protein Degradation (CHAMP) uses to ability of heat shock protein 90 (HSP90) to interact directly with many different E3 ubiquitin ligases and assist in the degradation of misfolded proteins through the ubiquitin-proteasome system (UPS). Similar to PROTAC, CHAMP uses a heterobifunctional molecule composed by a moiety binding the protein of interest and a moiety recognizing HSP90.

Similar to PROTACs, the TPD molecular glues are small molecules directly inducing close proximity of an E3 ligase and a protein-of-interest, thereby leading to its ubiquitination and subsequent degradation.

Autophagy-targeting chimeras (AUTACs), autophagosome-tethering compounds (ATTECs), autophagy-targeting chimeras (AUTOTACs) and chaperone-mediated autophagy (CMA)-based TPD use the intracellular autophagic degradation pathway, where small, membrane-bound vesicles assemble and engulf material for degradation, via the lysosome. AUTACs/ATTECs/AUTOTACs/CMA link a specific ligand for a protein of interest to a guanine tag (AUTACs), LC3-binding ligand (ATTECs), p62-binding ligand (AUTOTACs) or heat shock protein 70 (HSP70) ligand (CMA) resulting in tethering of the target protein to the autophagosome for subsequent degradation.

In hydrophobic tagging (HyT) TPD, known ligands for target proteins can be combined with different hydrophobic moieties and an additional carbon linker that terminates in one of several different bulky side groups (adamantane, tert-butyl carbamate-protected arginine, benzylic, ademantyl, tricyclic or cyclohexyl). Hydrophobic moieties may induce local regions of protein unfolding, significant protein structural changes, and ultimately protein destabilization that may be recognized by the cell's protein quality control machinery. Alternatively, these groups may mimic hydrophobic patches of unfolded regions of proteins, triggering recognition by the unfolded protein response, leading to proteasomal degradation.

In the present invention the targeted protein degradation/degrader (TPD) compound may also comprise at least one peptide or variant as defined herein or a peptide essentially consisting of or comprising the sequence from aa. 1 to aa. 287 of MATR3 or the full-length sequence of MATR3.

### Reversible bicyclization

Compared to small-molecule drugs, peptides are highly selective and efficacious and, at the same time, relatively safe and well-tolerated. However, peptides are inherently susceptible to proteolytic degradation. Additionally, peptides are generally impermeable to the cell membrane, largely limiting their applications to extracellular targets. Compared to their linear counterparts, cyclic peptides have reduced conformational freedom, which makes them more resistant to proteolysis and allows them to bind to their molecular targets with higher affinity and specificity. In particular, a short sequence motif (FΦRRRR, where Φ is L-2-naphthylalanine) efficiently transport cyclic peptides inside cells and could be used as general transporters of cyclic peptides into mammalian cells (ACS Chem. Biol. 2013, 8:423-431). However, many peptide ligands must be in their extended conformations to be biologically active and are not compatible with the above cyclization approaches. To this end, a reversible bicyclization strategy, which allows the entire CPP-cargo fusion to be converted into a bicyclic structure by the formation of a pair of disulfide bonds, was recently described. When outside the cell, the peptide exists as a highly constrained bicycle, which possesses enhanced cell permeability and proteolytic stability. Upon entering the cytosol, the disulfide bonds are reduced by the intracellular glutathione (GSH) to produce the linear, biologically active peptide. The bicyclic system permits the formation of a small CPP ring for optimal cellular uptake11 and a separate cargo ring to accommodate peptides of different lengths (Angew Chem Int Ed Engl. 2017 Feb 1;56(6):1525-1529, incorporated by reference).

### Linkers

Regarding the MATRIN-3 (MATR3) (peptide) fusion proteins (e.g., SA, Fc, the cell-penetrating peptide (CPP), antibody MATRIN-3 (MATR3) (peptide) fusion proteins) used in the present invention, the heterologous protein/peptide, e.g., SA, CPP, antibody and MATRIN-3 (MATR3) moieties or fragments or peptides can be directly bonded to each other in the contiguous polypeptide chain, or preferably indirectly bonded to each other through a suitable linker. The linker is preferably a peptide linker. Peptide linkers are commonly used in fusion polypeptides and methods for selecting or designing linkers are well-known. (See, e.g., Chen X et al. Adv. Drug Deliv. Rev. 65(10): 135701369 (2013) and Wriggers W et al., Biopolymers 80:736-746 (2005)). Peptide linkers generally are categorized as i) flexible linkers, ii) helix forming linkers, and iii) cleavable linkers, and examples of each type are known in the art. Preferably, a flexible linker is included in the fusion polypeptides described herein. Flexible linkers may contain a majority of amino acids that are sterically unhindered, such as glycine and alanine. The hydrophilic amino acid Ser is also conventionally used in flexible linkers. Examples of flexible linkers include, polyglycines, polyalanines poly(Gly-Ala), and poly(Gly-Ser).

Peptide linkers can be of a suitable length. The peptide linker sequence may be at least 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, or more amino acid residues in length. For example, a peptide linker can be from about 5 to about 50 amino acids in length; from about 10 to about 40 amino acids in length; from about 15 to about 30 amino acids in length; or from about 15 to about 20 amino acids in length. Variation in peptide linker length may retain or enhance activity, giving rise to superior efficacy in activity studies. The peptide linker sequence may be comprised of a naturally, or non- naturally, occurring amino acids. In some aspect, the amino acids glycine and serine comprise the amino acids within the linker sequence. The linker region may be e.g. as disclosed in WO2020/152367, herein incorporated by reference. In more embodiments, a linker may contain glycine (G) and serine (S) in a random or preferably a repeated pattern. In other embodiments, a linker may contain glycine (G), serine (S) and proline (P) in a random or preferably repeated pattern. In a particular example, the linker is GPPGS (SEQ ID NO:20). In general, the linker is not immunogenic when administered in a patient, such as a human. Thus, linkers may be chosen such that they have low immunogenicity or are thought to have low immunogenicity.

The linkers described herein are exemplary, and the linker can include other amino acids, such as Glu and Lys, if desired. The peptide linkers may include multiple repeats of, for example, (G4S) (SEQ ID NO:21), (G3S) GGGS (SEQ ID NO:22), (G2S) GGS and/or (GlySer), if desired. In certain aspects, the peptide linkers may include multiple repeats of, for example, (SG4) SGGGG (SEQ ID NO:24), (SG3) SGGG (SEQ ID NO:25), (SG2) SGG or (SerGly). In other aspects, the peptide linkers may include combinations and multiples of repeating amino acid sequence units. In other aspects, Ser can be replaced with Ala e.g., (G4A, GGGGA) (SEQ ID NO:29) or (GA). In certain aspects, peptide linkers may also include cleavable linkers. In a particular embodiment, a MATRIN-3 (MATR3) (peptide) fusion or conjugate used in the present methods of the invention comprises a MATRIN-3 (MATR3) moiety or fragment or or peptide or portion linked to a heterologous protein/peptide (e.g., CPP or Degrader) or a conjugate moiety with a linker, wherein the linker has the amino acid sequence GGSSEAAEAAEAAEAAEAAEAAE (SEQ ID NO: 32). Additional non-limiting examples of linkers are described in PCT Publication No. WO2015/ 197446, which is incorporated herein by reference in its entirety.

Regarding the MATRIN-3 (MATR3) conjugates (e.g., the MATRIN-3 (MATR3) FA conjugates) used in the present methods of the invention, the MATRIN-3 (MATR3) moiety or fragments and conjugate moiety, e.g., fatty acid moiety, can be joined by a linker as follows.

The linker separates the MATRIN-3 (MATR3) moiety or fragments and the conjugate moiety, e.g., fatty acid moiety. In particular embodiments, its chemical structure is not critical since it serves primarily as a spacer. In a specific embodiment, the linker is a chemical moiety that contains two reactive groups/functional groups, one of which can react with the MATRIN-3 (MATR3) moiety or fragments and the other with the conjugate moiety, e.g., fatty acid moiety. The two reactive/functional groups of the linker are linked via a linking moiety or spacer, structure of which is not critical as long as it does not interfere with the coupling of the linker to the MATRIN-3 (MATR3) moiety or fragments and the conjugate moiety, e.g., fatty acid moiety, such as for example fatty acid moieties of Formula Al, A2 or A3. 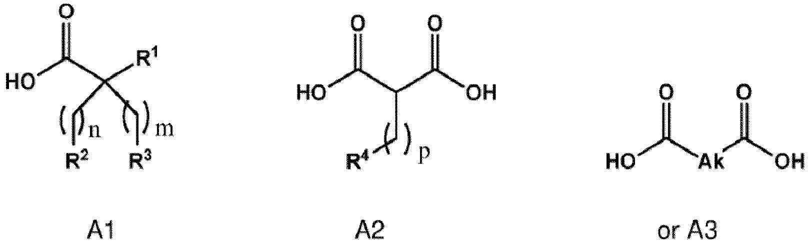
R¹ is CO₂H or H;
R², R³ and R⁴ are independently of each other H, OH, CO₂H, -CH=CH₂ or -C=CH;
Ak is a branched C₆-C₃₀alkylene;
n, m and p are independently of each other an integer between 6 and 30; and which does not

The linker can be made up of amino acids linked together by peptide bonds. The amino acids can be natural or non-natural amino acids. In some embodiments of the present invention, the linker is made up of from 1 to 20 amino acids linked by peptide bonds, wherein the amino acids are selected from the 20 naturally occurring amino acids. In various embodiments, the 1 to 20 amino acids are selected from the amino acids glycine, serine, alanine, methionine, asparagine, glutamine, cysteine, glutamic acid and lysine, or amide derivatives thereof such as lysine amide. In some embodiments, a linker is made up of a majority of amino acids that are sterically unhindered, such as glycine and alanine. In some embodiments, linkers are polyglycines, polyalanines, combinations of glycine and alanine (such as poly(Gly-Ala)), or combinations of glycine and serine (such as poly(Gly-Ser)). In some embodiments, a linker is made up of a majority of amino acids selected from histidine, alanine, methionine, glutamine, asparagine, and glycine. In some embodiments, the linker contains a poly-histidine moiety. In other embodiments, the linker contains glutamic acid, glutamine, lysine or lysine amide or combination thereof.

In some embodiment, the linker may have more than two available reactive functional groups and can therefore serve as a way to link more than one fatty acid moiety. For example, amino acids such as Glutamine, Glutamic acid, Serine, or Lysine can provide several points of attachment for a fatty acid moiety: the side chain of the amino acid and the functionality at the N-terminus or the C-terminus.

In some embodiments, the linker comprises 1 to 20 amino acids which are selected from non-natural amino acids. While a linker of 1-10 amino acid residues is preferred for conjugation with the fatty acid moiety, the present invention contemplates linkers of any length or composition. The linkers described herein are exemplary, and linkers that are much longer and which include other residues are contemplated by the present invention. Non-peptide linkers are also contemplated by the present invention.

In other embodiments, the linker comprise one or more alkyl groups, alkenyl groups, cycloalkyl groups, aryl groups, heteroaryl groups, heterocyclic groups, polyethylene glycol and/or one or more natural or unnatural amino acids, or combination thereof, wherein each of the alkyl, alkenyl, cycloalkyl, aryl, heteroaryl, heterocyclyl, polyethylene glycol and/or the natural or unnatural amino acids are optionally combined and linked together, or linked to the MATRIN-3 (MATR3) moiety or peptide and/or to the fatty acid moiety, via a chemical group selected from -C(0)0-, - OC(O)-, - NHC(O)-, -C(0)NH-, -0-, -NH-, -S-, -C(O)-, -OC(0)NH-, -NHC(0)-0-, =NH-0-, =NH-NH- or =NH-N(alkyl)-.

Linkers containing alkyl spacer are for example -NH-(CH2)Z-C(0)- or -S-(CH2)Z- C(O)- or -0-(CH2)z-C(0)-, -NH-(CH2)Z-NH- , -0-C(0)-(CH2)z-C(0)-0-, -C(0)-(CH2)z-0-, - NHC(0)-(CH2)z-C(0)-NH- and the like wherein z is 2-20 can be used. These alkyl linkers can further be substituted by any non-sterically hindering group, including, but not limited to, a lower alkyl (e.g., Ci-C6), lower acyl, halogen (e.g., CI, Br), CN, NH2, or phenyl.

The linker can also be of polymeric nature. The linker may include polymer chains or units that are biostable or biodegradable. Polymers with repeat linkage may have varying degrees of stability under physiological conditions depending on bond lability. Polymers may contain bonds such as polycarbonates (-O-C(O)-O-), polyesters (-C(O)-O-), polyurethanes (-NH- C(O)-O-), polyamide (-C(O)-NH-). These bonds are provided by way of examples and are not intended to limit the type of bonds employable in the polymer chains or linkers of the invention. Suitable polymers include, for example, polyethylene glycol (PEG), polyvinyl pyrrolidone, polyvinyl alcohol, polyamino acids, divinylether maleic anhydride, N-(2-hydroxypropyl)- methacrylicamide, dextran, dextran derivatives, polypropylene glycol, polyoxyethylated polyol, heparin, heparin fragments, polysaccharides, cellulose and cellulose derivatives, starch and starch derivatives, polyalkylene glycol and derivatives thereof, copolymers of polyalkylene glycols and derivatives thereof, polyvinyl ethyl ether, and the like and mixtures thereof. A polymer linker is for example polyethylene glycol (PEG). The PEG linker can be linear or branched. A molecular weight of the PEG linker in the present invention is not restricted to any particular size, but certain embodiments have a molecular weight between 100 to 5000 Dalton for example 500 to 1500 Dalton.

The linking moiety (or spacer) contains appropriate functional-reactive groups at both terminals that form a bridge between an amino group of the peptide or polypeptide/protein (e.g. N-terminus or side chain of a lysine) and a functional/reactive group on the fatty acid moiety (e.g the carboxylic acid functionality of the fatty acid moiety). Alternatively, the linking moiety (or spacer) contains appropriate functional-reactive groups at both terminals that form a bridge between an acid carboxylic group of the peptide or polypeptide/protein (e.g. C-terminus) and a functional/reactive group on the fatty acid moiety (e.g the carboxylic acid functionality of the fatty acid moiety of formula Al, A2 and A3 as above).

The linker may comprise several linking moieties (or spacer) of different nature (for example a combination of amino acids, heterocyclyl moiety, PEG and/or alkyl moieties). In this instance, each linking moiety contains appropriate functional-reactive groups at both terminals that form a bridge between an amino group of the peptide or polypeptide/protein (e.g. the N- terminus or the side chain of a lysine) and the next linking moiety of different nature and/or contains appropriate functional-reactive groups that form a bridge between the prior linking moiety of different nature and the fatty acid moiety. In other instance, each linking moiety contains appropriate functional-reactive groups at both terminals that form a bridge between an acid carboxylic group of the peptide or polypeptide/protein (e.g. the C-terminus) and the next linking moiety of different nature and/or contains appropriate functional-reactive groups that form a bridge between the prior linking moiety of different nature and the fatty acid moiety.

Additionally, a linking moiety may have more than 2 terminal functional groups and can therefore be linked to more than one fatty acid moiety. Example of these multi-functional group moieties are glutamic acid, lysine, or serine. The side chain of the amino acid can also serve as a point of attachment for another fatty acid moiety.

The modified peptides or polypeptides and/or peptide -polypeptide partial construct (i.e. peptide/polypeptide attached to a partial linker) include reactive groups which can react with available reactive functionalities on the fatty acid moiety (or modified fatty acid moiety: i.e. already attached a partial linker) to form a covalent bond. Reactive groups are chemical groups capable of forming a covalent bond. Reactive groups are located at one site of conjugation and can generally be carboxy, phosphoryl, acyl group, ester, or mixed anhydride, maleimide, N-hydroxysuccinimide, tetrazine, alkyne, imidate, pyridine-2-yl-disulfanyl, thereby capable of forming a covalent bond with functionalities like amino group, hydroxyl group, alkene group, hydrazine group, hydroxylamine group, an azide group, or a thiol group at the other site of conjugation.

Reactive groups of particular interest for conjugating a MATRIN-3 (MATR3) moiety or fragment or peptide to a linker and/or a linker to the fatty acid moiety and/or to conjugate various linking moieties of different nature together are N-hydroxysuccinimide, alkyne (more particularly cyclooctyne).

Functionalities include: 1. thiol groups for reacting with maleimides, tosyl sulfone or pyridine-2-yldisulfanyl; 2. amino groups (for example amino functionality of an amino acid) for bonding to carboxylic acid or activated carboxylic acid (e.g. amide bond formation via N-hydroxysuccinamide chemistry), phosphoryl groups, acyl group or mixed anhydride; 3. Azide to undergo a Huisgen cycloaddition with a terminal alkyne and more particularly cyclooctyne (more commonly known as click chemistry); 4. carbonyl group to react with hydroxylamine or hydrazine to form oxime or hydrazine respectively; 5. Alkene and more particularly strained alkene to react with tetrazine in an aza [4+2] addition. While several examples of linkers and functionalities/reactive group are described herein, the methods of the present invention contemplate linkers of any length and composition.

### MATRIN-3 (MATR3) Fusion Polypeptides

In specific aspects, MATRIN-3 (MATR3) fusion polypeptides described herein as useful for administration for the present methods of treatment of the invention may contain a MATRIN-3 (MATR3) fragment and a heterologous moiety, and optionally a linker. In a particular embodiment, a MATRIN-3 (MATR3) fusion polypeptide described herein as useful for administration for the present methods of treatment of the invention may contain a MATRIN-3 (MATR3) fragment and a heterologous moiety which is SA, a cell-penetrating peptide (CPP), an antibody or a variant thereof, and optionally a linker.

In specific aspects, MATRIN-3 (MATR3) fusion polypeptides described herein as useful for administration for the present methods of treatment of the invention may contain a MATRIN-3 (MATR3) moiety or fragments or peptide and SA, a cell-penetrating peptide (CPP) moiety, antibody moiety or a variant thereof, and optionally a linker. In one embodiment, the fusion polypeptide is a contiguous amino acid chain in which the SA, CPP, MCPP moiety is located N-terminally to the MATRIN-3 (MATR3) moiety. The C-terminus of the SA, CPP or antibody moiety can be directly bonded to the N-terminus of the MATRIN-3 (MATR3) moiety or fragment. Preferably, the C-terminus of the SA, CPP, antibody moiety is indirectly bonded to the N-terminus of the MATRIN-3 (MATR3) moiety or fragment or peptide through a peptide linker.

The SA, CPP or antibody moiety and MATRIN-3 (MATR3) moiety or fragment can be from any desired species. For example, the fusion protein can contain SA, CPP, antibody and MATRIN-3 (MATR3) moieties or fragments that are from human, mouse, rat, dog, cat, horse, or any other desired species. The SA, CPP, antibody and MATRIN-3 (MATR3) moieties or fragments or peptides are generally from the same species, but fusion peptides in which the SA, CPP or antibody moiety is from one species and the MATRIN-3 (MATR3) moiety or fragment is from another species (e.g., mouse SA, CPP or antibody and human MATRIN-3 (MATR3)) are also encompassed by this disclosure.

In some embodiments, the fusion polypeptide comprises mouse serum albumin (SA), CPP or functional variant thereof and mature human MATRIN-3 (MATR3) peptide or functional variant thereof.

In preferred embodiments, the SA moiety is HSA, CPP moiety is B-MSP or a functional variant thereof and the MATRIN-3 (MATR3) moiety or fragment is the mature human MATR3 peptide or a functional variant thereof. When present, the optional linker is preferably a flexible peptide linker. If desired, the fusion polypeptide can further comprise a linker that links the C- terminus of the SA moiety to the N-terminus of the MATRIN-3 moiety.

If desired, the fusion polypeptide can contain additional amino acid sequence. For example, an affinity tag can be included to facilitate detecting and/or purifying the fusion polypeptide.

### MATRIN-3 (MATR3) Conjugates

Various embodiments of the MATRIN-3 (MATR3) (peptide) conjugates, e.g., MATRIN-3 (MATR3) fatty acid conjugates, that can be used in the present methods of treatment of the invention are described herein. It will be recognized that features specified in each embodiment may be combined with other specified features to provide further embodiments.

In a specific embodiment, a MATRIN-3 (MATR3) (peptide) conjugate for the methods provided here comprises a MATRIN-3 (MATR3) peptide or a functional variant thereof conjugated to a moiety, such as a fatty acid moiety, optionally comprising a linker. In some embodiment of the invention, the fatty acid residue is a lipophilic residue.

In another embodiment the fatty acid residue is negatively charged at physiological pH. In another embodiment the fatty acid residue comprises a group which can be negatively charged. One preferred group which can be negatively charged is a carboxylic acid group.

In another embodiment of the invention, the fatty acid residue binds non-covalently to albumin or other plasma proteins. In yet another embodiment of the invention the fatty acid residue is selected from a straight chain alkyl group, a branched alkyl group, a group which has an (O-carboxylic acid group, a partially or completely hydrogenated cyclopentanophenanthrene skeleton.

In another embodiment the fatty acid residue is a cibacronyl residue. In another embodiment the fatty acid residue has from 6 to 40 carbon atoms, from 8 to 26 carbon atoms or from 8 to 20 carbon atoms.

In another embodiment, the fatty acid residue is an acyl group selected from the group comprising R-C(O)- wherein R is a C4-38 linear or branched alkyl or a C4-38 linear or branched alkenyl where each said alkyl and alkenyl are optionally substituted with one or more substituents selected from -C02H, hydroxyl, -SO3H, halo and -NHC(0)C(0)OH. The acyl group (R-C(O)-) derives from the reaction of the corresponding carboxylic acid R-C(0)OH with an amino group on the MATRIN-3 (MATR3) polypeptide.

In another embodiment the fatty acid residue is an acyl group selected from the group comprising CH3(CH2)r-CO, wherein r is an integer from 4 to 38, preferably an integer from 4 to 24, more preferred selected from the group comprising CH3(CH2)6CO-, CH3(CH2)s- CO-, CH3(CH2)10-CO-, CH3(CH2)12-CO-, CH3(CH2)14-CO-, CH3(CH2)16-CO-, CH3(CH2)18-CO-, CH3(CH2)20-CO and CH3(CH2)22-CO-.

In another embodiment the fatty acid residue is an acyl group of a straight-chain or branched alkane.

In another embodiment the fatty acid residue is an acyl group selected from the group comprising HOOC-(CH2)sCO-, wherein s is an integer from 4 to 38, preferably an integer from 4 to 24, more preferred selected from the group comprising HOOC(CH2)i4-CO-, HOOC(CH2)16-CO-, HOOC(CH2)18-CO-, HOOC(CH2)20-CO- and HOOC(CH2)22-CO-.

In another embodiment the fatty acid residue is a group of the formula CH3-(CH2)X- CO-NH-CH(CH2C02H)-C(0)- wherein x is an integer of from 8 to 24.

In yet another embodiment the fatty acid residue is selected from the group consisting of: CH3-(CH2)6_24-C02H; CF3-(CF2)4_9-CH2CH2-C02H; CF3-(CF2)4.9-CH2CH2-0-CH2-C02H; C02H-(CH2)6.24-C02H; S02H-(CH2)6.24-C02H; wherein the fatty acid is linked to an amino group on MATRIN-3 (MATR3) polypeptide (N-terminus or side chain of a lysine) or to an amino group on a linker via one of its carboxylic functionalities.

Specific examples of fatty acid are: 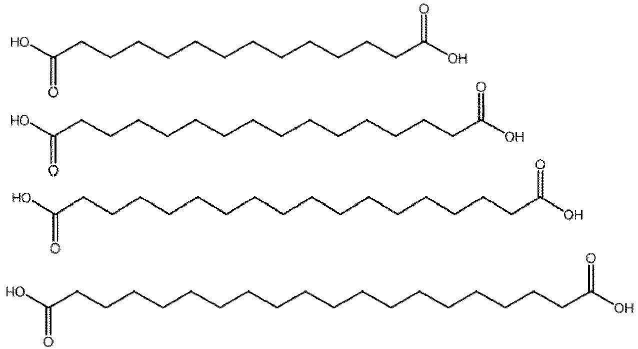 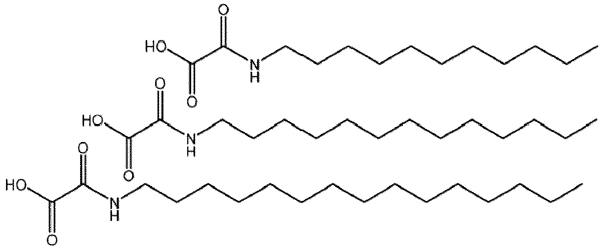 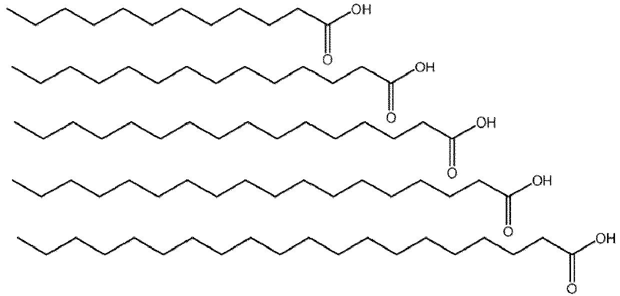 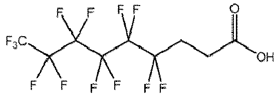 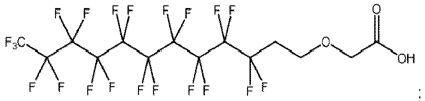 wherein the fatty acid is linked to the N-terminus of MATRIN-3 (MATR3) or to an amino group on the side chain of MATRIN-3 (MATR3) or to an amino group on a linker via one of its carboxylic acid functionalities.

Examples of fatty acid linked to one or two Glutamic acid amino acids are: 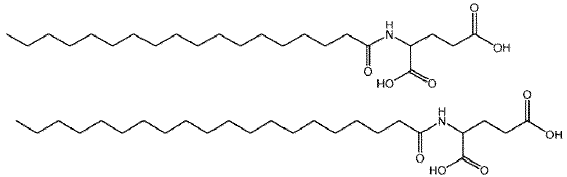 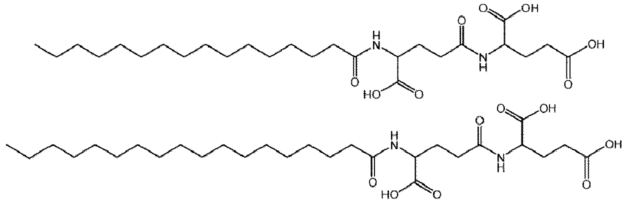 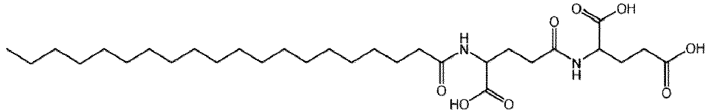 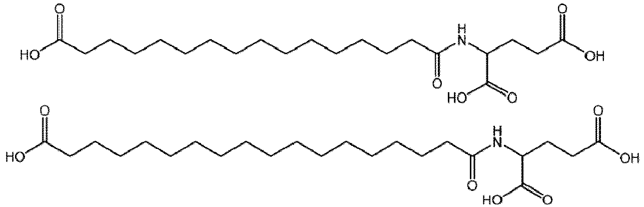 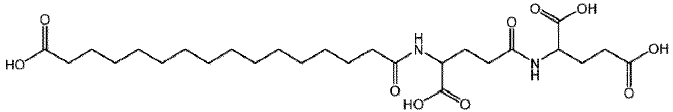 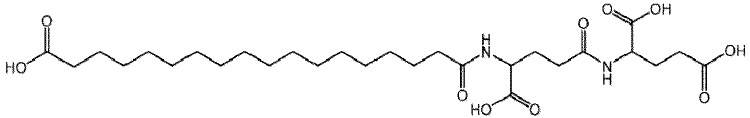 wherein the chiral carbon atoms independently are either R or S and wherein the fatty acid-linker moiety is linked to the N-terminus of MATRIN-3 (MATR3) or to an amino group on the side chain of MATRIN-3 (MATR3) or to an amino group on another linking moiety via one of the Glutamic acid's carboxylic acid functionalities.

Example of fatty acid moiety(ies) linked to a Lysine or/and Lysine amide amino acids are: 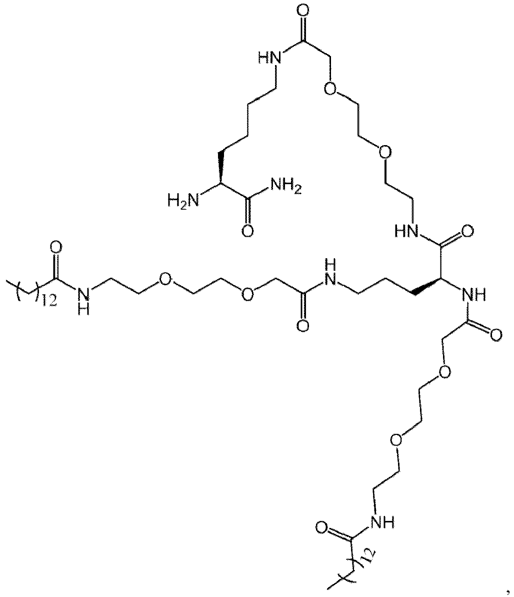 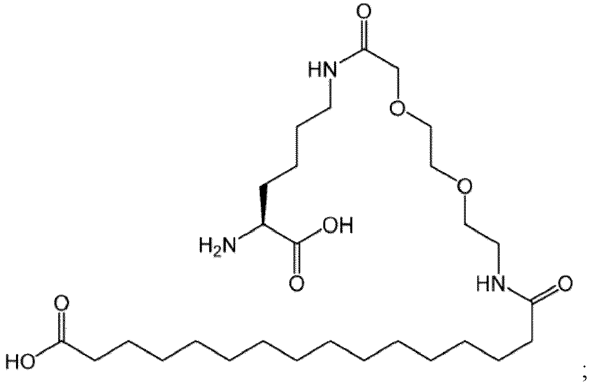 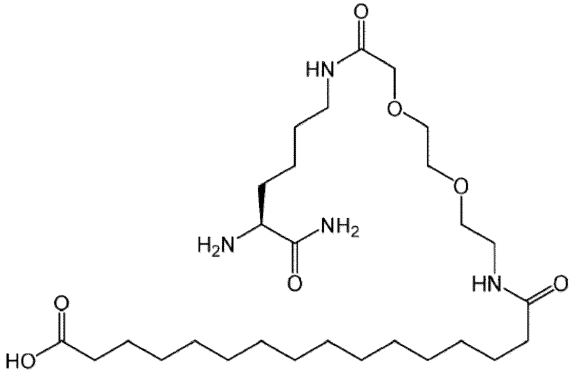 wherein the primary amino group of the lysine is attached the C-terminus of MATRIN-3 (MATR3) or to a carboxylic acid functionality on a side chain of MATRIN-3 (MATR3); or to a carboxylic acid functionality on another linking moiety.

Another specific example of linkers to be used with above fatty acids is 4- sulfamoylbutanoic acid:

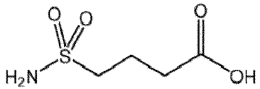

Examples of fatty acids linked to the above linker are: 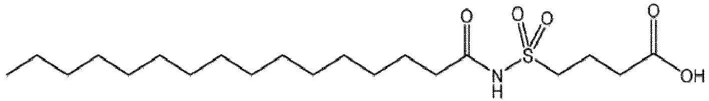 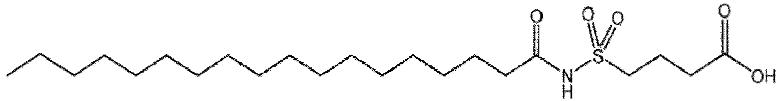 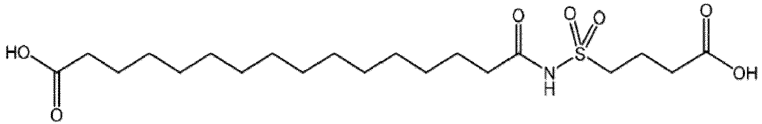 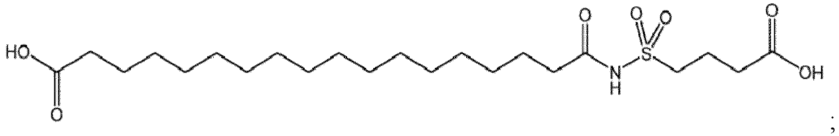 wherein the fatty acid-linker moiety is linked to the N-terminus of MATRIN-3 (MATR3) or to an amino group on the side chain of MATRIN-3 (MATR3) or to an amino group on another linking moiety via the carboxylic acid functionality on the sulfamoyl butanoic acid moiety.

Other examples of fatty acid-linker constructs are further disclosed in US 2013/0040884, Albumin-binding conjugates comprising fatty acid and PEG (Novo Nordisk) which is incorporated by reference.

Such constructs are preferably linked to the N-terminus of MATRIN-3 (MATR3) via a carboxylic acid functionality.

In embodiment 1, the invention pertains to a conjugate comprising a MATRIN-3 (MATR3) moiety or peptides linked to a fatty acid moiety via a linker wherein the fatty acid moiety has the following Formulae Al, A2 or A3: 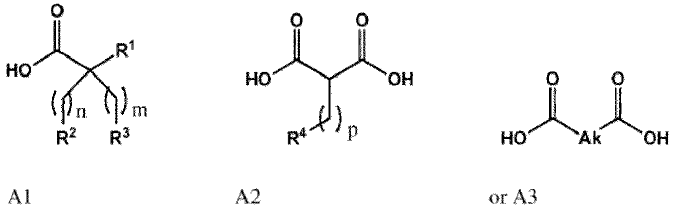
R¹ is C02H, H;
R², R³ and R⁴ are independently of each other H, OH, C02H, -CH=CH2or -C=CH;
Ak is a branched C6-C3 alkylene;
n, m, and p are independently of each other and integer between 6 and 30; or an amide, an ester, or a pharmaceutically acceptable salt thereof.

Preferred embodiments are also disclosed in WO2017/109706 incorporated by reference.

The invention pertains to conjugate according to any of the preceding conjugate's embodiments wherein the linker comprises an oligo ethylene glycol moiety as disclosed in WO2017/109706, incorporated by reference.

The invention pertains to conjugate according to any of the preceding conjugate's embodiments wherein the linker comprises (or further comprises) a heterocyclic moiety as disclosed in WO2017/109706, incorporated by reference.

Such heterocyclyl containing linkers are obtained for example by azide-alkyne Huisgen cycloaddition, which more commonly known as click chemistry. More particularly, some of the heterocyclyl depicted supra result from the reaction of a cycloalkyne with an azide - containing moiety.

Cycloalkyne are readily available from commercial sources and can therefore be functionalized via cycloaddition with a moiety containing an azide functionality (e.g. a linker containing a terminal azide functionality). Examples of the use of cyclic alkyne click chemistry in protein labeling has been described in US 2009/0068738 which is herein incorporated by reference.

These reagents which are readily available and/or commercially available are attached directly or via a linker as described supra to the peptide or polypeptide of interest. The alkyne, maleimide or tetrazine reactive groups are reacted with a functional group (azide, thiol, and alkene respectively) which is present on the fatty acid moiety or on a linker-fatty acid construct (such as for example a PEG-fatty acid construct).

In a further embodiment, the invention pertains to a conjugate according to any of the preceding conjugate's embodiments wherein the linker comprises or further comprises one or more amino acids independently selected from histidine, methionine, alanine, glutamine, asparagine, and glycine. In one particular aspect of this embodiment, the linker comprises 1 to 6 amino acids selected from histidine, alanine, and methionine.

The invention also pertains to a conjugate according to any one of the preceding conjugate's embodiments wherein the MATRIN-3 (MATR3) moiety is MATRIN-3 (MATR3)), or related proteins and homologs, variants, fragments, and other modified forms thereof. In a further embodiment, the invention pertains to a conjugate according to any one of the preceding conjugate's embodiments wherein the MATRIN-3 (MATR3) moiety is a MATRIN-3 (MATR3) variant.

### Nucleic Acids and Host Cells

The invention also relates to nucleic acids that encode MATR3 fragments, or fusion polypeptides containing MATR3 or MATR3 fragments or peptides described herein particularly as useful for administration for the present methods of treatment of the invention, including vectors that can be used to produce the polypeptides. The nucleic acids are preferably isolated and/or recombinant. In certain embodiments, the nucleic acid encodes a fusion polypeptide in which HSA, CPP or antibody or a functional variant thereof is located N-terminally to human mature MATRIN-3 (MATR3) fragments or a functional variant thereof. If desired the nucleic acid can further encode a linker (e.g., a flexible peptide linker) that bonds the C-terminus of the SA, CPP, antibody or a functional variant thereof to the N-terminus of human mature MATRIN-3 (MATR3) fragments or a functional variant thereof. If desired, the nucleic acid can also encode a leader, or signal, sequence to direct cellular processing and secretion of the fusion polypeptide.

In preferred embodiments, the nucleic acid encodes a fusion polypeptide in which the SA moiety is HSA or a functional variant thereof and the MATRIN-3 (MATR3) moiety or fragment is the mature human MATRIN-3 fragment or a functional variant thereof. When present, the optional linker is preferably a flexible peptide linker. In particular embodiments, the nucleic acid encodes a fusion polypeptide that comprises A) an SA moiety selected from the group consisting of HSA(25-609) (SEQ ID NO:10) and B) a MATRIN-3 fragments selected from the group consisting of sequences of SEQ ID Nos. 1, 3-8, 11-19.

If desired, the encoded fusion polypeptide can further comprise a linker that links the C-terminus of the SA, CPP or antibody moiety to the N-terminus of the MATRIN-3 (MATR3) moiety or peptide. Preferably, the linker is as disclosed in WO2020/152367, herein incorporated by reference.

For expression in host cells, the nucleic acid encoding a fusion polypeptide can be present in a suitable vector and after introduction into a suitable host, the sequence can be expressed to produce the encoded fusion polypeptide according to standard cloning and expression techniques, which are known in the art (e.g., as described in Sambrook, J., Fritsh, E. F., and Maniatis, T. Molecular Cloning: A Laboratory Manual 2nd ed., Cold Spring Harbor Laboratory, Cold Spring Harbor Laboratory Press, Cold Spring Harbor, N.Y., 1989). The invention also relates to such vectors comprising a nucleic acid sequence according to the invention.

A recombinant expression vector can be designed for expression of a MATRIN-3 (MATR3) fusion polypeptide in prokaryotic (e.g., E. coli) or eukaryotic cells (e.g., insect cells, yeast cells, or mammalian cells). Representative host cells include many E. coli strains, mammalian cell lines, such as CHO, CHO-K1, and HEK293; insect cells, such as Sf9 cells; and yeast cells, such as S. cerevisiae and P. pastoris. Alternatively, the recombinant expression vector can be transcribed and translated in vitro, for example using T7 promoter regulatory sequences and T7 polymerase and an in vitro translation system. Vectors suitable for expression in host cells and cell-free in vitro systems are well known in the art. Generally, such a vector contains one or more expression control elements that are operably linked to the sequence encoding the fusion polypeptide.

Expression control elements include, for example, promoters, enhancers, splice sites, poly adenylation signals and the like. Usually a promoter is located upstream and operably linked to the nucleic acid sequence encoding the fusion polypeptide. The vector can comprise or be associated with any suitable promoter, enhancer, and other expression-control elements. Examples of such elements include strong expression promoters (e.g., a human CMV IE promoter/enhancer, an RSV promoter, SV40 promoter, SL3-3 promoter, MMTV promoter, or HIV LTR promoter, EF1 alpha promoter, CAG promoter) and effective poly (A) termination sequences. Additional elements that can be present in a vector to facilitate cloning and propagation include, for example, an origin of replication for plasmid product in E. coli, an antibiotic resistance gene as a selectable marker, and/or a convenient cloning site (e.g., a polylinker).

In another aspect of the instant disclosure, host cells comprising the nucleic acids and vectors disclosed herein are provided. In various embodiments, the vector or nucleic acid is integrated into the host cell genome, which in other embodiments the vector or nucleic acid is extra-chromosomal. If desired the host cells can be isolated.

Recombinant cells, such as yeast, bacterial (e.g., E. coli), and mammalian cells (e.g., immortalized mammalian cells) comprising such a nucleic acid, vector, or combinations of either or both thereof are provided. In various embodiments, cells comprising a non-integrated nucleic acid, such as a plasmid, cosmid, phagemid, or linear expression element, which comprises a sequence coding for expression of a fusion polypeptide comprising the human MATRIN-3 (MATR3) fragment or a functional variant thereof fused or not with SA, CPP or an antibody or the functional variant thereof and, are provided.

A vector comprising a nucleic acid sequence encoding a MATRIN-3 (MATR3) fusion polypeptide or MATR-3 peptide disclosed herein can be introduced into a host cell using any suitable method, such as by transformation, transfection, or transduction. Suitable methods are well known in the art. In one example, a nucleic acid encoding a fusion polypeptide comprising the SA, CPP or antibody or the functional variant thereof and human MATRIN-3 (MATR3) peptide or the functional variant thereof can be positioned in and/or delivered to a host cell or host animal via a viral vector. Any suitable viral vector can be used in this capacity.

The invention also provides a method for producing a fusion polypeptide as described herein, comprising maintaining a recombinant host cell comprising a recombinant nucleic acid of the invention under conditions suitable for expression of the recombinant nucleic acid, whereby the recombinant nucleic acid is expressed, and a fusion polypeptide is produced. In some embodiments, the method further comprises isolating the fusion polypeptide.

In the present invention a preferred mode of treatment is by a gene therapy-type approach in which MATR3 fragments, peptide, variant, fusion thereof or a TPD, such as PROTAC, containing the same will be delivered using vectors, preferably AAV derived vectors, preferably with a muscle-specific promoter, preferably the vector is administered intramuscularly or systemically for FSHD; preferably lentivirus derived vectors, preferably with a B cell-specific promoter, preferably the vector is administered ex-vivo for ALL.

The cells of the invention may be used for transplants in the patient, so cell therapy is contemplated in the present invention.

In various embodiments, the peptide of the invention is comprised within a vector. A vector is a tool that allows or facilitates the transfer of an entity from one environment to another. By way of example, some vectors used in recombinant DNA techniques allow entities, such as a segment of DNA (such as a heterologous DNA segment, such as a heterologous cDNA segment), to be transferred into a target cell. Examples of vectors used in recombinant DNA techniques include plasmids, chromosomes, artificial chromosomes, or viruses.

Examples of viral vectors include lentiviral vectors, retroviral vectors, Murine Leukemia Virus (MLV) vectors, adenovirus vectors, pox viral vectors and vaccinia viral vectors. Examples of retroviral vectors include murine leukemia virus (MLV), human immunodeficiency virus (HIV-1), equine infectious anemia virus (EIAV), mouse mammary tumor virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other retroviridiae including lentiviruses. A detailed list of retroviruses may be found in Coffm et al., 1997, "retroviruses", Cold Spring Harbour Laboratory Press Eds: JM Coffin, SM Hughes, HE Varmus pp 758- 763.

Preferably, the viral vector is a targeted vector, that is it has a tissue tropism which is altered compared to the native virus, so that the vector is targeted to particular cells.

More preferably, the viral vector preferentially targets a certain hematopoietic cell type or hematopoietic cell types. Preferably the viral vector targets only hematopoietic cells that have a hematopoietic disorder.

In a preferred embodiment the vector is derivable from a lentivirus or from an adeno-associated virus.

### Therapeutic Methods and Pharmaceutical Compositions

DUX4 is a homeodomain-containing transcription factor and an important regulator of early human development as it plays an essential role in activating the embryonic genome during the 2-to 8-cell stage of development *(*Nat. Genet. 49, 925-934 (2017); Nat. Genet. 49, 935-940 (2017); Nat. Genet. 49, 941-945 (2017). As such, it is not typically expressed in healthy somatic cells, and importantly it is silent in healthy skeletal muscle or B-cells.

The present invention refers to the treatment of a condition associated with an aberrant expression and/or function of DUX4 protein and/or of rearranged DUX4 protein (such as DUX4-IGH). Such condition includes muscular dystrophy and cancer, such as leukemia.

For instance, facioscapulohumeral muscular dystrophy (FSHD) is one of the most prevalent neuromuscular disorders *(*Neurology 83, 1056-9 (2014) and leads to significant lifetime morbidity, with up to 25% of patients requiring wheelchair. The disease is characterized by rostro-caudal progressive and asymmetric weakness in a specific subset of muscles. Symptoms typically appear as asymmetric weakness of the facial (facio), shoulder (scapulo), and upper arm (humeral) muscles, and progress to affect nearly all skeletal muscle groups. Extra-muscular manifestations can occur in severe cases, including retinal vasculopathy, hearing loss, respiratory defects, cardiac involvement, mental retardation, and epilepsy *(*Curr. Neurol. Neurosci. Rep. 16, 66 (2016). FSHD is not caused by a classical form of gene mutation that results in loss or altered protein function. Likewise, it differs from typical muscular dystrophies by the absence of sarcolemma defects (J. Cell Biol. 191, 1049-1060 (2010). Instead, FSHD is linked to epigenetic alterations affecting the D4Z4 macrosatellite repeat array in 4q35 and causing chromatin relaxation leading to inappropriate gain of expression of the D4Z4-embedded double homeobox 4 *(DUX4)* gene *(*Curr. Neurol. Neurosci. Rep. 16, 66 (2016).

Acute lymphoblastic leukemia (ALL) is the most common cancer among children and the most frequent cause of death from cancer before 20 years of age. Approximately 80-85% of pediatric ALL is of B cell origin and results from arrest at an immature B-precursor cell stage *(*N. Engl. J. Med. 373, 1541-52 (2015). The underlying etiology of most cases of childhood ALL remains largely unknown. Nevertheless, sentinel chromosomal translocations occur frequently, and recurrent ALL-associated translocations can be initiating events that drive leukemogenesis (J. Clin. Oncol. 33, 2938-48 (2015). Importantly, the characterization of gene expression, biochemical and functional consequences of these mutations may provide a window of therapeutic opportunity. Indeed, therapeutic strategies tailored to target ALL-associated driver lesions and pathways may increase anti-leukemia efficacy and decrease relapse, as well as reduce undesirable off-target toxicities *(*J. Clin. Oncol. 33, 2938-48 (2015). Recently, recurrent DUX4 rearrangements were reported in up to 10% of B-ALL patients *(*Nat. Genet. 48, 569-74 (2016); EBioMedicine 8, 173-83 (2016); Nat. Commun. 7, 11790 (2016); Nat. Genet. 48, 1481-1489 (2016). Nearly all cases exhibit rearrangement of DUX4 to the immunoglobulin heavy chain (IGH) enhancer region resulting in truncation of DUX4 C terminus and addition of amino acids from read-through into the IGH locus. The rearrangement has two functional consequences. First, the translocation hijacks the IGH enhancer resulting in overexpression of DUX4 in the B cell lineage. Second, the truncation of DUX4 C terminus and the appendage of amino acids encoded by the IGH locus changes the biology of the resulting rearranged DUX4 (DUX4-r also called DUX4-IGH) protein. While DUX4 is pro-apoptotic, DUX4-r induces transformation in NIH-3T3 fibroblasts and is required for the proliferation of DUX4-r expressing NALM6 ALL cells *(*Nat. Genet. 48, 569-74 (2016); Nat. Genet. 48, 1481-1489 (2016). Moreover, expression of DUX4-r in mouse pro-B cells is sufficient to give rise to leukemia. In contrast, mouse pro-B cells expressing wild-type DUX4 undergo cell death *(*Nat. Genet. 48, 569-74 (2016). The DUX4 rearrangement is a clonal event acquired early in leukemogenesis and the expression of DUX4-r is maintained in leukemias at relapse *(*Nat. Genet. 48, 569-74 (2016); Nat. Genet. 48, 1481-1489 (2016), strongly supporting DUX4-r as an oncogenic driver.

There are no drugs currently approved to prevent or treat a condition associated with an aberrant expression and/or function of DUX4 protein and/or of rearranged DUX4 protein (DUX4-r or DUX4-IGH), such as FSHD or DUX-r associated ALL. For the first time, the inventors identified a molecule (MATR3) able to inhibit the activity of both DUX4 and DUX4-r for the treatment of muscular dystrophies, or cancer such as FSHD and DUX-r associated ALL.

Where the invention makes use of a polypeptide, they may be administered directly e.g. as the polypeptide itself or by introducing nucleic acid constructs/viral vectors encoding the polypeptide into cells under conditions that allow for expression of the polypeptide in a cell of interest. Transfer of the polynucleotide, polypeptide may be performed by any of the methods known in the art which physically or chemically permeabilize the cell membrane or by the use of liposomes. Cell-penetrating peptides may also be used to transfer a polypeptide into a cell.

The polynucleotides for use in the invention may be contained in a gene transfer vector. The vector may be delivered to a target site by a viral or non-viral vector. Non-limiting examples of a suitable virus vector include adenovirus, adeno- associated virus, and a retrovirus including lentivirus. The vector may be an expression vector. Expression vectors as described herein comprise regions of nucleic acid containing sequences capable of being transcribed. Thus, sequences encoding mRNA, tRNA and rRNA are included within this definition.

Expression vectors preferably comprise a polynucleotide for use in the invention is preferably operably linked to a control sequence that is capable of providing for the expression of the coding sequence by the host cell. The term "operably linked" means that the components described are in a relationship permitting them to function in their intended manner. A regulatory sequence "operably linked" to a coding sequence is ligated in such a way that expression of the coding sequence is achieved under condition compatible with the control sequences. The control sequences may be modified, for example by the addition of further transcriptional regulatory elements to make the level of transcription directed by the control sequences more responsive to transcriptional modulators.

Non-viral delivery systems include but are not limited to DNA transfection methods and calcium phosphate precipitation. Here, transfection includes a process using a non-viral vector to deliver a gene to a target mammalian cell. Typical transfection methods include electroporation, DNA biolistic, lipid-mediated transfection, compacted DNA-mediated transfection, liposomes, immunoliposomes, nanoparticles, lipofectin, cationic agent-mediated, cationic facial amphiphiles (CFAs) (Nature Biotechnology 1996 14; 556), and combinations thereof. An effective amount of the therapeutic vector or the peptide or the fusion polypeptide, usually in the form of a pharmaceutical composition, is administered to a subject in need thereof. The therapeutic vector or the fusion polypeptide can be administered in a single dose or multiple doses, and the amount administered, and dosing regimen will depend upon the particular therapeutic vector or fusion protein selected, the severity of the subject's condition and other factors. A clinician of ordinary skill can determine appropriate dosing and dosage regimen based on a number of other factors, for example, the individual's age, sensitivity, tolerance, and overall well-being.

The administration can be performed by any suitable route using suitable methods, such as parenterally (e.g., intravenous, subcutaneous, intraperitoneal, intramuscular, intrathecal injections or infusion), orally, topically, intranasally or by inhalation. Parental administration is generally preferred. Intravenous administration is preferred.

MATRIN-3 (MATR3) fragments or peptides therapeutic vectors or MATRIN-3 (MATR3) fusion polypeptides of the present invention can be administered to the subject in need thereof alone or with one or more other agents. When the therapeutic vector or fusion polypeptide is administered with another agent, the agents can be administered concurrently or sequentially to provide overlap in the therapeutic effects of the agents. Examples of other agents that can be administered in combination with the therapeutic vector or the fusion polypeptide include: anti-inflammatory agents, antioxidants, chemotherapy, radiotherapy.

The invention also relates to pharmaceutical compositions comprising a MATRIN-3 (MATR3) (peptide) conjugate or a MATRIN-3 (MATR3) (peptide) fusion polypeptide as described herein (e.g., comprising a fusion polypeptide comprising SA, CPP, antibody or a functional variant thereof and human MATRIN-3 (MATR3) protein fragment or peptide or a functional variant thereof). Such pharmaceutical compositions can comprise a therapeutically effective amount of the fusion polypeptide and a pharmaceutically or physiologically acceptable carrier. The carrier is generally selected to be suitable for the intended mode of administration and can include agents for modifying, maintaining, or preserving, for example, the pH, osmolarity, viscosity, clarity, color, isotonicity, odor, sterility, stability, rate of dissolution or release, adsorption, or penetration of the composition. Typically, these carriers include aqueous or alcoholic/aqueous solutions, emulsions, or suspensions, including saline and/or buffered media.

Suitable agents for inclusion in the pharmaceutical compositions include, but are not limited to, amino acids (such as glycine, glutamine, asparagine, arginine, or lysine), antimicrobials, antioxidants (such as ascorbic acid, sodium sulfite, or sodium hydrogen-sulfite), buffers (such as borate, bicarbonate, Tris-HCl, citrates, phosphates, or other organic acids), bulking agents (such as mannitol or glycine), chelating agents (such as ethylenediamine tetra acetic acid (EDTA)), complexing agents (such as caffeine, polyvinylpyrrolidone, beta- cyclodextrin, or hydroxypropyl-beta-cyclodextrin), fillers, monosaccharides, disaccharides, and other carbohydrates (such as glucose, mannose, or dextrins), proteins (such as free serum albumin, gelatin, or immunoglobulins), coloring, flavoring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as Polysorbate 20 or Polysorbate 80; Triton; tromethamine; lecithin; cholesterol or tyloxapal), stability enhancing agents (such as sucrose or sorbitol), tonicity enhancing agents (such as alkali metal halides, such as sodium or potassium chloride, or mannitol sorbitol), delivery vehicles, diluents, excipients and/or pharmaceutical adjuvants.

Parenteral vehicles include sodium chloride solution, Ringer's dextrose, dextrose and sodium chloride and lactated Ringer's. Suitable physiologically acceptable thickeners such as carboxymethylcellulose, polyvinylpyrrolidone, gelatin and alginates may be included. Intravenous vehicles include fluid and nutrient replenishers and electrolyte replenishers, such as those based on Ringer's dextrose. In some case it will be preferable to include agents to adjust tonicity of the composition, for example, sugars, polyalcohols such as mannitol, sorbitol, or sodium chloride in a pharmaceutical composition. For example, in many cases it is desirable that the composition is substantially isotonic. Preservatives and other additives, such as antimicrobials, antioxidants, chelating agents, and inert gases, may also be present. The precise formulation will depend on the route of administration. Additional relevant principle, methods and components for pharmaceutical formulations are well known. (See, e.g., Allen, Loyd V. Ed, (2012) Remington's Pharmaceutical Sciences, 22nd Edition).

When parenteral administration is contemplated, the pharmaceutical compositions are usually in the form of a sterile, pyrogen-free, parenterally acceptable composition. A particularly suitable vehicle for parenteral injection is a sterile, isotonic solution, properly preserved. The pharmaceutical composition can be in the form of a lyophilizate, such as a lyophilized cake.

In certain embodiments, the pharmaceutical composition is for subcutaneous administration. Suitable formulation components and methods for subcutaneous administration of polypeptide therapeutics (e.g., antibodies, fusion proteins and the like) are known in the art. See, e.g., Published United States Patent Application No 2011/0044977 and US Patent No. 8,465,739 and US Patent No. 8,476,239. Typically, the pharmaceutical compositions for subcutaneous administration contain suitable stabilizers (e.g, amino acids, such as methionine, and or saccharides such as sucrose), buffering agents and tonicifying agents.

### Definitions

The term "amino acid mimetic," as used herein, refers to chemical compounds that have a structure that is different from the general chemical structure of an amino acid, but functions in a manner similar to a naturally occurring amino acid.

"Conservative" amino acid replacements or substitutions refer to replacing one amino acid with another that has a side chain with similar size, shape and/or chemical characteristics. Examples of conservative amino acid replacements include replacing one amino acid with another amino acid within the following groups: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M).

The term "effective amount" refers to an amount sufficient to achieve the desired therapeutic effect, under the conditions of administration, such as an amount sufficient to bind DUX4 or DUX4-r, prevent the interaction with DNA of DUX4 or DUX4-r, inhibit the activation of specific target genes by DUX4 or DUX4-r, reduce the toxic effects of DUX4 or reduce the cancer activity of DUX4-r. For example, a "therapeutically-effective amount" of a MATRIN-3 (MATR3) therapeutic agent administered to a patient exhibiting, suffering, or prone to suffer from a condition associated with aberrant expression and/or function of DUX4, such as FSHD or ALL is such an amount which causes an improvement in the pathological symptoms, disease progression, physiological conditions associated with or induces resistance to succumbing to the afore mentioned disorders.

"Functional variant" and "biologically active variant" refer to a polypeptide or peptide that contains an amino acid sequence that differs from a reference polypeptide or peptide (e.g., HSA, human IgFc, CPP, antibody, TPD, human wild type mature MATRIN-3 (MATR3) peptide) by sequence replacement, deletion, or addition (e.g. HSA, human IgFc, CPP, antibody or TPD fusion polypeptide), and/or addition of non-polypeptide moieties (e.g. PEG, fatty acids) but retains desired functional activity of the reference polypeptide or peptide. The amino acid sequence of a functional variant can include one or more amino acid replacements, additions, or deletions relative to the reference polypeptide, and include fragments of the reference polypeptide or peptide that retain the desired activity.

For example, a functional variant of HSA, human IgFc, CPP, antibody (e.g., reversible bicyclization) prolongs the serum half-life of the fusion polypeptides described herein in comparison to the half-life of MATRIN-3 (MATR3), while retaining the reference MATRIN-3 (MATR3) (e.g., human MATRIN-3 (MATR3)) polypeptide's activity (e.g., reduced expression of DUX4 or DUX4-r or target genes) activity. Polypeptide or peptide variants possessing a somewhat decreased level of activity relative to their wild-type versions can nonetheless be considered to be functional or biologically active polypeptide variants, although ideally a biologically active polypeptide possesses similar or enhanced biological properties relative to its wild-type protein counterpart (a protein that contains the reference amino acid sequence).

"Identity" means, in relation to nucleotide or amino acid sequence of a nucleic acid or polypeptide molecule, the overall relatedness between two such molecules. Calculation of the percent sequence identity (nucleotide or amino acid sequence identity) of two sequences, for example, can be performed by aligning the two sequences for optimal comparison purposes (e.g., gaps can be introduced in one or both of a first and a second nucleic acid or amino acid sequence for optimal alignment). The nucleotides or amino acids at corresponding positions are then compared. When a position in the first sequence is occupied by the same nucleotide or amino acid as the corresponding position in the second sequence, then the molecules are identical at that position. The percent identity between the two sequences is a function of the number of identical positions shared by the sequences, considering the number of gaps, and the length of each gap, which needs to be introduced for optimal alignment of the two sequences. The comparison of sequences and determination of percent identity between two sequences can be accomplished using a mathematical algorithm. For example, the percent identity between two sequences can be determined using methods such as those described by the National Center for Biotechnology Information (http://www.ncbi.nlm.nih.gov/). For example, the percent identity between two sequences can be determined using Clustal 2.0 multiple sequence alignment program and default parameters. Larkin MA et al. (2007) "Clustal W and Clustal X version 2.0." Bioinformatics 23(21): 2947-2948.

The term "moiety," as used herein, refers to a portion of a fusion polypeptide (e.g., SA-MATRIN3 fragment, CPP-MATRIN3 fragment, antibody-MATRIN-3 (MATR3) fragment) or fatty acid-conjugate described herein . The fusion proteins used in the methods of the present invention include, e.g., a MATRIN-3 (MATR3) moiety or peptide, which contains an amino acid sequence derived from MATRIN-3 (MATR3), and a SA, CPP or antibody moiety, which contains an amino acid sequence derived from SA, CPP or antibody. The fatty acid conjugates used in the methods of the present invention include, e.g., a MATRIN-3 (MATR3) moiety or fragment, which contains an amino acid sequence derived from MATRIN-3 (MATR3), and a fatty acid moiety, e.g., a fatty acid comprising one of the Formulae further described herein. The term "moiety" can also refer to a linker or functional molecule (e.g., PEG) comprising a fatty acid conjugate or fusion protein used in the methods of the present invention. The fusion protein optionally contains a linker moiety, which links the MATRIN-3 (MATR3) moiety or fragment and the SA, CPP or antibody moiety, in the fusion polypeptide.

Without wishing to be bound by any particular theory, it is believed that the MATRIN-3 (MATR3) moiety or peptide confers biological function of bind DUX4 or DUX4-r, prevent the interaction with DNA of DUX4 or DUX4-r, inhibit the activation of specific target genes by DUX4 or DUX4-r, reduce the toxic effects of DUX4, or reduce the cancer activity of DUX4-r, while the SA, CPP or antibody moiety prolongs the serum half-life, improves expression and stability, and increase delivery to skeletal muscle of the fusion polypeptides described herein.

The term "naturally occurring" when used in connection with biological materials such as nucleic acid molecules, polypeptides, host cells, and the like, refers to materials that are found in nature and are not manipulated by man. Similarly, "non-naturally occurring" as used herein refers to a material that is not found in nature or that has been structurally modified or synthesized by man. When used in connection with nucleotides, the term "naturally occurring" refers to the bases adenine (A), cytosine (C), guanine (G), thymine (T), and uracil (U). When used in connection with amino acids, the term "naturally occurring" refers to the 20 conventional amino acids (i.e., alanine (A), cysteine (C), aspartic acid (D), glutamic acid (E), phenylalanine (F), glycine (G), histidine (H), isoleucine (I), lysine (K), leucine (L), methionine (M), asparagine (N), proline (P), glutamine (Q), arginine (R), serine (S), threonine (T), valine (V), tryptophan (W), and tyrosine (Y)), as well as selenocysteine, pyrrolysine (PYL), and pyrroline-carboxy- lysine (PCL).

As used herein, the terms "variant," "mutant," as well as any like terms, when used in reference to MATRIN-3 (MATR3) (or fragments or peptides thereof) or antibody or specific versions thereof (e.g., "MATRIN-3 (MATR3) fragment or peptide variant," "human MATRIN-3 (MATR3) fragment or peptide variant," etc.) define protein or polypeptide sequences that comprise modifications, truncations, deletions, or other variants of naturally occurring (i.e., wild-type) protein or polypeptide counterparts or corresponding native sequences. "MATRIN-3 (MATR3) fragment or peptide variant," for instance, is described relative to the wild-type (i.e., naturally occurring) MATRIN-3 (MATR3) protein or fragment or peptide as described herein and known in the literature.

A "subject" is an individual to whom a MATRIN-3 (MATR3) fusion polypeptide or MATRIN-3 (MATR3) conjugate (e.g., usually in the form of a pharmaceutical composition) is administered. The subject is preferably a human, but "subject" includes animals, mammals, pet, and livestock animals, such as cows, sheep, goats, horses, dogs, cats, rabbits, guinea pigs, rats, mice, or other bovine, ovine, equine, canine, feline, rodent or murine species, poultry, and fish.

The term "MATRIN-3 (MATR3) therapeutic agent" as used herein means a MATRIN-3 (MATR3) polypeptide or fragment, MATRIN-3 (MATR3) fragment variant, MATRIN-3 (MATR3) (peptide) fusion protein, or MATRIN-3 (MATR3) (peptide) conjugate (e.g., a MATRIN-3 (MATR3) fatty acid conjugate), or a pharmaceutical composition comprising one or more of the same, that is administered to a subject in order to treat in a condition associated with aberrant expression and/or function of DUX4, such as FSHD or ALL

The terms "conjugate" and "fatty acid conjugate" are used interchangeably and are intended to refer to the entity formed as a result of a covalent attachment of a polypeptide or protein (or fragment and/or variant thereof) and a fatty acid moiety, optionally via a linker.

One of ordinary skill in the art will appreciate that various amino acid substitutions, e.g, conservative amino acid substitutions, may be made in the sequence of any of the polypeptide or protein described herein, without necessarily decreasing its activity. As used herein, "amino acid commonly used as a substitute thereof' includes conservative substitutions (i.e., substitutions with amino acids of comparable chemical characteristics). For the purposes of conservative substitution, the non-polar (hydrophobic) amino acids include alanine, leucine, isoleucine, valine, glycine, proline, phenylalanine, tryptophan, and methionine. The polar (hydrophilic), neutral amino acids include serine, threonine, cysteine, tyrosine, asparagine, and glutamine. The positively charged (basic) amino acids include arginine, lysine, and histidine. The negatively charged (acidic) amino acids include aspartic acid and glutamic acid. Examples of amino acid substitutions include substituting an L-amino acid for its corresponding D-amino acid, substituting cysteine for homocysteine or other non-natural amino acids having a thiol- containing side chain, substituting a lysine for homolysine, diaminobutyric acid, diaminopropionic acid, ornithine or other non-natural amino acids having an amino containing side chain, or substituting an alanine for norvaline or the like.

The term "amino acid," as used herein, refers to naturally occurring amino acids, unnatural amino acids, amino acid analogues and amino acid mimetics that function in a manner similar to the naturally occurring amino acids, all in their D and L stereoisomers if their structure allows such stereoisomeric forms. Amino acids are referred to herein by either their name, their commonly known three letter symbols or by the one-letter symbols recommended by the IUPAC-IUB Biochemical Nomenclature Commission.

The term "naturally occurring" refers to materials which are found in nature and are not manipulated by man. Similarly, "non-naturally occurring," "un-natural," and the like, as used herein, refers to a material that is not found in nature or that has been structurally modified or synthesized by man. When used in connection with amino acids, the term "naturally occurring" refers to the 20 conventional amino acids (i.e., alanine (A or Ala), cysteine (C or Cys), aspartic acid (D or Asp), glutamic acid (E or Glu), phenylalanine (F or Phe), glycine (G or Gly), histidine (H or His), isoleucine (I or He), lysine (K or Lys), leucine (L or Leu), methionine (M or Met), asparagine (N or Asn), proline (P or Pro), glutamine (Q or Gin), arginine (R or Arg), serine (S or Ser), threonine (T or Thr), valine (V or Val), tryptophan (W or Trp), and tyrosine (Y or Tyr)). The terms "non-natural amino acid" and "unnatural amino acid," as used herein, are interchangeably intended to represent amino acid structures that cannot be generated biosynthetically in any organism using unmodified or modified genes from any organism, whether the same or different. The terms refer to an amino acid residue that is not present in the naturally occurring (wild type) protein sequence or the sequences of the present invention.

These include, but are not limited to, modified amino acids and/or amino acid analogues that are not one of the 20 naturally occurring amino acids, selenocysteine, pyrrolysine (Pyl), or pyrroline-carboxy-lysine (Pel, e.g., as described in PCT patent publication WO2010/48582). Such non-natural amino acid residues can be introduced by substitution of naturally occurring amino acids, and/or by insertion of non-natural amino acids into the naturally occurring (wild type) protein sequence or the sequences of the invention. The non-natural amino acid residue also can be incorporated such that a desired functionality is imparted to the molecule, for example, the ability to link a functional moiety (e.g., PEG). When used in connection with amino acids, the symbol "U" shall mean "non-natural amino acid" and "unnatural amino acid," as used herein.

The term "analogue" as used herein referring to a polypeptide or protein means a modified peptide or protein wherein one or more amino acid residues of the peptide/protein have been substituted by other amino acid residues and/or wherein one or more amino acid residues have been deleted from the peptide/protein and/or wherein one or more amino acid residues have been added the peptide/protein. Such addition or deletion of amino acid residues can take place at the N-terminal of the peptide and/or at the C-terminal of the peptide.

The terms "MATRIN-3 (MATR3) polypeptide" and "MATRIN-3 (MATR3) protein" are used interchangeably and mean a naturally occurring wild-type polypeptide expressed in a mammal, such as a human or a mouse. For purposes of this disclosure, the term "MATRIN-3 (MATR3) protein" can be used interchangeably to refer to any full-length MATRIN-3 (MATR3) polypeptide, which consists of 847 amino acid residues; (NCBI Ref. Seq. NP_954659.1) contains four known functional domains: Zinc finger 1, RNA recognition motif 1, RNA recognition motif 2 and Zinc finger 2, as also disclosed in WO2020152367, herein enclosed by reference, and in the NCBI Ref. Seq. NP_954659.1.

The term "MATRIN-3 (MATR3) peptide variant" encompasses a MATRIN-3 (MATR3) peptide or fragment in which a naturally occurring MATRIN-3 (MATR3) peptide or fragment sequence has been modified. Such modifications include, but are not limited to, one or more amino acid substitutions, including substitutions with non-naturally occurring amino acids non-naturally occurring amino acid analogs and amino acid mimetics.

In one aspect, the term "MATRIN-3 (MATR3) peptide variant" refers to a MATRIN-3 (MATR3) protein sequence in which at least one residue normally found at a given position of a native MATRIN-3 (MATR3) peptide is deleted or is replaced by a residue not normally found at that position in the native MATRIN-3 (MATR3) sequence. In some cases it will be desirable to replace a single residue normally found at a given position of a native MATRIN-3 (MATR3) peptide with more than one residue that is not normally found at the position; in still other cases it may be desirable to maintain the native MATRIN-3 (MATR3) peptide sequence and insert one or more residues at a given position in the protein; in still other cases it may be desirable to delete a given residue entirely; all of these constructs are encompassed by the term "MATRIN-3 (MATR3) peptide variant". The variant comprised in the present invention are e.g. peptides with a higher binding affinity DUX4/DUX4-r, protein stability, solubility, etc compared to the native MATRIN-3 (MATR3) peptides.

The methods of the present invention also encompass nucleic acid molecules encoding such MATRIN-3 (MATR3) variant peptide sequences. In various embodiments, a MATRIN-3 (MATR3) peptide variant comprises an amino acid sequence that is at least about 85 percent identical to a naturally occurring MATRIN-3 (MATR3) peptide. In other embodiments, a MATRIN-3 (MATR3) peptide comprises an amino acid sequence that is at least about 80%, 85%, 90%, or about 95%, 96%, 97%, 98%, or 99% identical to a naturally occurring MATRIN-3 (MATR3) polypeptide or peptide amino acid sequence. Such MATRIN-3 (MATR3) mutant peptides (or variant) preferably, but need not, possess at least one activity of a wild-type MATRIN-3 (MATR3) peptide or of wild-type MATRIN-3 (MATR3) polypeptide, such as:
- inhibits DUX4-induced toxicity in particular in HEK293 cells,
- blocks induction of DUX4 targets, in particular in HEK293 cells,
- interacts with the DNA-binding domain of DUX4,
- inhibits DUX4 directly by blocking its ability to bind DNA,
- inhibits the expression of DUX4 and DUX4 targets in particular in FSHD muscle cells,
- rescues viability and myogenic differentiation in particular of FSHD muscle cells,
- inhibits the expression of DUX4-r and DUX4r targets in particular in ALL cells and
- blocks proliferation in particular of ALL cells;
- the ability to treat, prevent, or ameliorate condition associated with an aberrant expression and/or function of at least one DUX4 protein and/or of at least one rearranged DUX4 protein, such as muscular dystrophy or cancer such as FSHD or ALL.

Although the MATRIN-3 (MATR3) polypeptides or fragments and MATRIN-3 (MATR3) mutant polypeptides or fragments, and the constructs comprising such polypeptides are primarily disclosed in terms of human MATRIN-3 (MATR3), the invention is not so limited and extends to MATRIN-3 (MATR3) polypeptides or fragments and MATRIN-3 (MATR3) mutant polypeptides or fragments and the constructs comprising such polypeptides where the MATRIN-3 (MATR3) polypeptides or fragments and MATRIN-3 (MATR3) mutant polypeptides or fragments are derived from other species (e.g., cynomolgus monkeys, mice and rats). In some instances, a MATRIN-3 (MATR3) polypeptide or fragments or a MATRIN-3 (MATR3) mutant polypeptide can be used to treat or ameliorate a disorder in a subject in a mature form of a MATRIN-3 (MATR3) mutant polypeptide or fragment that is derived from the same species as the subject. A MATRIN-3 (MATR3) mutant polypeptide or peptide or fragment is preferably biologically active. In various respective embodiments, a MATRIN-3 (MATR3) polypeptide or peptide or a MATRIN-3 (MATR3) mutant polypeptide or peptide has a biological activity that is equivalent to, greater to or less than that of the naturally occurring form of the mature MATRIN-3 (MATR3) protein. Examples of biological activities include the ability to bind DUX4 or DUX4-r, prevent the interaction with DNA of DUX4 or DUX4-r, inhibit the activation of specific target genes by DUX4 or DUX4-r, reduce the toxic effects of DUX4, or reduce the cancer activity of DUX4-r. As used herein in the context of the structure of a polypeptide or protein or peptide, the term "N-terminus" (or "amino terminus") and "C-terminus" (or "carboxyl terminus") refer to the extreme amino and carboxyl ends of the polypeptide, respectively.

The term "therapeutic polypeptide" or "therapeutic protein" or "therapeutic peptide" as used herein means a polypeptide or protein or peptide which is being developed for therapeutic use, or which has been developed for therapeutic use.

The terms "comprising", "comprises" and "comprised of" as used herein are synonymous with "including" or "includes"; or "containing" or "contains", and are inclusive or open-ended and do not exclude additional, non-recited members, elements, or steps. The terms "comprising", "comprises" and "comprised of" also include the term "consisting of" and "essentially consisting of'.

The term "essentially consisting of" includes the term "consisting of'.

The term "comprised in" means e.g. that the indicated sequence is a portion or a fragment of the larger indicated sequence. The term "comprised in" may also indicate that the indicated sequence includes the first and/or the last amino acid of the larger indicated sequence.

The present invention will be described by means of non-limiting examples, referring to the following figures:
**Figure 1****. MATR3 2-288 inhibits expression of DUX4 and its targets in primary FSHD muscle cells.**
   Expression of DUX4, DUX4 targets (MBD3L2, TRIM43 and TRIM 48) and Dystrophin (as control) upon lentiviral transduction of primary FSHD muscle cells with control GFP vector (CTRL) or GFP_MATR3 (MATR3 2-288) constructs.
**Figure 2****. MATR3 2-288 significantly impacts the DUX4-dependent gene signature in FSHD.** RNA-sequencing performed on RNA extracted from primary FSHD muscle cells transduced with EV or MATR3 2-288. Gene significantly (adj p-value <0.05) downregulated (Log2FC<1) by MATR3 2-288 significantly overlap with genes significantly upregulated by DUX4 (Jagannathan et al 2006), while genes significantly upregulated by MATR3 2-288 significantly overlap with gene significantly downregulated by DUX4.
**Figure 3****. MATR3 2-288 rescues apoptosis in FSHD and is safe to healthy primary muscle cells.**
   Left. Live, real-time, single cell apoptosis assays in primary FSHD muscle cells transduced with a control BFP lentivirus (CTRL) or a lentivirus expressing BFP/MATR3 2-288 (MATR3 2-288) with cell death monitored over time.
   **Right.** Cell apoptosis monitored in primary muscle cells from healthy donors transduced with CTRL or MATR3 2-288.
   (N=3, mean ±SD, ^{∗}p≤0.05, ****p ≤0.0001).

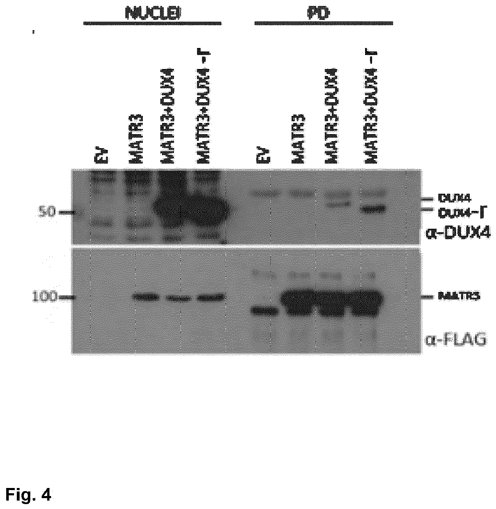

**Figure 4****. MATR3 interacts with DUX4-r.**
   Nuclear extracts of HEK-293T cells transfected with empty vector (EV), Flag-MATR3, Flag-MATR3 plus DUX4 or Flag-MATR3 plus DUX4-r were immunoprecipitated with antibodies against Flag and revealed with anti-DUX4/DUX4-r and anti-Flag antibodies. Left part of the blot input (nuclei). Right side of the blot co-immunoprecipitation.

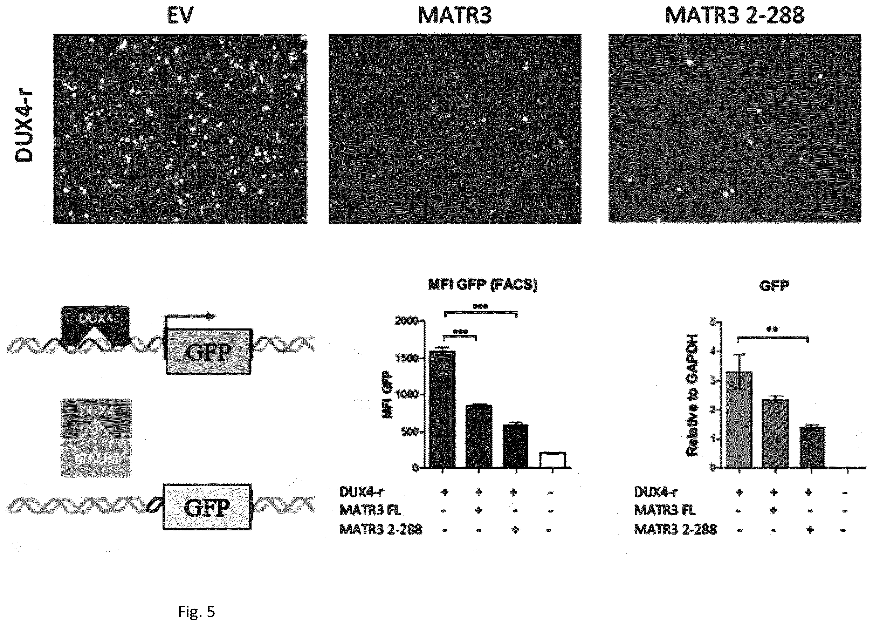

**Figure 5****. MATR3 2-288 inhibits DUX4-r dependent transactivation.**
   HEK-293T cells co-transfected with DUX4-r and a DUX4-r dependent GFP reporter in combination with empty vector (EV), MATR3 or MATR3 2-288.
   Top. Representative fluorescent microscopy images.
   **Bottom left.** Reporter expression quantification by flow cytometry.
   **Bottom right.** Expression of the indicated targets by RT-qPCR.
   (N=3, mean ±SD, ^{∗}p≤0.05, ^{∗∗}p≤0.01; ***p ≤0.001).

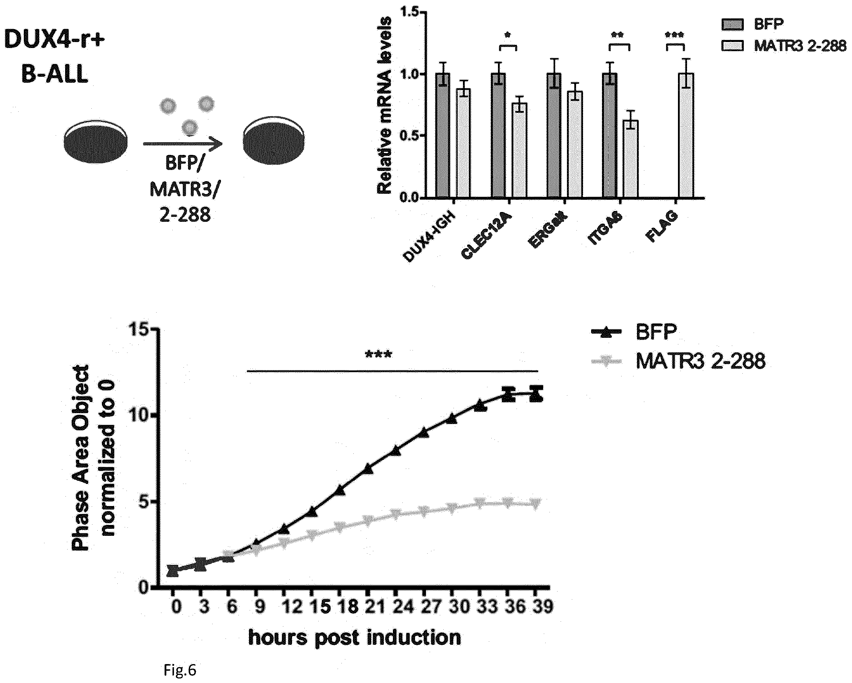

**Figure 6****. MATR3 2-288 inhibits expression** of DUX4-r **targets and DUX4-r dependent tumor spheroid formation.**
   Top. Relative gene expression analyses of the indicated DUX4-r targets in NALM6 (DUX4-r positive) B-ALL cells upon delivery of BFP or BFP-MATR3 2-288 lentiviruses. (N=3, mean ±SD, ^{∗}p≤0.05, ^{∗∗}p≤0.01; ***p ≤0.001).
   **Bottom.** REH (DUX4-r negative) B-ALL cells expressing doxycycline-inducible DUX4-r transduced with BFP or BFP-MATR3 2-288. Tumor spheroid formation quantified by live imaging. Values are normalized to time 0 (doxycycline administration).
   (N=6, mean ±SD, ***p ≤0.001).

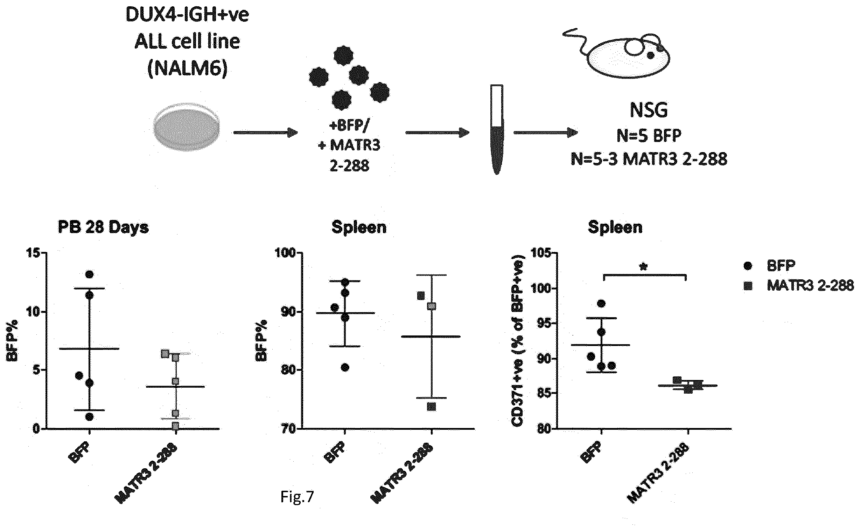

**Figure 7****. MATR3 2-288 inhibits DUX4-r target expression in vivo.**
   Top. Schematic representation of the NALM6 B-ALL xenograft. NALM6 were transduced with BFP or BFP-MATR3 2-288, sorted and injected in immunocompromised recipient NSG mice.
   **Bottom.** Engraftment measured by flow cytometry as % of BFP +ve cells in peripheral blood at 28 days (left) and in the Spleen at sacrifice (center).
   **Bottom right.** CD371 expression measured in engrafted cells (BFP+ve) in the Spleen at sacrifice.
   (Error bars represent SD of independent biological replicates. ^{∗}p≤0.05).

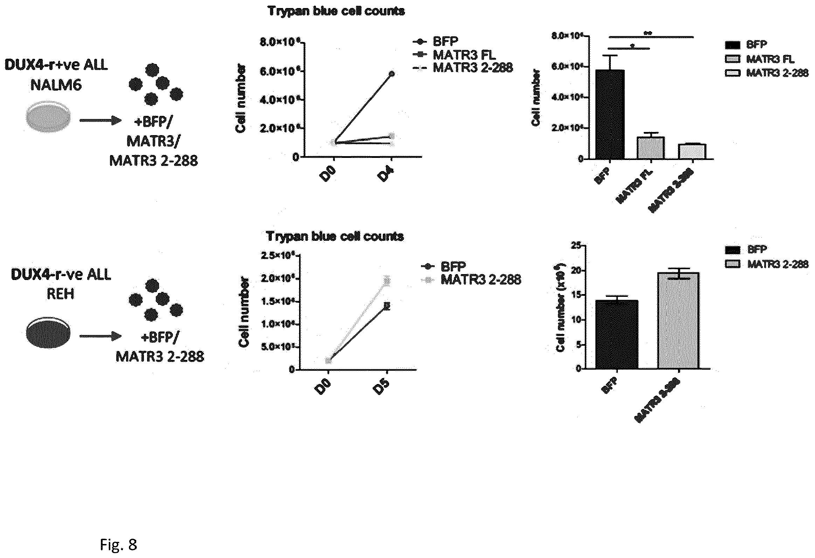

**Figure 8****. MATR3 2-288 inhibits proliferation selectively of DUX4-r positive B-ALL cells.** NALM6 (DUX4-r positive, Top) or REH (DUX4-r negative, Bottom) B-ALL cells were transduced with BFP or BFP-MATR3 2-288 lentiviruses. Proliferation was measured by trypan blue cell count. Center. Cell number at different times. Right. Cell number at the end of experiment.
   (N=3, mean ±SD, ^{∗}p<0.05, ^{∗∗}p<0.01).

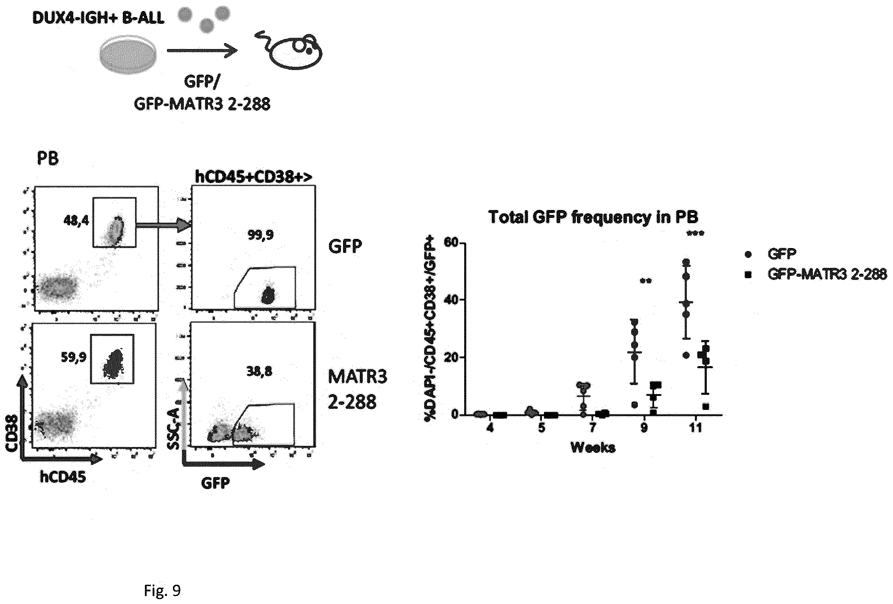

**Figure 9****. MATR3 2-288 inhibits DUX4-r leukemogenic activity in vivo in PDX. Top.** Schematic representation of DUX4-r positive patient derived leukemia (PDX) cells transduced with GFP or GFP-MATR3 2-288 and transplanted in immunodeficient NSG mice.
   **Bottom left.** Representative FACS analysis of peripheral blood (PB) from transplanted mice.
   **Bottom right.** Percentage of GFP+ve cells in PB up to 11 weeks from transplantation. (N=5 GFP, N=4 GFP-MATR3 2-288, mean ±SD, ^{∗∗}p≤0.01; ^{∗∗∗}p ≤0.001).

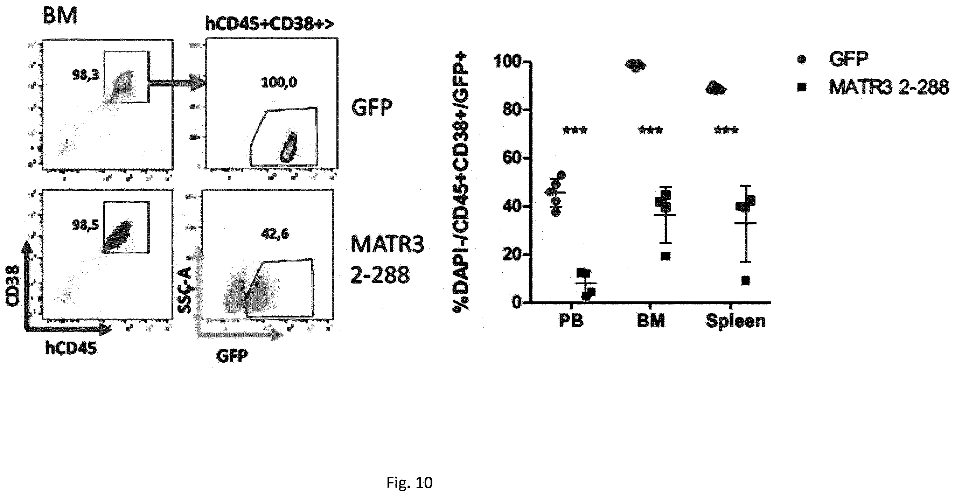

**Figure 10****. MATR3 2-288 impairs DUX4-r leukemia expansion in vivo.**
   **Left.** Total white blood cell counts (WBCC) in the bone marrow (BM) of DUX4-r positive PDX transduced with GFP or GFP-MATR3 2-288 and transplanted in NSG mice.
   **Right.** Percentage of GFP+ve cells in peripheral blood (PB), BM and Spleen at sacrifice.
   (N=5 GFP, N=4 GFP-MATR3 2-288, mean ±SD, ^{∗∗}p≤0.01; ***p ≤0.001).

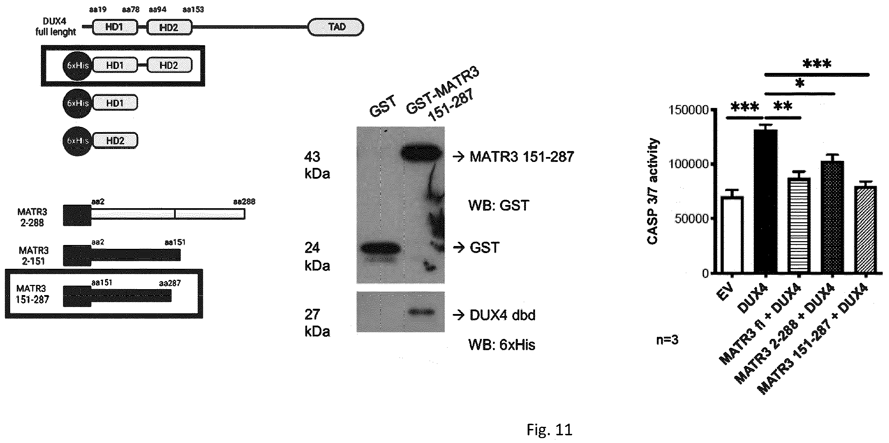

**Figure 11****. MATR3 151-287 is sufficient to bind the DNA-binding domain of DUX4 and inhibit DUX4 toxic activity.**
   **Left.** Schematic representation of the DUX4 regions expressed and purified from E. coli fused to HIS, and the MATR3 regions expressed and purified from E. coli fused to GST.
   **Center.** GST pulldown showing direct interaction between DUX4 dbd (HD1+HD2) and MATR3 151-287. No interaction was detected with DUX4 HD1 or HD2, or with MATR3 2-151 (not shown).
   **Right.** Caspase 3/7 assay in HEK293 transfected with empty vector (EV), DUX4 or DUX4 in combination with the indicated MATR3 regions.
   (N=3, mean ±SD, ^{∗}p<0.05, ^{∗∗}p≤0.01; ***p ≤0.001).
**Figure 12****.**
   Pulldown assay with purified His-DUX4 dbd and biotinylated MATR3 Pep 1, (residues 149-208); Pep 2, (residues 188-248), Pep 3 (residues 228-287) analyzed by immunoblotting with antibodies against His tag (to detect DUX4 dbd) or streptavidin (to detect Pep 1-3). Pep 2 and Pep 3 interact with DUX4 dbd.

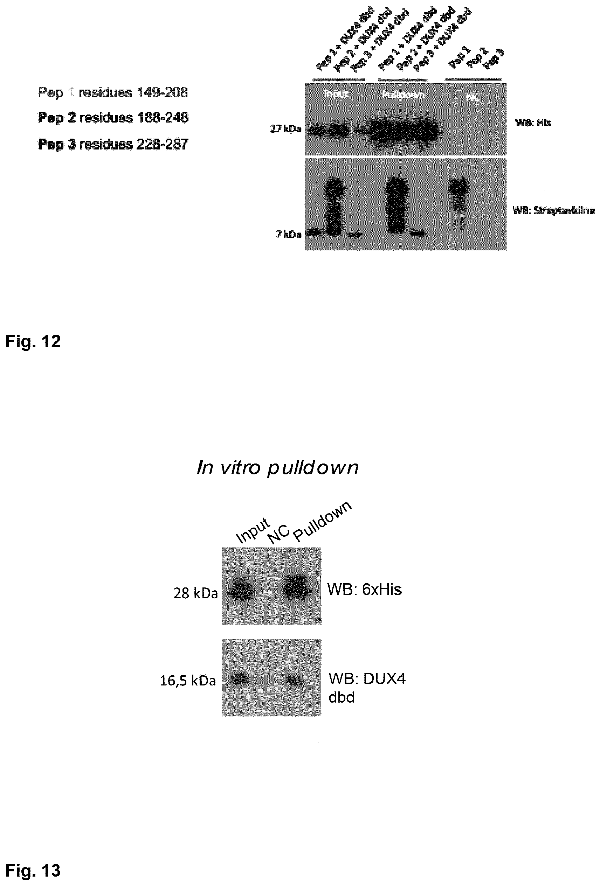

**Figure 13****. MATR3 210-255 directly binds to the DNA-binding domain of DUX4.**
   Pulldown experiment with purified MATR3 210-255 peptide and recombinant DUX4 dbd showing that MATR3 210-255.

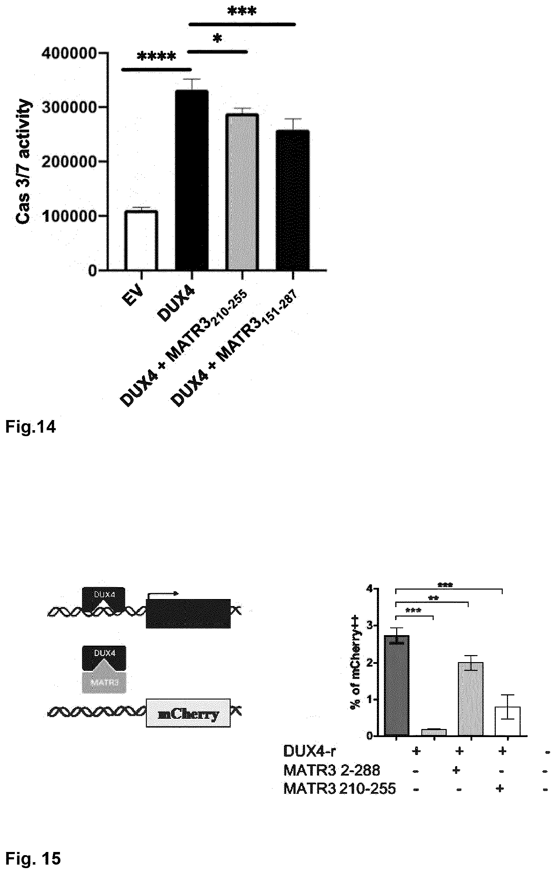

**Figure 14****. MATR3 210-255 inhibits DUX4-dependent toxicity.**
   Caspase 3/7 assay in HEK293 transfected with empty vector (EV), DUX4 or DUX4 in combination with the indicated MATR3 regions. MATR3 210-255 protects from DUX4-induced apoptosis.
   (N=3, mean ±SD, ^{∗}p<0.05, ^{∗∗∗}p ≤0.001; ^{∗∗∗∗}p ≤0.0001).
**Figure 15****. MATR3 210-255 inhibits DUX4-r dependent transactivation.**
   HEK-293T cells co-transfected with DUX4-r and a DUX4-r dependent GFP reporter in combination with empty vector (EV), MATR3 2-288 or MATR3 210-255.
   **Left.** Reporter expression quantification by flow cytometry.
   **Right.** Percentage of GFP positive cells.
   (N=3, mean ±SD, ^{∗}p<0.05, ^{∗∗}p≤0.01; ^{∗∗∗}p ≤0.001).

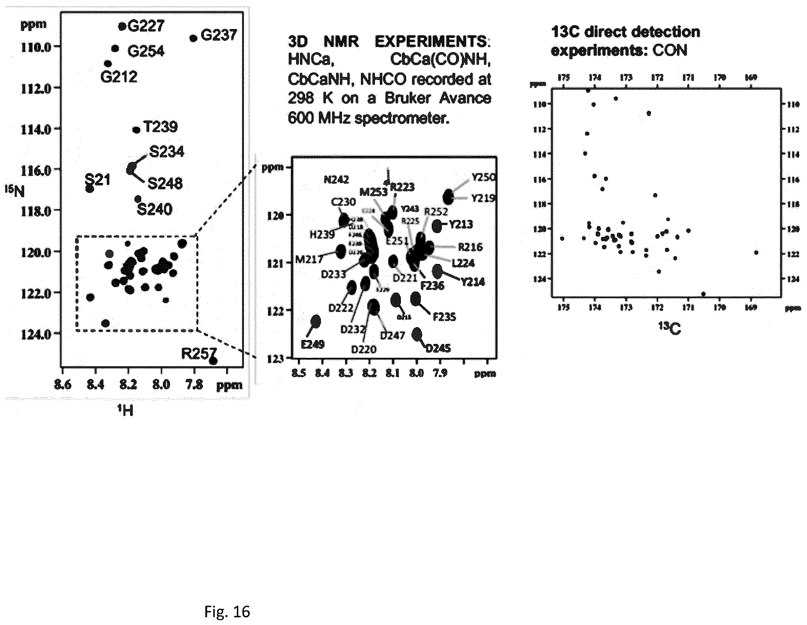

**Figure 16****. Complete MATR3 210-255 resonance backbone assignment.**
   To assign the backbone resonances, a combination of classical 3D heteronuclear experiments and Carbon detected experiments were used.
   **Left.** NMR 1H-15N HSQC spectrum of MATR3₂₁₀₋₂₅₅. The labels indicate the assignment of the resonances to the single amino acids amide (NH) bonds.
   **Center.** The assignment performed acquiring 3D experiments on 13C/15N samples.
   **Right.** 15N, 13C CON spectrum correlating amide 15N atoms of a residue (i) with 13C CO of residues (i) and (i-1).

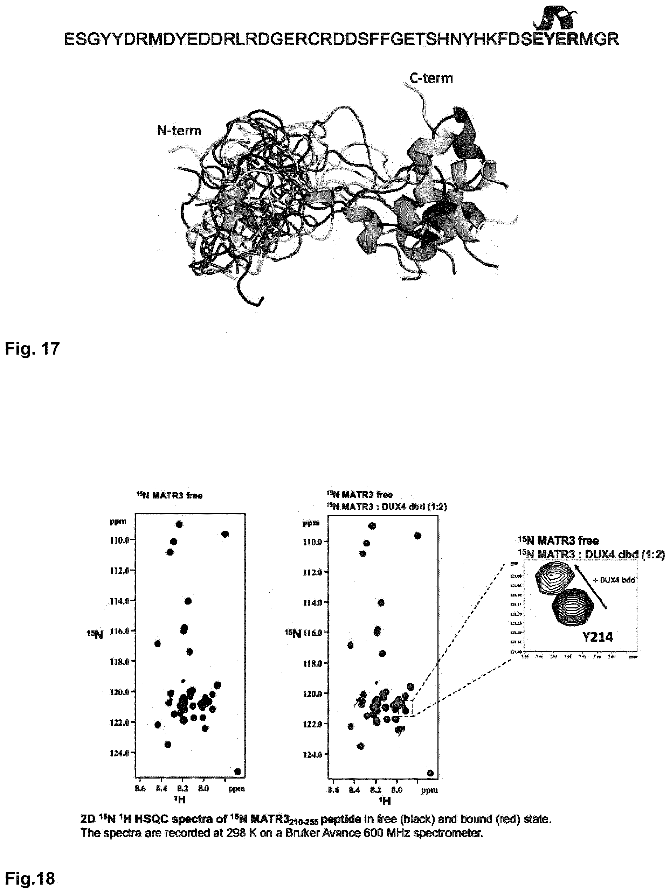

**Figure 17****. MATR3 210-255 putative structure determination.**
   Nuclear Overhauser Enhancement Spectroscopy (NOESY, 1H-1H in water and D20 and 15N) was used to collect distances (distance between hydrogens, d< 5 Ångström). These distances have been used to calculate the peptide structure with the ARIA2.3.2 software. The figure shows the superposition of 10 calculated structures compatible with the experimental restrains. The calculations show that the peptide is predominantly unstructured with a small helix at the C-terminus. The residues showing alpha-helical propensity are highlighted in bold on the lines sequence.
**Figure 18****. Confirmation of MATR3 210-255 binding to DUX4/DUX4-r DNA-binding domain.**
   **Left.** 1H-15N HSQC spectrum of free MATR3 peptide.
   **Right.** Superposition of 1H-15N HSQC spectrum of MATR3 peptide without (Black) or with 2-fold excess of unlabeled DUX4/DUX4-r DNA-binding domain (dbd). In the insert is represented the shift of Y214 upon addition of 2-fold excess of DUX4 dbd.

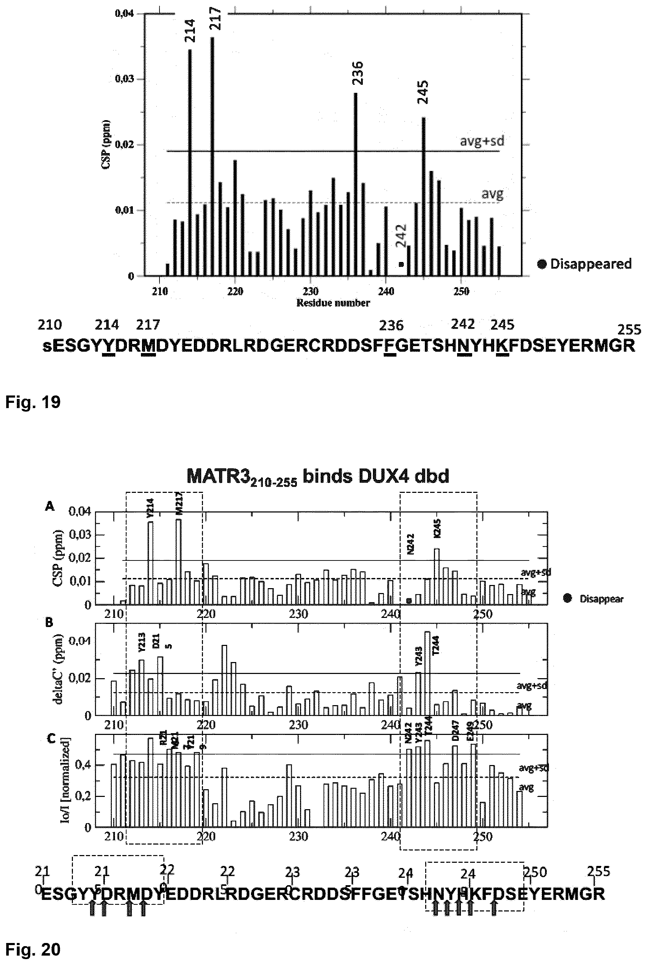

**Figure 19****. Identification of the MATR3 residues more affected by DUX4/DUX4-r dbd.**
   The histogram is a quantitative representation of the peaks displacements observed in the 1H-15N HSQC experiment of MATR3 upon addition of DUX4/DUX4-r dbd. Residues shifting more than the average (avg) + standard deviation (sd) are considered significantly shifting. The peak corresponding to residue 242 disappears during binding (red). The sequence of MATR3 210-255 with highlighted the 5 amino acids most affected by DUX4/DUX4-r binding is at the bottom.
**Figure 20****. Identification of the most relevant MATR3 residues for DUX4/DUX4-r binding.**
   **A.** Histogram showing a quantitative representation of the chemical shift perturbations (CSPs) of the amide group (NH) induced in 15N labelled MATR3 210-255 by DUX4/DUX4-r dbd (ratio 1:2) as a function of peptide sequence.
   **B.** Histogram showing chemical shift perturbations of carbonyl carbon (C') of MATR3 210-255 bound to DUX4/DUX4-r dbd (ratio 1:0.75).
   **C.** Histogram showing the intensity perturbations of peptide bound of MATR3 210-255 bound to DUX4 dbd (ratio 1:0.75).

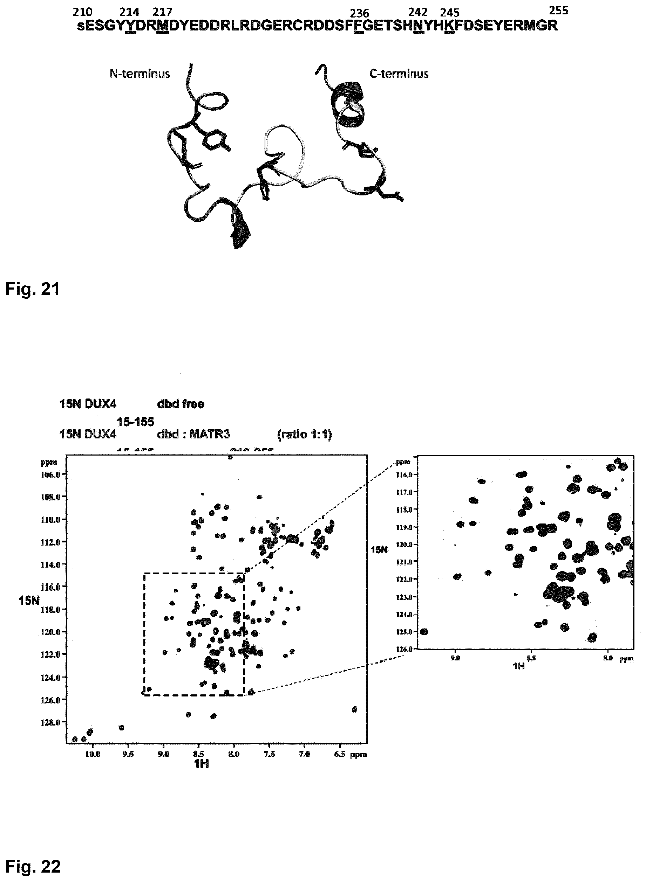

**Figure 21****. MATR3 210-255 structure showing the residues more affected by DUX4/DUX4-r binding.**
   Representative MATR3 210-255 structure extracted from the bundle of NMR structures. The sidechains of the residues mostly perturbed by DUX4 binding are represented in red.
**Figure 22****. NMR spectroscopy with labeled DUX4 and unlabeled MATR3.**
   Superposition of 2D ¹⁵N ¹H HSQC spectrum of free ¹⁵N DUX4₁₅₋₁₅₅ (black) and bound to MATR3₂₁₀₋₂₅₅ (red) state.

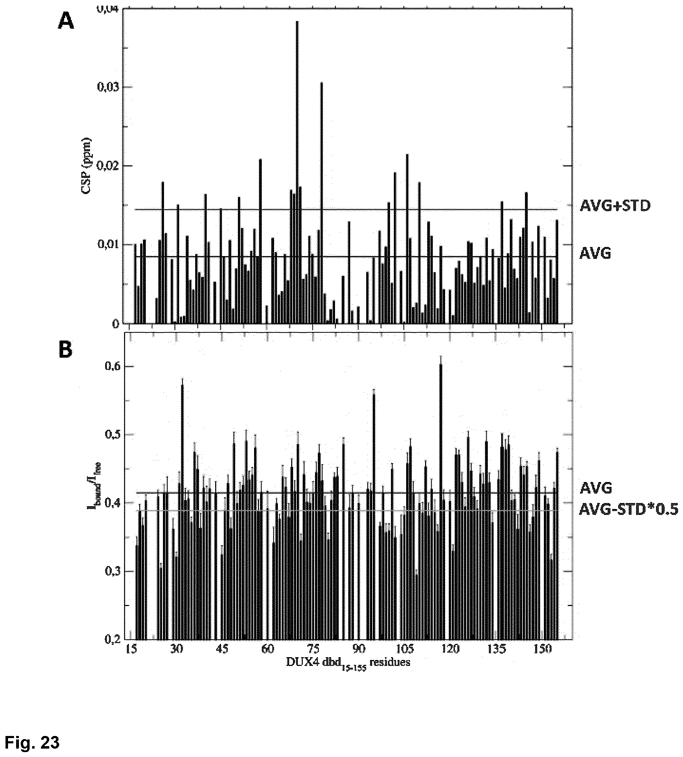

**Figure 23****. Identification of MATR3₂₁₀₋₂₅₅ binding site on DUX4₁₅₋₁₅₅.**
   **A.** Histogram showing chemical shift perturbations (CSPs) of the amide group (NH) induced in 15N labelled DUX4₁₅₋₁₅₅ by MATR3₂₁₀₋₂₅₅ (ratio 1:1) as a function of peptide sequence. **B.** Histogram showing the intensity reductions of the amide group (NH) induced in 15N labelled DUX4₁₅₋₁₅₅ by MATR3₂₁₀₋₂₅₅ (ratio 1:1).

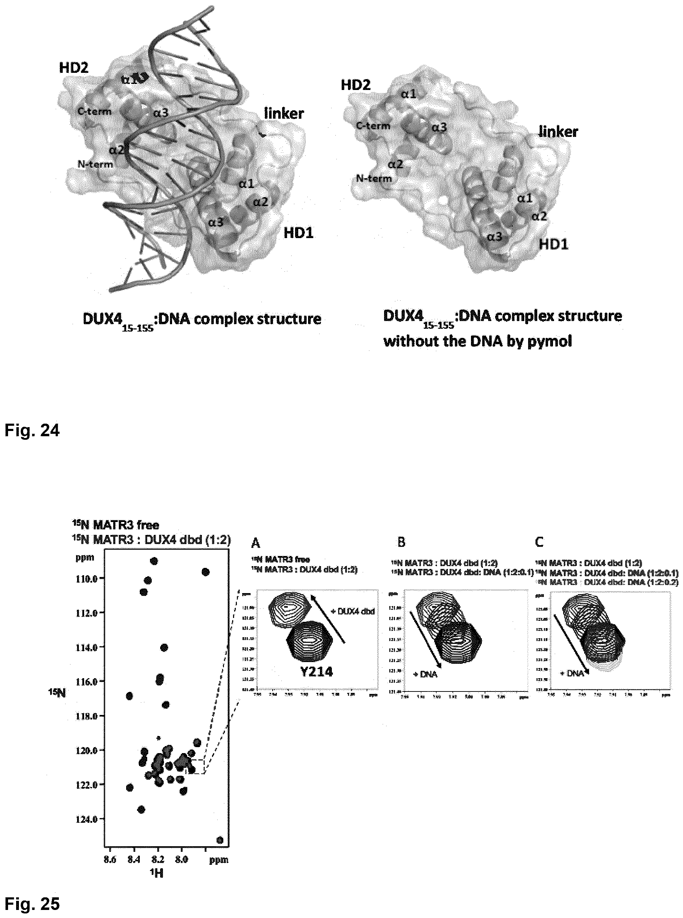

**Figure 24****. Mapping on DUX4₁₅₋₁₅₅:DNA complex crystal structure of the residues mostly affected by MATR₂₁₀₋₂₅₅.**
   **Left.** Crystal structure of DUX4₁₅₋₁₅₅ in complex with DNA. The most affected residues by the binding of MATR3₂₁₀₋₂₅₅ on DUX4₁₅₋₁₅₅ are reporter in cyan on crystal structure. **Right.** Same as in Left, but with the DNA not shown.
**Figure 25****. MATR3 210-255 and DNA compete for binding to DUX4/DUX4-r.**
   **A.** Zoom in the superposition of the HSQC spectrum of free 15N MATR3 210-255 (black) or in the presence of unlabeled DUX4dbd (red). The peak corresponding to the amide resonances of Y214 shifts upon binding. **B.** Zoom in the superposition of HSQC spectrum of free 15N MATR3 210-255 (black), in the presence of unlabeled DUX4 dbd (1:2) (red) or in the presence of an oligonucleotide containing the consensus DUX4/DUX4-r DNA binding site (1:2:0.1) (blue). **C.** The peak corresponding to the amide resonances of Y214 shifts back towards the free position upon addition of increasing amounts (1:2:0.2) of DNA (green), indicating that DNA competes with the interaction with DUX4 dbd.

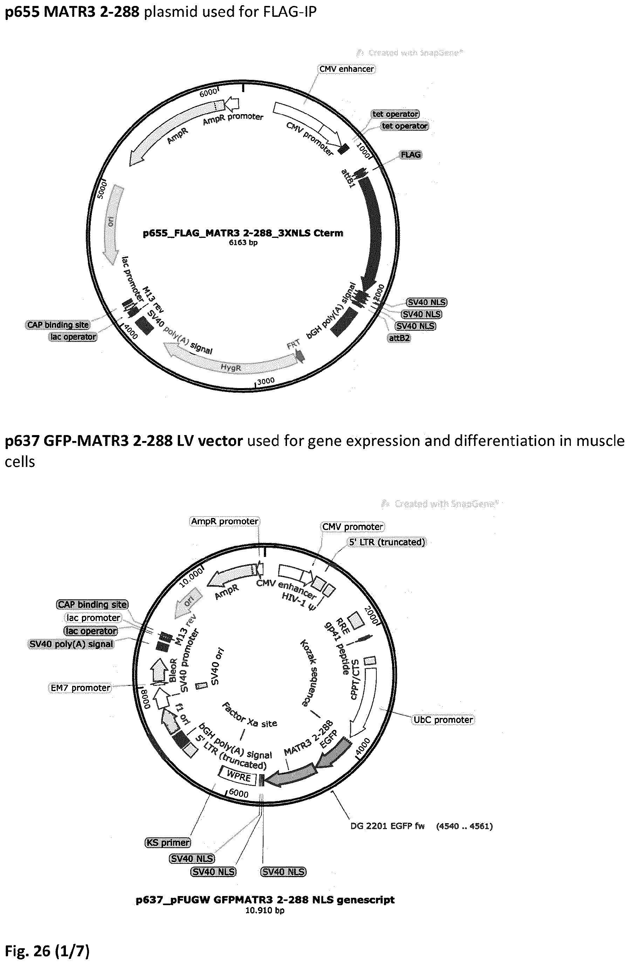

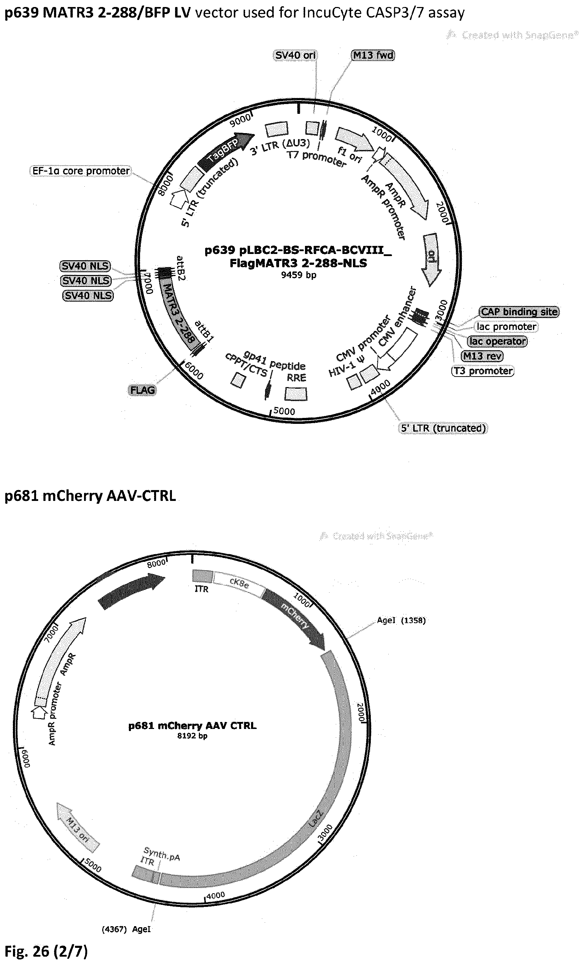

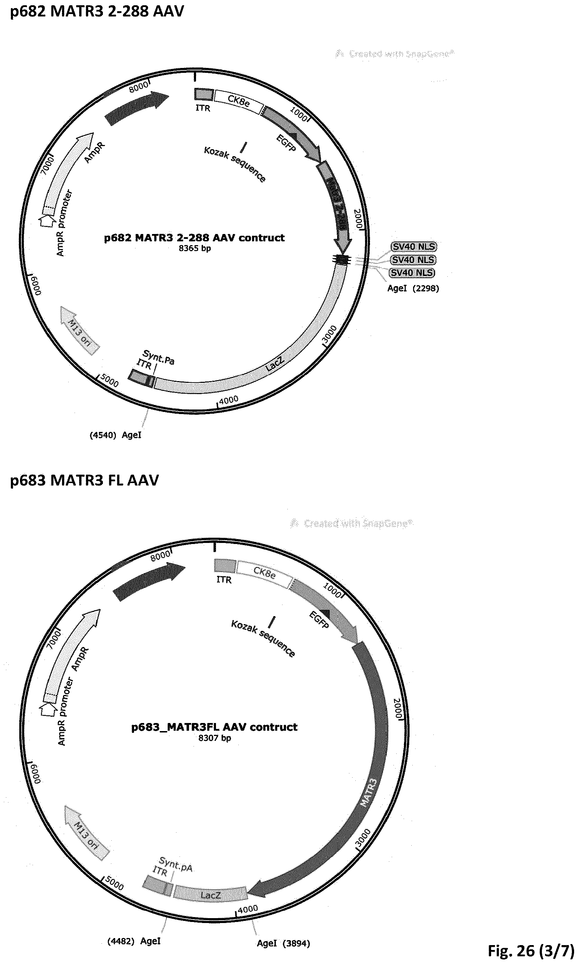

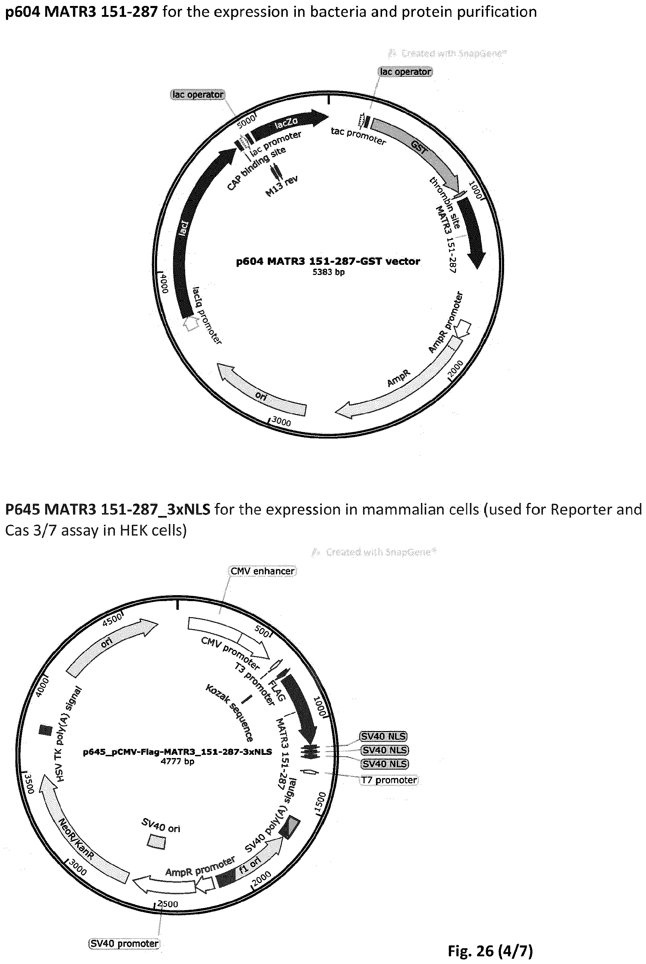

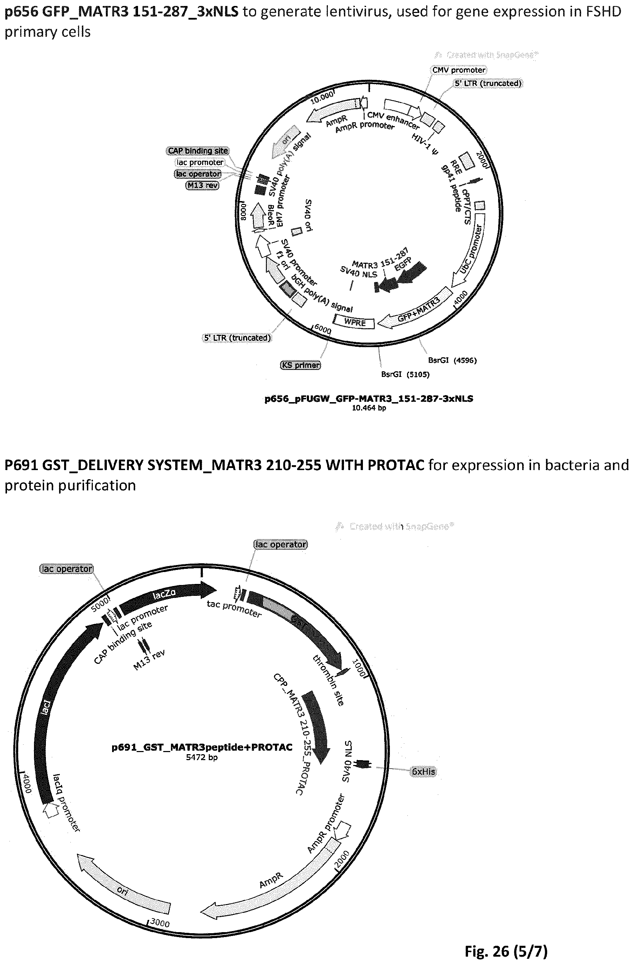

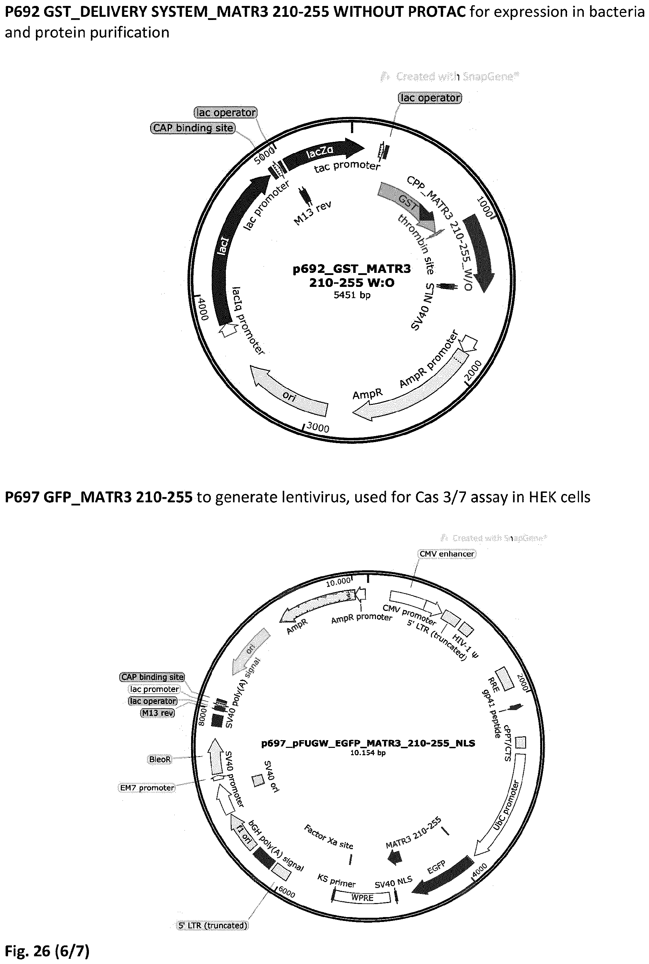

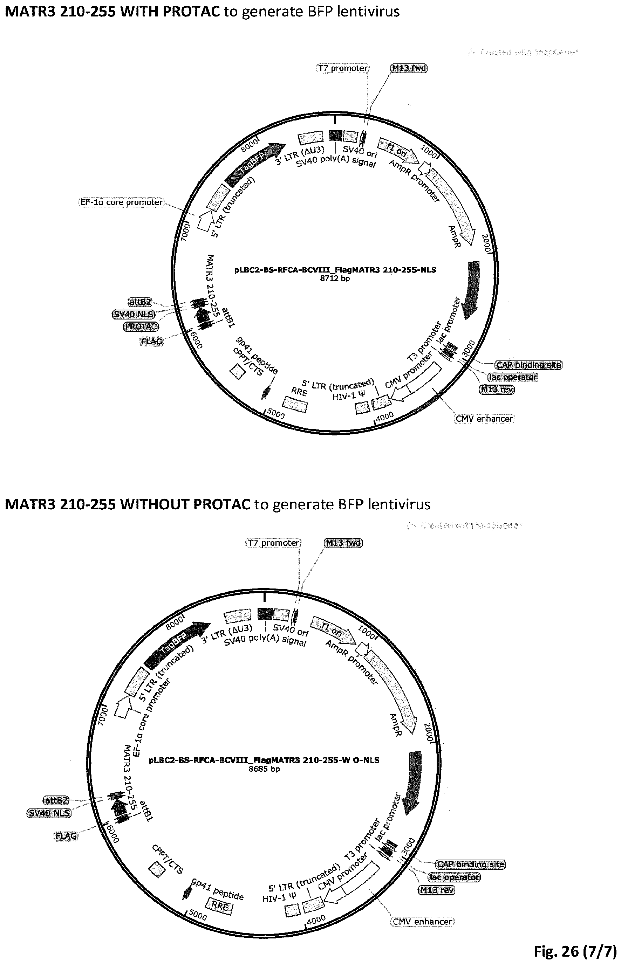

**Figure 26****:** Maps of the plasmids.

### SEQUENCES

**MATR3 1-288 (SEQ ID No. 1)** 
1 msksfqqssl srdsqghgrd lsaagiglla aatqslsmpa slgrmnqgta rlaslmnlgm
61 ssslnqqgah salssastss hnlqsifnig srgplplssq hrgdadqasn ilasfglsar
121 dldelsrype dkitpenlpq illqlkrrrt eegptlsygr dgrsatrepp yrvprddwee
181 krhfrrdsfd drgpslnpvl dydhgsrsqe sgyydrmdye ddrlrdgerc rddsffgets
241 hnyhkfdsey ermgrgpgpl qerslfekkr gappssnied fhgllpkg
 (corresponding to aa. 1-288 of the aa. sequence disclosed with NCBI Accession number: NP_954659.1)
**MATR3 1-287 (SEQ ID No. 17)** 
1 msksfqqssl srdsqghgrd lsaagiglla aatqslsmpa slgrmnqgta rlaslmnlgm
61 ssslnqqgah salssastss hnlqsifnig srgplplssq hrgdadqasn ilasfglsar
121 dldelsrype dkitpenlpq illqlkrrrt eegptlsygr dgrsatrepp yrvprddwee
181 krhfrrdsfd drgpslnpvl dydhgsrsqe sgyydrmdye ddrlrdgerc rddsffgets
241 hnyhkfdsey ermgrgpgpl qerslfekkr gappssnied fhgllpk
 (corresponding to aa. 1-287 of the aa. sequence disclosed with NCBI Accession number: NP_954659.1)
**MATR3 2-288 (SEQ ID No. 18)** 
2   sksfqqssl srdsqghgrd lsaagiglla aatqslsmpa slgrmnqgta rlaslmnlgm
61  ssslnqqgah salssastss hnlqsifnig srgplplssq hrgdadqasn ilasfglsar
121 dldelsrype dkitpenlpq illqlkrrrt eegptlsygr dgrsatrepp yrvprddwee
181 krhfrrdsfd drgpslnpvl dydhgsrsqe sgyydrmdye ddrlrdgerc rddsffgets
241 hnyhkfdsey ermgrgpgpl qerslfekkr gappssnied fhgllpkg
 (corresponding to aa. 2-288 of the aa. sequence disclosed with NCBI Accession number: NP_954659.1)
**MATR3 2-287 (SEQ ID No. 19)** 
2   sksfqqssl srdsqghgrd lsaagiglla aatqslsmpa slgrmnqgta rlaslmnlgm
61  ssslnqqgah salssastss hnlqsifnig srgplplssq hrgdadqasn ilasfglsar
121 dldelsrype dkitpenlpq illqlkrrrt eegptlsygr dgrsatrepp yrvprddwee
181 krhfrrdsfd drgpslnpvl dydhgsrsqe sgyydrmdye ddrlrdgerc rddsffgets
241 hnyhkfdsey ermgrgpgpl qerslfekkr gappssnied fhgllpk
 (corresponding to aa. 2-287 of the aa. sequence disclosed with NCBI Accession number: NP_954659.1)
**MATR3 2-322 (SEQ ID NO: 30)** 
2   sksfqqssl srdsqghgrd lsaagiglla aatqslsmpa slgrmnqgta rlaslmnlgm
 61 ssslnqqgah salssastss hnlqsifnig srgplplssq hrgdadqasn ilasfglsar
121 dldelsrype dkitpenlpq illqlkrrrt eegptlsygr dgrsatrepp yrvprddwee
181 krhfrrdsfd drgpslnpvl dydhgsrsqe sgyydrmdye ddrlrdgerc rddsffgets
241 hnyhkfdsey ermgrgpgpl qerslfekkr gappssnied fhgllpkgyp hlcsicdlpv
301 hsnkewsqhi ngashsrrcq ll
 (corresponding to aa. 2-322 of the aa. sequence disclosed with NCBI Accession number: NP_954659.1)
**MATR3 2-798 (SEQ ID NO:31)** 
2   sksfqqssl srdsqghgrd lsaagiglla aatqslsmpa slgrmnqgta rlaslmnlgm
61  ssslnqqgah salssastss hnlqsifnig srgplplssq hrgdadqasn ilasfglsar
121 dldelsrype dkitpenlpq illqlkrrrt eegptlsygr dgrsatrepp yrvprddwee
181 krhfrrdsfd drgpslnpvl dydhgsrsqe sgyydrmdye ddrlrdgerc rddsffgets
241 hnyhkfdsey ermgrgpgpl qerslfekkr gappssnied fhgllpkgyp hlcsicdlpv
301 hsnkewsqhi ngashsrrcq llleiypewn pdndtghtmg dpfmlqqstn papgilgppp
361 psfhlggpav gprgnlgagn gnlqgprhmq kgrvetsrvv himdfqrgkn lryqllqlve
421 pfgvisnhli lnkineafie mattedaqaa vdyytttpal vfgkpvrvhl sqkykrikkp
481 egkpdqkfdq kqelgrvihl snlphsgysd savlklaepy gkiknyilmr mksqafieme
541 tredamamvd hclkkalwfq grcvkvdlse kykklvlrip nrgidllkkd ksrkrsyspd
601 gkespsdkks ktdgsqktes stegkeqeek sgedgekdtk ddqteqepnm llesedellv
661 deeeaaalle sgssvgdetd lanlgdvasd gkkepsdkav kkdgsasaaa kkklkkvdki
721 eeldqeneaa lengikneen tepgaessen addpnkdtse nadgqsdenk ddytipdeyr
781 igpyqpnvpv gidyvipk
 (corresponding to aa. 2-798 of the aa. sequence disclosed with NCBI Accession number: NP_954659.1)
**MATR3 188-288 (SEQ ID NO:6)** 
sfd drgpslnpvl dydhgsrsqe sgyydrmdye ddrlrdgerc rddsffgets hnyhkfdsey
ermgrgpgpl qerslfekkr gappssnied fhgllpkg
 (corresponding to aa. 188-288 of the aa. sequence disclosed with NCBI Accession number: NP_954659.1)
**MATR3 209-288 of MATR3 (SEQ ID NO:7)** 
qe sgyydrmdye ddrlrdgerc rddsffgets hnyhkfdsey ermgrgpgpl qerslfekkr
gappssnied fhgllpkg
 (corresponding to aa. 209-288 of the aa. sequence disclosed with NCBI Accession number: NP_954659.1)
**MATR3 210-288 of MATR3 (SEQ ID NO:8)** 
e sgyydrmdye ddrlrdgerc rddsffgets hnyhkfdsey ermgrgpgpl qerslfekkr
gappssnied fhgllpkg
 (corresponding to aa. 210-288 of the aa. sequence disclosed with NCBI Accession number: NP_954659.1)
**MATR3 151-287 of MATR3 (SEQ ID NO:11)** 
eegptlsygr dgrsatrepp yrvprddwee krhfrrdsfd drgpslnpvl dydhgsrsqe sgyydrmdye
ddrlrdgerc rddsffgets hnyhkfdsey ermgrgpgpl qerslfekkr gappssnied fhgllpk
 (corresponding to aa. 151-287 of the aa. sequence disclosed with NCBI Accession number: NP_954659.1)
**MATR3 209-287 of MATR3 (SEQ ID NO:12)** 
qe sgyydrmdye ddrlrdgerc rddsffgets hnyhkfdsey ermgrgpgpl qerslfekkr
gappssnied fhgllpk
 (corresponding to aa. 209-287 of the aa. sequence disclosed with NCBI Accession number: NP_954659.1)
**MATR3 210-287 of MATR3 (SEQ ID NO:13)** 
e sgyydrmdye ddrlrdgerc rddsffgets hnyhkfdsey ermgrgpgpl qerslfekkr gappssnied
fhgllpk
 (corresponding to aa. 210-287 of the aa. sequence disclosed with NCBI Accession number: NP_954659.1)
**MATR3 188-287 (SEQ ID NO:14)** 
sfd drgpslnpvl dydhgsrsqe sgyydrmdye ddrlrdgerc rddsffgets hnyhkfdsey
ermgrgpgpl qerslfekkr gappssnied fhgllpk
 (corresponding to aa. 188-287 of the aa. sequence disclosed with NCBI Accession number: NP_954659.1)
**MATR3 149-287 (SEQ ID NO:15)** 
rt eegptlsygr dgrsatrepp yrvprddwee krhfrrdsfd drgpslnpvl dydhgsrsqe
sgyydrmdye ddrlrdgerc rddsffgets hnyhkfdsey ermgrgpgpl qerslfekkr gappssnied
fhgllpk
 (corresponding to aa. 149-287 of the aa. sequence disclosed with NCBI Accession number: NP_954659.1)
**MATR3 151-288 of MATR3 (SEQ ID NO:16)** 
eegptlsygr dgrsatrepp yrvprddwee krhfrrdsfd drgpslnpvl dydhgsrsqe sgyydrmdye
ddrlrdgerc rddsffgets hnyhkfdsey ermgrgpgpl qerslfekkr gappssnied fhgllpkg
 (corresponding to aa. 151-288 of the aa. sequence disclosed with NCBI Accession number: NP_954659.1)

### SYNTHETIC PEPTIDES:

**Peptide 1 (SEQ ID No. 2) aa. 149-208 of MATR3**
   Sequence: (Biotin)-RTEEGPTLSYGRDGRSATREPPYRVPRDDWEEK RHFRRDSFDDRGPSLNPVLDYDHGSRS-NH2
**Peptide 2 (SEQ ID No. 3) aa. 188-248 of MATR3**
   Sequence: (Biotin)- SFDDRGPSLNPVLDYDHGSRSQESGYYDRMD YEDDRLRDGERCRDDSFFGETSHNYHKFDS-NH2
**Peptide 3 (SEQ ID No. 4) aa. 228-287 of MATR3**
   Sequence: (Biotin)- ERCRDDSFFGETSHNYHKFDSEYERMGRGPG PLQERSLFEKKRGAPPSSNIEDFHGLLPK -NH2
**MATR3 210-255 - REGION CONSIDERED FOR NMR (46 AA) (SEQ ID No. 5)**
   210-ESGYYDRMDYEDDRLRDGERCRDD SFFGETSHNYHKFDSEYERMGR-25 5
**p655 MATR3 2-288** plasmid used for FLAG-IP (see figure 26 which refers to the herein mentioned plasmids)

### CMV Enhancer promoter

### FLAG

### MATR3 2-288

### 3XNLS

### bG_Hpol_yA_signal

### Nucleotide sequence:

gacggatcgggagatctcccgatcccctatggtgcactctcagtacaatctgctctgatgccgcatagttaagccagtatctgctccctgct
tgtgtgttggaggtcgctgagtagtgcgcgagcaaaatttaagctacaacaaggcaaggcttgaccgacaattgcatgaagaatctgctt
agggttaggcgttttgcgctgcttcgcgatgtacgggccagatatacgcgttgacattgattattgactagttattaatagtaatcaatta
cggggtcattagttcatagcccatatatggagttccgcgttacataacttacggtaaatggcccgcctggctgaccgcccaacgacc

cccgcccattgacgtcaataatgacgtatgttcccatagtaacgccaatagggactttccattgacgtcaatgggtggagtatttacg
gtaaactgcccacttggcagtacatcaagtgtatcatatgccaagtacgccccctattgacgtcaatgacggtaaatggcccgcctg
gcattatgcccagtacatgaccttatgggactttcctacttggcagtacatctacgtattagtcatcgctattaccatggtgatgcggtt
ttggcagtacatcaatgggcgtggatagcggtttgactcacggggatttccaagtctccaccccattgacgtcaatgggagtttgttt
tggcaccaaaatcaacgggactttccaaaatgtcgtaacaactccgccccattgacgcaaatgggcggtaggcgtgtacggtggg
aggtctatataagcagagctctccctatcagtgatagagatctccctatcagtgatagagatcgtcgacgagctcgtttagtgaaccgtc
agatcgcctggagacgccatccacgctgttttgacctccatagaagacaccgggaccgatccagcctccggactctagcgtttaaactta
agcttggtaccgagctcggatccactagtccagtgtggtggaattctgcagatatcacaagtttGTACAAAAAAGCAGGCTCCA
TGGATTACAAGGATGACGACGATAAGAGCCCGGGCGGATCcTCCAAGTCATTCCAGCAGTCATCTCTCA
GTAGGGACTCACAGGGTCATGGGCGTGACCTGTCTGCGGCAGGAATAGGCCTTCTTGCTGCTGCTACC
CAGTCTTTAAGTATGCCAGCATCTCTTGGAAGGATGAACCAGGGTACTGCACGCCTTGCTAGTTTAAT
GAATCTTGGAATGAGTTCTTCATTGAATCAACAAGGAGCTCATAGTGCACTGTCTTCTGCTAGTACTTC
TTCCCATAATTTGCAGTCTATATTTAACATTGGAAGTAGAGGTCCACTCCCTTTATCTTCTCAACACCGT
GGAGATGCAGACCAGGCCAGTAACATTTTGGCCAGCTTTGGTCTGTCTGCTAGAGACTTAGATGAACT
GAGTCGTTATCCAGAGGACAAGATTACTCCTGAGAATTTGCCCCAAATCCTTCTACAGCTTAAAAGGA
GGAGAACTGAAGAAGGCCCTACCTTGAGTTATGGTAGAGATGGCAGATCTGCTACACGGGAGCCACC
ATACAGAGTACCTAGGGATGATTGGGAAGAAAAAAGGCACTTTAGAAGAGATAGTTTTGATGATCGT
GGTCCTAGTCTCAACCCAGTGCTTGATTATGACCATGGAAGTCGTTCTCAAGAATCTGGTTATTATGAC
AGAATGGATTATGAAGATGACAGATTAAGAGATGGAGAAAGGTGTAGGGATGATTCTTTTTTTGGTG
AGACCTCGCATAACTATCATAAATTTGACAGTGAGTATGAGAGAATGGGACGTGGTCCTGGCCCCTTA
CAAGAGAGATCTCTCTTTGAGAAAAAGAGAGGCGCTCCTCCAAGTAGCAATATTGAAGACTTCCATGG
ACTCTTACCGAAGGGTAGGGATCCCAAGAAAAAACGCAAGGTGGAAGATCCTAAGAAAAAGCGGAA
AGTGGAAGACCCAAAAAAGAAGAGAAAGGTAGACTGAGACCCAGCTTTcttgtacaaagtggtgacgtaagct
aggggcccgtttaaacccgctgatcagcctcgactgtgccttctagttgccagccatctgttgtttgcccctcccccgtgccttccttgaccct
ggaaggtgccactcccactgtcctttcctaataaaatgaggaaattgcatcgcattgtctgagtaggtgtcattctattctggggggtgggg
tggggcaggacagcaagggggaggattgggaagacaatagcaggcatgctggggatgcggtgggctctatggcttctgaggcggaaa
gaaccagctggggctctagggggtatccccacgcgccctgtagcggcgcattaagcgcggcgggtgtggtggttacgcgcagcgtgacc
gctacacttgccagcgccctagcgcccgctcctttcgctttcttcccttcctttctcgccacgttcgccggctttccccgtcaagctctaaatc
gggggctccctttagggttccgatttagtgctttacggcacctcgaccccaaaaaacttgattagggtgatggttcacgtacctagaagttc
ctattccgaagttcctattctctagaaagtataggaacttccttggccaaaaagcctgaactcaccgcgacgtctgtcgagaagtttctgat
cgaaaagttcgacagcgtctccgacctgatgcagctctcggagggcgaagaatctcgtgctttcagcttcgatgtaggagggcgtggata
tgtcctgcgggtaaatagctgcgccgatggtttctacaaagatcgttatgtttatcggcactttgcatcggccgcgctcccgattccggaag
tgcttgacattggggaattcagcgagagcctgacctattgcatctcccgccgtgcacagggtgtcacgttgcaagacctgcctgaaaccg
aactgcccgctgttctgcagccggtcgcggaggccatggatgcgatcgctgcggccgatcttagccagacgagcgggttcggcccattcg
gaccgcaaggaatcggtcaatacactacatggcgtgatttcatatgcgcgattgctgatccccatgtgtatcactggcaaactgtgatgga
cgacaccgtcagtgcgtccgtcgcgcaggctctcgatgagctgatgctttgggccgaggactgccccgaagtccggcacctcgtgcacgc
ggatttcggctccaacaatgtcctgacggacaatggccgcataacagcggtcattgactggagcgaggcgatgttcggggattcccaata
cgaggtcgccaacatcttcttctggaggccgtggttggcttgtatggagcagcagacgcgctacttcgagcggaggcatccggagcttgc
aggatcgccgcggctccgggcgtatatgctccgcattggtcttgaccaactctatcagagcttggttgacggcaatttcgatgatgcagctt
gggcgcagggtcgatgcgacgcaatcgtccgatccggagccgggactgtcgggcgtacacaaatcgcccgcagaagcgcggccgtctg
gaccgatggctgtgtagaagtactcgccgatagtggaaaccgacgccccagcactcgtccgagggcaaaggaatagcacgtactacga
gatttcgattccaccgccgccttctatgaaaggttgggcttcggaatcgttttccgggacgccggctggatgatcctccagcgcggggatct
catgctggagttcttcgcccaccccaacttgtttattgcagcttataatggttacaaataaagcaatagcatcacaaatttcacaaataaa
gcatttttttcactgcattctagttgtggtttgtccaaactcatcaatgtatcttatcatgtctgtataccgtcgacctctagctagagcttggc
gtaatcatggtcatagctgtttcctgtgtgaaattgttatccgctcacaattccacacaacatacgagccggaagcataaagtgtaaagcc
tggggtgcctaatgagtgagctaactcacattaattgcgttgcgctcactgcccgctttccagtcgggaaacctgtcgtgccagctgcatta
atgaatcggccaacgcgcggggagaggcggtttgcgtattgggcgctcttccgcttcctcgctcactgactcgctgcgctcggtcgttcgg

ctgcggcgagcggtatcagctcactcaaaggcggtaatacggttatccacagaatcaggggataacgcaggaaagaacatgtgagcaa
aaggccagcaaaaggccaggaaccgtaaaaaggccgcgttgctggcgtttttccataggctccgcccccctgacgagcatcacaaaaat
cgacgctcaagtcagaggtggcgaaacccgacaggactataaagataccaggcgtttccccctggaagctccctcgtgcgctctcctgtt
ccgaccctgccgcttaccggatacctgtccgcctttctcccttcgggaagcgtggcgctttctcatagctcacgctgtaggtatctcagttcg
gtgtaggtcgttcgctccaagctgggctgtgtgcacgaaccccccgttcagcccgaccgctgcgccttatccggtaactatcgtcttgagtc
caacccggtaagacacgacttatcgccactggcagcagccactggtaacaggattagcagagcgaggtatgtaggcggtgctacagag
ttcttgaagtggtggcctaactacggctacactagaagaacagtatttggtatctgcgctctgctgaagccagttaccttcggaaaaagag
ttggtagctcttgatccggcaaacaaaccaccgctggtagcggtggtttttttgtttgcaagcagcagattacgcgcagaaaaaaaggat
ctcaagaagatcctttgatcttttctacggggtctgacgctcagtggaacgaaaactcacgttaagggattttggtcatgagattatcaaa
aaggatcttcacctagatccttttaaattaaaaatgaagttttaaatcaatctaaagtatatatgagtaaacttggtctgacagttaccaat
gcttaatcagtgaggcacctatctcagcgatctgtctatttcgttcatccatagttgcctgactccccgtcgtgtagataactacgatacggg
agggcttaccatctggccccagtgctgcaatgataccgcgagacccacgctcaccggctccagatttatcagcaataaaccagccagcc
ggaagggccgagcgcagaagtggtcctgcaactttatccgcctccatccagtctattaattgttgccgggaagctagagtaagtagttcgc
cagttaatagtttgcgcaacgttgttgccattgctacaggcatcgtggtgtcacgctcgtcgtttggtatggcttcattcagctccggttccc
aacgatcaaggcgagttacatgatcccccatgttgtgcaaaaaagcggttagctccttcggtcctccgatcgttgtcagaagtaagttggc
cgcagtgttatcactcatggttatggcagcactgcataattctcttactgtcatgccatccgtaagatgcttttctgtgactggtgagtactc
aaccaagtcattctgagaatagtgtatgcggcgaccgagttgctcttgcccggcgtcaatacgggataataccgcgccacatagcagaa
ctttaaaagtgctcatcattggaaaacgttcttcggggcgaaaactctcaaggatcttaccgctgttgagatccagttcgatgtaacccact
cgtgcacccaactgatcttcagcatcttttactttcaccagcgtttctgggtgagcaaaaacaggaaggcaaaatgccgcaaaaaaggga
ataagggcgacacggaaatgttgaatactcatactcttcctttttcaatattattgaagcatttatcagggttattgtctcatgagcggatac
atatttgaatgtatttagaaaaataaacaaataggggttccgcgcacatttccccgaaaagtgccacctgacgtc (SEQ ID NO:33)

### Aminoacid sequence:

MDYKDDDDKSPGGSSKSFQQSSLSRDSQGHGRDLSAAGIGLLAAATQSLSMPASLGRMNQGTARLASL
MNLGMSSSLNQQGAHSALSSASTSSHNLQSIFNIGSRGPLPLSSQHRGDADQASNILASFGLSARDLDELS
RYPEDKITPENLPQILLQLKRRRTEEGPTLSYGRDGRSATREPPYRVPRDDWEEKRHFRRDSFDDRGPSLNP
VLDYDHGSRSQESGYYDRMDYEDDRLRDGERCRDDSFFGETSHNYHKFDSEYERMGRGPGPLQERSLFE
KKRGAPPSSNIEDFHGLLPKGRDPKKKRKVEDPKKKRKVEDPKKKRKVD* (SEQ ID NO:34)

**p637 GFP-MATR3 2-288 LV vector** used for gene expression and differentiation in muscle cells

### PROMOTER

### KOZAK

### EGFP

### MATR3 2-288

### 3XNLS

### Nucleotide sequence:

gtcgacggatcgggagatctcccgatcccctatggtgcactctcagtacaatctgctctgatgccgcatagttaagccagtatctgctccct
gcttgtgtgttggaggtcgctgagtagtgcgcgagcaaaatttaagctacaacaaggcaaggcttgaccgacaattgcatgaagaatctg
cttagggttaggcgttttgcgctgcttcgcgatgtacgggccagatatacgcgttgacattgattattgactagttattaatagtaatcaatt
acggggtcattagttcatagcccatatatggagttccgcgttacataacttacggtaaatggcccgcctggctgaccgcccaacgaccccc
gcccattgacgtcaataatgacgtatgttcccatagtaacgccaatagggactttccattgacgtcaatgggtggagtatttacggtaaac
tgcccacttggcagtacatcaagtgtatcatatgccaagtacgccccctattgacgtcaatgacggtaaatggcccgcctggcattatgcc
cagtacatgaccttatgggactttcctacttggcagtacatctacgtattagtcatcgctattaccatggtgatgcggttttggcagtacatc
aatgggcgtggatagcggtttgactcacggggatttccaagtctccaccccattgacgtcaatgggagtttgttttggcaccaaaatcaac

gggactttccaaaatgtcgtaacaactccgccccattgacgcaaatgggcggtaggcgtgtacggtgggaggtctatataagcagcgcgt
tttgcctgtactgggtctctctggttagaccagatctgagcctgggagctctctggctaactagggaacccactgcttaagcctcaataaag
cttgccttgagtgcttcaagtagtgtgtgcccgtctgttgtgtgactctggtaactagagatccctcagacccttttagtcagtgtggaaaat
ctctagcagtggcgcccgaacagggacttgaaagcgaaagggaaaccagaggagctctctcgacgcaggactcggcttgctgaagcgc
gcacggcaagaggcgaggggcggcgactggtgagtacgccaaaaattttgactagcggaggctagaaggagagagatgggtgcgag
agcgtcagtattaagcgggggagaattagatcgcgatgggaaaaaattcggttaaggccagggggaaagaaaaaatataaattaaaa
catatagtatgggcaagcagggagctagaacgattcgcagttaatcctggcctgttagaaacatcagaaggctgtagacaaatactggg
acagctacaaccatcccttcagacaggatcagaagaacttagatcattatataatacagtagcaaccctctattgtgtgcatcaaaggat
agagataaaagacaccaaggaagctttagacaagatagaggaagagcaaaacaaaagtaagaccaccgcacagcaagcggccgct
gatcttcagacctggaggaggagatatgagggacaattggagaagtgaattatataaatataaagtagtaaaaattgaaccattagga
gtagcacccaccaaggcaaagagaagagtggtgcagagagaaaaaagagcagtgggaataggagctttgttccttgggttcttgggag
cagcaggaagcactatgggcgcagcgtcaatgacgctgacggtacaggccagacaattattgtctggtatagtgcagcagcagaacaa
tttgctgagggctattgaggcgcaacagcatctgttgcaactcacagtctggggcatcaagcagctccaggcaagaatcctggctgtgga
aagatacctaaaggatcaacagctcctggggatttggggttgctctggaaaactcatttgcaccactgctgtgccttggaatgctagttgg
agtaataaatctctggaacagatttggaatcacacgacctggatggagtgggacagagaaattaacaattacacaagcttaatacactc
cttaattgaagaatcgcaaaaccagcaagaaaagaatgaacaagaattattggaattagataaatgggcaagtttgtggaattggttta
acataacaaattggctgtggtatataaaattattcataatgatagtaggaggcttggtaggtttaagaatagtttttgctgtactttctatag
tgaatagagttaggcagggatattcaccattatcgtttcagacccacctcccaaccccgaggggacccgacaggcccgaaggaatagaa
gaagaaggtggagagagagacagagacagatccattcgattagtgaacggatcggcactgcgtgcgccaattctgcagacaaatggca
gtattcatccacaattttaaaagaaaaggggggattggggggtacagtgcaggggaaagaatagtagacataatagcaacagacatac
aaactaaagaattacaaaaacaaattacaaaaattcaaaattttcgggtttattacagggacagcagagatccagtttggttaattaacc
cgtgtcggctccagatctggcctccgcgccgggttttggcgcctcccgcgggcgcccccctcctcacggcgagcgctgccacgtcaga
cgaagggcgcagcgagcgtcctgatccttccgcccggacgctcaggacagcggcccgctgctcataagactcggccttagaacccc
agtatcagcagaaggacattttaggacgggacttgggtgactctagggcactggttttctttccagagagcggaacaggcgagga
aaagtagtcccttctcggcgattctgcggagggatctccgtggggcggtgaacgccgatgattatataaggacgcgccgggtgtgg
cacagctagttccgtcgcagccgggatttgggtcgcggttcttgtttgtggatcgctgtgatcgtcacttggtgagtagcgggctgctg
ggctggccggggctttcgtggccgccgggccgctcggtgggacggaagcgtgtggagagaccgccaagggctgtagtctgggtcc
gcgagcaaggttgccctgaactgggggttggggggagcgcagcaaaatggcggctgttcccgagtcttgaatggaagacgcttg
tgaggcgggctgtgaggtcgttgaaacaaggtggggggcatggtgggcggcaagaacccaaggtcttgaggccttcgctaatgc
gggaaagctcttattcgggtgagatgggctggggcaccatctggggaccctgacgtgaagtttgtcactgactggagaactcggtt
tgtcgtctgttgcgggggcggcagttatggcggtgccgttgggcagtgcacccgtacctttgggagcgcgcgccctcgtcgtgtcgtg
acgtcacccgttctgttggcttataatgcagggtggggccacctgccggtaggtgtgcggtaggcttttctccgtcgcaggacgcag
ggttcgggcctagggtaggctctcctgaatcgacaggcgccggacctctggtgaggggagggataagtgaggcgtcagtttctttg
gtcggttttatgtacctatcttcttaagtagctgaagctccggttttgaactatgcgctcggggttggcgagtgtgttttgtgaagttttt
taggcaccttttgaaatgtaatcatttgggtcaatatgtaattttcagtgttagactagtaaattgtccgctaaattctggccgtttttg
gcttttttgttagacgaagcttgggctgcaggtcgactctagaggatccccgggtaccggtcgccaccatggtgagcaagggcgaggag
ctgttcaccggggtggtgcccatcctggtcgagctggacggcgacgtaaacggccacaagttcagcgtgtccggcgagggcgagggcga
tgccacctacggcaagctgaccctgaagttcatctgcaccaccggcaagctgcccgtgccctggcccaccctcgtgaccaccctgaccta
cggcgtgcagtgcttcagccgctaccccgaccacatgaagcagcacgacttcttcaagtccgccatgcccgaaggctacgtccaggagc
gcaccatcttcttcaaggacgacggcaactacaagacccgcgccgaggtgaagttcgagggcgacaccctggtgaaccgcatcgagctg
aagggcatcgacttcaaggaggacggcaacatcctggggcacaagctggagtacaactacaacagccacaacgtctatatcatggccg
acaagcagaagaacggcatcaaggtgaacttcaagatccgccacaacatcgaggacggcagcgtgcagctcgccgaccactaccagc
agaacacccccatcggcgacggccccgtgctgctgcccgacaaccactacctgagcacccagtccgccctgagcaaagaccccaacga
gaagcgcgatcacatggtcctgctggagttcgtgaccgccgccgggatcactctcggcatggacgagctgtacTCCAAGTCATTCC
AGCAGTCATCTCTCAGTAGGGACTCACAGGGTCATGGGCGTGACCTGTCTGCGGCAGGAATAGGCCTT
CTTGCTGCTGCTACCCAGTCTTTAAGTATGCCAGCATCTCTTGGAAGGATGAACCAGGGTACTGCACGC
CTTGCTAGTTTAATGAATCTTGGAATGAGTTCTTCATTGAATCAACAAGGAGCTCATAGTGCACTGTCT

TCTGCTAGTACTTCTTCCCATAATTTGCAGTCTATATTTAACATTGGAAGTAGAGGTCCACTCCCTTTAT
CTTCTCAACACCGTGGAGATGCAGACCAGGCCAGTAACATTTTGGCCAGCTTTGGTCTGTCTGCTAGA
GACTTAGATGAACTGAGTCGTTATCCAGAGGACAAGATTACTCCTGAGAATTTGCCCCAAATCCTTCTA
CAGCTTAAAAGGAGGAGAACTGAAGAAGGCCCTACCTTGAGTTATGGTAGAGATGGCAGATCTGCTA
CACGGGAGCCACCATACAGAGTACCTAGGGATGATTGGGAAGAAAAAAGGCACTTTAGAAGAGATA
GTTTTGATGATCGTGGTCCTAGTCTCAACCCAGTGCTTGATTATGACCATGGAAGTCGTTCTCAAGAAT
CTGGTTATTATGACAGAATGGATTATGAAGATGACAGATTAAGAGATGGAGAAAGGTGTAGGGATG
ATTCTTTTTTTGGTGAGACCTCGCATAACTATCATAAATTTGACAGTGAGTATGAGAGAATGGGACGT
GGTCCTGGCCCCTTACAAGAGAGATCTCTCTTTGAGAAAAAGAGAGGCGCTCCTCCAAGTAGCAATAT
TGAAGACTTCCATGGACTCTTACCGAAGGGTagggatcccaagaaaaaacgcaaggtggaagatcctaagaaaaa
gcggaaagtggaagacccaaaaaagaagagaaaggtagacTAATGTACAAGTAAGCTAGCTCGAGCTAGAATTa
attcgatatcaagcttatcgataatcaacctctggattacaaaatttgtgaaagattgactggtattcttaactatgttgctccttttacgcta
tgtggatacgctgctttaatgcctttgtatcatgctattgcttcccgtatggctttcattttctcctccttgtataaatcctggttgctgtctcttt
atgaggagttgtggcccgttgtcaggcaacgtggcgtggtgtgcactgtgtttgctgacgcaacccccactggttggggcattgccaccac
ctgtcagctcctttccgggactttcgctttccccctccctattgccacggcggaactcatcgccgcctgccttgcccgctgctggacaggggc
tcggctgttgggcactgacaattccgtggtgttgtcggggaaatcatcgtcctttccttggctgctcgcctgtgttgccacctggattctgcg
cgggacgtccttctgctacgtcccttcggccctcaatccagcggaccttccttcccgcggcctgctgccggctctgcggcctcttccgcgtct
tcgccttcgccctcagacgagtcggatctccctttgggccgcctccccgcatcgataccgtcgacctcgagacctagaaaaacatggagc
aatcacaagtagcaatacagcagctaccaatgctgattgtgcctggctagaagcacaagaggaggaggaggtgggttttccagtcacac
ctcaggtacctttaagaccaatgacttacaaggcagctgtagatcttagccactttttaaaagaaaaggggggactggaagggctaattc
actcccaacgaagacaagatatccttgatctgtggatctaccacacacaaggctacttccctgattggcagaactacacaccagggccag
ggatcagatatccactgacctttggatggtgctacaagctagtaccagttgagcaagagaaggtagaagaagccaatgaaggagagaa
cacccgcttgttacaccctgtgagcctgcatgggatggatgacccggagagagaagtattagagtggaggtttgacagccgcctagcatt
tcatcacatggcccgagagctgcatccggactgtactgggtctctctggttagaccagatctgagcctgggagctctctggctaactaggg
aacccactgcttaagcctcaataaagcttgccttgagtgcttcaagtagtgtgtgcccgtctgttgtgtgactctggtaactagagatccct
cagacccttttagtcagtgtggaaaatctctagcagggcccgtttaaacccgctgatcagcctcgactgtgccttctagttgccagccatct
gttgtttgcccctcccccgtgccttccttgaccctggaaggtgccactcccactgtcctttcctaataaaatgaggaaattgcatcgcattgt
ctgagtaggtgtcattctattctggggggtggggtggggcaggacagcaagggggaggattgggaagacaatagcaggcatgctgggg
atgcggtgggctctatggcttctgaggcggaaagaaccagctggggctctagggggtatccccacgcgccctgtagcggcgcattaagc
gcggcgggtgtggtggttacgcgcagcgtgaccgctacacttgccagcgccctagcgcccgctcctttcgctttcttcccttcctttctcgcc
acgttcgccggctttccccgtcaagctctaaatcgggggctccctttagggttccgatttagtgctttacggcacctcgaccccaaaaaact
tgattagggtgatggttcacgtagtgggccatcgccctgatagacggtttttcgccctttgacgttggagtccacgttctttaatagtggact
cttgttccaaactggaacaacactcaaccctatctcggtctattcttttgatttataagggattttgccgatttcggcctattggttaaaaaat
gagctgatttaacaaaaatttaacgcgaattaattctgtggaatgtgtgtcagttagggtgtggaaagtccccaggctccccagcaggca
gaagtatgcaaagcatgcatctcaattagtcagcaaccaggtgtggaaagtccccaggctccccagcaggcagaagtatgcaaagcat
gcatctcaattagtcagcaaccatagtcccgcccctaactccgcccatcccgcccctaactccgcccagttccgcccattctccgccccatg
gctgactaattttttttatttatgcagaggccgaggccgcctctgcctctgagctattccagaagtagtgaggaggcttttttggaggcctag
gcttttgcaaaaagctcccgggagcttgtatatccattttcggatctgatcagcacgtgttgacaattaatcatcggcatagtatatcggca
tagtataatacgacaaggtgaggaactaaaccatggccaagttgaccagtgccgttccggtgctcaccgcgcgcgacgtcgccggagcg
gtcgagttctggaccgaccggctcgggttctcccgggacttcgtggaggacgacttcgccggtgtggtccgggacgacgtgaccctgttca
tcagcgcggtccaggaccaggtggtgccggacaacaccctggcctgggtgtgggtgcgcggcctggacgagctgtacgccgagtggtcg
gaggtcgtgtccacgaacttccgggacgcctccgggccggccatgaccgagatcggcgagcagccgtgggggcgggagttcgccctgc
gcgacccggccggcaactgcgtgcacttcgtggccgaggagcaggactgacacgtgctacgagatttcgattccaccgccgccttctatg
aaaggttgggcttcggaatcgttttccgggacgccggctggatgatcctccagcgcggggatctcatgctggagttcttcgcccaccccaa
cttgtttattgcagcttataatggttacaaataaagcaatagcatcacaaatttcacaaataaagcatttttttcactgcattctagttgtgg
tttgtccaaactcatcaatgtatcttatcatgtctgtataccgtcgacctctagctagagcttggcgtaatcatggtcatagctgtttcctgtg
tgaaattgttatccgctcacaattccacacaacatacgagccggaagcataaagtgtaaagcctggggtgcctaatgagtgagctaactc

acattaattgcgttgcgctcactgcccgctttccagtcgggaaacctgtcgtgccagctgcattaatgaatcggccaacgcgcggggaga
ggcggtttgcgtattgggcgctcttccgcttcctcgctcactgactcgctgcgctcggtcgttcggctgcggcgagcggtatcagctcactca
aaggcggtaatacggttatccacagaatcaggggataacgcaggaaagaacatgtgagcaaaaggccagcaaaaggccaggaaccg
taaaaaggccgcgttgctggcgtttttccataggctccgcccccctgacgagcatcacaaaaatcgacgctcaagtcagaggtggcgaa
acccgacaggactataaagataccaggcgtttccccctggaagctccctcgtgcgctctcctgttccgaccctgccgcttaccggatacct
gtccgcctttctcccttcgggaagcgtggcgctttctcatagctcacgctgtaggtatctcagttcggtgtaggtcgttcgctccaagctggg
ctgtgtgcacgaaccccccgttcagcccgaccgctgcgccttatccggtaactatcgtcttgagtccaacccggtaagacacgacttatcg
ccactggcagcagccactggtaacaggattagcagagcgaggtatgtaggcggtgctacagagttcttgaagtggtggcctaactacgg
ctacactagaagaacagtatttggtatctgcgctctgctgaagccagttaccttcggaaaaagagttggtagctcttgatccggcaaaca
aaccaccgctggtagcggtggtttttttgtttgcaagcagcagattacgcgcagaaaaaaaggatctcaagaagatcctttgatcttttct
acggggtctgacgctcagtggaacgaaaactcacgttaagggattttggtcatgagattatcaaaaaggatcttcacctagatccttttaa
attaaaaatgaagttttaaatcaatctaaagtatatatgagtaaacttggtctgacagttaccaatgcttaatcagtgaggcacctatctc
agcgatctgtctatttcgttcatccatagttgcctgactccccgtcgtgtagataactacgatacgggagggcttaccatctggccccagtg
ctgcaatgataccgcgagacccacgctcaccggctccagatttatcagcaataaaccagccagccggaagggccgagcgcagaagtgg
tcctgcaactttatccgcctccatccagtctattaattgttgccgggaagctagagtaagtagttcgccagttaatagtttgcgcaacgttgt
tgccattgctacaggcatcgtggtgtcacgctcgtcgtttggtatggcttcattcagctccggttcccaacgatcaaggcgagttacatgat
cccccatgttgtgcaaaaaagcggttagctccttcggtcctccgatcgttgtcagaagtaagttggccgcagtgttatcactcatggttatg
gcagcactgcataattctcttactgtcatgccatccgtaagatgcttttctgtgactggtgagtactcaaccaagtcattctgagaatagtgt
atgcggcgaccgagttgctcttgcccggcgtcaatacgggataataccgcgccacatagcagaactttaaaagtgctcatcattggaaaa
cgttcttcggggcgaaaactctcaaggatcttaccgctgttgagatccagttcgatgtaacccactcgtgcacccaactgatcttcagcatc
ttttactttcaccagcgtttctgggtgagcaaaaacaggaaggcaaaatgccgcaaaaaagggaataagggcgacacggaaatgttga
atactcatactcttcctttttcaatattattgaagcatttatcagggttattgtctcatgagcggatacatatttgaatgtatttagaaaaata
aacaaataggggttccgcgcacatttccccgaaaagtgccacctgac (SEQ ID NO:35)

### Aminoacid sequence:

MVSKGEELFTGVVPILVELDGDVNGHKFSVSGEGEGDATYGKLTLKFICTTGKLPVPWPTLVTTLTYGVQCFS
RYPDHMKQHDFFKSAMPEGYVQERTIFFKDDGNYKTRAEVKFEGDTLVNRIELKGIDFKEDGNILGHKLEYN
YNSHNVYIMADKQKNGIKVNFKIRHNIEDGSVQLADHYQQNTPIGDGPVLLPDNHYLSTQSALSKDPNEKR
DHMVLLEFVTAAGITLGMDELYSKSFQQSSLSRDSQGHGRDLSAAGIGLLAAATQSLSMPASLGRMNQGT
ARLASLMNLGMSSSLNQQGAHSALSSASTSSHNLQSIFNIGSRGPLPLSSQHRGDADQASNILASFGLSAR
DLDELSRYPEDKITPENLPQILLQLKRRRTEEGPTLSYGRDGRSATREPPYRVPRDDWEEKRHFRRDSFDDR
GPSLNPVLDYDHGSRSQESGYYDRMDYEDDRLRDGERCRDDSFFGETSHNYHKFDSEYERMGRGPGPLQ
ERSLFEKKRGAPPSSNIEDFHGLLPKGRDPKKKRKVEDPKKKRKVEDPKKKRKVD* (SEQ ID NO:36)

**p639 MATR3 2-288/BFP LV** vector used for IncuCyte CASP3/7 assay

### CMV Enhancer promoter

### FLAG

### MATR3 2-288

### 3XNLS

### EF1aPromoter

### BFP

### Nucleotide sequence:

AAATTTCACAAATAAAGCATTTTTTTCACTGCATTCTAGTTGTGGTTTGTCCAAACTCATCAATGTATCTTA
TCATGTCTGGCTCTAGCTATCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTCCAGAAGTAGTGAGGAGG

CTTTTTTGGAGGCCTAGGCTTTTGCGTCGAGACGTACCCAATTCGCCCTATAGTGAGTCGTATTACGCGC
GCTCACTGGCCGTCGTTTTACAACGTCGTGACTGGGAAAACCCTGGCGTTACCCAACTTAATCGCCTTGC
AGCACATCCCCCTTTCGCCAGCTGGCGTAATAGCGAAGAGGCCCGCACCGATCGCCCTTCCCAACAGTT
GCGCAGCCTGAATGGCGAATGGCGCGACGCGCCCTGTAGCGGCGCATTAAGCGCGGCGGGTGTGGTG
GTTACGCGCAGCGTGACCGCTACACTTGCCAGCGCCCTAGCGCCCGCTCCTTTCGCTTTCTTCCCTTCCTT
TCTCGCCACGTTCGCCGGCTTTCCCCGTCAAGCTCTAAATCGGGGGCTCCCTTTAGGGTTCCGATTTAGT
GCTTTACGGCACCTCGACCCCAAAAAACTTGATTAGGGTGATGGTTCACGTAGTGGGCCATCGCCCTGA
TAGACGGTTTTTCGCCCTTTGACGTTGGAGTCCACGTTCTTTAATAGTGGACTCTTGTTCCAAACTGGAAC
AACACTCAACCCTATCTCGGTCTATTCTTTTGATTTATAAGGGATTTTGCCGATTTCGGCCTATTGGTTAA
AAAATGAGCTGATTTAACAAAAATTTAACGCGAATTTTAACAAAATATTAACGTTTACAATTTCCCAGGT
GGCACTTTTCGGGGAAATGTGCGCGGAACCCCTATTTGTTTATTTTTCTAAATACATTCAAATATGTATCC
GCTCATGAGACAATAACCCTGATAAATGCTTCAATAATATTGAAAAAGGAAGAGTATGAGTATTCAACA
TTTCCGTGTCGCCCTTATTCCCTTTTTTGCGGCATTTTGCCTTCCTGTTTTTGCTCACCCAGAAACGCTGGT
GAAAGTAAAAGATGCTGAAGATCAGTTGGGTGCACGAGTGGGTTACATCGAACTGGATCTCAACAGCG
GTAAGATCCTTGAGAGTTTTCGCCCCGAAGAACGTTTTCCAATGATGAGCACTTTTAAAGTTCTGCTATG
TGGCGCGGTATTATCCCGTATTGACGCCGGGCAAGAGCAACTCGGTCGCCGCATACACTATTCTCAGAA
TGACTTGGTTGAGTACTCACCAGTCACAGAAAAGCATCTTACGGATGGCATGACAGTAAGAGAATTATG
CAGTGCTGCCATAACCATGAGTGATAACACTGCGGCCAACTTACTTCTGACAACGATCGGAGGACCGAA
GGAGCTAACCGCTTTTTTGCACAACATGGGGGATCATGTAACTCGCCTTGATCGTTGGGAACCGGAGCT
GAATGAAGCCATACCAAACGACGAGCGTGACACCACGATGCCTGTAGCAATGGCAACAACGTTGCGCA
AACTATTAACTGGCGAACTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCTGATAAATCTGGAGCCG
GTGAGCGTGGGTCTCGCGGTATCATTGCAGCACTGGGGCCAGATGGTAAGCCCTCCCGTATCGTAGTTA
TCTACACGACGGGGAGTCAGGCAACTATGGATGAACGAAATAGACAGATCGCTGAGATAGGTGCCTCA
CTGATTAAGCATTGGTAACTGTCAGACCAAGTTTACTCATATATACTTTAGATTGATTTAAAACTTCATTT
TTAATTTAAAAGGATCTAGGTGAAGATCCTTTTTGATAATCTCATGACCAAAATCCCTTAACGTGAGTTTT
CGTTCCACTGAGCGTCAGACCCCGTAGAAAAGATCAAAGGATCTTCTTGAGATCCTTTTTTTCTGCGCGT
AATCTGCTGCTTGCAAACAAAAAAACCACCGCTACCAGCGGTGGTTTGTTTGCCGGATCAAGAGCTACC
AACTCTTTTTCCGAAGGTAACTGGCTTCAGCAGAGCGCAGATACCAAATACTGTCCTTCTAGTGTAGCCG
TAGTTAGGCCACCACTTCAAGAACTCTGTAGCACCGCCTACATACCTCGCTCTGCTAATCCTGTTACCAGT
GGCTGCTGCCAGTGGCGATAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAGCGAACGACCTACACCGAA
CTGAGATACCTACAGCGTGAGCTATGAGAAAGCGCCACGCTTCCCGAAGGGAGAAAGGCGGACAGGTA
TCCGGTAAGCGGCAGGGTCGGAACAGGAGAGCGCACGAGGGAGCTTCCAGGGGGAAACGCCTGGTAT
CTTTATAGTCCTGTCGGGTTTCGCCACCTCTGACTTGAGCGTCGATTTTTGTGATGCTCGTCAGGGGGGC
GGAGCCTATGGAAAAACGCCAGCAACGCGGCCTTTTTACGGTTCCTGGCCTTTTGCTGGCCTTTTGCTCA
CATGTTCTTTCCTGCGTTATCCCCTGATTCTGTGGATAACCGTATTACCGCCTTTGAGTGAGCTGATACCG
CTCGCCGCAGCCGAACGACCGAGCGCAGCGAGTCAGTGAGCGAGGAAGCGGAAGAGCGCCCAATACG
CAAACCGCCTCTCCCCGCGCGTTGGCCGATTCATTAATGCAGCTGGCACGACAGGTTTCCCGACTGGAA
AGCGGGCAGTGAGCGCAACGCAATTAATGTGAGTTAGCTCACTCATTAGGCACCCCAGGCTTTACACTT
TATGCTTCCGGCTCGTATGTTGTGTGGAATTGTGAGCGGATAACAATTTCACACAGGAAACAGCTATGA
CCATGATTACGCCAAGCGCGCAATTAACCCTCACTAAAGGGAACAAAAGCTGGAGCTGCAAGCTTGGCC
ATTGCATACGTTGTATCCATATCATAATATGTACATTTATATTGGCTCATGTCCAACATTACCGCCATGTTg
acattgattattgactagttattaatagtaatcaattacggggtcattagttcatagcccatatatggagttccgcgttacataactta
cggtaaatggcccgcctggctgaccgcccaacgacccccgcccattgacgtcaataatgacgtatgttcccatagtaacgccaatag
ggactttccattgacgtcaatgggtggagtatttacggtaaactgcccacttggcagtacatcaagtgtatcatatgccaagtacgc
cccctattgacgtcaatgacggtaaatggcccgcctggcattatgcccagtacatgaccttatgggactttcctacttggcagtacatc

tacgtattagtcatcgctattaccatggtgatgcggttttggcagtacatcaatgggcgtggatagcggtttgactcacggggatttc
caagtctccaccccattgacgtcaatgggagtttgttttggcaccaaaatcaacgggactttccaaaatgtcgtaacaactccgcccc
attgacgcaaatgggcggtaggcgtgtacggtgggaggtctatataagcagagctCGTTTAGTGAACCGGGGTCTCTCT
GGTTAGACCAGATCTGAGCCTGGGAGCTCTCTGGCTAACTAGGGAACCCACTGCTTAAGCCTCAATAAA
GCTTGCCTTGAGTGCTTCAAGTAGTGTGTGCCCGTCTGTTGTGTGACTCTGGTAACTAGAGATCCCTCAG
ACCCTTTTAGTCAGTGTGGAAAATCTCTAGCAGTGGCGCCCGAACAGGGACCTGAAAGCGAAAGGGAA
ACCAGAGCTCTCTCGACGCAGGACTCGGCTTGCTGAAGCGCGCACGGCAAGAGGCGAGGGGCGGCGA
CTGGTGAGTACGCCAAAAATTTTGACTAGCGGAGGCTAGAAGGAGAGAGATGGGTGCGAGAGCGTCA
GTATTAAGCGGGGGAGAATTAGATCGCGATGGGAAAAAATTCGGTTAAGGCCAGGGGGAAAGAAAAA
ATATAAATTAAAACATATAGTATGGGCAAGCAGGGAGCTAGAACGATTCGCAGTTAATCCTGGCCTGTT
AGAAACATCAGAAGGCTGTAGACAAATACTGGGACAGCTACAACCATCCCTTCAGACAGGATCAGAAG
AACTTAGATCATTATATAATACAGTAGCAACCCTCTATTGTGTGCATCAAAGGATAGAGATAAAAGACAC
CAAGGAAGCTTTAGACAAGATAGAGGAAGAGCAAAACAAAAGTAAGACCACCGCACAGCAAGCGGCC
GCTGATCTTCAGACCTGGAGGAGGAGATATGAGGGACAATTGGAGAAGTGAATTATATAAATATAAAG
TAGTAAAAATTGAACCATTAGGAGTAGCACCCACCAAGGCAAAGAGAAGAGTGGTGCAGAGAGAAAA
AAGAGCAGTGGGAATAGGAGCTTTGTTCCTTGGGTTCTTGGGAGCAGCAGGAAGCACTATGGGCGCAG
CCTCAATGACGCTGACGGTACAGGCCAGACAATTATTGTCTGGTATAGTGCAGCAGCAGAACAATTTGC
TGAGGGCTATTGAGGCGCAACAGCATCTGTTGCAACTCACAGTCTGGGGCATCAAGCAGCTCCAGGCAA
GAATCCTGGCTGTGGAAAGATACCTAAAGGATCAACAGCTCCTGGGGATTTGGGGTTGCTCTGGAAAAC
TCATTTGCACCACTGCTGTGCCTTGGAATGCTAGTTGGAGTAATAAATCTCTGGAACAGATTGGAATCAC
ACGACCTGGATGGAGTGGGACAGAGAAATTAACAATTACACAAGCTTAATACACTCCTTAATTGAAGAA
TCGCAAAACCAGCAAGAAAAGAATGAACAAGAATTATTGGAATTAGATAAATGGGCAAGTTTGTGGAA
TTGGTTTAACATAACAAATTGGCTGTGGTATATAAAATTATTCATAATGATAGTAGGAGGCTTGGTAGGT
TTAAGAATAGTTTTTGCTGTACTTTCTATAGTGAATAGAGTTAGGCAGGGATATTCACCATTATCGTTTCA
GACCCACCTCCCAACCCCGAGGGGACCCGACAGGCCCGAAGGAATAGAAGAAGAAGGTGGAGAGAGA
GACAGAGACAGATCCATTCGATTAGTGAACGGATCTCGACGGTATCGGTTAACTTTTAAAAGAAAAGGG
GGGATTGGGGGGTACAGTGCAGGGGAAAGAATAGTAGACATAATAGCAACAGACATACAAACTAAAG
AATTACAAAAACAAATTACAAAAATTCAAAATTTTATCGATCACGAGACTAGCCTCGAGAAGCTTGATTA
TATACTAGTATGAAACCCCACCTGTAGGTTTGGCAAGCTAGCTGCAGTAACGCCATTTTGCAAGGCATG
GAAAAATACCAAACCAAGAATAGAGAAGTTCAGATCAAGGGCGGGTACATGAAAATAGCTAACGTTGG
GCCAAACAGGATATCTGCGGTGAGCAGTTTCGGCCCCGGCCCGGGGCCAAGAACAGATGGTCACCGCA
GTTTCGGCCCCGGCCCGAGGCCAAGAACAGATGGTCCCCAGATATGGCCCAACCCTCAGCAGTTTCTTA
AGACCCATCAGATGTTTCCAGGCTCCCCCAAGGACCTGAAATGACCCTGCGCCTTATTTGAATTAACCAA
TCAGCCTGCTTCTCGCTTCTGTTCGCGCGCTTCTGCTTCCCGAGCTCTATAAAAGAGCTCACAACCCCTCA
CTCGGCTTAATTAAAAGCTTTATATAATCAGCATCACAAGTTTGTACAAAAAAGCAGGCTCCATGGATTA
CAAGGATGACGACGATAAGAGCCCGGGCggatccTCCAAGTCATTCCAGCAGTCATCTCTCAGTAGGGA
CTCACAGGGTCATGGGCGTGACCTGTCTGCGGCAGGAATAGGCCTTCTTGCTGCTGCTACCCAGTCTTT
AAGTATGCCAGCATCTCTTGGAAGGATGAACCAGGGTACTGCACGCCTTGCTAGTTTAATGAATCTTG
GAATGAGTTCTTCATTGAATCAACAAGGAGCTCATAGTGCACTGTCTTCTGCTAGTACTTCTTCCCATA
ATTTGCAGTCTATATTTAACATTGGAAGTAGAGGTCCACTCCCTTTATCTTCTCAACACCGTGGAGATG
CAGACCAGGCCAGTAACATTTTGGCCAGCTTTGGTCTGTCTGCTAGAGACTTAGATGAACTGAGTCGT
TATCCAGAGGACAAGATTACTCCTGAGAATTTGCCCCAAATCCTTCTACAGCTTAAAAGGAGGAGAAC
TGAAGAAGGCCCTACCTTGAGTTATGGTAGAGATGGCAGATCTGCTACACGGGAGCCACCATACAGA
GTACCTAGGGATGATTGGGAAGAAAAAAGGCACTTTAGAAGAGATAGTTTTGATGATCGTGGTCCTA
GTCTCAACCCAGTGCTTGATTATGACCATGGAAGTCGTTCTCAAGAATCTGGTTATTATGACAGAATG
GATTATGAAGATGACAGATTAAGAGATGGAGAAAGGTGTAGGGATGATTCTTTTTTTGGTGAGACCT
CGCATAACTATCATAAATTTGACAGTGAGTATGAGAGAATGGGACGTGGTCCTGGCCCCTTACAAGA

GAGATCTCTCTTTGAGAAAAAGAGAGGCGCTCCTCCAAGTAGCAATATTGAAGACTTCCATGGACTCT
TACCGAAGGGTagggatcccaagaaaaaacgcaaggtggaagatcctaagaaaaagcggaaagtggaagacccaaaaa
agaagagaaaggtagacTAATAAGACCCAGCTTTCTTGTACAAAGTGGTGATGCTCTAGAATGCATGCATG
CCGTACGATCGCCGCCTCAGGCCTGAGTTCGAAGTATACGTCGACAATCAACCTCTGGATTACAAAATTT
GTGAAAGATTGACTGGTATTCTTAACTATGTTGCTCCTTTTACGCTATGTGGATACGCTGCTTTAATGCCT
TTGTATCATGCTATTGCTTCCCGTATGGCTTTCATTTTCTCCTCCTTGTATAAATCCTGGTTGCTGTCTCTTT
ATGAGGAGTTGTGGCCCGTTGTCAGGCAACGTGGCGTGGTGTGCACTGTGTTTGCTGACGCAACCCCCA
CTGGTTGGGGCATTGCCACCACCTGTCAGCTCCTTTCCGGGACTTTCGCTTTCCCCCTCCCTATTGCCACG
GCGGAACTCATCGCCGCCTGCCTTGCCCGCTGCTGGACAGGGGCTCGGCTGTTGGGCACTGACAATTCC
GTGGTGTTGTCGGGGAAGCTGACGTCCTTTCCATGGCTGCTCGCCTGTGTTGCCACCTGGATTCTGCGC
GGGACGTCTTCTGCTACGTCCCTTCGGCCTCAATCAGCGGACTTCCTTCCCGCGGCCTGCTGCCGGCTCT
GCGGCCTCTTCCGCGTCTTCGCCTTCGCCCTCAGACGAGTCGGATCTCCCTTTGGGCCGCCTCCCCGCCT
GGAATTCCTGCAGCCCGGGGGATCAAGGATCTGCGATCGCTCCGGTGCCCGTCAGTGGGCAGAGCGCA
CATCGCCCACAGTCCCCGAGAAGTTGGGGGGAGGGGTCGGCAATTGAACGGGTGCCTAGAGAAGGTG
GCGCGGGGTAAACTGGGAAAGTGATGTCGTGTACTGGCTCCGCCTTTTTCCCGAGGGTGGGGGAGAAC
CGTATATAAGTGCAGTAGTCGCCGTGAACGTTCTTTTTCGCAACGGGTTTGCCGCCAGAACACAGCTGAA
GCTTCGAGGGGCTCGCATCTCTCCTTCACGCGCCCGCCGCCCTACCTGAGGCCGCCATCCACGCCGGTTG
AGTCGCGTTCTGCCGCCTCCCGCCTGTGGTGCCTCCTGAACTGCGTCCGCCGTCTAGGTAAGTTTAAAGC
TCAGGTCGAGACCGGGCCTTTGTCCGGCGCTCCCTTGGAGCCTACCTAGACTCAGCCGGCTCTCCACGCT
TTGCCTGACCCTGCTTGCTCAACTCTACGTCTTTGTTTCGTTTTCTGTTCTGCGCCGTTACAGATCCAAGCT
GTGACCGGCGCCTACGATCCACCGGTCGCCACCATGAGCGAGCTGATTAAGGAGAACATGCACATGAA
GCTGTACATGGAGGGCACCGTGGACAACCATCACTTCAAGTGCACATCCGAGGGCGAAGGCAAGCCCT
ACGAGGGCACCCAGACCATGAGAATCAAGGTGGTCGAGGGCGGCCCTCTCCCCTTCGCCTTCGACATCC
TGGCTACTAGCTTCCTCTACGGCAGCAAGACCTTCATCAACCACACCCAGGGCATCCCCGACTTCTTCAA
GCAGTCCTTCCCTGAGGGCTTCACATGGGAGAGAGTCACCACATACGAAGACGGGGGCGTGCTGACCG
CTACCCAGGACACCAGCCTCCAGGACGGCTGCCTCATCTACAACGTCAAGATCAGAGGGGTGAACTTCA
CATCCAACGGCCCTGTGATGCAGAAGAAAACACTCGGCTGGGAGGCCTTCACCGAGACGCTGTACCCCG
CTGACGGCGGCCTGGAAGGCAGAAACGACATGGCCCTGAAGCTCGTGGGCGGGAGCCATCTGATCGCA
AACATCAAGACCACATATAGATCCAAGAAACCCGCTAAGAACCTCAAGATGCCTGGCGTCTACTATGTG
GACTACAGACTGGAAAGAATCAAGGAGGCCAACAACGAGACCTACGTCGAGCAGCACGAGGTGGCAG
TGGCCAGATACTGCGACCTCCCTAGCAAACTGGGGCACAAGCTTAATTAAAGCGGCCGCGTCGAGAGAT
GGGGGAGGCTAACTGAGCTAGCTTAGTCATGCACCGGGCTGCAGGAATTCGAGCTCGGTACCTTTAAG
ACCAATGACTTACAAGGCAGCTGTAGATCTTAGCCACTTTTTAAAAGAAAAGGGGGGACTGGAAGGGC
TAATTCACTCCCAACGAAGACAAGATCTGCTTTTTGCTTGTACTGGGTCTCTCTGGTTAGACCAGATCTGA
GCCTGGGAGCTCTCTGGCTAACTAGGGAACCCACTGCTTAAGCCTCAATAAAGCTTGCCTTGAGTGCTTC
AAGTAGTGTGTGCCCGTCTGTTGTGTGACTCTGGTAACTAGAGATCCCTCAGACCCTTTTAGTCAGTGTG
GAAAATCTCTAGCAGTAGTAGTTCATGTCATCTTATTATTCAGTATTTATAACTTGCAAAGAAATGAATAT
CAGAGAGTGAGAGGAACTTGTTTATTGCAGCTTATAATGGTTACAAATAAAGCAATAGCATCAC (SEQ
ID NO:37)

### Aminoacid sequence:

MDYKDDDDKSPGGSSKSFQQSSLSRDSQGHGRDLSAAGIGLLAAATQSLSMPASLGRMNQGTARLASL
MNLGMSSSLNQQGAHSALSSASTSSHNLQSIFNIGSRGPLPLSSQHRGDADQASNILASFGLSARDLDELS
RYPEDKITPENLPQILLQLKRRRTEEGPTLSYGRDGRSATREPPYRVPRDDWEEKRHFRRDSFDDRGPSLNP
VLDYDHGSRSQESGYYDRMDYEDDRLRDGERCRDDSFFGETSHNYHKFDSEYERMGRGPGPLQERSLFE
KKRGAPPSSNIEDFHGLLPKGRDPKKKRKVEDPKKKRKVEDPKKKRKVD* (SEQ ID NO:38)

### p681 mCherry AAV-CTRL

### ITRs

### CK8ePromoter

### mCherry

### IacZ

### Synth.pA

### ITRs

Aatgcagctgcgcgctcgctcgctcactgaggccgcccgggcaaagcccgggcgtcgggcgacctttggtcgcccggcctcagtgagcg
agcgagcgcgcagagagggagtggccaactccatcactaggggttccttgtagttaatgattaacccgccatgctacttatctacaaact
ctagcatgctgcccatgtaaggaggcaaggcctggggacacccgagatgcctggttataattaacccagacatgtggctgccccc
ccccccccaacacctgctgcctctaaaaataaccctgcatgccatgttcccggcgaagggccagctgtcccccgccagctagactcag
cacttagtttaggaaccagtgagcaagtcagcccttggggcagcccatacaaggccatggggctgggcaagctgcacgcctgggt
ccggggtgggcacggtgcccgggcaacgagctgaaagctcatctgctctcaggggcccctccctggggacagcccctcctggctag
tcacaccctgtaggctcctctatataacccaggggcacaggggctgccctcattctaccaccacctccacagcacagacagacactc
aggagccagccagcgtcgaggttaacc
atggtgagcaagggcgaggaggataacatggccatcatcaaggagttcatgcgcttcaaggtgcacatggagggctccgtgaacggc
cacgagttcgagatcgagggcgagggcgagggccgcccctacgagggcacccagaccgccaagctgaaggtgaccaagggtggccc
cctgcccttcgcctgggacatcctgtcccctcagttcatgtacggctccaaggcctacgtgaagcaccccgccgacatccccgactacttg
aagctgtccttccccgagggcttcaagtgggagcgcgtgatgaacttcgaggacggcggcgtggtgaccgtgacccaggactcctccct
gcaggacggcgagttcatctacaaggtgaagctgcgcggcaccaacttcccctccgacggccccgtaatgcagaagaagaccatggg
ctgggaggcctcctccgagcggatgtaccccgaggacggcgccctgaagggcgagatcaagcagaggctgaagctgaaggacggcg
gccactacgacgctgaggtcaagaccacctacaaggccaagaagcccgtgcagctgcccggcgcctacaacgtcaacatcaagttgg
acatcacctcccacaacgaggactacaccatcgtggaacagtacgaacgcgccgagggccgccactccaccggcggcatggacgagc
tgtacaagtag
GGGGATCCGACCGGTGCTAGCGGCCGC
caattgcaacgtcgtgactgggaaaaccctggcgttacccaacttaatcgccttgcagcacatccccctttcgccagctggcgtaatagcg
aagaggcccgcaccgatcgcccttcccaacagttgcgcagcctgaatggcgaatggcgctttgcctggtttccggcaccagaagcggtgc
cggaaagctggctggagtgcgatcttcctgaggccgatactgtcgtcgtcccctcaaactggcagatgcacggttacgatgcgcccatct
acaccaacgtgacctatcccattacggtcaatccgccgtttgttcccacggagaatccgacgggttgttactcgctcacatttaatgttgat
gaaagctggctacaggaaggccagacgcgaattatttttgatggcgttaactcggcgtttcatctgtggtgcaacgggcgctgggtcggtt
acggccaggacagtcgtttgccgtctgaatttgacctgagcgcatttttacgcgccggagaaaaccgcctcgcggtgatggtgctgcgctg
gagtgacggcagttatctggaagatcaggatatgtggcggatgagcggcattttccgtgacgtctcgttgctgcataaaccgactacaca
aatcagcgatttccatgttgccactcgctttaatgatgatttcagccgcgctgtactggaggctgaagttcagatgtgcggcgagttgcgtg
actacctacgggtaacagtttctttatggcagggtgaaacgcaggtcgccagcggcaccgcgcctttcggcggtgaaattatcgatgagc
gtggtggttatgccgatcgcgtcacactacgtctgaacgtcgaaaacccgaaactgtggagcgccgaaatcccgaatctctatcgtgcgg
tggttgaactgcacaccgccgacggcacgctgattgaagcagaagcctgcgatgtcggtttccgcgaggtgcggattgaaaatggtctgc
tgctgctgaacggcaagccgttgctgattcgaggcgttaaccgtcacgagcatcatcctctgcatggtcaggtcatggatgagcagacga
tggtgcaggatatcctgctgatgaagcagaacaactttaacgccgtgcgctgttcgcattatccgaaccatccgctgtggtacacgctgtg
cgaccgctacggcctgtatgtggtggatgaagccaatattgaaacccacggcatggtgccaatgaatcgtctgaccgatgatccgcgctg
gctaccggcgatgagcgaacgcgtaacgcgaatggtgcagcgcgatcgtaatcacccgagtgtgatcatctggtcgctggggaatgaat
caggccacggcgctaatcacgacgcgctgtatcgctggatcaaatctgtcgatccttcccgcccggtgcagtatgaaggcggcggagccg
acaccacggccaccgatattatttgcccgatgtacgcgcgcgtggatgaagaccagcccttcccggctgtgccgaaatggtccatcaaaa
aatggctttcgctacctggagagacgcgcccgctgatcctttgcgaatacgcccacgcgatgggtaacagtcttggcggtttcgctaaata
ctggcaggcgtttcgtcagtatccccgtttacagggcggcttcgtctgggactgggtggatcagtcgctgattaaatatgatgaaaacggc
aacccgtggtcggcttacggcggtgattttggcgatacgccgaacgatcgccagttctgtatgaacggtctggtctttgccgaccgcacgc

cgcatccagcgctgacggaagcaaaacaccagcagcagtttttccagttccgtttatccgggcaaaccatcgaagtgaccagcgaatac
ctgttccgtcatagcgataacgagctcctgcactggatggtggcgctggatggtaagccgctggcaagcggtgaagtgcctctggatgtc
gctccacaaggtaaacagttgattgaactgcctgaactaccgcagccggagagcgccgggcaactctggctcacagtacgcgtagtgca
accgaacgcgaccgcatggtcagaagccgggcacatcagcgcctggcagcagtggcgtctggcggaaaacctcagtgtgacgctcccc
gccgcgtcccacgccatcccgcatctgaccaccagcgaaatggatttttgcatcgagctgggtaataagcgttggcaatttaaccgccagt
caggctttctttcacagatgtggattggcgataaaaaacaactgctgacgccgctgcgcgatcagttcacccgtgcaccgctggataacg
acattggcgtaagtgaagcgacccgcattgaccctaacgcctgggtcgaacgctggaaggcggcgggccattaccaggccgaagcagc
gttgttgcagtgcacggcagatacacttgctgatgcggtgctgattacgaccgctcacgcgtggcagcatcaggggaaaaccttatttatc
agccggaaaacctaccggattgatggtagtggtcaaatggcgattaccgttgatgttgaagtggcgagcgatacaccgcatccggcgcg
gattggcctgaactgccagctggcgcaggtagcagagcgggtaaactggctcggattagggccgcaagaaaactatcccgaccgcctta
ctgccgcctgttttgaccgctgggatctgccattgtcagacatgtataccccgtacgtcttcccgagcgaaaacggtctgcgctgcgggac
gcgcgaattgaattatggcccacaccagtggcgcggcgacttccagttcaacatcagccgctacagtcaacagcaactgatggaaacca
gccatcgccatctgctgcacgcggaagaaggcacatggctgaatatcgacggtttccatatggggattggtggcgacgactcctggagcc
cgtcagtatcggcg
TTAACCGGT
ggctagtaataaaagatccttattttcattggatctgtgtgttggttttttgtgtgggtaccgtttgtagataagtagcatggcgggttaatca
ttaactacaaggaacccctagtgatggagttggccactccctctctgcgcgctcgctcgctcactgaggccgggcgaccaaaggtcgccc
gacgcccgggctttgcccgggcggcctcagtgagcgagcgagcgcgcagctggcgtaatagcgaagaggcccgcaccgatcgcccttc
ccaacagttgcgcagcctgaatggcgaatggcgattccgttgcaatggctggcggtaatattgttctggatattaccagcaaggccgata
gtttgagttcttctactcaggcaagtgatgttattactaatcaaagaagtattgcgacaacggttaatttgcgtgatggacagactcttttac
tcggtggcctcactgattataaaaacacttctcaggattctggcgtaccgttcctgtctaaaatccctttaatcggcctcctgtttagctccc
gctctgattctaacgaggaaagcacgttatacgtgctcgtcaaagcaaccatagtacgcgccctgtagcggcgcattaagcgcggcgggt
gtggtggttacgcgcagcgtgaccgctacacttgccagcgccctagcgcccgctcctttcgctttcttcccttcctttctcgccacgttcgccg
gctttccccgtcaagctctaaatcgggggctccctttagggttccgatttagtgctttacggcacctcgaccccaaaaaacttgattagggt
gatggttcacgtagtgggccatcgccctgatagacggtttttcgccctttgacgttggagtccacgttctttaatagtggactcttgttcca
aactggaacaacactcaaccctatctcggtctattcttttgatttataagggattttgccgatttcggcctattggttaaaaaatgagctgatt
taacaaaaatttaacgcgaattttaacaaaatattaacgtttacaatttaaatatttgcttatacaatcttcctgtttttggggcttttctgatt
atcaaccggggtacatatgattgacatgctagttttacgattaccgttcatcgattctcttgtttgctccagactctcaggcaatgacctgat
agcctttgtagagacctctcaaaaatagctaccctctccggcatgaatttatcagctagaacggttgaatatcatattgatggtgatttgac
tgtctccggcctttctcacccgtttgaatctttacctacacattactcaggcattgcatttaaaatatatgagggttctaaaaatttttatcctt
gcgttgaaataaaggcttctcccgcaaaagtattacagggtcataatgtttttggtacaaccgatttagctttatgctctgaggctttattgc
ttaattttgctaattctttgccttgcctgtatgatttattggatgttggaattcctgatgcggtattttctccttacgcatctgtgcggtatttcac
accgcatatggtgcactctcagtacaatctgctctgatgccgcatagttaagccagccccgacacccgccaacacccgctgacgcgccct
gacgggcttgtctgctcccggcatccgcttacagacaagctgtgaccgtctccgggagctgcatgtgtcagaggttttcaccgtcatcacc
gaaacgcgcgagacgaaagggcctcgtgatacgcctatttttataggttaatgtcatgataataatggtttcttagacgtcaggtggcact
tttcggggaaatgtgcgcggaacccctatttgtttatttttctaaatacattcaaatatgtatccgctcatgagacaataaccctgataaatg
cttcaataatattgaaaaaggaagagtatgagtattcaacatttccgtgtcgcccttattcccttttttgcggcattttgccttcctgtttttgc
tcacccagaaacgctggtgaaagtaaaagatgctgaagatcagttgggtgcacgagtgggttacatcgaactggatctcaacagcggta
agatccttgagagttttcgccccgaagaacgttttccaatgatgagcacttttaaagttctgctatgtggcgcggtattatcccgtattgacg
ccgggcaagagcaactcggtcgccgcatacactattctcagaatgacttggttgagtactcaccagtcacagaaaagcatcttacggatg
gcatgacagtaagagaattatgcagtgctgccataaccatgagtgataacactgcggccaacttacttctgacaacgatcggaggaccg
aaggagctaaccgcttttttgcacaacatgggggatcatgtaactcgccttgatcgttgggaaccggagctgaatgaagccataccaaac
gacgagcgtgacaccacgatgcctgtagcaatggcaacaacgttgcgcaaactattaactggcgaactacttactctagcttcccggcaa
caattaatagactggatggaggcggataaagttgcaggaccacttctgcgctcggcccttccggctggctggtttattgctgataaatctg
gagccggtgagcgtgggtctcgcggtatcattgcagcactggggccagatggtaagccctcccgtatcgtagttatctacacgacgggga
gtcaggcaactatggatgaacgaaatagacagatcgctgagataggtgcctcactgattaagcattggtaactgtcagaccaagtttact
catatatactttagattgatttaaaacttcatttttaatttaaaaggatctaggtgaagatcctttttgataatctcatgaccaaaatccctta

acgtgagttttcgttccactgagcgtcagaccccgtagaaaagatcaaaggatcttcttgagatcctttttttctgcgcgtaatctgctgctt
gcaaacaaaaaaaccaccgctaccagcggtggtttgtttgccggatcaagagctaccaactctttttccgaaggtaactggcttcagcag
agcgcagataccaaatactgtccttctagtgtagccgtagttaggccaccacttcaagaactctgtagcaccgcctacatacctcgctctg
ctaatcctgttaccagtggctgctgccagtggcgataagtcgtgtcttaccgggttggactcaagacgatagttaccggataaggcgcagc
ggtcgggctgaacggggggttcgtgcacacagcccagcttggagcgaacgacctacaccgaactgagatacctacagcgtgagctatg
agaaagcgccacgcttcccgaagggagaaaggcggacaggtatccggtaagcggcagggtcggaacaggagagcgcacgagggagc
ttccagggggaaacgcctggtatctttatagtcctgtcgggtttcgccacctctgacttgagcgtcgatttttgtgatgctcgtcaggggggc
ggagcctatggaaaaacgccagcaacgcggcctttttacggttcctggccttttgctggccttttgctcacatgttctttcctgcgttatcccc
tgattctgtggataaccgtattaccgcctttgagtgagctgataccgctcgccgcagccgaacgaccgagcgcagcgagtcagtgagcga
ggaagcggaagagcgcccaatacgcaaaccgcctctccccgcgcgttggccgattcattaatg (SEQ ID NO:39)

### Amino acid sequences for

### mCherry

MVSKGEEDNMAIIKEFMRFKVHMEGSVNGHEFEIEGEGEGRPYEGTQTAKLKVTKGGPLPFAWDILSPQ
FMYGSKAYVKHPADIPDYLKLSFPEGFKWERVMNFEDGGVVTVTQDSSLQDGEFIYKVKLRGTNFPSDGP
VMQKKTMGWEASSERMYPEDGALKGEIKQRLKLKDGGHYDAEVKTTYKAKKPVQLPGAYNVNIKLDITS
HNEDYTIVEQYERAEGRHSTGGMDELYK* (SEQ ID NO:40)

### p682 MATR3 2-288 AAV

### ITRs

### CK8e Promoter

### KOZAK seg

### GFP

### MATR3 2-288

### 3xNLS

### LacZ

### Synth.pA-ITRs

CAGCTGCGCGCTCGCTCGCTCACTGAGGCCGCCCGGGCAAAGCCCGGGCGTCGGGCGACCTTTGGTCG
CCCGGCCTCAGTGAGCGAGCGAGCGCGCAGAGAGGGAGTGGCCAACTCCATCACTAGGGGTTCCTTGT
AGTTAATGATTAACCCGCCATGCTACTTATCTACAAACTCTAGCATGCtgcccatgtaaggaggcaaggcctggg
gacacccgagatgcctggttataattaacccagacatgtggctgcccccccccccccaacacctgctgcctctaaaaataaccctgca
tgccatgttcccggcgaagggccagctgtcccccgccagctagactcagcacttagtttaggaaccagtgagcaagtcagcccttgg
ggcagcccatacaaggccatggggctgggcaagctgcacgcctgggtccggggtgggcacggtgcccgggcaacgagctgaaa
gctcatctgctctcaggggcccctccctggggacagcccctcctggctagtcacaccctgtaggctcctctatataacccaggggcac
aggggctgccctcattctaccaccacctccacagcacagacagacactcaggagccagccagcGTCGAGGTTAACGCCACC
ATGGTGAGCAAGGGCGAGGAGCTGTTCACCGGGGTGGTGCCCATCCTGGTCGAGCTGGACGGCGACG
TAAACGGCCACAAGTTCAGCGTGTCCGGCGAGGGCGAGGGCGATGCCACCTACGGCAAGCTGACCCTG
AAGTTCATCTGCACCACCGGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAAGTCCGCCATGCCCGAAG
GCTACGTCCAGGAGCGCACCATCTTCTTCAAGGACGACGGCAACTACAAGACCCGCGCCGAGGTGAAG
TTCGAGGGCGACACCCTGGTGAACCGCATCGAGCTGAAGGGCATCGACTTCAAGGAGGACGGCAACAT
CCTGGGGCACAAGCTGGAGTACAACTACAACAGCCACAACGTCTATATCATGGCCGACAAGCAGAAGA
ACGGCATCAAGGTGAACTTCAAGATCCGCCACAACATCGAGGACGGCAGCGTGCAGCTCGCCGACCAC
TACCAGCAGAACACCCCCATCGGCGACGGCCCCGTGCTGCTGCCCGACAACCACTACCTGAGCACCCAG

TCCGCCCTGAGCAAAGACCCCAACGAGAAGCGCGATCACATGGTCCTGCTGGAGTTCGTGACCGCCGCC
GGGATCACTCTCGGCATGGACGAGCTGTACTCCAAGTCATTCCAGCAGTCATCTCTCAGTAGGGACTCA
CAGGGTCATGGGCGTGACCTGTCTGCGGCAGGAATAGGCCTTCTTGCTGCTGCTACCCAGTCTTTAAG
TATGCCAGCATCTCTTGGAAGGATGAACCAGGGTACTGCACGCCTTGCTAGTTTAATGAATCTTGGAA
TGAGTTCTTCATTGAATCAACAAGGAGCTCATAGTGCACTGTCTTCTGCTAGTACTTCTTCCCATAATTT
GCAGTCTATATTTAACATTGGAAGTAGAGGTCCACTCCCTTTATCTTCTCAACACCGTGGAGATGCAGA
CCAGGCCAGTAACATTTTGGCCAGCTTTGGTCTGTCTGCTAGAGACTTAGATGAACTGAGTCGTTATCC
AGAGGACAAGATTACTCCTGAGAATTTGCCCCAAATCCTTCTACAGCTTAAAAGGAGGAGAACTGAA
GAAGGCCCTACCTTGAGTTATGGTAGAGATGGCAGATCTGCTACACGGGAGCCACCATACAGAGTAC
CTAGGGATGATTGGGAAGAAAAAAGGCACTTTAGAAGAGATAGTTTTGATGATCGTGGTCCTAGTCT
CAACCCAGTGCTTGATTATGACCATGGAAGTCGTTCTCAAGAATCTGGTTATTATGACAGAATGGATT
ATGAAGATGACAGATTAAGAGATGGAGAAAGGTGTAGGGATGATTCTTTTTTTGGTGAGACCTCGCA
TAACTATCATAAATTTGACAGTGAGTATGAGAGAATGGGACGTGGTCCTGGCCCCTTACAAGAGAGA
TCTCTCTTTGAGAAAAAGAGAGGCGCTCCTCCAAGTAGCAATATTGAAGACTTCCATGGACTCTTACCG
AAGGGTAGGGATCCCAAGAAAAAACGCAAGGTGGAAGATCCTAAGAAAAAGCGGAAAGTGGAAGAC
CCAAAAAAGAAGAGAAAGGTATAAACCGGT
gcagggtgaaacgcaggtcgccagcggcaccgcgcctttcggcggtgaaattatcgatgagcgtggtggttatgccgatcgcgtcacact
acgtctgaacgtcgaaaacccgaaactgtggagcgccgaaatcccgaatctctatcgtgcggtggttgaactgcacaccgccgacggca
cgctgattgaagcagaagcctgcgatgtcggtttccgcgaggtgcggattgaaaatggtctgctgctgctgaacggcaagccgttgctga
ttcgaggcgttaaccgtcacgagcatcatcctctgcatggtcaggtcatggatgagcagacgatggtgcaggatatcctgctgatgaagc
agaacaactttaacgccgtgcgctgttcgcattatccgaaccatccgctgtggtacacgctgtgcgaccgctacggcctgtatgtggtgga
tgaagccaatattgaaacccacggcatggtgccaatgaatcgtctgaccgatgatccgcgctggctaccggcgatgagcgaacgcgtaa
cgcgaatggtgcagcgcgatcgtaatcacccgagtgtgatcatctggtcgctggggaatgaatcaggccacggcgctaatcacgacgcg
ctgtatcgctggatcaaatctgtcgatccttcccgcccggtgcagtatgaaggcggcggagccgacaccacggccaccgatattatttgcc
cgatgtacgcgcgcgtggatgaagaccagcccttcccggctgtgccgaaatggtccatcaaaaaatggctttcgctacctggagagacg
cgcccgctgatcctttgcgaatacgcccacgcgatgggtaacagtcttggcggtttcgctaaatactggcaggcgtttcgtcagtatcccc
gtttacagggcggcttcgtctgggactgggtggatcagtcgctgattaaatatgatgaaaacggcaacccgtggtcggcttacggcggtg
attttggcgatacgccgaacgatcgccagttctgtatgaacggtctggtctttgccgaccgcacgccgcatccagcgctgacggaagcaa
aacaccagcagcagtttttccagttccgtttatccgggcaaaccatcgaagtgaccagcgaatacctgttccgtcatagcgataacgagct
cctgcactggatggtggcgctggatggtaagccgctggcaagcggtgaagtgcctctggatgtcgctccacaaggtaaacagttgattga
actgcctgaactaccgcagccggagagcgccgggcaactctggctcacagtacgcgtagtgcaaccgaacgcgaccgcatggtcagaa
gccgggcacatcagcgcctggcagcagtggcgtctggcggaaaacctcagtgtgacgctccccgccgcgtcccacgccatcccgcatct
gaccaccagcgaaatggatttttgcatcgagctgggtaataagcgttggcaatttaaccgccagtcaggctttctttcacagatgtggatt
ggcgataaaaaacaactgctgacgccgctgcgcgatcagttcacccgtgcaccgctggataacgacattggcgtaagtgaagcgacccg
cattgaccctaacgcctgggtcgaacgctggaaggcggcgggccattaccaggccgaagcagcgttgttgcagtgcacggcagatacac
ttgctgatgcggtgctgattacgaccgctcacgcgtggcagcatcaggggaaaaccttatttatcagccggaaaacctaccggattgatg
gtagtggtcaaatggcgattaccgttgatgttgaagtggcgagcgatacaccgcatccggcgcggattggcctgaactgccagctggcgc
aggtagcagagcgggtaaactggctcggattagggccgcaagaaaactatcccgaccgccttactgccgcctgttttgaccgctgggatc
tgccattgtcagacatgtataccccgtacgtcttcccgagcgaaaacggtctgcgctgcgggacgcgcgaattgaattatggcccacacc
agtggcgcggcgacttccagttcaacatcagccgctacagtcaacagcaactgatggaaaccagccatcgccatctgctgcacgcggaa
gaaggcacatggctgaatatcgacggtttccatatggggattggtggcgacgactcctggagcccgtcagtatcggcgTAAaccggt
GGCTAGTAATAAAAGATCCTTATTTTCATTGGATCTGTGTGTTGGTTTTTTGTGTGGGTACCGTTTGTAGA
TAAGTAGCATGGCGGGTTAATCATTAACTACAAGGAACCCCTAGTGATGGAGTTGGCCACTCCCTCTCT
GCGCGCTCGCTCGCTCACTGAGGCCGGGCGACCAAAGGTCGCCCGACGCCCGGGCTTTGCCCGGGCGG
CCTCAGTGAGCGAGCGAGCGCGCAGCTGGCGTAATAGCGAAGAGGCCCGCACCGATCGCCCTTCCCAA
CAGTTGCGCAGCCTGAATGGCGAATGGCGATTCCGTTGCAATGGCTGGCGGTAATATTGTTCTGGATAT
TACCAGCAAGGCCGATAGTTTGAGTTCTTCTACTCAGGCAAGTGATGTTATTACTAATCAAAGAAGTATT

GCGACAACGGTTAATTTGCGTGATGGACAGACTCTTTTACTCGGTGGCCTCACTGATTATAAAAACACTT
CTCAGGATTCTGGCGTACCGTTCCTGTCTAAAATCCCTTTAATCGGCCTCCTGTTTAGCTCCCGCTCTGAT
TCTAACGAGGAAAGCACGTTATACGTGCTCGTCAAAGCAACCATAGTACGCGCCCTGTAGCGGCGCATT
AAGCGCGGCGGGTGTGGTGGTTACGCGCAGCGTGACCGCTACACTTGCCAGCGCCCTAGCGCCCGCTC
CTTTCGCTTTCTTCCCTTCCTTTCTCGCCACGTTCGCCGGCTTTCCCCGTCAAGCTCTAAATCGGGGGCTCC
CTTTAGGGTTCCGATTTAGTGCTTTACGGCACCTCGACCCCAAAAAACTTGATTAGGGTGATGGTTCACG
TAGTGGGCCATCGCCCTGATAGACGGTTTTTCGCCCTTTGACGTTGGAGTCCACGTTCTTTAATAGTGGA
CTCTTGTTCCAAACTGGAACAACACTCAACCCTATCTCGGTCTATTCTTTTGATTTATAAGGGATTTTGCC
GATTTCGGCCTATTGGTTAAAAAATGAGCTGATTTAACAAAAATTTAACGCGAATTTTAACAAAATATTA
ACGTTTACAATTTAAATATTTGCTTATACAATCTTCCTGTTTTTGGGGCTTTTCTGATTATCAACCGGGGTA
CATATGATTGACATGCTAGTTTTACGATTACCGTTCATCGATTCTCTTGTTTGCTCCAGACTCTCAGGCAA
TGACCTGATAGCCTTTGTAGAGACCTCTCAAAAATAGCTACCCTCTCCGGCATGAATTTATCAGCTAGAA
CGGTTGAATATCATATTGATGGTGATTTGACTGTCTCCGGCCTTTCTCACCCGTTTGAATCTTTACCTACA
CATTACTCAGGCATTGCATTTAAAATATATGAGGGTTCTAAAAATTTTTATCCTTGCGTTGAAATAAAGGC
TTCTCCCGCAAAAGTATTACAGGGTCATAATGTTTTTGGTACAACCGATTTAGCTTTATGCTCTGAGGCTT
TATTGCTTAATTTTGCTAATTCTTTGCCTTGCCTGTATGATTTATTGGATGTTGGAATTCCTGATGCGGTAT
TTTCTCCTTACGCATCTGTGCGGTATTTCACACCGCATATGGTGCACTCTCAGTACAATCTGCTCTGATGC
CGCATAGTTAAGCCAGCCCCGACACCCGCCAACACCCGCTGACGCGCCCTGACGGGCTTGTCTGCTCCC
GGCATCCGCTTACAGACAAGCTGTGACCGTCTCCGGGAGCTGCATGTGTCAGAGGTTTTCACCGTCATC
ACCGAAACGCGCGAGACGAAAGGGCCTCGTGATACGCCTATTTTTATAGGTTAATGTCATGATAATAAT
GGTTTCTTAGACGTCAGGTGGCACTTTTCGGGGAAATGTGCGCGGAACCCCTATTTGTTTATTTTTCTAA
ATACATTCAAATATGTATCCGCTCATGAGACAATAACCCTGATAAATGCTTCAATAATATTGAAAAAGGA
AGAGTATGAGTATTCAACATTTCCGTGTCGCCCTTATTCCCTTTTTTGCGGCATTTTGCCTTCCTGTTTTTG
CTCACCCAGAAACGCTGGTGAAAGTAAAAGATGCTGAAGATCAGTTGGGTGCACGAGTGGGTTACATC
GAACTGGATCTCAACAGCGGTAAGATCCTTGAGAGTTTTCGCCCCGAAGAACGTTTTCCAATGATGAGC
ACTTTTAAAGTTCTGCTATGTGGCGCGGTATTATCCCGTATTGACGCCGGGCAAGAGCAACTCGGTCGCC
GCATACACTATTCTCAGAATGACTTGGTTGAGTACTCACCAGTCACAGAAAAGCATCTTACGGATGGCAT
GACAGTAAGAGAATTATGCAGTGCTGCCATAACCATGAGTGATAACACTGCGGCCAACTTACTTCTGAC
AACGATCGGAGGACCGAAGGAGCTAACCGCTTTTTTGCACAACATGGGGGATCATGTAACTCGCCTTGA
TCGTTGGGAACCGGAGCTGAATGAAGCCATACCAAACGACGAGCGTGACACCACGATGCCTGTAGCAA
TGGCAACAACGTTGCGCAAACTATTAACTGGCGAACTACTTACTCTAGCTTCCCGGCAACAATTAATAGA
CTGGATGGAGGCGGATAAAGTTGCAGGACCACTTCTGCGCTCGGCCCTTCCGGCTGGCTGGTTTATTGC
TGATAAATCTGGAGCCGGTGAGCGTGGGTCTCGCGGTATCATTGCAGCACTGGGGCCAGATGGTAAGC
CCTCCCGTATCGTAGTTATCTACACGACGGGGAGTCAGGCAACTATGGATGAACGAAATAGACAGATCG
CTGAGATAGGTGCCTCACTGATTAAGCATTGGTAACTGTCAGACCAAGTTTACTCATATATACTTTAGAT
TGATTTAAAACTTCATTTTTAATTTAAAAGGATCTAGGTGAAGATCCTTTTTGATAATCTCATGACCAAAA
TCCCTTAACGTGAGTTTTCGTTCCACTGAGCGTCAGACCCCGTAGAAAAGATCAAAGGATCTTCTTGAGA
TCCTTTTTTTCTGCGCGTAATCTGCTGCTTGCAAACAAAAAAACCACCGCTACCAGCGGTGGTTTGTTTGC
CGGATCAAGAGCTACCAACTCTTTTTCCGAAGGTAACTGGCTTCAGCAGAGCGCAGATACCAAATACTG
TCCTTCTAGTGTAGCCGTAGTTAGGCCACCACTTCAAGAACTCTGTAGCACCGCCTACATACCTCGCTCTG
CTAATCCTGTTACCAGTGGCTGCTGCCAGTGGCGATAAGTCGTGTCTTACCGGGTTGGACTCAAGACGA
TAGTTACCGGATAAGGCGCAGCGGTCGGGCTGAACGGGGGGTTCGTGCACACAGCCCAGCTTGGAGC
GAACGACCTACACCGAACTGAGATACCTACAGCGTGAGCTATGAGAAAGCGCCACGCTTCCCGAAGGG
AGAAAGGCGGACAGGTATCCGGTAAGCGGCAGGGTCGGAACAGGAGAGCGCACGAGGGAGCTTCCA
GGGGGAAACGCCTGGTATCTTTATAGTCCTGTCGGGTTTCGCCACCTCTGACTTGAGCGTCGATTTTTGT
GATGCTCGTCAGGGGGGCGGAGCCTATGGAAAAACGCCAGCAACGCGGCCTTTTTACGGTTCCTGGCC
TTTTGCTGGCCTTTTGCTCACATGTTCTTTCCTGCGTTATCCCCTGATTCTGTGGATAACCGTATTACCGCC

TTTGAGTGAGCTGATACCGCTCGCCGCAGCCGAACGACCGAGCGCAGCGAGTCAGTGAGCGAGGAAGC
GGAAGAGCGCCCAATACGCAAACCGCCTCTCCCCGCGCGTTGGCCGATTCATTAATG (SEQ ID NO:41)

### Amino acid sequences for

GFP
MVSKGEELFTGVVPILVELDGDVNGHKFSVSGEGEGDATYGKLTLKFICTTGKLPVPWPTLVTTLTYGVQCFS
RYPDHMKQHDFFKSAMPEGYVQERTIFFKDDGNYKTRAEVKFEGDTLVNRIELKGIDFKEDGNILGHKLEYN
YNSHNVYIMADKQKNGIKVNFKIRHNIEDGSVQLADHYQQNTPIGDGPVLLPDNHYLSTQSALSKDPNEKR
DHMVLLEFVTAAGITLGMDELY (SEQ ID NO:42)

### MATR3 2-288

SKSFQQSSLSRDSQGHGRDLSAAGIGLLAAATQSLSMPASLGRMNQGTARLASLMNLGMSSSLNQQGA
HSALSSASTSSHNLQSIFNIGSRGPLPLSSQHRGDADQASNILASFGLSARDLDELSRYPEDKITPENLPQILL
QLKRRRTEEGPTLSYGRDGRSATREPPYRVPRDDWEEKRHFRRDSFDDRGPSLNPVLDYDHGSRSQESGY
YDRMDYEDDRLRDGERCRDDSFFGETSHNYHKFDSEYERMGRGPGPLQERSLFEKKRGAPPSSNIEDFHG
LLPKG (SEQ ID NO:43)

### 3xNLS

***PKKKRKV*** (SEQ ID NO:44)
***PKKKRKV***
**PKKKRKV**

### p683 MATR3 FL AAV

### ITRs

### CK8e Promoter

### KOZAK seg

### GFP

### MATR3 (2-847)

### LacZ

### Synth.pA-ITRs

CAGCTGCGCGCTCGCTCGCTCACTGAGGCCGCCCGGGCAAAGCCCGGGCGTCGGGCGACCTTTGGTCG
CCCGGCCTCAGTGAGCGAGCGAGCGCGCAGAGAGGGAGTGGCCAACTCCATCACTAGGGGTTCCTTGT
AGTTAATGATTAACCCGCCATGCTACTTATCTACAAACTCTAGCATGCtgcccatgtaaggaggcaaggcctggg
gacacccgagatgcctggttataattaacccagacatgtggctgcccccccccccccaacacctgctgcctctaaaaataaccctgca
tgccatgttcccggcgaagggccagctgtcccccgccagctagactcagcacttagtttaggaaccagtgagcaagtcagcccttgg
ggcagcccatacaaggccatggggctgggcaagctgcacgcctgggtccggggtgggcacggtgcccgggcaacgagctgaaa
gctcatctgctctcaggggcccctccctggggacagcccctcctggctagtcacaccctgtaggctcctctatataacccaggggcac
aggggctgccctcattctaccaccacctccacagcacagacagacactcaggagccagccagcGTCGAGGTTAACGCCACC
ATGGTGAGCAAGGGCGAGGAGCTGTTCACCGGGGTGGTGCCCATCCTGGTCGAGCTGGACGGCGACG
TAAACGGCCACAAGTTCAGCGTGTCCGGCGAGGGCGAGGGCGATGCCACCTACGGCAAGCTGACCCTG
AAGTTCATCTGCACCACCGGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAAGTCCGCCATGCCCGAAG
GCTACGTCCAGGAGCGCACCATCTTCTTCAAGGACGACGGCAACTACAAGACCCGCGCCGAGGTGAAG
TTCGAGGGCGACACCCTGGTGAACCGCATCGAGCTGAAGGGCATCGACTTCAAGGAGGACGGCAACAT
CCTGGGGCACAAGCTGGAGTACAACTACAACAGCCACAACGTCTATATCATGGCCGACAAGCAGAAGA

ACGGCATCAAGGTGAACTTCAAGATCCGCCACAACATCGAGGACGGCAGCGTGCAGCTCGCCGACCAC
TACCAGCAGAACACCCCCATCGGCGACGGCCCCGTGCTGCTGCCCGACAACCACTACCTGAGCACCCAG
TCCGCCCTGAGCAAAGACCCCAACGAGAAGCGCGATCACATGGTCCTGCTGGAGTTCGTGACCGCCGCC
GGGATCACTCTCGGCATGGACGAGCTGTAC
TCCAAGTCATTCCAGCAGTCATCTCTCAGTAGGGACTCACAGGGTCATGGGCGTGACCTGTCTGCGGC
AGGAATAGGCCTTCTTGCTGCTGCTACCCAGTCTTTAAGTATGCCAGCATCTCTTGGAAGGATGAACCA
GGGTACTGCACGCCTTGCTAGTTTAATGAATCTTGGAATGAGTTCTTCATTGAATCAACAAGGAGCTC
ATAGTGCACTGTCTTCTGCTAGTACTTCTTCCCATAATTTGCAGTCTATATTTAACATTGGAAGTAGAGG
TCCACTCCCTTTATCTTCTCAACACCGTGGAGATGCAGACCAGGCCAGTAACATTTTGGCCAGCTTTGG
TCTGTCTGCTAGAGACTTAGATGAACTGAGTCGTTATCCAGAGGACAAGATTACTCCTGAGAATTTGC
CCCAAATCCTTCTACAGCTTAAAAGGAGGAGAACTGAAGAAGGCCCTACCTTGAGTTATGGTAGAGAT
GGCAGATCTGCTACACGGGAGCCACCATACAGAGTACCTAGGGATGATTGGGAAGAAAAAAGGCAC
TTTAGAAGAGATAGTTTTGATGATCGTGGTCCTAGTCTCAACCCAGTGCTTGATTATGACCATGGAAGT
CGTTCTCAAGAATCTGGTTATTATGACAGAATGGATTATGAAGATGACAGATTAAGAGATGGAGAAA
GGTGTAGGGATGATTCTTTTTTTGGTGAGACCTCGCATAACTATCATAAATTTGACAGTGAGTATGAG
AGAATGGGACGTGGTCCTGGCCCCTTACAAGAGAGATCTCTCTTTGAGAAAAAGAGAGGCGCTCCTCC
AAGTAGCAATATTGAAGACTTCCATGGACTCTTACCGAAGGGTTATCCCCATCTGTGCTCTATATGTGA
TTTGCCAGTTCATTCTAATAAGGAGTGGAGTCAACATATCAATGGAGCAAGTCACAGTCGTCGATGCC
AGCTTCTTCTTGAAATCTACCCAGAATGGAATCCTGACAATGATACAGGACACACAATGGGTGATCCA
TTCATGTTGCAGCAGTCTACAAATCCAGCACCAGGAATTCTGGGACCTCCACCTCCCTCATTTCATCTTG
GGGGACCAGCAGTTGGACCAAGAGGAAATCTGGGTGCTGGAAATGGAAACCTGCAAGGACCTAGAC
ACATGCAGAAAGGCAGAGTGGAAACTAGCAGAGTTGTTCACATCATGGATTTTCAACGAGGGAAAAA
CTTGAGATACCAGCTATTACAGCTGGTAGAACCATTTGGAGTCATTTCAAATCATCTGATTCTAAATAA
AATTAATGAGGCATTTATTGAAATGGCAACCACAGAGGATGCTCAGGCCGCAGTGGATTATTACACA
ACCACACCAGCGTTAGTATTTGGCAAGCCAGTGAGAGTTCATTTATCCCAGAAGTATAAAAGAATAAA
GAAACCTGAAGGAAAGCCAGATCAGAAGTTTGATCAAAAGCAAGAGCTTGGACGTGTGATACATCTC
AGCAATTTGCCGCATTCTGGCTATTCTGATAGTGCTGTTCTCAAGCTTGCTGAGCCTTATGGGAAAATA
AAGAATTACATATTGATGAGGATGAAAAGTCAGGCTTTTATTGAGATGGAGACAAGAGAAGATGCA
ATGGCAATGGTTGACCATTGTTTGAAAAAAGCCCTTTGGTTTCAGGGGAGATGTGTGAAGGTTGACCT
GTCTGAGAAATATAAAAAACTGGTTCTGAGGATTCCAAACAGAGGCATTGATTTACTGAAAAAAGAT
AAATCCCGAAAAAGATCTTACTCTCCAGATGGCAAAGAATCTCCAAGTGATAAGAAATCCAAAACTGA
TGGTTCCCAGAAGACTGAGAGTTCAACCGAAGGTAAAGAACAAGAAGAGAAGTCCGGTGAAGATGG
TGAGAAAGACACAAAGGATGACCAGACAGAGCAGGAACCTAATATGCTTCTTGAATCTGAAGATGAG
CTACTTGTAGATGAAGAAGAAGCAGCAGCACTGCTAGAAAGTGGCAGTTCAGTGGGAGACGAGACC
GATCTTGCTAATTTAGGTGATGTGGCTTCTGATGGGAAAAAGGAACCATCAGATAAAGCTGTGAAAA
AAGATGGAAGTGCTTCAGCAGCAGCAAAGAAAAAGCTTAAAAAGGTGGACAAGATCGAGGAACTTG
ATCAAGAAAACGAAGCAGCGTTGGAAAATGGAATTAAAAATGAGGAAAACACAGAACCAGGTGCTG
AATCTTCTGAGAACGCTGATGATCCCAACAAAGATACAAGTGAAAACGCAGATGGTCAAAGTGATGA
GAACAAGGACGACTATACAATCCCAGATGAGTATAGAATTGGACCATATCAGCCCAATGTTCCTGTTG
GTATAGACTATGTGATACCTAAAACAGGGTTTTACTGTAAGCTGTGTTCACTCTTTTATACAAATGAAG
AAGTTGCAAAGAATACTCATTGCAGCAGCCTTCCTCATTATCAGAAATTAAAGAAATTTCTGAATAAAT
TGGCAGAAGAACGCAGACAGAAGAAGGAAACTTAA
ACCGGTGCGGGCCATTACCAGGCCGAAGCAGCGTTGTTGCAGTGCACGGCAGATACACTTGCTGATGC
GGTGCTGATTACGACCGCTCACGCGTGGCAGCATCAGGGGAAAACCTTATTTATCAGCCGGAAAACCTA
CCGGATTGATGGTAGTGGTCAAATGGCGATTACCGTTGATGTTGAAGTGGCGAGCGATACACCGCATCC
GGCGCGGATTGGCCTGAACTGCCAGCTGGCGCAGGTAGCAGAGCGGGTAAACTGGCTCGGATTAGGG
CCGCAAGAAAACTATCCCGACCGCCTTACTGCCGCCTGTTTTGACCGCTGGGATCTGCCATTGTCAGACA

TGTATACCCCGTACGTCTTCCCGAGCGAAAACGGTCTGCGCTGCGGGACGCGCGAATTGAATTATGGCC
CACACCAGTGGCGCGGCGACTTCCAGTTCAACATCAGCCGCTACAGTCAACAGCAACTGATGGAAACCA
GCCATCGCCATCTGCTGCACGCGGAAGAAGGCACATGGCTGAATATCGACGGTTTCCATATGGGGATTG
GTGGCGACGACTCCTGGAGCCCGTCAGTATCGGCGTAAACCGGT
GGCTAGTAATAAAAGATCCTTATTTTCATTGGATCTGTGTGTTGGTTTTTTGTGTGGGTACCGTTTGTAGA
TAAGTAGCATGGCGGGTTAATCATTAACTACAAGGAACCCCTAGTGATGGAGTTGGCCACTCCCTCTCT
GCGCGCTCGCTCGCTCACTGAGGCCGGGCGACCAAAGGTCGCCCGACGCCCGGGCTTTGCCCGGGCGG
CCTCAGTGAGCGAGCGAGCGCGCAGCTGGCGTAATAGCGAAGAGGCCCGCACCGATCGCCCTTCCCAA
CAGTTGCGCAGCCTGAATGGCGAATGGCGATTCCGTTGCAATGGCTGGCGGTAATATTGTTCTGGATAT
TACCAGCAAGGCCGATAGTTTGAGTTCTTCTACTCAGGCAAGTGATGTTATTACTAATCAAAGAAGTATT
GCGACAACGGTTAATTTGCGTGATGGACAGACTCTTTTACTCGGTGGCCTCACTGATTATAAAAACACTT
CTCAGGATTCTGGCGTACCGTTCCTGTCTAAAATCCCTTTAATCGGCCTCCTGTTTAGCTCCCGCTCTGAT
TCTAACGAGGAAAGCACGTTATACGTGCTCGTCAAAGCAACCATAGTACGCGCCCTGTAGCGGCGCATT
AAGCGCGGCGGGTGTGGTGGTTACGCGCAGCGTGACCGCTACACTTGCCAGCGCCCTAGCGCCCGCTC
CTTTCGCTTTCTTCCCTTCCTTTCTCGCCACGTTCGCCGGCTTTCCCCGTCAAGCTCTAAATCGGGGGCTCC
CTTTAGGGTTCCGATTTAGTGCTTTACGGCACCTCGACCCCAAAAAACTTGATTAGGGTGATGGTTCACG
TAGTGGGCCATCGCCCTGATAGACGGTTTTTCGCCCTTTGACGTTGGAGTCCACGTTCTTTAATAGTGGA
CTCTTGTTCCAAACTGGAACAACACTCAACCCTATCTCGGTCTATTCTTTTGATTTATAAGGGATTTTGCC
GATTTCGGCCTATTGGTTAAAAAATGAGCTGATTTAACAAAAATTTAACGCGAATTTTAACAAAATATTA
ACGTTTACAATTTAAATATTTGCTTATACAATCTTCCTGTTTTTGGGGCTTTTCTGATTATCAACCGGGGTA
CATATGATTGACATGCTAGTTTTACGATTACCGTTCATCGATTCTCTTGTTTGCTCCAGACTCTCAGGCAA
TGACCTGATAGCCTTTGTAGAGACCTCTCAAAAATAGCTACCCTCTCCGGCATGAATTTATCAGCTAGAA
CGGTTGAATATCATATTGATGGTGATTTGACTGTCTCCGGCCTTTCTCACCCGTTTGAATCTTTACCTACA
CATTACTCAGGCATTGCATTTAAAATATATGAGGGTTCTAAAAATTTTTATCCTTGCGTTGAAATAAAGGC
TTCTCCCGCAAAAGTATTACAGGGTCATAATGTTTTTGGTACAACCGATTTAGCTTTATGCTCTGAGGCTT
TATTGCTTAATTTTGCTAATTCTTTGCCTTGCCTGTATGATTTATTGGATGTTGGAATTCCTGATGCGGTAT
TTTCTCCTTACGCATCTGTGCGGTATTTCACACCGCATATGGTGCACTCTCAGTACAATCTGCTCTGATGC
CGCATAGTTAAGCCAGCCCCGACACCCGCCAACACCCGCTGACGCGCCCTGACGGGCTTGTCTGCTCCC
GGCATCCGCTTACAGACAAGCTGTGACCGTCTCCGGGAGCTGCATGTGTCAGAGGTTTTCACCGTCATC
ACCGAAACGCGCGAGACGAAAGGGCCTCGTGATACGCCTATTTTTATAGGTTAATGTCATGATAATAAT
GGTTTCTTAGACGTCAGGTGGCACTTTTCGGGGAAATGTGCGCGGAACCCCTATTTGTTTATTTTTCTAA
ATACATTCAAATATGTATCCGCTCATGAGACAATAACCCTGATAAATGCTTCAATAATATTGAAAAAGGA
AGAGTATGAGTATTCAACATTTCCGTGTCGCCCTTATTCCCTTTTTTGCGGCATTTTGCCTTCCTGTTTTTG
CTCACCCAGAAACGCTGGTGAAAGTAAAAGATGCTGAAGATCAGTTGGGTGCACGAGTGGGTTACATC
GAACTGGATCTCAACAGCGGTAAGATCCTTGAGAGTTTTCGCCCCGAAGAACGTTTTCCAATGATGAGC
ACTTTTAAAGTTCTGCTATGTGGCGCGGTATTATCCCGTATTGACGCCGGGCAAGAGCAACTCGGTCGCC
GCATACACTATTCTCAGAATGACTTGGTTGAGTACTCACCAGTCACAGAAAAGCATCTTACGGATGGCAT
GACAGTAAGAGAATTATGCAGTGCTGCCATAACCATGAGTGATAACACTGCGGCCAACTTACTTCTGAC
AACGATCGGAGGACCGAAGGAGCTAACCGCTTTTTTGCACAACATGGGGGATCATGTAACTCGCCTTGA
TCGTTGGGAACCGGAGCTGAATGAAGCCATACCAAACGACGAGCGTGACACCACGATGCCTGTAGCAA
TGGCAACAACGTTGCGCAAACTATTAACTGGCGAACTACTTACTCTAGCTTCCCGGCAACAATTAATAGA
CTGGATGGAGGCGGATAAAGTTGCAGGACCACTTCTGCGCTCGGCCCTTCCGGCTGGCTGGTTTATTGC
TGATAAATCTGGAGCCGGTGAGCGTGGGTCTCGCGGTATCATTGCAGCACTGGGGCCAGATGGTAAGC
CCTCCCGTATCGTAGTTATCTACACGACGGGGAGTCAGGCAACTATGGATGAACGAAATAGACAGATCG
CTGAGATAGGTGCCTCACTGATTAAGCATTGGTAACTGTCAGACCAAGTTTACTCATATATACTTTAGAT
TGATTTAAAACTTCATTTTTAATTTAAAAGGATCTAGGTGAAGATCCTTTTTGATAATCTCATGACCAAAA
TCCCTTAACGTGAGTTTTCGTTCCACTGAGCGTCAGACCCCGTAGAAAAGATCAAAGGATCTTCTTGAGA

TCCTTTTTTTCTGCGCGTAATCTGCTGCTTGCAAACAAAAAAACCACCGCTACCAGCGGTGGTTTGTTTGC
CGGATCAAGAGCTACCAACTCTTTTTCCGAAGGTAACTGGCTTCAGCAGAGCGCAGATACCAAATACTG
TCCTTCTAGTGTAGCCGTAGTTAGGCCACCACTTCAAGAACTCTGTAGCACCGCCTACATACCTCGCTCTG
CTAATCCTGTTACCAGTGGCTGCTGCCAGTGGCGATAAGTCGTGTCTTACCGGGTTGGACTCAAGACGA
TAGTTACCGGATAAGGCGCAGCGGTCGGGCTGAACGGGGGGTTCGTGCACACAGCCCAGCTTGGAGC
GAACGACCTACACCGAACTGAGATACCTACAGCGTGAGCTATGAGAAAGCGCCACGCTTCCCGAAGGG
AGAAAGGCGGACAGGTATCCGGTAAGCGGCAGGGTCGGAACAGGAGAGCGCACGAGGGAGCTTCCA
GGGGGAAACGCCTGGTATCTTTATAGTCCTGTCGGGTTTCGCCACCTCTGACTTGAGCGTCGATTTTTGT
GATGCTCGTCAGGGGGGCGGAGCCTATGGAAAAACGCCAGCAACGCGGCCTTTTTACGGTTCCTGGCC
TTTTGCTGGCCTTTTGCTCACATGTTCTTTCCTGCGTTATCCCCTGATTCTGTGGATAACCGTATTACCGCC
TTTGAGTGAGCTGATACCGCTCGCCGCAGCCGAACGACCGAGCGCAGCGAGTCAGTGAGCGAGGAAGC
GGAAGAGCGCCCAATACGCAAACCGCCTCTCCCCGCGCGTTGGCCGATTCATTAATG (SEQ ID NO:45)

### Amino acid sequences for

GFP
MVSKGEELFTGVVPILVELDGDVNGHKFSVSGEGEGDATYGKLTLKFICTTGKLPVPWPTLVTTLTYGVQCFS
RYPDHMKQHDFFKSAMPEGYVQERTIFFKDDGNYKTRAEVKFEGDTLVNRIELKGIDFKEDGNILGHKLEYN
YNSHNVYIMADKQKNGIKVNFKIRHNIEDGSVQLADHYQQNTPIGDGPVLLPDNHYLSTQSALSKDPNEKR
DHMVLLEFVTAAGITLGMDELY (SEQ ID NO:46)

### MATR3 (2-847)

SKSFQQSSLSRDSQGHGRDLSAAGIGLLAAATQSLSMPASLGRMNQGTARLASLMNLGMSSSLNQQGA
HSALSSASTSSHNLQSIFNIGSRGPLPLSSQHRGDADQASNILASFGLSARDLDELSRYPEDKITPENLPQILL
QLKRRRTEEGPTLSYGRDGRSATREPPYRVPRDDWEEKRHFRRDSFDDRGPSLNPVLDYDHGSRSQESGY
YDRMDYEDDRLRDGERCRDDSFFGETSHNYHKFDSEYERMGRGPGPLQERSLFEKKRGAPPSSNIEDFHG
LLPKGYPHLCSICDLPVHSNKEWSQHINGASHSRRCQLLLEIYPEWNPDNDTGHTMGDPFMLQQSTNPA
PGILGPPPPSFHLGGPAVGPRGNLGAGNGNLQGPRHMQKGRVETSRVVHIMDFQRGKNLRYQLLQLVE
PFGVISNHLILNKINEAFIEMATTEDAQAAVDYYTTTPALVFGKPVRVHLSQKYKRIKKPEGKPDQKFDQK
QELGRVIHLSNLPHSGYSDSAVLKLAEPYGKIKNYILMRMKSQAFIEMETREDAMAMVDHCLKKALWFQ
GRCVKVDLSEKYKKLVLRIPNRGIDLLKKDKSRKRSYSPDGKESPSDKKSKTDGSQKTESSTEGKEQEEKSGE
DGEKDTKDDQTEQEPNMLLESEDELLVDEEEAAALLESGSSVGDETDLANLGDVASDGKKEPSDKAVKKD
GSASAAAKKKLKKVDKIEELDQENEAALENGIKNEENTEPGAESSENADDPNKDTSENADGQSDENKDDY
TIPDEYRIGPYQPNVPVGIDYVIPKTGFYCKLCSLFYTNEEVAKNTHCSSLPHYQKLKKFLNKLAEERRQKKE
T* (SEQ ID NO:47)

**p604 MATR3 151-287** for the expression in bacteria and protein purification

### Tac promoter

### Lac operator

### GST

### Thrombin site

### MATR3 151-287

ACGTTATCGACTGCACGGTGCACCAATGCTTCTGGCGTCAGGCAGCCATCGGAAGCTGTGGTATGGCTG
TGCAGGTCGTAAATCACTGCATAATTCGTGTCGCTCAAGGCGCACTCCCGTTCTGGATAATGTTTTTTGC
GCCGACATCATAACGGTTCTGGCAAATATTCTGAAATGAGCTGTTGACAATTAATCATCGGCTCGTATAA
TGTGTGGAATTGTGAGCGGATAACAATTTCACACAGGAAACAGTATTCttgacaattaatcatcggctcgtataa

tgTGTGGAATTGTGAGCGGATAACAATTTCACACAGGAAACAGTATTCATGTCCCCTATACTAGGTTATT
GGAAAATTAAGGGCCTTGTGCAACCCACTCGACTTCTTTTGGAATATCTTGAAGAAAAATATGAAGAGC
ATTTGTATGAGCGCGATGAAGGTGATAAATGGCGAAACAAAAAGTTTGAATTGGGTTTGGAGTTTCCCA
ATCTTCCTTATTATATTGATGGTGATGTTAAATTAACACAGTCTATGGCCATCATACGTTATATAGCTGAC
AAGCACAACATGTTGGGTGGTTGTCCAAAAGAGCGTGCAGAGATTTCAATGCTTGAAGGAGCGGTTTT
GGATATTAGATACGGTGTTTCGAGAATTGCATATAGTAAAGACTTTGAAACTCTCAAAGTTGATTTTCTT
AGCAAGCTACCTGAAATGCTGAAAATGTTCGAAGATCGTTTATGTCATAAAACATATTTAAATGGTGATC
ATGTAACCCATCCTGACTTCATGTTGTATGACGCTCTTGATGTTGTTTTATACATGGACCCAATGTGCCTG
GATGCGTTCCCAAAATTAGTTTGTTTTAAAAAACGTATTGAAGCTATCCCACAAATTGATAAGTACTTGA
AATCCAGCAAGTATATAGCATGGCCTTTGCAGGGCTGGCAAGCCACGTTTGGTGGTGGCGACCATCCTC
CAAAATCGGATCTGGTTCCGCGTGGATCTCGTCGTGCATCTGTTGGATCCGAAGAAGGCCCTACCTTGA
GTTATGGTAGAGATGGCAGATCTGCTACACGGGAGCCACCATACAGAGTACCTAGGGATGATTGGGA
AGAAAAAAGGCACTTTAGAAGAGATAGTTTTGATGATCGTGGTCCTAGTCTCAACCCAGTGCTTGATT
ATGACCATGGAAGTCGTTCTCAAGAATCTGGTTATTATGACAGAATGGATTATGAAGATGACAGATTA
AGAGATGGAGAAAGGTGTAGGGATGATTCTTTTTTTGGTGAGACCTCGCATAACTATCATAAATTTGA
CAGTGAGTATGAGAGAATGGGACGTGGTCCTGGCCCCTTACAAGAGAGATCTCTCTTTGAGAAAAAG
AGAGGCGCTCCTCCAAGTAGCAATATTGAAGACTTCCATGGACTCTTACCGAAGTAACCGGGAATTCA
TCGTGACTGACTGACGATCTGCCTCGCGCGTTTCGGTGATGACGGTGAAAACCTCTGACACATGCAGCT
CCCGGAGACGGTCACAGCTTGTCTGTAAGCGGATGCCGGGAGCAGACAAGCCCGTCAGGGCGCGTCAG
CGGGTGTTGGCGGGTGTCGGGGCGCAGCCATGACCCAGTCACGTAGCGATAGCGGAGTGTATAATTCT
TGAAGACGAAAGGGCCTCGTGATACGCCTATTTTTATAGGTTAATGTCATGATAATAATGGTTTCTTAGA
CGTCAGGTGGCACTTTTCGGGGAAATGTGCGCGGAACCCCTATTTGTTTATTTTTCTAAATACATTCAAAT
ATGTATCCGCTCATGAGACAATAACCCTGATAAATGCTTCAATAATATTGAAAAAGGAAGAGTATGAGT
ATTCAACATTTCCGTGTCGCCCTTATTCCCTTTTTTGCGGCATTTTGCCTTCCTGTTTTTGCTCACCCAGAA
ACGCTGGTGAAAGTAAAAGATGCTGAAGATCAGTTGGGTGCACGAGTGGGTTACATCGAACTGGATCT
CAACAGCGGTAAGATCCTTGAGAGTTTTCGCCCCGAAGAACGTTTTCCAATGATGAGCACTTTTAAAGTT
CTGCTATGTGGCGCGGTATTATCCCGTGTTGACGCCGGGCAAGAGCAACTCGGTCGCCGCATACACTAT
TCTCAGAATGACTTGGTTGAGTACTCACCAGTCACAGAAAAGCATCTTACGGATGGCATGACAGTAAGA
GAATTATGCAGTGCTGCCATAACCATGAGTGATAACACTGCGGCCAACTTACTTCTGACAACGATCGGA
GGACCGAAGGAGCTAACCGCTTTTTTGCACAACATGGGGGATCATGTAACTCGCCTTGATCGTTGGGAA
CCGGAGCTGAATGAAGCCATACCAAACGACGAGCGTGACACCACGATGCCTGCAGCAATGGCAACAAC
GTTGCGCAAACTATTAACTGGCGAACTACTTACTCTAGCTTCCCGGCAACAATTAATAGACTGGATGGAG
GCGGATAAAGTTGCAGGACCACTTCTGCGCTCGGCCCTTCCGGCTGGCTGGTTTATTGCTGATAAATCTG
GAGCCGGTGAGCGTGGGTCTCGCGGTATCATTGCAGCACTGGGGCCAGATGGTAAGCCCTCCCGTATC
GTAGTTATCTACACGACGGGGAGTCAGGCAACTATGGATGAACGAAATAGACAGATCGCTGAGATAGG
TGCCTCACTGATTAAGCATTGGTAACTGTCAGACCAAGTTTACTCATATATACTTTAGATTGATTTAAAAC
TTCATTTTTAATTTAAAAGGATCTAGGTGAAGATCCTTTTTGATAATCTCATGACCAAAATCCCTTAACGT
GAGTTTTCGTTCCACTGAGCGTCAGACCCCGTAGAAAAGATCAAAGGATCTTCTTGAGATCCTTTTTTTCT
GCGCGTAATCTGCTGCTTGCAAACAAAAAAACCACCGCTACCAGCGGTGGTTTGTTTGCCGGATCAAGA
GCTACCAACTCTTTTTCCGAAGGTAACTGGCTTCAGCAGAGCGCAGATACCAAATACTGTCCTTCTAGTG
TAGCCGTAGTTAGGCCACCACTTCAAGAACTCTGTAGCACCGCCTACATACCTCGCTCTGCTAATCCTGTT
ACCAGTGGCTGCTGCCAGTGGCGATAAGTCGTGTCTTACCGGGTTGGACTCAAGACGATAGTTACCGGA
TAAGGCGCAGCGGTCGGGCTGAACGGGGGGTTCGTGCACACAGCCCAGCTTGGAGCGAACGACCTACA
CCGAACTGAGATACCTACAGCGTGAGCTATGAGAAAGCGCCACGCTTCCCGAAGGGAGAAAGGCGGAC
AGGTATCCGGTAAGCGGCAGGGTCGGAACAGGAGAGCGCACGAGGGAGCTTCCAGGGGGAAACGCCT
GGTATCTTTATAGTCCTGTCGGGTTTCGCCACCTCTGACTTGAGCGTCGATTTTTGTGATGCTCGTCAGG
GGGGCGGAGCCTATGGAAAAACGCCAGCAACGCGGCCTTTTTACGGTTCCTGGCCTTTTGCTGGCCTTT

TGCTCACATGTTCTTTCCTGCGTTATCCCCTGATTCTGTGGATAACCGTATTACCGCCTTTGAGTGAGCTG
ATACCGCTCGCCGCAGCCGAACGACCGAGCGCAGCGAGTCAGTGAGCGAGGAAGCGGAAGAGCGCCT
GATGCGGTATTTTCTCCTTACGCATCTGTGCGGTATTTCACACCGCATAAATTCCGACACCATCGAATGGT
GCAAAACCTTTCGCGGTATGGCATGATAGCGCCCGGAAGAGAGTCAATTCAGGGTGGTGAATGTGAAA
CCAGTAACGTTATACGATGTCGCAGAGTATGCCGGTGTCTCTTATCAGACCGTTTCCCGCGTGGTGAACC
AGGCCAGCCACGTTTCTGCGAAAACGCGGGAAAAAGTGGAAGCGGCGATGGCGGAGCTGAATTACATT
CCCAACCGCGTGGCACAACAACTGGCGGGCAAACAGTCGTTGCTGATTGGCGTTGCCACCTCCAGTCTG
GCCCTGCACGCGCCGTCGCAAATTGTCGCGGCGATTAAATCTCGCGCCGATCAACTGGGTGCCAGCGTG
GTGGTGTCGATGGTAGAACGAAGCGGCGTCGAAGCCTGTAAAGCGGCGGTGCACAATCTTCTCGCGCA
ACGCGTCAGTGGGCTGATCATTAACTATCCGCTGGATGACCAGGATGCCATTGCTGTGGAAGCTGCCTG
CACTAATGTTCCGGCGTTATTTCTTGATGTCTCTGACCAGACACCCATCAACAGTATTATTTTCTCCCATG
AAGACGGTACGCGACTGGGCGTGGAGCATCTGGTCGCATTGGGTCACCAGCAAATCGCGCTGTTAGCG
GGCCCATTAAGTTCTGTCTCGGCGCGTCTGCGTCTGGCTGGCTGGCATAAATATCTCACTCGCAATCAAA
TTCAGCCGATAGCGGAACGGGAAGGCGACTGGAGTGCCATGTCCGGTTTTCAACAAACCATGCAAATG
CTGAATGAGGGCATCGTTCCCACTGCGATGCTGGTTGCCAACGATCAGATGGCGCTGGGCGCAATGCG
CGCCATTACCGAGTCCGGGCTGCGCGTTGGTGCGGATATCTCGGTAGTGGGATACGACGATACCGAAG
ACAGCTCATGTTATATCCCGCCGTTAACCACCATCAAACAGGATTTTCGCCTGCTGGGGCAAACCAGCGT
GGACCGCTTGCTGCAACTCTCTCAGGGCCAGGCGGTGAAGGGCAATCAGCTGTTGCCCGTCTCACTGGT
GAAAAGAAAAACCACCCTGGCGCCCAATACGCAAACCGCCTCTCCCCGCGCGTTGGCCGATTCATTAAT
GCAGCTGGCACGACAGGTTTCCCGACTGGAAAGCGGGCAGTGAGCGCAACGCAATTAATGTGAGTTAG
CTCACTCATTAGGCACCCCAGGCTTTACACTTTATGCTTCCGGCTCGTATGTTGTGTGGAATTGTGAGCG
GATAACAATTTCACACAGGAAACAGCTATGACCATGATTACGGATTCACTGGCCGTCGTTTTACAACGTC
GTGACTGGGAAAACCCTGGCGTTACCCAACTTAATCGCCTTGCAGCACATCCCCCTTTCGCCAGCTGGCG
TAATAGCGAAGAGGCCCGCACCGATCGCCCTTCCCAACAGTTGCGCAGCCTGAATGGCGAATGGCGCTT
TGCCTGGTTTCCGGCACCAGAAGCGGTGCCGGAAAGCTGGCTGGAGTGCGATCTTCCTGAGGCCGATA
CTGTCGTCGTCCCCTCAAACTGGCAGATGCACGGTTACGATGCGCCCATCTACACCAACGTAACCTATCC
CATTACGGTCAATCCGCCGTTTGTTCCCACGGAGAATCCGACGGGTTGTTACTCGCTCACATTTAATGTT
GATGAAAGCTGGCTACAGGAAGGCCAGACGCGAATTATTTTTGATGGCGTTGGAATT (SEQ ID NO:48)

### Aminoacid sequence:

MSPILGYWKIKGLVQPTRLLLEYLEEKYEEHLYERDEGDKWRNKKFELGLEFPNLPYYIDGDVKLTQSMAIIRYI
ADKHNMLGGCPKERAEISMLEGAVLDIRYGVSRIAYSKDFETLKVDFLSKLPEMLKMFEDRLCHKTYLNGDH
VTHPDFMLYDALDVVLYMDPMCLDAFPKLVCFKKRIEAIPQIDKYLKSSKYIAWPLQGWQATFGGGDHPPK
SDLVPRGSRRASVGSEEGPTLSYGRDGRSATREPPYRVPRDDWEEKRHFRRDSFDDRGPSLNPVLDYDHG
SRSQESGYYDRMDYEDDRLRDGERCRDDSFFGETSHNYHKFDSEYERMGRGPGPLQERSLFEKKRGAPPS
SNIEDFHGLLPK

* (SEQ ID NO:49)

**P645 MATR3 151-287_3xNLS** for the expression in mammalian cells (used for Reporter and Cas 3/7 assay in HEK cells)

### CMV enhancer promoter

### Kozak sequence

### Flag

### MATR3 151-287

### 3xNLS

ATGCATTAGTTATTAATAGTAATCAATTACGGGGTCATTAGTTCATAGCCCATATATGGAGTTCCGcgttac
ataacttacggtaaatggcccgcctggctgaccgcccaacgacccccgcccattgacgtcaataatgacgtatgttcccatagtaac
gccaatagggactttccattgacgtcaatgggtggagtatttacggtaaactgcccacttggcagtacatcaagtgtatcatatgcc
aagtacgccccctattgacgtcaatgacggtaaatggcccgcctggcattatgcccagtacatgaccttatgggactttcctacttgg
cagtacatctacgtattagtcatcgctattaccatggtgatgcggttttggcagtacatcaatgggcgtggatagcggtttgactcac
ggggatttccaagtctccaccccattgacgtcaatgggagtttgttttggcaccaaaatcaacgggactttccaaaatgtcgtaaca
actccgccccattgacgcaaatgggcggtaggcgtgtacggtgggaggtctatataagcagagctGGTTTAGTGAACCGTC
AGATCCGCTAGCGATTACGCCAAGCTCGAAATTAACCCTCACTAAAGGGAACAAAAGCTGGAGCTCCAC
CGCGGTGGCGGCCGCCACCATGGATTACAAGGATGACGACGATAAGAGCCCGGGCggatccGAAGAAG
GCCCTACCTTGAGTTATGGTAGAGATGGCAGATCTGCTACACGGGAGCCACCATACAGAGTACCTAGG
GATGATTGGGAAGAAAAAAGGCACTTTAGAAGAGATAGTTTTGATGATCGTGGTCCTAGTCTCAACC
CAGTGCTTGATTATGACCATGGAAGTCGTTCTCAAGAATCTGGTTATTATGACAGAATGGATTATGAA
GATGACAGATTAAGAGATGGAGAAAGGTGTAGGGATGATTCTTTTTTTGGTGAGACCTCGCATAACT
ATCATAAATTTGACAGTGAGTATGAGAGAATGGGACGTGGTCCTGGCCCCTTACAAGAGAGATCTCTC
TTTGAGAAAAAGAGAGGCGCTCCTCCAAGTAGCAATATTGAAGACTTCCATGGACTCTTACCGAAGAA
GGATCCCAAGAAAAAACGCAAGGTGGAAGATCCTAAGAAAAAGCGGAAAGTGGAAGACCCAAAAAA
GAAGAGAAAGGTAGACTGActcgaggGGGGGCCCGGTACCTTAATTAATTAAGGTACCAGGTAAGTGTA
CCCAATTCGCCCTATAGTGAGTCGTATTACAATTCACTCGATCGCCCTTCCCAACAGTTGCGCAGCCTGA
ATGGCGAATGGAGATCCAATTTTTAAGTGTATAATGTGTTAAACTACTGATTCTAATTGTTTGTGTATTTT
AGATTCACAGTCCCAAGGCTCATTTCAGGCCCCTCAGTCCTCACAGTCTGTTCATGATCATAATCAGCCAT
ACCACATTTGTAGAGGTTTTACTTGCTTTAAAAAACCTCCCACACCTCCCCCTGAACCTGAAACATAAAAT
GAATGCAATTGTTGTTGTTAACTTGTTTATTGCAGCTTATAATGGTTACAAATAAAGCAATAGCATCACA
AATTTCACAAATAAAGCATTTTTTTCACTGCATTCTAGTTGTGGTTTGTCCAAACTCATCAATGTATCTTAA
CGCGTAAATTGTAAGCGTTAATATTTTGTTAAAATTCGCGTTAAATTTTTGTTAAATCAGCTCATTTTTTAA
CCAATAGGCCGAAATCGGCAAAATCCCTTATAAATCAAAAGAATAGACCGAGATAGGGTTGAGTGTTGT
TCCAGTTTGGAACAAGAGTCCACTATTAAAGAACGTGGACTCCAACGTCAAAGGGCGAAAAACCGTCTA
TCAGGGCGATGGCCCACTACGTGAACCATCACCCTAATCAAGTTTTTTGGGGTCGAGGTGCCGTAAAGC
ACTAAATCGGAACCCTAAAGGGAGCCCCCGATTTAGAGCTTGACGGGGAAAGCCGGCGAACGTGGCGA
GAAAGGAAGGGAAGAAAGCGAAAGGAGCGGGCGCTAGGGCGCTGGCAAGTGTAGCGGTCACGCTGC
GCGTAACCACCACACCCGCCGCGCTTAATGCGCCGCTACAGGGCGCGTCAGGTGGCACTTTTCGGGGAA
ATGTGCGCGGAACCCCTATTTGTTTATTTTTCTAAATACATTCAAATATGTATCCGCTCATGAGACAATAA
CCCTGATAAATGCTTCAATAATATTGAAAAAGGAAGAATCCTGAGGCGGAAAGAACCAGCTGTGGAAT
GTGTGTCAGTTAGGGTGTGGAAAGTCCCCAGGCTCCCCAGCAGGCAGAAGTATGCAAAGCATGCATCTC
AATTAGTCAGCAACCAGGTGTGGAAAGTCCCCAGGCTCCCCAGCAGGCAGAAGTATGCAAAGCATGCA
TCTCAATTAGTCAGCAACCATAGTCCCGCCCCTAACTCCGCCCATCCCGCCCCTAACTCCGCCCAGTTCCG
CCCATTCTCCGCCCCATGGCTGACTAATTTTTTTTATTTATGCAGAGGCCGAGGCCGCCTCGGCCTCTGAG
CTATTCCAGAAGTAGTGAGGAGGCTTTTTTGGAGGCCTAGGCTTTTGCAAAGATCGATCAAGAGACAGG
ATGAGGATCGTTTCGCATGATTGAACAAGATGGATTGCACGCAGGTTCTCCGGCCGCTTGGGTGGAGA
GGCTATTCGGCTATGACTGGGCACAACAGACAATCGGCTGCTCTGATGCCGCCGTGTTCCGGCTGTCAG
CGCAGGGGCGCCCGGTTCTTTTTGTCAAGACCGACCTGTCCGGTGCCCTGAATGAACTGCAAGACGAGG
CAGCGCGGCTATCGTGGCTGGCCACGACGGGCGTTCCTTGCGCAGCTGTGCTCGACGTTGTCACTGAAG
CGGGAAGGGACTGGCTGCTATTGGGCGAAGTGCCGGGGCAGGATCTCCTGTCATCTCACCTTGCTCCTG
CCGAGAAAGTATCCATCATGGCTGATGCAATGCGGCGGCTGCATACGCTTGATCCGGCTACCTGCCCAT
TCGACCACCAAGCGAAACATCGCATCGAGCGAGCACGTACTCGGATGGAAGCCGGTCTTGTCGATCAG
GATGATCTGGACGAAGAACATCAGGGGCTCGCGCCAGCCGAACTGTTCGCCAGGCTCAAGGCGAGCAT
GCCCGACGGCGAGGATCTCGTCGTGACCCATGGCGATGCCTGCTTGCCGAATATCATGGTGGAAAATG

GCCGCTTTTCTGGATTCATCGACTGTGGCCGGCTGGGTGTGGCGGACCGCTATCAGGACATAGCGTTGG
CTACCCGTGATATTGCTGAAGAACTTGGCGGCGAATGGGCTGACCGCTTCCTCGTGCTTTACGGTATCGC
CGCTCCCGATTCGCAGCGCATCGCCTTCTATCGCCTTCTTGACGAGTTCTTCTGAGCGGGACTCTGGGGT
TCGAAATGACCGACCAAGCGACGCCCAACCTGCCATCACGAGATTTCGATTCCACCGCCGCCTTCTATGA
AAGGTTGGGCTTCGGAATCGTTTTCCGGGACGCCGGCTGGATGATCCTCCAGCGCGGGGATCTCATGCT
GGAGTTCTTCGCCCACCCTAGGGGGAGGCTAACTGAAACACGGAAGGAGACAATACCGGAAGGAACCC
GCGCTATGACGGCAATAAAAAGACAGAATAAAACGCACGGTGTTGGGTCGTTTGTTCATAAACGCGGG
GTTCGGTCCCAGGGCTGGCACTCTGTCGATACCCCACCGAGACCCCATTGGGGCCAATACGCCCGCGTT
TCTTCCTTTTCCCCACCCCACCCCCCAAGTTCGGGTGAAGGCCCAGGGCTCGCAGCCAACGTCGGGGCG
GCAGGCCCTGCCATAGCCTCAGGTTACTCATATATACTTTAGATTGATTTAAAACTTCATTTTTAATTTAA
AAGGATCTAGGTGAAGATCCTTTTTGATAATCTCATGACCAAAATCCCTTAACGTGAGTTTTCGTTCCACT
GAGCGTCAGACCCCGTAGAAAAGATCAAAGGATCTTCTTGAGATCCTTTTTTTCTGCGCGTAATCTGCTG
CTTGCAAACAAAAAAACCACCGCTACCAGCGGTGGTTTGTTTGCCGGATCAAGAGCTACCAACTCTTTTT
CCGAAGGTAACTGGCTTCAGCAGAGCGCAGATACCAAATACTGTCCTTCTAGTGTAGCCGTAGTTAGGC
CACCACTTCAAGAACTCTGTAGCACCGCCTACATACCTCGCTCTGCTAATCCTGTTACCAGTGGCTGCTGC
CAGTGGCGATAAGTCGTGTCTTACCGGGTTGGACTCAAGACGATAGTTACCGGATAAGGCGCAGCGGT
CGGGCTGAACGGGGGGTTCGTGCACACAGCCCAGCTTGGAGCGAACGACCTACACCGAACTGAGATAC
CTACAGCGTGAGCTATGAGAAAGCGCCACGCTTCCCGAAGGGAGAAAGGCGGACAGGTATCCGGTAA
GCGGCAGGGTCGGAACAGGAGAGCGCACGAGGGAGCTTCCAGGGGGAAACGCCTGGTATCTTTATAG
TCCTGTCGGGTTTCGCCACCTCTGACTTGAGCGTCGATTTTTGTGATGCTCGTCAGGGGGGCGGAGCCTA
TGGAAAAACGCCAGCAACGCGGCCTTTTTACGGTTCCTGGCCTTTTGCTGGCCTTTTGCTCACATGTTCTT
TCCTGCGTTATCCCCTGATTCTGTGGATAACCGTATTACCGCC (SEQ ID NO:50)

### Aminoacid sequence:

MDYKDDDDKSPGGSEEGPTLSYGRDGRSATREPPYRVPRDDWEEKRHFRRDSFDDRGPSLNPVLDYDHG
SRSQESGYYDRMDYEDDRLRDGERCRDDSFFGETSHNYHKFDSEYERMGRGPGPLQERSLFEKKRGAPPS
SNIEDFHGLLPKKDPKKKRKVEDPKKKRKVEDPKKKRKVD* (SEQ ID NO:51)

**p656 GFP_MATR3 151-287_3xNLS** to generate lentivirus, used for gene expression in FSHD primary cells

### UbC promoter

### Kozak sequence

### EGFP

### MATR3 151-287

### 3xNLS

GTCGACGGATCGGGAGATCTCCCGATCCCCTATGGTGCACTCTCAGTACAATCTGCTCTGATGCCGCATA
GTTAAGCCAGTATCTGCTCCCTGCTTGTGTGTTGGAGGTCGCTGAGTAGTGCGCGAGCAAAATTTAAGC
TACAACAAGGCAAGGCTTGACCGACAATTGCATGAAGAATCTGCTTAGGGTTAGGCGTTTTGCGCTGCT
TCGCGATGTACGGGCCAGATATACGCGTTGACATTGATTATTGACTAGTTATTAATAGTAATCAATTACG
GGGTCATTAGTTCATAGCCCATATATGGAGTTCCGCGTTACATAACTTACGGTAAATGGCCCGCCTGGCT
GACCGCCCAACGACCCCCGCCCATTGACGTCAATAATGACGTATGTTCCCATAGTAACGCCAATAGGGA
CTTTCCATTGACGTCAATGGGTGGAGTATTTACGGTAAACTGCCCACTTGGCAGTACATCAAGTGTATCA
TATGCCAAGTACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGGTGATGCGGTT

TTGGCAGTACATCAATGGGCGTGGATAGCGGTTTGACTCACGGGGATTTCCAAGTCTCCACCCCATTGA
CGTCAATGGGAGTTTGTTTTGGCACCAAAATCAACGGGACTTTCCAAAATGTCGTAACAACTCCGCCCCA
TTGACGCAAATGGGCGGTAGGCGTGTACGGTGGGAGGTCTATATAAGCAGCGCGTTTTGCCTGTACTG
GGTCTCTCTGGTTAGACCAGATCTGAGCCTGGGAGCTCTCTGGCTAACTAGGGAACCCACTGCTTAAGC
CTCAATAAAGCTTGCCTTGAGTGCTTCAAGTAGTGTGTGCCCGTCTGTTGTGTGACTCTGGTAACTAGAG
ATCCCTCAGACCCTTTTAGTCAGTGTGGAAAATCTCTAGCAGTGGCGCCCGAACAGGGACTTGAAAGCG
AAAGGGAAACCAGAGGAGCTCTCTCGACGCAGGACTCGGCTTGCTGAAGCGCGCACGGCAAGAGGCG
AGGGGCGGCGACTGGTGAGTACGCCAAAAATTTTGACTAGCGGAGGCTAGAAGGAGAGAGATGGGTG
CGAGAGCGTCAGTATTAAGCGGGGGAGAATTAGATCGCGATGGGAAAAAATTCGGTTAAGGCCAGGG
GGAAAGAAAAAATATAAATTAAAACATATAGTATGGGCAAGCAGGGAGCTAGAACGATTCGCAGTTAA
TCCTGGCCTGTTAGAAACATCAGAAGGCTGTAGACAAATACTGGGACAGCTACAACCATCCCTTCAGAC
AGGATCAGAAGAACTTAGATCATTATATAATACAGTAGCAACCCTCTATTGTGTGCATCAAAGGATAGA
GATAAAAGACACCAAGGAAGCTTTAGACAAGATAGAGGAAGAGCAAAACAAAAGTAAGACCACCGCA
CAGCAAGCGGCCGCTGATCTTCAGACCTGGAGGAGGAGATATGAGGGACAATTGGAGAAGTGAATTAT
ATAAATATAAAGTAGTAAAAATTGAACCATTAGGAGTAGCACCCACCAAGGCAAAGAGAAGAGTGGTG
CAGAGAGAAAAAAGAGCAGTGGGAATAGGAGCTTTGTTCCTTGGGTTCTTGGGAGCAGCAGGAAGCAC
TATGGGCGCAGCGTCAATGACGCTGACGGTACAGGCCAGACAATTATTGTCTGGTATAGTGCAGCAGCA
GAACAATTTGCTGAGGGCTATTGAGGCGCAACAGCATCTGTTGCAACTCACAGTCTGGGGCATCAAGCA
GCTCCAGGCAAGAATCCTGGCTGTGGAAAGATACCTAAAGGATCAACAGCTCCTGGGGATTTGGGGTT
GCTCTGGAAAACTCATTTGCACCACTGCTGTGCCTTGGAATGCTAGTTGGAGTAATAAATCTCTGGAACA
GATTTGGAATCACACGACCTGGATGGAGTGGGACAGAGAAATTAACAATTACACAAGCTTAATACACTC
CTTAATTGAAGAATCGCAAAACCAGCAAGAAAAGAATGAACAAGAATTATTGGAATTAGATAAATGGG
CAAGTTTGTGGAATTGGTTTAACATAACAAATTGGCTGTGGTATATAAAATTATTCATAATGATAGTAGG
AGGCTTGGTAGGTTTAAGAATAGTTTTTGCTGTACTTTCTATAGTGAATAGAGTTAGGCAGGGATATTCA
CCATTATCGTTTCAGACCCACCTCCCAACCCCGAGGGGACCCGACAGGCCCGAAGGAATAGAAGAAGA
AGGTGGAGAGAGAGACAGAGACAGATCCATTCGATTAGTGAACGGATCGGCACTGCGTGCGCCAATTC
TGCAGACAAATGGCAGTATTCATCCACAATTTTAAAAGAAAAGGGGGGATTGGGGGGTACAGTGCAGG
GGAAAGAATAGTAGACATAATAGCAACAGACATACAAACTAAAGAATTACAAAAACAAATTACAAAAAT
TCAAAATTTTCGGGTTTATTACAGGGACAGCAGAGATCCAGTTTGGTTAATTAACCCGTGTCGGCTCCAG
ATCTggcctccgcgccgggttttggcgcctcccgcgggcgcccccctcctcacggcgagcgctgccacgtcagacgaagggcgca
gcgagcgtcctgatccttccgcccggacgctcaggacagcggcccgctgctcataagactcggccttagaaccccagtatcagcag
aaggacattttaggacgggacttgggtgactctagggcactggttttctttccagagagcggaacaggcgaggaaaagtagtccc
ttctcggcgattctgcggagggatctccgtggggcggtgaacgccgatgattatataaggacgcgccgggtgtggcacagctagtt
ccgtcgcagccgggatttgggtcgcggttcttgtttgtggatcgctgtgatcgtcacttggtgagtagcgggctgctgggctggccgg
ggctttcgtggccgccgggccgctcggtgggacggaagcgtgtggagagaccgccaagggctgtagtctgggtccgcgagcaag
gttgccctgaactgggggttggggggagcgcagcaaaatggcggctgttcccgagtcttgaatggaagacgcttgtgaggcggg
ctgtgaggtcgttgaaacaaggtggggggcatggtgggcggcaagaacccaaggtcttgaggccttcgctaatgcgggaaagct
cttattcgggtgagatgggctggggcaccatctggggaccctgacgtgaagtttgtcactgactggagaactcggtttgtcgtctgtt
gcgggggcggcagttatggcggtgccgttgggcagtgcacccgtacctttgggagcgcgcgccctcgtcgtgtcgtgacgtcaccc
gttctgttggcttataatgcagggtggggccacctgccggtaggtgtgcggtaggcttttctccgtcgcaggacgcagggttcgggc
ctagggtaggctctcctgaatcgacaggcgccggacctctggtgaggggagggataagtgaggcgtcagtttctttggtcggtttta
tgtacctatcttcttaagtagctgaagctccggttttgaactatgcgctcggggttggcgagtgtgttttgtgaagttttttaggcacctt
ttgaaatgtaatcatttgggtcaatatgtaattttcagtgttagactagtaaattgtccgctaaattctggccgtttttggcttttttgtta
gacGAAGCTTGGGCTGCAGGTCGACTCTAGAGGATCCCCGGGTACCGGTCGCCACCATGGTGAGCAAG
GGCGAGGAGCTGTTCACCGGGGTGGTGCCCATCCTGGTCGAGCTGGACGGCGACGTAAACGGCCACAA
GTTCAGCGTGTCCGGCGAGGGCGAGGGCGATGCCACCTACGGCAAGCTGACCCTGAAGTTCATCTGCA
CCACCGGCAAGCTGCCCGTGCCCTGGCCCACCCTCGTGACCACCCTGACCTACGGCGTGCAGTGCTTCA

GCCGCTACCCCGACCACATGAAGCAGCACGACTTCTTCAAGTCCGCCATGCCCGAAGGCTACGTCCAGG
AGCGCACCATCTTCTTCAAGGACGACGGCAACTACAAGACCCGCGCCGAGGTGAAGTTCGAGGGCGAC
ACCCTGGTGAACCGCATCGAGCTGAAGGGCATCGACTTCAAGGAGGACGGCAACATCCTGGGGCACAA
GCTGGAGTACAACTACAACAGCCACAACGTCTATATCATGGCCGACAAGCAGAAGAACGGCATCAAGG
TGAACTTCAAGATCCGCCACAACATCGAGGACGGCAGCGTGCAGCTCGCCGACCACTACCAGCAGAACA
CCCCCATCGGCGACGGCCCCGTGCTGCTGCCCGACAACCACTACCTGAGCACCCAGTCCGCCCTGAGCA
AAGACCCCAACGAGAAGCGCGATCACATGGTCCTGCTGGAGTTCGTGACCGCCGCCGGGATCACTCTCG
GCATGGACGAGCTGTACAAGGAAGAAGGCCCTACCTTGAGTTATGGTAGAGATGGCAGATCTGCTAC
ACGGGAGCCACCATACAGAGTACCTAGGGATGATTGGGAAGAAAAAAGGCACTTTAGAAGAGATAG
TTTTGATGATCGTGGTCCTAGTCTCAACCCAGTGCTTGATTATGACCATGGAAGTCGTTCTCAAGAATC
TGGTTATTATGACAGAATGGATTATGAAGATGACAGATTAAGAGATGGAGAAAGGTGTAGGGATGA
TTCTTTTTTTGGTGAGACCTCGCATAACTATCATAAATTTGACAGTGAGTATGAGAGAATGGGACGTG
GTCCTGGCCCCTTACAAGAGAGATCTCTCTTTGAGAAAAAGAGAGGCGCTCCTCCAAGTAGCAATATT
GAAGACTTCCATGGACTCTTACCGAAGGGTAGGGATCCCAAGAAAAAACGCAAGGTGGAAGATCCTA
AGAAAAAGCGGAAAGTGGAAGACCCAAAAAAGAAGAGAAAGGTAGACTAATGTACAAGTAAAGCGG
CCGCGACTCTAGAATTCGATATCAAGCTTATCGATAATCAACCTCTGGATTACAAAATTTGTGAAAGATT
GACTGGTATTCTTAACTATGTTGCTCCTTTTACGCTATGTGGATACGCTGCTTTAATGCCTTTGTATCATGC
TATTGCTTCCCGTATGGCTTTCATTTTCTCCTCCTTGTATAAATCCTGGTTGCTGTCTCTTTATGAGGAGTT
GTGGCCCGTTGTCAGGCAACGTGGCGTGGTGTGCACTGTGTTTGCTGACGCAACCCCCACTGGTTGGGG
CATTGCCACCACCTGTCAGCTCCTTTCCGGGACTTTCGCTTTCCCCCTCCCTATTGCCACGGCGGAACTCA
TCGCCGCCTGCCTTGCCCGCTGCTGGACAGGGGCTCGGCTGTTGGGCACTGACAATTCCGTGGTGTTGT
CGGGGAAATCATCGTCCTTTCCTTGGCTGCTCGCCTGTGTTGCCACCTGGATTCTGCGCGGGACGTCCTT
CTGCTACGTCCCTTCGGCCCTCAATCCAGCGGACCTTCCTTCCCGCGGCCTGCTGCCGGCTCTGCGGCCT
CTTCCGCGTCTTCGCCTTCGCCCTCAGACGAGTCGGATCTCCCTTTGGGCCGCCTCCCCGCATCGATACCG
TCGACCTCGAGACCTAGAAAAACATGGAGCAATCACAAGTAGCAATACAGCAGCTACCAATGCTGATTG
TGCCTGGCTAGAAGCACAAGAGGAGGAGGAGGTGGGTTTTCCAGTCACACCTCAGGTACCTTTAAGAC
CAATGACTTACAAGGCAGCTGTAGATCTTAGCCACTTTTTAAAAGAAAAGGGGGGACTGGAAGGGCTA
ATTCACTCCCAACGAAGACAAGATATCCTTGATCTGTGGATCTACCACACACAAGGCTACTTCCCTGATT
GGCAGAACTACACACCAGGGCCAGGGATCAGATATCCACTGACCTTTGGATGGTGCTACAAGCTAGTAC
CAGTTGAGCAAGAGAAGGTAGAAGAAGCCAATGAAGGAGAGAACACCCGCTTGTTACACCCTGTGAGC
CTGCATGGGATGGATGACCCGGAGAGAGAAGTATTAGAGTGGAGGTTTGACAGCCGCCTAGCATTTCA
TCACATGGCCCGAGAGCTGCATCCGGACTGTACTGGGTCTCTCTGGTTAGACCAGATCTGAGCCTGGGA
GCTCTCTGGCTAACTAGGGAACCCACTGCTTAAGCCTCAATAAAGCTTGCCTTGAGTGCTTCAAGTAGTG
TGTGCCCGTCTGTTGTGTGACTCTGGTAACTAGAGATCCCTCAGACCCTTTTAGTCAGTGTGGAAAATCT
CTAGCAGGGCCCGTTTAAACCCGCTGATCAGCCTCGACTGTGCCTTCTAGTTGCCAGCCATCTGTTGTTT
GCCCCTCCCCCGTGCCTTCCTTGACCCTGGAAGGTGCCACTCCCACTGTCCTTTCCTAATAAAATGAGGA
AATTGCATCGCATTGTCTGAGTAGGTGTCATTCTATTCTGGGGGGTGGGGTGGGGCAGGACAGCAAGG
GGGAGGATTGGGAAGACAATAGCAGGCATGCTGGGGATGCGGTGGGCTCTATGGCTTCTGAGGCGGA
AAGAACCAGCTGGGGCTCTAGGGGGTATCCCCACGCGCCCTGTAGCGGCGCATTAAGCGCGGCGGGTG
TGGTGGTTACGCGCAGCGTGACCGCTACACTTGCCAGCGCCCTAGCGCCCGCTCCTTTCGCTTTCTTCCCT
TCCTTTCTCGCCACGTTCGCCGGCTTTCCCCGTCAAGCTCTAAATCGGGGGCTCCCTTTAGGGTTCCGATT
TAGTGCTTTACGGCACCTCGACCCCAAAAAACTTGATTAGGGTGATGGTTCACGTAGTGGGCCATCGCC
CTGATAGACGGTTTTTCGCCCTTTGACGTTGGAGTCCACGTTCTTTAATAGTGGACTCTTGTTCCAAACTG
GAACAACACTCAACCCTATCTCGGTCTATTCTTTTGATTTATAAGGGATTTTGCCGATTTCGGCCTATTGG
TTAAAAAATGAGCTGATTTAACAAAAATTTAACGCGAATTAATTCTGTGGAATGTGTGTCAGTTAGGGTG
TGGAAAGTCCCCAGGCTCCCCAGCAGGCAGAAGTATGCAAAGCATGCATCTCAATTAGTCAGCAACCAG
GTGTGGAAAGTCCCCAGGCTCCCCAGCAGGCAGAAGTATGCAAAGCATGCATCTCAATTAGTCAGCAAC

CATAGTCCCGCCCCTAACTCCGCCCATCCCGCCCCTAACTCCGCCCAGTTCCGCCCATTCTCCGCCCCATG
GCTGACTAATTTTTTTTATTTATGCAGAGGCCGAGGCCGCCTCTGCCTCTGAGCTATTCCAGAAGTAGTG
AGGAGGCTTTTTTGGAGGCCTAGGCTTTTGCAAAAAGCTCCCGGGAGCTTGTATATCCATTTTCGGATCT
GATCAGCACGTGTTGACAATTAATCATCGGCATAGTATATCGGCATAGTATAATACGACAAGGTGAGGA
ACTAAACCATGGCCAAGTTGACCAGTGCCGTTCCGGTGCTCACCGCGCGCGACGTCGCCGGAGCGGTC
GAGTTCTGGACCGACCGGCTCGGGTTCTCCCGGGACTTCGTGGAGGACGACTTCGCCGGTGTGGTCCG
GGACGACGTGACCCTGTTCATCAGCGCGGTCCAGGACCAGGTGGTGCCGGACAACACCCTGGCCTGGG
TGTGGGTGCGCGGCCTGGACGAGCTGTACGCCGAGTGGTCGGAGGTCGTGTCCACGAACTTCCGGGAC
GCCTCCGGGCCGGCCATGACCGAGATCGGCGAGCAGCCGTGGGGGCGGGAGTTCGCCCTGCGCGACC
CGGCCGGCAACTGCGTGCACTTCGTGGCCGAGGAGCAGGACTGACACGTGCTACGAGATTTCGATTCC
ACCGCCGCCTTCTATGAAAGGTTGGGCTTCGGAATCGTTTTCCGGGACGCCGGCTGGATGATCCTCCAG
CGCGGGGATCTCATGCTGGAGTTCTTCGCCCACCCCAACTTGTTTATTGCAGCTTATAATGGTTACAAAT
AAAGCAATAGCATCACAAATTTCACAAATAAAGCATTTTTTTCACTGCATTCTAGTTGTGGTTTGTCCAAA
CTCATCAATGTATCTTATCATGTCTGTATACCGTCGACCTCTAGCTAGAGCTTGGCGTAATCATGGTCATA
GCTGTTTCCTGTGTGAAATTGTTATCCGCTCACAATTCCACACAACATACGAGCCGGAAGCATAAAGTGT
AAAGCCTGGGGTGCCTAATGAGTGAGCTAACTCACATTAATTGCGTTGCGCTCACTGCCCGCTTTCCAGT
CGGGAAACCTGTCGTGCCAGCTGCATTAATGAATCGGCCAACGCGCGGGGAGAGGCGGTTTGCGTATT
GGGCGCTCTTCCGCTTCCTCGCTCACTGACTCGCTGCGCTCGGTCGTTCGGCTGCGGCGAGCGGTATCA
GCTCACTCAAAGGCGGTAATACGGTTATCCACAGAATCAGGGGATAACGCAGGAAAGAACATGTGAGC
AAAAGGCCAGCAAAAGGCCAGGAACCGTAAAAAGGCCGCGTTGCTGGCGTTTTTCCATAGGCTCCGCC
CCCCTGACGAGCATCACAAAAATCGACGCTCAAGTCAGAGGTGGCGAAACCCGACAGGACTATAAAGA
TACCAGGCGTTTCCCCCTGGAAGCTCCCTCGTGCGCTCTCCTGTTCCGACCCTGCCGCTTACCGGATACCT
GTCCGCCTTTCTCCCTTCGGGAAGCGTGGCGCTTTCTCATAGCTCACGCTGTAGGTATCTCAGTTCGGTG
TAGGTCGTTCGCTCCAAGCTGGGCTGTGTGCACGAACCCCCCGTTCAGCCCGACCGCTGCGCCTTATCCG
GTAACTATCGTCTTGAGTCCAACCCGGTAAGACACGACTTATCGCCACTGGCAGCAGCCACTGGTAACA
GGATTAGCAGAGCGAGGTATGTAGGCGGTGCTACAGAGTTCTTGAAGTGGTGGCCTAACTACGGCTAC
ACTAGAAGAACAGTATTTGGTATCTGCGCTCTGCTGAAGCCAGTTACCTTCGGAAAAAGAGTTGGTAGC
TCTTGATCCGGCAAACAAACCACCGCTGGTAGCGGTGGTTTTTTTGTTTGCAAGCAGCAGATTACGCGCA
GAAAAAAAGGATCTCAAGAAGATCCTTTGATCTTTTCTACGGGGTCTGACGCTCAGTGGAACGAAAACT
CACGTTAAGGGATTTTGGTCATGAGATTATCAAAAAGGATCTTCACCTAGATCCTTTTAAATTAAAAATG
AAGTTTTAAATCAATCTAAAGTATATATGAGTAAACTTGGTCTGACAGTTACCAATGCTTAATCAGTGAG
GCACCTATCTCAGCGATCTGTCTATTTCGTTCATCCATAGTTGCCTGACTCCCCGTCGTGTAGATAACTAC
GATACGGGAGGGCTTACCATCTGGCCCCAGTGCTGCAATGATACCGCGAGACCCACGCTCACCGGCTCC
AGATTTATCAGCAATAAACCAGCCAGCCGGAAGGGCCGAGCGCAGAAGTGGTCCTGCAACTTTATCCGC
CTCCATCCAGTCTATTAATTGTTGCCGGGAAGCTAGAGTAAGTAGTTCGCCAGTTAATAGTTTGCGCAAC
GTTGTTGCCATTGCTACAGGCATCGTGGTGTCACGCTCGTCGTTTGGTATGGCTTCATTCAGCTCCGGTT
CCCAACGATCAAGGCGAGTTACATGATCCCCCATGTTGTGCAAAAAAGCGGTTAGCTCCTTCGGTCCTCC
GATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGT
GTATGCGGCGACCGAGTTGCTCTTGCCCGGCGTCAATACGGGATAATACCGCGCCACATAGCAGAACTT
TAAAAGTGCTCATCATTGGAAAACGTTCTTCGGGGCGAAAACTCTCAAGGATCTTACCGCTGTTGAGATC
CAGTTCGATGTAACCCACTCGTGCACCCAACTGATCTTCAGCATCTTTTACTTTCACCAGCGTTTCTGGGT
GAGCAAAAACAGGAAGGCAAAATGCCGCAAAAAAGGGAATAAGGGCGACACGGAAATGTTGAATACT
CATACTCTTCCTTTTTCAATATTATTGAAGCATTTATCAGGGTTATTGTCTCATGAGCGGATACATATTTGA
ATGTATTTAGAAAAATAAACAAATAGGGGTTCCGCGCACATTTCCCCGAAAAGTGCCACCTGAC (SEQ ID
NO:52)

### Aminoacid sequence:

MVSKGEELFTGVVPILVELDGDVNGHKFSVSGEGEGDATYGKLTLKFICTTGKLPVPWPTLVTTLTYGVQCFS
RYPDHMKQHDFFKSAMPEGYVQERTIFFKDDGNYKTRAEVKFEGDTLVNRIELKGIDFKEDGNILGHKLEYN
YNSHNVYIMADKQKNGIKVNFKIRHNIEDGSVQLADHYQQNTPIGDGPVLLPDNHYLSTQSALSKDPNEKR
DHMVLLEFVTAAGITLGMDELYKEEGPTLSYGRDGRSATREPPYRVPRDDWEEKRHFRRDSFDDRGPSLN
PVLDYDHGSRSQESGYYDRMDYEDDRLRDGERCRDDSFFGETSHNYHKFDSEYERMGRGPGPLQERSLF
EKKRGAPPSSNIEDFHGLLPKGRDPKKKRKVEDPKKKRKVEDPKKKRKVD* (SEQ ID NO:53)

**P691 GST_DELIVERY SYSTEM_MATR3 210-255 WITH PROTAC** for expression in bacteria and protein purification

### Tac promoter

### Lac operator

### GST

Thrombin site (cutting site indicated by | in the aa sequence)

### Delivery system MATR3 210-255 + PROTAC

### NLS

### 6xHis

ACGTTATCGACTGCACGGTGCACCAATGCTTCTGGCGTCAGGCAGCCATCGGAAGCTGTGGTATGGCTG
TGCAGGTCGTAAATCACTGCATAATTCGTGTCGCTCAAGGCGCACTCCCGTTCTGGATAATGTTTTTTGC
GCCGACATCATAACGGTTCTGGCAAATATTCTGAAATGAGCTGTTGACAATTAATCATCGGCTCGTATAA
TGTGTGGAAttgtgagcggataacaaTTTCACACAGGAAACAGTATTCATGTCCCCTATACTAGGTTATTGGA
AAATTAAGGGCCTTGTGCAACCCACTCGACTTCTTTTGGAATATCTTGAAGAAAAATATGAAGAGCATTT
GTATGAGCGCGATGAAGGTGATAAATGGCGAAACAAAAAGTTTGAATTGGGTTTGGAGTTTCCCAATCT
TCCTTATTATATTGATGGTGATGTTAAATTAACACAGTCTATGGCCATCATACGTTATATAGCTGACAAGC
ACAACATGTTGGGTGGTTGTCCAAAAGAGCGTGCAGAGATTTCAATGCTTGAAGGAGCGGTTTTGGATA
TTAGATACGGTGTTTCGAGAATTGCATATAGTAAAGACTTTGAAACTCTCAAAGTTGATTTTCTTAGCAA
GCTACCTGAAATGCTGAAAATGTTCGAAGATCGTTTATGTCATAAAACATATTTAAATGGTGATCATGTA
ACCCATCCTGACTTCATGTTGTATGACGCTCTTGATGTTGTTTTATACATGGACCCAATGTGCCTGGATGC
GTTCCCAAAATTAGTTTGTTTTAAAAAACGTATTGAAGCTATCCCACAAATTGATAAGTACTTGAAATCCA
GCAAGTATATAGCATGGCCTTTGCAGGGCTGGCAAGCCACGTTTGGTGGTGGCGACCATCCTCCAAAAT
CGGATCTGGTTCCGCGTGGATCTGGCCGCAAAAAACGCCGCCAGCGCCGCCCGCCGCAGGGCCTG
TTTGAAGCGATTGAAGGCTTTATTGAAAACGGCTGGGAAGGCATGATTGATGGCTGGTATGGCAACA
ACACCCATGATCTGGTGGGCGATGTGCGCCTGGCGGGCGTGCAGAGCGTGGCGAGCAGCCGCCGCCA
TAAACGCTTTGCGGGCGTGGGCGATCGCATGAAACAGATTGAAGATAAAATTGAAGAAATTCTGAGC
AAAATTTATCCGAAAAAAAAACGCAAAGTGCATATTGAAAACGAAATTGCGCGCATTAAAAAACTGA
TTGGCGAACGCGGCAGCGGCAGCGAAAGCGGCTATTATGATCGCATGGATTATGAAGATGATCGCCT
GCGCGATGGCGAACGCTGCCGCGATGATAGCTTTTTTGGCGAAACCAGCCATAACTATCATAAATTTG
ATAGCGAATATGAACGCATGGGCCGCGGCAGCGGCAGCGCGCTGGCGCCGTATATTCCGCCGAAAAA
AAAACGCAAAGTGCATCATCATCATCATCATTAAGAATTCATCGTGACTGACTGACGATCTGCCTCGCGC
GTTTCGGTGATGACGGTGAAAACCTCTGACACATGCAGCTCCCGGAGACGGTCACAGCTTGTCTGTAAG
CGGATGCCGGGAGCAGACAAGCCCGTCAGGGCGCGTCAGCGGGTGTTGGCGGGTGTCGGGGCGCAGC
CATGACCCAGTCACGTAGCGATAGCGGAGTGTATAATTCTTGAAGACGAAAGGGCCTCGTGATACGCCT
ATTTTTATAGGTTAATGTCATGATAATAATGGTTTCTTAGACGTCAGGTGGCACTTTTCGGGGAAATGTG
CGCGGAACCCCTATTTGTTTATTTTTCTAAATACATTCAAATATGTATCCGCTCATGAGACAATAACCCTG

ATAAATGCTTCAATAATATTGAAAAAGGAAGAGTATGAGTATTCAACATTTCCGTGTCGCCCTTATTCCCT
TTTTTGCGGCATTTTGCCTTCCTGTTTTTGCTCACCCAGAAACGCTGGTGAAAGTAAAAGATGCTGAAGA
TCAGTTGGGTGCACGAGTGGGTTACATCGAACTGGATCTCAACAGCGGTAAGATCCTTGAGAGTTTTCG
CCCCGAAGAACGTTTTCCAATGATGAGCACTTTTAAAGTTCTGCTATGTGGCGCGGTATTATCCCGTGTT
GACGCCGGGCAAGAGCAACTCGGTCGCCGCATACACTATTCTCAGAATGACTTGGTTGAGTACTCACCA
GTCACAGAAAAGCATCTTACGGATGGCATGACAGTAAGAGAATTATGCAGTGCTGCCATAACCATGAGT
GATAACACTGCGGCCAACTTACTTCTGACAACGATCGGAGGACCGAAGGAGCTAACCGCTTTTTTGCAC
AACATGGGGGATCATGTAACTCGCCTTGATCGTTGGGAACCGGAGCTGAATGAAGCCATACCAAACGA
CGAGCGTGACACCACGATGCCTGCAGCAATGGCAACAACGTTGCGCAAACTATTAACTGGCGAACTACT
TACTCTAGCTTCCCGGCAACAATTAATAGACTGGATGGAGGCGGATAAAGTTGCAGGACCACTTCTGCG
CTCGGCCCTTCCGGCTGGCTGGTTTATTGCTGATAAATCTGGAGCCGGTGAGCGTGGGTCTCGCGGTAT
CATTGCAGCACTGGGGCCAGATGGTAAGCCCTCCCGTATCGTAGTTATCTACACGACGGGGAGTCAGGC
AACTATGGATGAACGAAATAGACAGATCGCTGAGATAGGTGCCTCACTGATTAAGCATTGGTAACTGTC
AGACCAAGTTTACTCATATATACTTTAGATTGATTTAAAACTTCATTTTTAATTTAAAAGGATCTAGGTGA
AGATCCTTTTTGATAATCTCATGACCAAAATCCCTTAACGTGAGTTTTCGTTCCACTGAGCGTCAGACCCC
GTAGAAAAGATCAAAGGATCTTCTTGAGATCCTTTTTTTCTGCGCGTAATCTGCTGCTTGCAAACAAAAA
AACCACCGCTACCAGCGGTGGTTTGTTTGCCGGATCAAGAGCTACCAACTCTTTTTCCGAAGGTAACTGG
CTTCAGCAGAGCGCAGATACCAAATACTGTCCTTCTAGTGTAGCCGTAGTTAGGCCACCACTTCAAGAAC
TCTGTAGCACCGCCTACATACCTCGCTCTGCTAATCCTGTTACCAGTGGCTGCTGCCAGTGGCGATAAGT
CGTGTCTTACCGGGTTGGACTCAAGACGATAGTTACCGGATAAGGCGCAGCGGTCGGGCTGAACGGGG
GGTTCGTGCACACAGCCCAGCTTGGAGCGAACGACCTACACCGAACTGAGATACCTACAGCGTGAGCTA
TGAGAAAGCGCCACGCTTCCCGAAGGGAGAAAGGCGGACAGGTATCCGGTAAGCGGCAGGGTCGGAA
CAGGAGAGCGCACGAGGGAGCTTCCAGGGGGAAACGCCTGGTATCTTTATAGTCCTGTCGGGTTTCGC
CACCTCTGACTTGAGCGTCGATTTTTGTGATGCTCGTCAGGGGGGCGGAGCCTATGGAAAAACGCCAGC
AACGCGGCCTTTTTACGGTTCCTGGCCTTTTGCTGGCCTTTTGCTCACATGTTCTTTCCTGCGTTATCCCCT
GATTCTGTGGATAACCGTATTACCGCCTTTGAGTGAGCTGATACCGCTCGCCGCAGCCGAACGACCGAG
CGCAGCGAGTCAGTGAGCGAGGAAGCGGAAGAGCGCCTGATGCGGTATTTTCTCCTTACGCATCTGTG
CGGTATTTCACACCGCATAAATTCCGACACCATCGAATGGTGCAAAACCTTTCGCGGTATGGCATGATAG
CGCCCGGAAGAGAGTCAATTCAGGGTGGTGAATGTGAAACCAGTAACGTTATACGATGTCGCAGAGTA
TGCCGGTGTCTCTTATCAGACCGTTTCCCGCGTGGTGAACCAGGCCAGCCACGTTTCTGCGAAAACGCG
GGAAAAAGTGGAAGCGGCGATGGCGGAGCTGAATTACATTCCCAACCGCGTGGCACAACAACTGGCG
GGCAAACAGTCGTTGCTGATTGGCGTTGCCACCTCCAGTCTGGCCCTGCACGCGCCGTCGCAAATTGTC
GCGGCGATTAAATCTCGCGCCGATCAACTGGGTGCCAGCGTGGTGGTGTCGATGGTAGAACGAAGCGG
CGTCGAAGCCTGTAAAGCGGCGGTGCACAATCTTCTCGCGCAACGCGTCAGTGGGCTGATCATTAACTA
TCCGCTGGATGACCAGGATGCCATTGCTGTGGAAGCTGCCTGCACTAATGTTCCGGCGTTATTTCTTGAT
GTCTCTGACCAGACACCCATCAACAGTATTATTTTCTCCCATGAAGACGGTACGCGACTGGGCGTGGAG
CATCTGGTCGCATTGGGTCACCAGCAAATCGCGCTGTTAGCGGGCCCATTAAGTTCTGTCTCGGCGCGTC
TGCGTCTGGCTGGCTGGCATAAATATCTCACTCGCAATCAAATTCAGCCGATAGCGGAACGGGAAGGCG
ACTGGAGTGCCATGTCCGGTTTTCAACAAACCATGCAAATGCTGAATGAGGGCATCGTTCCCACTGCGA
TGCTGGTTGCCAACGATCAGATGGCGCTGGGCGCAATGCGCGCCATTACCGAGTCCGGGCTGCGCGTT
GGTGCGGATATCTCGGTAGTGGGATACGACGATACCGAAGACAGCTCATGTTATATCCCGCCGTTAACC
ACCATCAAACAGGATTTTCGCCTGCTGGGGCAAACCAGCGTGGACCGCTTGCTGCAACTCTCTCAGGGC
CAGGCGGTGAAGGGCAATCAGCTGTTGCCCGTCTCACTGGTGAAAAGAAAAACCACCCTGGCGCCCAA
TACGCAAACCGCCTCTCCCCGCGCGTTGGCCGATTCATTAATGCAGCTGGCACGACAGGTTTCCCGACTG
GAAAGCGGGCAGTGAGCGCAACGCAATTAATGTGAGTTAGCTCACTCATTAGGCACCCCAGGCTTTACA
CTTTATGCTTCCGGCTCGTATGTTGTGTGGAATTGTGAGCGGATAACAATTTCACACAGGAAACAGCTAT
GACCATGATTACGGATTCACTGGCCGTCGTTTTACAACGTCGTGACTGGGAAAACCCTGGCGTTACCCA

ACTTAATCGCCTTGCAGCACATCCCCCTTTCGCCAGCTGGCGTAATAGCGAAGAGGCCCGCACCGATCGC
CCTTCCCAACAGTTGCGCAGCCTGAATGGCGAATGGCGCTTTGCCTGGTTTCCGGCACCAGAAGCGGTG
CCGGAAAGCTGGCTGGAGTGCGATCTTCCTGAGGCCGATACTGTCGTCGTCCCCTCAAACTGGCAGATG
CACGGTTACGATGCGCCCATCTACACCAACGTAACCTATCCCATTACGGTCAATCCGCCGTTTGTTCCCAC
GGAGAATCCGACGGGTTGTTACTCGCTCACATTTAATGTTGATGAAAGCTGGCTACAGGAAGGCCAGAC
GCGAATTATTTTTGATGGCGTTGGAATT (SEQ ID NO:54)

### Aminoacid sequence:

MSPILGYWKIKGLVQPTRLLLEYLEEKYEEHLYERDEGDKWRNKKFELGLEFPNLPYYIDGDVKLTQSMAIIRYI
ADKHNMLGGCPKERAEISMLEGAVLDIRYGVSRIAYSKDFETLKVDFLSKLPEMLKMFEDRLCHKTYLNGDH
VTHPDFMLYDALDVVLYMDPMCLDAFPKLVCFKKRIEAIPQIDKYLKSSKYIAWPLQGWQATFGGGDHPPK
SDLVPR|GSGRKKRRQRRRPPQGLFEAIEGFIENGWEGMIDGWYGNNTHDLVGDVRLAGVQSVASSRR
HKRFAGVGDRMKQIEDKIEEILSKIYHIENEIARIKKLIGERGSGSESGYYDRMDYEDDRLRDGERCRDDSFF
GETSHNYHKFDSEYERMGRGSGSALAPYIPPKKKRKVHHHHHH* (SEQ ID NO:55)

### Specific regions of the peptide:

### TAT (cell penetrating peptide)

### INF7

### N

### Ne

### Leu-Zipper

### MATR3₂₁₀₋₂₅₅ peptide flanked by linkers

### VHL recruiting seguence (PROTAC)

### NLS

### 6xHis

MGRKKRRQRRRPPQGLFEAIEGFIENGWEGMIDGWYGNNTHDLVGDVRLAGVQSVASSRRHKRFAGVGD
RMKQIEDKIEEILSKIYHIENEIARIKKLIGERGSGSESGYYDRMDYEDDRLRDGERCRDDSFFGETSHNYHKF
DSEYERMGRGSGSALAPYIPPKKKRKVHHHHHH* (SEQ ID NO:56)

**P692 GST_DELIVERY SYSTEM**_**MATR3 210-255 WITHOUT PROTAC** for expression in bacteria and protein purification

### Tac promoter

### Lac operator

### GST

### Thrombin site (cutting site indicated by | in the aa sequence)

### Delivery system MATR3 210-255 (no PROTAC)

### NLS

### 6xHis

ACGTTATCGACTGCACGGTGCACCAATGCTTCTGGCGTCAGGCAGCCATCGGAAGCTGTGGTATGGCTG
TGCAGGTCGTAAATCACTGCATAATTCGTGTCGCTCAAGGCGCACTCCCGTTCTGGATAATGTTTTTTGC
GCCGACATCATAACGGTTCTGGCAAATATTCTGAAATGAGCTGTTGACAATTAATCATCGGCTCGTATAA
TGTGTGGAAttgtgagcggataacaaTTTCACACAGGAAACAGTATTCATGTCCCCTATACTAGGTTATTGGA

AAATTAAGGGCCTTGTGCAACCCACTCGACTTCTTTTGGAATATCTTGAAGAAAAATATGAAGAGCATTT
GTATGAGCGCGATGAAGGTGATAAATGGCGAAACAAAAAGTTTGAATTGGGTTTGGAGTTTCCCAATCT
TCCTTATTATATTGATGGTGATGTTAAATTAACACAGTCTATGGCCATCATACGTTATATAGCTGACAAGC
ACAACATGTTGGGTGGTTGTCCAAAAGAGCGTGCAGAGATTTCAATGCTTGAAGGAGCGGTTTTGGATA
TTAGATACGGTGTTTCGAGAATTGCATATAGTAAAGACTTTGAAACTCTCAAAGTTGATTTTCTTAGCAA
GCTACCTGAAATGCTGAAAATGTTCGAAGATCGTTTATGTCATAAAACATATTTAAATGGTGATCATGTA
ACCCATCCTGACTTCATGTTGTATGACGCTCTTGATGTTGTTTTATACATGGACCCAATGTGCCTGGATGC
GTTCCCAAAATTAGTTTGTTTTAAAAAACGTATTGAAGCTATCCCACAAATTGATAAGTACTTGAAATCCA
GCAAGTATATAGCATGGCCTTTGCAGGGCTGGCAAGCCACGTTTGGTGGTGGCGACCATCCTCCAAAAT
CGGATCTGGTTCCGCGTGGATCTGGCCGCAAAAAACGCCGCCAGCGCCGCCGCCCGCCGCAGGGCCTG
TTTGAAGCGATTGAAGGCTTTATTGAAAACGGCTGGGAAGGCATGATTGATGGCTGGTATGGCAACA
ACACCCATGATCTGGTGGGCGATGTGCGCCTGGCGGGCGTGCAGAGCGTGGCGAGCAGCCGCCGCCA
TAAACGCTTTGCGGGCGTGGGCGATCGCATGAAACAGATTGAAGATAAAATTGAAGAAATTCTGAGC
AAAATTTATCATATTGAAAACGAAATTGCGCGCATTAAAAAACTGATTGGCGAACGCGGCAGCGGCA
GCGAAAGCGGCTATTATGATCGCATGGATTATGAAGATGATCGCCTGCGCGATGGCGAACGCTGCCG
CGATGATAGCTTTTTTGGCGAAACCAGCCATAACTATCATAAATTTGATAGCGAATATGAACGCATGG
GCCGCGGCAGCGGCAGCCCGAAAAAAAAACGCAAAGTGCATCATCATCATCATCATTAAGAATTCATC
GTGACTGACTGACGATCTGCCTCGCGCGTTTCGGTGATGACGGTGAAAACCTCTGACACATGCAGCTCC
CGGAGACGGTCACAGCTTGTCTGTAAGCGGATGCCGGGAGCAGACAAGCCCGTCAGGGCGCGTCAGC
GGGTGTTGGCGGGTGTCGGGGCGCAGCCATGACCCAGTCACGTAGCGATAGCGGAGTGTATAATTCTT
GAAGACGAAAGGGCCTCGTGATACGCCTATTTTTATAGGTTAATGTCATGATAATAATGGTTTCTTAGAC
GTCAGGTGGCACTTTTCGGGGAAATGTGCGCGGAACCCCTATTTGTTTATTTTTCTAAATACATTCAAAT
ATGTATCCGCTCATGAGACAATAACCCTGATAAATGCTTCAATAATATTGAAAAAGGAAGAGTATGAGT
ATTCAACATTTCCGTGTCGCCCTTATTCCCTTTTTTGCGGCATTTTGCCTTCCTGTTTTTGCTCACCCAGAA
ACGCTGGTGAAAGTAAAAGATGCTGAAGATCAGTTGGGTGCACGAGTGGGTTACATCGAACTGGATCT
CAACAGCGGTAAGATCCTTGAGAGTTTTCGCCCCGAAGAACGTTTTCCAATGATGAGCACTTTTAAAGTT
CTGCTATGTGGCGCGGTATTATCCCGTGTTGACGCCGGGCAAGAGCAACTCGGTCGCCGCATACACTAT
TCTCAGAATGACTTGGTTGAGTACTCACCAGTCACAGAAAAGCATCTTACGGATGGCATGACAGTAAGA
GAATTATGCAGTGCTGCCATAACCATGAGTGATAACACTGCGGCCAACTTACTTCTGACAACGATCGGA
GGACCGAAGGAGCTAACCGCTTTTTTGCACAACATGGGGGATCATGTAACTCGCCTTGATCGTTGGGAA
CCGGAGCTGAATGAAGCCATACCAAACGACGAGCGTGACACCACGATGCCTGCAGCAATGGCAACAAC
GTTGCGCAAACTATTAACTGGCGAACTACTTACTCTAGCTTCCCGGCAACAATTAATAGACTGGATGGAG
GCGGATAAAGTTGCAGGACCACTTCTGCGCTCGGCCCTTCCGGCTGGCTGGTTTATTGCTGATAAATCTG
GAGCCGGTGAGCGTGGGTCTCGCGGTATCATTGCAGCACTGGGGCCAGATGGTAAGCCCTCCCGTATC
GTAGTTATCTACACGACGGGGAGTCAGGCAACTATGGATGAACGAAATAGACAGATCGCTGAGATAGG
TGCCTCACTGATTAAGCATTGGTAACTGTCAGACCAAGTTTACTCATATATACTTTAGATTGATTTAAAAC
TTCATTTTTAATTTAAAAGGATCTAGGTGAAGATCCTTTTTGATAATCTCATGACCAAAATCCCTTAACGT
GAGTTTTCGTTCCACTGAGCGTCAGACCCCGTAGAAAAGATCAAAGGATCTTCTTGAGATCCTTTTTTTCT
GCGCGTAATCTGCTGCTTGCAAACAAAAAAACCACCGCTACCAGCGGTGGTTTGTTTGCCGGATCAAGA
GCTACCAACTCTTTTTCCGAAGGTAACTGGCTTCAGCAGAGCGCAGATACCAAATACTGTCCTTCTAGTG
TAGCCGTAGTTAGGCCACCACTTCAAGAACTCTGTAGCACCGCCTACATACCTCGCTCTGCTAATCCTGTT
ACCAGTGGCTGCTGCCAGTGGCGATAAGTCGTGTCTTACCGGGTTGGACTCAAGACGATAGTTACCGGA
TAAGGCGCAGCGGTCGGGCTGAACGGGGGGTTCGTGCACACAGCCCAGCTTGGAGCGAACGACCTACA
CCGAACTGAGATACCTACAGCGTGAGCTATGAGAAAGCGCCACGCTTCCCGAAGGGAGAAAGGCGGAC
AGGTATCCGGTAAGCGGCAGGGTCGGAACAGGAGAGCGCACGAGGGAGCTTCCAGGGGGAAACGCCT
GGTATCTTTATAGTCCTGTCGGGTTTCGCCACCTCTGACTTGAGCGTCGATTTTTGTGATGCTCGTCAGG
GGGGCGGAGCCTATGGAAAAACGCCAGCAACGCGGCCTTTTTACGGTTCCTGGCCTTTTGCTGGCCTTT

TGCTCACATGTTCTTTCCTGCGTTATCCCCTGATTCTGTGGATAACCGTATTACCGCCTTTGAGTGAGCTG
ATACCGCTCGCCGCAGCCGAACGACCGAGCGCAGCGAGTCAGTGAGCGAGGAAGCGGAAGAGCGCCT
GATGCGGTATTTTCTCCTTACGCATCTGTGCGGTATTTCACACCGCATAAATTCCGACACCATCGAATGGT
GCAAAACCTTTCGCGGTATGGCATGATAGCGCCCGGAAGAGAGTCAATTCAGGGTGGTGAATGTGAAA
CCAGTAACGTTATACGATGTCGCAGAGTATGCCGGTGTCTCTTATCAGACCGTTTCCCGCGTGGTGAACC
AGGCCAGCCACGTTTCTGCGAAAACGCGGGAAAAAGTGGAAGCGGCGATGGCGGAGCTGAATTACATT
CCCAACCGCGTGGCACAACAACTGGCGGGCAAACAGTCGTTGCTGATTGGCGTTGCCACCTCCAGTCTG
GCCCTGCACGCGCCGTCGCAAATTGTCGCGGCGATTAAATCTCGCGCCGATCAACTGGGTGCCAGCGTG
GTGGTGTCGATGGTAGAACGAAGCGGCGTCGAAGCCTGTAAAGCGGCGGTGCACAATCTTCTCGCGCA
ACGCGTCAGTGGGCTGATCATTAACTATCCGCTGGATGACCAGGATGCCATTGCTGTGGAAGCTGCCTG
CACTAATGTTCCGGCGTTATTTCTTGATGTCTCTGACCAGACACCCATCAACAGTATTATTTTCTCCCATG
AAGACGGTACGCGACTGGGCGTGGAGCATCTGGTCGCATTGGGTCACCAGCAAATCGCGCTGTTAGCG
GGCCCATTAAGTTCTGTCTCGGCGCGTCTGCGTCTGGCTGGCTGGCATAAATATCTCACTCGCAATCAAA
TTCAGCCGATAGCGGAACGGGAAGGCGACTGGAGTGCCATGTCCGGTTTTCAACAAACCATGCAAATG
CTGAATGAGGGCATCGTTCCCACTGCGATGCTGGTTGCCAACGATCAGATGGCGCTGGGCGCAATGCG
CGCCATTACCGAGTCCGGGCTGCGCGTTGGTGCGGATATCTCGGTAGTGGGATACGACGATACCGAAG
ACAGCTCATGTTATATCCCGCCGTTAACCACCATCAAACAGGATTTTCGCCTGCTGGGGCAAACCAGCGT
GGACCGCTTGCTGCAACTCTCTCAGGGCCAGGCGGTGAAGGGCAATCAGCTGTTGCCCGTCTCACTGGT
GAAAAGAAAAACCACCCTGGCGCCCAATACGCAAACCGCCTCTCCCCGCGCGTTGGCCGATTCATTAAT
GCAGCTGGCACGACAGGTTTCCCGACTGGAAAGCGGGCAGTGAGCGCAACGCAATTAATGTGAGTTAG
CTCACTCATTAGGCACCCCAGGCTTTACACTTTATGCTTCCGGCTCGTATGTTGTGTGGAATTGTGAGCG
GATAACAATTTCACACAGGAAACAGCTATGACCATGATTACGGATTCACTGGCCGTCGTTTTACAACGTC
GTGACTGGGAAAACCCTGGCGTTACCCAACTTAATCGCCTTGCAGCACATCCCCCTTTCGCCAGCTGGCG
TAATAGCGAAGAGGCCCGCACCGATCGCCCTTCCCAACAGTTGCGCAGCCTGAATGGCGAATGGCGCTT
TGCCTGGTTTCCGGCACCAGAAGCGGTGCCGGAAAGCTGGCTGGAGTGCGATCTTCCTGAGGCCGATA
CTGTCGTCGTCCCCTCAAACTGGCAGATGCACGGTTACGATGCGCCCATCTACACCAACGTAACCTATCC
CATTACGGTCAATCCGCCGTTTGTTCCCACGGAGAATCCGACGGGTTGTTACTCGCTCACATTTAATGTT
GATGAAAGCTGGCTACAGGAAGGCCAGACGCGAATTATTTTTGATGGCGTTGGAATT (SEQ ID NO:57)

### Aminoacid sequence:

MSPILGYWKIKGLVQPTRLLLEYLEEKYEEHLYERDEGDKWRNKKFELGLEFPNLPYYIDGDVKLTQSMAIIRYI
ADKHNMLGGCPKERAEISMLEGAVLDIRYGVSRIAYSKDFETLKVDFLSKLPEMLKMFEDRLCHKTYLNGDH
VTHPDFMLYDALDVVLYMDPMCLDAFPKLVCFKKRIEAIPQIDKYLKSSKYIAWPLQGWQATFGGGDHPPK
SDLVPR|GSGRKKRRQRRRPPQGLFEAIEGFIENGWEGMIDGWYGNNTHDLVGDVRLAGVQSVASSRR
HKRFAGVGDRMKQIEDKIEEILSKIYHIENEIARIKKLIGERGSGSESGYYDRMDYEDDRLRDGERCRDDSFF
GETSHNYHKFDSEYERMGRGSGSPKKKRKVHHHHHH* (SEQ ID NO:58)

### Specific regions of the peptide:

### TAT (cell penetrating peptide)

### INF7

### N

### Ne

### Leu-Zipper

### MATR3₂₁₀₋₂₅₅ peptide flanked by linkers

### NLS

### 6xHis

MGRKKRRQRRRPPQGLFEAIEGFIENGWEGMIDGWYGNNTHDLVGDVRLAGVQSVASSRRHKRFAGVGD
RMKQIEDKIEEILSKIYHIENEIARIKKLIGERGSGSESGYYDRMDYEDDRLRDGERCRDDSFFGETSHNYHKF
DSEYERMGRGSGSPKKKRKVHHHHHH* (SEQ ID NO:59)

**P697 GFP_MATR3 210-255** to generate lentivirus, used for Cas 3/7 assay in HEK cells

### UbC promoter

### Kozak sequence

### EGFP

### Glycine linker

### MATR3 210-255-NLS

GTCGACGGATCGGGAGATCTCCCGATCCCCTATGGTGCACTCTCAGTACAATCTGCTCTGATGCCGCATA
GTTAAGCCAGTATCTGCTCCCTGCTTGTGTGTTGGAGGTCGCTGAGTAGTGCGCGAGCAAAATTTAAGC
TACAACAAGGCAAGGCTTGACCGACAATTGCATGAAGAATCTGCTTAGGGTTAGGCGTTTTGCGCTGCT
TCGCGATGTACGGGCCAGATATACGCGTTGACATTGATTATTGACTAGTTATTAATAGTAATCAATTACG
GGGTCATTAGTTCATAGCCCATATATGGAGTTCCGCGTTACATAACTTACGGTAAATGGCCCGCCTGGCT
GACCGCCCAACGACCCCCGCCCATTGACGTCAATAATGACGTATGTTCCCATAGTAACGCCAATAGGGA
CTTTCCATTGACGTCAATGGGTGGAGTATTTACGGTAAACTGCCCACTTGGCAGTACATCAAGTGTATCA
TATGCCAAGTACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGGTGATGCGGTT
TTGGCAGTACATCAATGGGCGTGGATAGCGGTTTGACTCACGGGGATTTCCAAGTCTCCACCCCATTGA
CGTCAATGGGAGTTTGTTTTGGCACCAAAATCAACGGGACTTTCCAAAATGTCGTAACAACTCCGCCCCA
TTGACGCAAATGGGCGGTAGGCGTGTACGGTGGGAGGTCTATATAAGCAGCGCGTTTTGCCTGTACTG
GGTCTCTCTGGTTAGACCAGATCTGAGCCTGGGAGCTCTCTGGCTAACTAGGGAACCCACTGCTTAAGC
CTCAATAAAGCTTGCCTTGAGTGCTTCAAGTAGTGTGTGCCCGTCTGTTGTGTGACTCTGGTAACTAGAG
ATCCCTCAGACCCTTTTAGTCAGTGTGGAAAATCTCTAGCAGTGGCGCCCGAACAGGGACTTGAAAGCG
AAAGGGAAACCAGAGGAGCTCTCTCGACGCAGGACTCGGCTTGCTGAAGCGCGCACGGCAAGAGGCG
AGGGGCGGCGACTGGTGAGTACGCCAAAAATTTTGACTAGCGGAGGCTAGAAGGAGAGAGATGGGTG
CGAGAGCGTCAGTATTAAGCGGGGGAGAATTAGATCGCGATGGGAAAAAATTCGGTTAAGGCCAGGG
GGAAAGAAAAAATATAAATTAAAACATATAGTATGGGCAAGCAGGGAGCTAGAACGATTCGCAGTTAA
TCCTGGCCTGTTAGAAACATCAGAAGGCTGTAGACAAATACTGGGACAGCTACAACCATCCCTTCAGAC
AGGATCAGAAGAACTTAGATCATTATATAATACAGTAGCAACCCTCTATTGTGTGCATCAAAGGATAGA
GATAAAAGACACCAAGGAAGCTTTAGACAAGATAGAGGAAGAGCAAAACAAAAGTAAGACCACCGCA
CAGCAAGCGGCCGCTGATCTTCAGACCTGGAGGAGGAGATATGAGGGACAATTGGAGAAGTGAATTAT
ATAAATATAAAGTAGTAAAAATTGAACCATTAGGAGTAGCACCCACCAAGGCAAAGAGAAGAGTGGTG
CAGAGAGAAAAAAGAGCAGTGGGAATAGGAGCTTTGTTCCTTGGGTTCTTGGGAGCAGCAGGAAGCAC
TATGGGCGCAGCGTCAATGACGCTGACGGTACAGGCCAGACAATTATTGTCTGGTATAGTGCAGCAGCA
GAACAATTTGCTGAGGGCTATTGAGGCGCAACAGCATCTGTTGCAACTCACAGTCTGGGGCATCAAGCA
GCTCCAGGCAAGAATCCTGGCTGTGGAAAGATACCTAAAGGATCAACAGCTCCTGGGGATTTGGGGTT
GCTCTGGAAAACTCATTTGCACCACTGCTGTGCCTTGGAATGCTAGTTGGAGTAATAAATCTCTGGAACA
GATTTGGAATCACACGACCTGGATGGAGTGGGACAGAGAAATTAACAATTACACAAGCTTAATACACTC
CTTAATTGAAGAATCGCAAAACCAGCAAGAAAAGAATGAACAAGAATTATTGGAATTAGATAAATGGG
CAAGTTTGTGGAATTGGTTTAACATAACAAATTGGCTGTGGTATATAAAATTATTCATAATGATAGTAGG

AGGCTTGGTAGGTTTAAGAATAGTTTTTGCTGTACTTTCTATAGTGAATAGAGTTAGGCAGGGATATTCA
CCATTATCGTTTCAGACCCACCTCCCAACCCCGAGGGGACCCGACAGGCCCGAAGGAATAGAAGAAGA
AGGTGGAGAGAGAGACAGAGACAGATCCATTCGATTAGTGAACGGATCGGCACTGCGTGCGCCAATTC
TGCAGACAAATGGCAGTATTCATCCACAATTTTAAAAGAAAAGGGGGGATTGGGGGGTACAGTGCAGG
GGAAAGAATAGTAGACATAATAGCAACAGACATACAAACTAAAGAATTACAAAAACAAATTACAAAAAT
TCAAAATTTTCGGGTTTATTACAGGGACAGCAGAGATCCAGTTTGGTTAATTAACCCGTGTCGGCTCCAG
ATCTggcctccgcgccgggttttggcgcctcccgcgggcgcccccctcctcacggcgagcgctgccacgtcagacgaagggcgca
gcgagcgtcctgatccttccgcccggacgctcaggacagcggcccgctgctcataagactcggccttagaaccccagtatcagcag
aaggacattttaggacgggacttgggtgactctagggcactggttttctttccagagagcggaacaggcgaggaaaagtagtccc
ttctcggcgattctgcggagggatctccgtggggcggtgaacgccgatgattatataaggacgcgccgggtgtggcacagctagtt
ccgtcgcagccgggatttgggtcgcggttcttgtttgtggatcgctgtgatcgtcacttggtgagtagcgggctgctgggctggccgg
ggctttcgtggccgccgggccgctcggtgggacggaagcgtgtggagagaccgccaagggctgtagtctgggtccgcgagcaag
gttgccctgaactgggggttggggggagcgcagcaaaatggcggctgttcccgagtcttgaatggaagacgcttgtgaggcggg
ctgtgaggtcgttgaaacaaggtggggggcatggtgggcggcaagaacccaaggtcttgaggccttcgctaatgcgggaaagct
cttattcgggtgagatgggctggggcaccatctggggaccctgacgtgaagtttgtcactgactggagaactcggtttgtcgtctgtt
gcgggggcggcagttatggcggtgccgttgggcagtgcacccgtacctttgggagcgcgcgccctcgtcgtgtcgtgacgtcaccc
gttctgttggcttataatgcagggtggggccacctgccggtaggtgtgcggtaggcttttctccgtcgcaggacgcagggttcgggc
ctagggtaggctctcctgaatcgacaggcgccggacctctggtgaggggagggataagtgaggcgtcagtttctttggtcggtttta
tgtacctatcttcttaagtagctgaagctccggttttgaactatgcgctcggggttggcgagtgtgttttgtgaagttttttaggcacctt
ttgaaatgtaatcatttgggtcaatatgtaattttcagtgttagactagtaaattgtccgctaaattctggccgtttttggcttttttgtta
gacGAAGCTTGGGCTGCAGGTCGACTCTAGAGGATCCCCGGGTACCGGTCGCCACCATGGTGAGCAAG
GGCGAGGAGCTGTTCACCGGGGTGGTGCCCATCCTGGTCGAGCTGGACGGCGACGTAAACGGCCACAA
GTTCAGCGTGTCCGGCGAGGGCGAGGGCGATGCCACCTACGGCAAGCTGACCCTGAAGTTCATCTGCA
CCACCGGCAAGCTGCCCGTGCCCTGGCCCACCCTCGTGACCACCCTGACCTACGGCGTGCAGTGCTTCA
GCCGCTACCCCGACCACATGAAGCAGCACGACTTCTTCAAGTCCGCCATGCCCGAAGGCTACGTCCAGG
AGCGCACCATCTTCTTCAAGGACGACGGCAACTACAAGACCCGCGCCGAGGTGAAGTTCGAGGGCGAC
ACCCTGGTGAACCGCATCGAGCTGAAGGGCATCGACTTCAAGGAGGACGGCAACATCCTGGGGCACAA
GCTGGAGTACAACTACAACAGCCACAACGTCTATATCATGGCCGACAAGCAGAAGAACGGCATCAAGG
TGAACTTCAAGATCCGCCACAACATCGAGGACGGCAGCGTGCAGCTCGCCGACCACTACCAGCAGAACA
CCCCCATCGGCGACGGCCCCGTGCTGCTGCCCGACAACCACTACCTGAGCACCCAGTCCGCCCTGAGCA
AAGACCCCAACGAGAAGCGCGATCACATGGTCCTGCTGGAGTTCGTGACCGCCGCCGGGATCACTCTCG
GCATGGACGAGCTGTACAAGGGAGGAGGAGGAGGAGAAAGCGGCTATTATGATCGCATGGATTATG
AAGATGATCGCCTGCGCGATGGCGAACGCTGCCGCGATGATAGCTTTTTTGGCGAAACCAGCCATAAC
TATCATAAATTTGATAGCGAATATGAACGCATGGGCCGCGGCAGCGGCAGCCCGAAAAAAAAACGCA
AAGTGTAATGTACAAGTAAGCTAGCTCGAGCTAGAATTAATTCGATATCAAGCTTATCGATAATCAACCT
CTGGATTACAAAATTTGTGAAAGATTGACTGGTATTCTTAACTATGTTGCTCCTTTTACGCTATGTGGATA
CGCTGCTTTAATGCCTTTGTATCATGCTATTGCTTCCCGTATGGCTTTCATTTTCTCCTCCTTGTATAAATCC
TGGTTGCTGTCTCTTTATGAGGAGTTGTGGCCCGTTGTCAGGCAACGTGGCGTGGTGTGCACTGTGTTT
GCTGACGCAACCCCCACTGGTTGGGGCATTGCCACCACCTGTCAGCTCCTTTCCGGGACTTTCGCTTTCC
CCCTCCCTATTGCCACGGCGGAACTCATCGCCGCCTGCCTTGCCCGCTGCTGGACAGGGGCTCGGCTGTT
GGGCACTGACAATTCCGTGGTGTTGTCGGGGAAATCATCGTCCTTTCCTTGGCTGCTCGCCTGTGTTGCC
ACCTGGATTCTGCGCGGGACGTCCTTCTGCTACGTCCCTTCGGCCCTCAATCCAGCGGACCTTCCTTCCCG
CGGCCTGCTGCCGGCTCTGCGGCCTCTTCCGCGTCTTCGCCTTCGCCCTCAGACGAGTCGGATCTCCCTTT
GGGCCGCCTCCCCGCATCGATACCGTCGACCTCGAGACCTAGAAAAACATGGAGCAATCACAAGTAGCA
ATACAGCAGCTACCAATGCTGATTGTGCCTGGCTAGAAGCACAAGAGGAGGAGGAGGTGGGTTTTCCA
GTCACACCTCAGGTACCTTTAAGACCAATGACTTACAAGGCAGCTGTAGATCTTAGCCACTTTTTAAAAG
AAAAGGGGGGACTGGAAGGGCTAATTCACTCCCAACGAAGACAAGATATCCTTGATCTGTGGATCTACC

ACACACAAGGCTACTTCCCTGATTGGCAGAACTACACACCAGGGCCAGGGATCAGATATCCACTGACCT
TTGGATGGTGCTACAAGCTAGTACCAGTTGAGCAAGAGAAGGTAGAAGAAGCCAATGAAGGAGAGAA
CACCCGCTTGTTACACCCTGTGAGCCTGCATGGGATGGATGACCCGGAGAGAGAAGTATTAGAGTGGA
GGTTTGACAGCCGCCTAGCATTTCATCACATGGCCCGAGAGCTGCATCCGGACTGTACTGGGTCTCTCTG
GTTAGACCAGATCTGAGCCTGGGAGCTCTCTGGCTAACTAGGGAACCCACTGCTTAAGCCTCAATAAAG
CTTGCCTTGAGTGCTTCAAGTAGTGTGTGCCCGTCTGTTGTGTGACTCTGGTAACTAGAGATCCCTCAGA
CCCTTTTAGTCAGTGTGGAAAATCTCTAGCAGGGCCCGTTTAAACCCGCTGATCAGCCTCGACTGTGCCT
TCTAGTTGCCAGCCATCTGTTGTTTGCCCCTCCCCCGTGCCTTCCTTGACCCTGGAAGGTGCCACTCCCAC
TGTCCTTTCCTAATAAAATGAGGAAATTGCATCGCATTGTCTGAGTAGGTGTCATTCTATTCTGGGGGGT
GGGGTGGGGCAGGACAGCAAGGGGGAGGATTGGGAAGACAATAGCAGGCATGCTGGGGATGCGGTG
GGCTCTATGGCTTCTGAGGCGGAAAGAACCAGCTGGGGCTCTAGGGGGTATCCCCACGCGCCCTGTAG
CGGCGCATTAAGCGCGGCGGGTGTGGTGGTTACGCGCAGCGTGACCGCTACACTTGCCAGCGCCCTAG
CGCCCGCTCCTTTCGCTTTCTTCCCTTCCTTTCTCGCCACGTTCGCCGGCTTTCCCCGTCAAGCTCTAAATC
GGGGGCTCCCTTTAGGGTTCCGATTTAGTGCTTTACGGCACCTCGACCCCAAAAAACTTGATTAGGGTG
ATGGTTCACGTAGTGGGCCATCGCCCTGATAGACGGTTTTTCGCCCTTTGACGTTGGAGTCCACGTTCTT
TAATAGTGGACTCTTGTTCCAAACTGGAACAACACTCAACCCTATCTCGGTCTATTCTTTTGATTTATAAG
GGATTTTGCCGATTTCGGCCTATTGGTTAAAAAATGAGCTGATTTAACAAAAATTTAACGCGAATTAATT
CTGTGGAATGTGTGTCAGTTAGGGTGTGGAAAGTCCCCAGGCTCCCCAGCAGGCAGAAGTATGCAAAG
CATGCATCTCAATTAGTCAGCAACCAGGTGTGGAAAGTCCCCAGGCTCCCCAGCAGGCAGAAGTATGCA
AAGCATGCATCTCAATTAGTCAGCAACCATAGTCCCGCCCCTAACTCCGCCCATCCCGCCCCTAACTCCGC
CCAGTTCCGCCCATTCTCCGCCCCATGGCTGACTAATTTTTTTTATTTATGCAGAGGCCGAGGCCGCCTCT
GCCTCTGAGCTATTCCAGAAGTAGTGAGGAGGCTTTTTTGGAGGCCTAGGCTTTTGCAAAAAGCTCCCG
GGAGCTTGTATATCCATTTTCGGATCTGATCAGCACGTGTTGACAATTAATCATCGGCATAGTATATCGG
CATAGTATAATACGACAAGGTGAGGAACTAAACCATGGCCAAGTTGACCAGTGCCGTTCCGGTGCTCAC
CGCGCGCGACGTCGCCGGAGCGGTCGAGTTCTGGACCGACCGGCTCGGGTTCTCCCGGGACTTCGTGG
AGGACGACTTCGCCGGTGTGGTCCGGGACGACGTGACCCTGTTCATCAGCGCGGTCCAGGACCAGGTG
GTGCCGGACAACACCCTGGCCTGGGTGTGGGTGCGCGGCCTGGACGAGCTGTACGCCGAGTGGTCGG
AGGTCGTGTCCACGAACTTCCGGGACGCCTCCGGGCCGGCCATGACCGAGATCGGCGAGCAGCCGTGG
GGGCGGGAGTTCGCCCTGCGCGACCCGGCCGGCAACTGCGTGCACTTCGTGGCCGAGGAGCAGGACT
GACACGTGCTACGAGATTTCGATTCCACCGCCGCCTTCTATGAAAGGTTGGGCTTCGGAATCGTTTTCCG
GGACGCCGGCTGGATGATCCTCCAGCGCGGGGATCTCATGCTGGAGTTCTTCGCCCACCCCAACTTGTTT
ATTGCAGCTTATAATGGTTACAAATAAAGCAATAGCATCACAAATTTCACAAATAAAGCATTTTTTTCACT
GCATTCTAGTTGTGGTTTGTCCAAACTCATCAATGTATCTTATCATGTCTGTATACCGTCGACCTCTAGCT
AGAGCTTGGCGTAATCATGGTCATAGCTGTTTCCTGTGTGAAATTGTTATCCGCTCACAATTCCACACAA
CATACGAGCCGGAAGCATAAAGTGTAAAGCCTGGGGTGCCTAATGAGTGAGCTAACTCACATTAATTGC
GTTGCGCTCACTGCCCGCTTTCCAGTCGGGAAACCTGTCGTGCCAGCTGCATTAATGAATCGGCCAACGC
GCGGGGAGAGGCGGTTTGCGTATTGGGCGCTCTTCCGCTTCCTCGCTCACTGACTCGCTGCGCTCGGTC
GTTCGGCTGCGGCGAGCGGTATCAGCTCACTCAAAGGCGGTAATACGGTTATCCACAGAATCAGGGGA
TAACGCAGGAAAGAACATGTGAGCAAAAGGCCAGCAAAAGGCCAGGAACCGTAAAAAGGCCGCGTTG
CTGGCGTTTTTCCATAGGCTCCGCCCCCCTGACGAGCATCACAAAAATCGACGCTCAAGTCAGAGGTGG
CGAAACCCGACAGGACTATAAAGATACCAGGCGTTTCCCCCTGGAAGCTCCCTCGTGCGCTCTCCTGTTC
CGACCCTGCCGCTTACCGGATACCTGTCCGCCTTTCTCCCTTCGGGAAGCGTGGCGCTTTCTCATAGCTCA
CGCTGTAGGTATCTCAGTTCGGTGTAGGTCGTTCGCTCCAAGCTGGGCTGTGTGCACGAACCCCCCGTTC
AGCCCGACCGCTGCGCCTTATCCGGTAACTATCGTCTTGAGTCCAACCCGGTAAGACACGACTTATCGCC
ACTGGCAGCAGCCACTGGTAACAGGATTAGCAGAGCGAGGTATGTAGGCGGTGCTACAGAGTTCTTGA
AGTGGTGGCCTAACTACGGCTACACTAGAAGAACAGTATTTGGTATCTGCGCTCTGCTGAAGCCAGTTA
CCTTCGGAAAAAGAGTTGGTAGCTCTTGATCCGGCAAACAAACCACCGCTGGTAGCGGTGGTTTTTTTG

TTTGCAAGCAGCAGATTACGCGCAGAAAAAAAGGATCTCAAGAAGATCCTTTGATCTTTTCTACGGGGT
CTGACGCTCAGTGGAACGAAAACTCACGTTAAGGGATTTTGGTCATGAGATTATCAAAAAGGATCTTCA
CCTAGATCCTTTTAAATTAAAAATGAAGTTTTAAATCAATCTAAAGTATATATGAGTAAACTTGGTCTGAC
AGTTACCAATGCTTAATCAGTGAGGCACCTATCTCAGCGATCTGTCTATTTCGTTCATCCATAGTTGCCTG
ACTCCCCGTCGTGTAGATAACTACGATACGGGAGGGCTTACCATCTGGCCCCAGTGCTGCAATGATACC
GCGAGACCCACGCTCACCGGCTCCAGATTTATCAGCAATAAACCAGCCAGCCGGAAGGGCCGAGCGCA
GAAGTGGTCCTGCAACTTTATCCGCCTCCATCCAGTCTATTAATTGTTGCCGGGAAGCTAGAGTAAGTAG
TTCGCCAGTTAATAGTTTGCGCAACGTTGTTGCCATTGCTACAGGCATCGTGGTGTCACGCTCGTCGTTT
GGTATGGCTTCATTCAGCTCCGGTTCCCAACGATCAAGGCGAGTTACATGATCCCCCATGTTGTGCAAAA
AAGCGGTTAGCTCCTTCGGTCCTCCGATCGTTGTCAGAAGTAAGTTGGCCGCAGTGTTATCACTCATGGT
TATGGCAGCACTGCATAATTCTCTTACTGTCATGCCATCCGTAAGATGCTTTTCTGTGACTGGTGAGTACT
CAACCAAGTCATTCTGAGAATAGTGTATGCGGCGACCGAGTTGCTCTTGCCCGGCGTCAATACGGGATA
ATACCGCGCCACATAGCAGAACTTTAAAAGTGCTCATCATTGGAAAACGTTCTTCGGGGCGAAAACTCTC
AAGGATCTTACCGCTGTTGAGATCCAGTTCGATGTAACCCACTCGTGCACCCAACTGATCTTCAGCATCT
TTTACTTTCACCAGCGTTTCTGGGTGAGCAAAAACAGGAAGGCAAAATGCCGCAAAAAAGGGAATAAG
GGCGACACGGAAATGTTGAATACTCATACTCTTCCTTTTTCAATATTATTGAAGCATTTATCAGGGTTATT
GTCTCATGAGCGGATACATATTTGAATGTATTTAGAAAAATAAACAAATAGGGGTTCCGCGCACATTTCC
CCGAAAAGTGCCACCTGAC (SEQ ID NO:60)

### Aminoacid sequence:

MVSKGEELFTGVVPILVELDGDVNGHKFSVSGEGEGDATYGKLTLKFICTTGKLPVPWPTLVTTLTYGVQCFS
RYPDHMKQHDFFKSAMPEGYVQERTIFFKDDGNYKTRAEVKFEGDTLVNRIELKGIDFKEDGNILGHKLEYN
YNSHNVYIMADKQKNGIKVNFKIRHNIEDGSVQLADHYQQNTPIGDGPVLLPDNHYLSTQSALSKDPNEKR
DHMVLLEFVTAAGITLGMDELYKGGGGGESGYYDRMDYEDDRLRDGERCRDDSFFGETSHNYHKFDSEYE
RMGRGSGSPKKKRKV* (SEQ ID NO:61)

**MATR3 210-255 WITH PROTAC** to generate BFP lentivirus

### CMV enhancer promoter

### Flag

### MATR3 210-255

### PROTAC

### NLS

### BFP

AAATTTCACAAATAAAGCATTTTTTTCACTGCATTCTAGTTGTGGTTTGTCCAAACTCATCAATGTATCTTA
TCATGTCTGGCTCTAGCTATCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTCCAGAAGTAGTGAGGAGG
CTTTTTTGGAGGCCTAGGCTTTTGCGTCGAGACGTACCCAATTCGCCCTATAGTGAGTCGTATTACGCGC
GCTCACTGGCCGTCGTTTTACAACGTCGTGACTGGGAAAACCCTGGCGTTACCCAACTTAATCGCCTTGC
AGCACATCCCCCTTTCGCCAGCTGGCGTAATAGCGAAGAGGCCCGCACCGATCGCCCTTCCCAACAGTT
GCGCAGCCTGAATGGCGAATGGCGCGACGCGCCCTGTAGCGGCGCATTAAGCGCGGCGGGTGTGGTG
GTTACGCGCAGCGTGACCGCTACACTTGCCAGCGCCCTAGCGCCCGCTCCTTTCGCTTTCTTCCCTTCCTT
TCTCGCCACGTTCGCCGGCTTTCCCCGTCAAGCTCTAAATCGGGGGCTCCCTTTAGGGTTCCGATTTAGT
GCTTTACGGCACCTCGACCCCAAAAAACTTGATTAGGGTGATGGTTCACGTAGTGGGCCATCGCCCTGA
TAGACGGTTTTTCGCCCTTTGACGTTGGAGTCCACGTTCTTTAATAGTGGACTCTTGTTCCAAACTGGAAC

AACACTCAACCCTATCTCGGTCTATTCTTTTGATTTATAAGGGATTTTGCCGATTTCGGCCTATTGGTTAA
AAAATGAGCTGATTTAACAAAAATTTAACGCGAATTTTAACAAAATATTAACGTTTACAATTTCCCAGGT
GGCACTTTTCGGGGAAATGTGCGCGGAACCCCTATTTGTTTATTTTTCTAAATACATTCAAATATGTATCC
GCTCATGAGACAATAACCCTGATAAATGCTTCAATAATATTGAAAAAGGAAGAGTATGAGTATTCAACA
TTTCCGTGTCGCCCTTATTCCCTTTTTTGCGGCATTTTGCCTTCCTGTTTTTGCTCACCCAGAAACGCTGGT
GAAAGTAAAAGATGCTGAAGATCAGTTGGGTGCACGAGTGGGTTACATCGAACTGGATCTCAACAGCG
GTAAGATCCTTGAGAGTTTTCGCCCCGAAGAACGTTTTCCAATGATGAGCACTTTTAAAGTTCTGCTATG
TGGCGCGGTATTATCCCGTATTGACGCCGGGCAAGAGCAACTCGGTCGCCGCATACACTATTCTCAGAA
TGACTTGGTTGAGTACTCACCAGTCACAGAAAAGCATCTTACGGATGGCATGACAGTAAGAGAATTATG
CAGTGCTGCCATAACCATGAGTGATAACACTGCGGCCAACTTACTTCTGACAACGATCGGAGGACCGAA
GGAGCTAACCGCTTTTTTGCACAACATGGGGGATCATGTAACTCGCCTTGATCGTTGGGAACCGGAGCT
GAATGAAGCCATACCAAACGACGAGCGTGACACCACGATGCCTGTAGCAATGGCAACAACGTTGCGCA
AACTATTAACTGGCGAACTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCTGATAAATCTGGAGCCG
GTGAGCGTGGGTCTCGCGGTATCATTGCAGCACTGGGGCCAGATGGTAAGCCCTCCCGTATCGTAGTTA
TCTACACGACGGGGAGTCAGGCAACTATGGATGAACGAAATAGACAGATCGCTGAGATAGGTGCCTCA
CTGATTAAGCATTGGTAACTGTCAGACCAAGTTTACTCATATATACTTTAGATTGATTTAAAACTTCATTT
TTAATTTAAAAGGATCTAGGTGAAGATCCTTTTTGATAATCTCATGACCAAAATCCCTTAACGTGAGTTTT
CGTTCCACTGAGCGTCAGACCCCGTAGAAAAGATCAAAGGATCTTCTTGAGATCCTTTTTTTCTGCGCGT
AATCTGCTGCTTGCAAACAAAAAAACCACCGCTACCAGCGGTGGTTTGTTTGCCGGATCAAGAGCTACC
AACTCTTTTTCCGAAGGTAACTGGCTTCAGCAGAGCGCAGATACCAAATACTGTCCTTCTAGTGTAGCCG
TAGTTAGGCCACCACTTCAAGAACTCTGTAGCACCGCCTACATACCTCGCTCTGCTAATCCTGTTACCAGT
GGCTGCTGCCAGTGGCGATAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAGCGAACGACCTACACCGAA
CTGAGATACCTACAGCGTGAGCTATGAGAAAGCGCCACGCTTCCCGAAGGGAGAAAGGCGGACAGGTA
TCCGGTAAGCGGCAGGGTCGGAACAGGAGAGCGCACGAGGGAGCTTCCAGGGGGAAACGCCTGGTAT
CTTTATAGTCCTGTCGGGTTTCGCCACCTCTGACTTGAGCGTCGATTTTTGTGATGCTCGTCAGGGGGGC
GGAGCCTATGGAAAAACGCCAGCAACGCGGCCTTTTTACGGTTCCTGGCCTTTTGCTGGCCTTTTGCTCA
CATGTTCTTTCCTGCGTTATCCCCTGATTCTGTGGATAACCGTATTACCGCCTTTGAGTGAGCTGATACCG
CTCGCCGCAGCCGAACGACCGAGCGCAGCGAGTCAGTGAGCGAGGAAGCGGAAGAGCGCCCAATACG
CAAACCGCCTCTCCCCGCGCGTTGGCCGATTCATTAATGCAGCTGGCACGACAGGTTTCCCGACTGGAA
AGCGGGCAGTGAGCGCAACGCAATTAATGTGAGTTAGCTCACTCATTAGGCACCCCAGGCTTTACACTT
TATGCTTCCGGCTCGTATGTTGTGTGGAATTGTGAGCGGATAACAATTTCACACAGGAAACAGCTATGA
CCATGATTACGCCAAGCGCGCAATTAACCCTCACTAAAGGGAACAAAAGCTGGAGCTGCAAGCTTGGCC
ATTGCATACGTTGTATCCATATCATAATATGTACATTTATATTGGCTCATGTCCAACATTACCGCCATGTTg
acattgattattgactagttattaatagtaatcaattacggggtcattagttcatagcccatatatggagttccgcgttacataactta
cggtaaatggcccgcctggctgaccgcccaacgacccccgcccattgacgtcaataatgacgtatgttcccatagtaacgccaatag
ggactttccattgacgtcaatgggtggagtatttacggtaaactgcccacttggcagtacatcaagtgtatcatatgccaagtacgc
cccctattgacgtcaatgacggtaaatggcccgcctggcattatgcccagtacatgaccttatgggactttcctacttggcagtacatc
tacgtattagtcatcgctattaccatggtgatgcggttttggcagtacatcaatgggcgtggatagcggtttgactcacggggatttc
caagtctccaccccattgacgtcaatgggagtttgttttggcaccaaaatcaacgggactttccaaaatgtcgtaacaactccgcccc
attgacgcaaatgggcggtaggcgtgtacggtgggaggtctatataagcagagctCGTTTAGTGAACCGGGGTCTCTCT
GGTTAGACCAGATCTGAGCCTGGGAGCTCTCTGGCTAACTAGGGAACCCACTGCTTAAGCCTCAATAAA
GCTTGCCTTGAGTGCTTCAAGTAGTGTGTGCCCGTCTGTTGTGTGACTCTGGTAACTAGAGATCCCTCAG
ACCCTTTTAGTCAGTGTGGAAAATCTCTAGCAGTGGCGCCCGAACAGGGACCTGAAAGCGAAAGGGAA
ACCAGAGCTCTCTCGACGCAGGACTCGGCTTGCTGAAGCGCGCACGGCAAGAGGCGAGGGGCGGCGA
CTGGTGAGTACGCCAAAAATTTTGACTAGCGGAGGCTAGAAGGAGAGAGATGGGTGCGAGAGCGTCA

GTATTAAGCGGGGGAGAATTAGATCGCGATGGGAAAAAATTCGGTTAAGGCCAGGGGGAAAGAAAAA
ATATAAATTAAAACATATAGTATGGGCAAGCAGGGAGCTAGAACGATTCGCAGTTAATCCTGGCCTGTT
AGAAACATCAGAAGGCTGTAGACAAATACTGGGACAGCTACAACCATCCCTTCAGACAGGATCAGAAG
AACTTAGATCATTATATAATACAGTAGCAACCCTCTATTGTGTGCATCAAAGGATAGAGATAAAAGACAC
CAAGGAAGCTTTAGACAAGATAGAGGAAGAGCAAAACAAAAGTAAGACCACCGCACAGCAAGCGGCC
GCTGATCTTCAGACCTGGAGGAGGAGATATGAGGGACAATTGGAGAAGTGAATTATATAAATATAAAG
TAGTAAAAATTGAACCATTAGGAGTAGCACCCACCAAGGCAAAGAGAAGAGTGGTGCAGAGAGAAAA
AAGAGCAGTGGGAATAGGAGCTTTGTTCCTTGGGTTCTTGGGAGCAGCAGGAAGCACTATGGGCGCAG
CCTCAATGACGCTGACGGTACAGGCCAGACAATTATTGTCTGGTATAGTGCAGCAGCAGAACAATTTGC
TGAGGGCTATTGAGGCGCAACAGCATCTGTTGCAACTCACAGTCTGGGGCATCAAGCAGCTCCAGGCAA
GAATCCTGGCTGTGGAAAGATACCTAAAGGATCAACAGCTCCTGGGGATTTGGGGTTGCTCTGGAAAAC
TCATTTGCACCACTGCTGTGCCTTGGAATGCTAGTTGGAGTAATAAATCTCTGGAACAGATTGGAATCAC
ACGACCTGGATGGAGTGGGACAGAGAAATTAACAATTACACAAGCTTAATACACTCCTTAATTGAAGAA
TCGCAAAACCAGCAAGAAAAGAATGAACAAGAATTATTGGAATTAGATAAATGGGCAAGTTTGTGGAA
TTGGTTTAACATAACAAATTGGCTGTGGTATATAAAATTATTCATAATGATAGTAGGAGGCTTGGTAGGT
TTAAGAATAGTTTTTGCTGTACTTTCTATAGTGAATAGAGTTAGGCAGGGATATTCACCATTATCGTTTCA
GACCCACCTCCCAACCCCGAGGGGACCCGACAGGCCCGAAGGAATAGAAGAAGAAGGTGGAGAGAGA
GACAGAGACAGATCCATTCGATTAGTGAACGGATCTCGACGGTATCGGTTAACTTTTAAAAGAAAAGGG
GGGATTGGGGGGTACAGTGCAGGGGAAAGAATAGTAGACATAATAGCAACAGACATACAAACTAAAG
AATTACAAAAACAAATTACAAAAATTCAAAATTTTATCGATCACGAGACTAGCCTCGAGAAGCTTGATTA
TATACTAGTATGAAACCCCACCTGTAGGTTTGGCAAGCTAGCTGCAGTAACGCCATTTTGCAAGGCATG
GAAAAATACCAAACCAAGAATAGAGAAGTTCAGATCAAGGGCGGGTACATGAAAATAGCTAACGTTGG
GCCAAACAGGATATCTGCGGTGAGCAGTTTCGGCCCCGGCCCGGGGCCAAGAACAGATGGTCACCGCA
GTTTCGGCCCCGGCCCGAGGCCAAGAACAGATGGTCCCCAGATATGGCCCAACCCTCAGCAGTTTCTTA
AGACCCATCAGATGTTTCCAGGCTCCCCCAAGGACCTGAAATGACCCTGCGCCTTATTTGAATTAACCAA
TCAGCCTGCTTCTCGCTTCTGTTCGCGCGCTTCTGCTTCCCGAGCTCTATAAAAGAGCTCACAACCCCTCA
CTCGGCTTAATTAAAAGCTTTATATAATCAGCATCACAAGTTTGTACAAAAAAGCAGGCTCCATGGATTA
CAAGGATGACGACGATAAGAGCCCGGGCGGATCCGAATCTGGTTATTATGACAGAATGGATTATGAA
GATGACAGATTAAGAGATGGAGAAAGGTGTAGGGATGATTCTTTTTTTGGTGAGACCTCGCATAACT
ATCATAAATTTGACAGTGAGTATGAGAGAATGGGACGTGGAAGCGGAAGCGCCCTGGCCCCCTACAT
CCCCGAAGACCCAAAAAAGAAGAGAAAGGTAGACTAAGACCCAGCTTTCTTGTACAAAGTGGTGATGC
TCTAGAATGCATGCATGCCGTACGATCGCCGCCTCAGGCCTGAGTTCGAAGTATACGTCGACAATCAAC
CTCTGGATTACAAAATTTGTGAAAGATTGACTGGTATTCTTAACTATGTTGCTCCTTTTACGCTATGTGGA
TACGCTGCTTTAATGCCTTTGTATCATGCTATTGCTTCCCGTATGGCTTTCATTTTCTCCTCCTTGTATAAAT
CCTGGTTGCTGTCTCTTTATGAGGAGTTGTGGCCCGTTGTCAGGCAACGTGGCGTGGTGTGCACTGTGT
TTGCTGACGCAACCCCCACTGGTTGGGGCATTGCCACCACCTGTCAGCTCCTTTCCGGGACTTTCGCTTTC
CCCCTCCCTATTGCCACGGCGGAACTCATCGCCGCCTGCCTTGCCCGCTGCTGGACAGGGGCTCGGCTGT
TGGGCACTGACAATTCCGTGGTGTTGTCGGGGAAGCTGACGTCCTTTCCATGGCTGCTCGCCTGTGTTGC
CACCTGGATTCTGCGCGGGACGTCTTCTGCTACGTCCCTTCGGCCTCAATCAGCGGACTTCCTTCCCGCG
GCCTGCTGCCGGCTCTGCGGCCTCTTCCGCGTCTTCGCCTTCGCCCTCAGACGAGTCGGATCTCCCTTTG
GGCCGCCTCCCCGCCTGGAATTCCTGCAGCCCGGGGGATCAAGGATCTGCGATCGCTCCGGTGCCCGTC
AGTGGGCAGAGCGCACATCGCCCACAGTCCCCGAGAAGTTGGGGGGAGGGGTCGGCAATTGAACGGG
TGCCTAGAGAAGGTGGCGCGGGGTAAACTGGGAAAGTGATGTCGTGTACTGGCTCCGCCTTTTTCCCGA
GGGTGGGGGAGAACCGTATATAAGTGCAGTAGTCGCCGTGAACGTTCTTTTTCGCAACGGGTTTGCCGC
CAGAACACAGCTGAAGCTTCGAGGGGCTCGCATCTCTCCTTCACGCGCCCGCCGCCCTACCTGAGGCCG
CCATCCACGCCGGTTGAGTCGCGTTCTGCCGCCTCCCGCCTGTGGTGCCTCCTGAACTGCGTCCGCCGTC
TAGGTAAGTTTAAAGCTCAGGTCGAGACCGGGCCTTTGTCCGGCGCTCCCTTGGAGCCTACCTAGACTC

AGCCGGCTCTCCACGCTTTGCCTGACCCTGCTTGCTCAACTCTACGTCTTTGTTTCGTTTTCTGTTCTGCGC
CGTTACAGATCCAAGCTGTGACCGGCGCCTACGATCCACCGGTCGCCACCATGAGCGAGCTGATTAAGG
AGAACATGCACATGAAGCTGTACATGGAGGGCACCGTGGACAACCATCACTTCAAGTGCACATCCGAG
GGCGAAGGCAAGCCCTACGAGGGCACCCAGACCATGAGAATCAAGGTGGTCGAGGGCGGCCCTCTCCC
CTTCGCCTTCGACATCCTGGCTACTAGCTTCCTCTACGGCAGCAAGACCTTCATCAACCACACCCAGGGC
ATCCCCGACTTCTTCAAGCAGTCCTTCCCTGAGGGCTTCACATGGGAGAGAGTCACCACATACGAAGAC
GGGGGCGTGCTGACCGCTACCCAGGACACCAGCCTCCAGGACGGCTGCCTCATCTACAACGTCAAGATC
AGAGGGGTGAACTTCACATCCAACGGCCCTGTGATGCAGAAGAAAACACTCGGCTGGGAGGCCTTCAC
CGAGACGCTGTACCCCGCTGACGGCGGCCTGGAAGGCAGAAACGACATGGCCCTGAAGCTCGTGGGCG
GGAGCCATCTGATCGCAAACATCAAGACCACATATAGATCCAAGAAACCCGCTAAGAACCTCAAGATGC
CTGGCGTCTACTATGTGGACTACAGACTGGAAAGAATCAAGGAGGCCAACAACGAGACCTACGTCGAG
CAGCACGAGGTGGCAGTGGCCAGATACTGCGACCTCCCTAGCAAACTGGGGCACAAGCTTAATTAAAG
CGGCCGCGTCGAGAGATGGGGGAGGCTAACTGAGCTAGCTTAGTCATGCACCGGGCTGCAGGAATTCG
AGCTCGGTACCTTTAAGACCAATGACTTACAAGGCAGCTGTAGATCTTAGCCACTTTTTAAAAGAAAAGG
GGGGACTGGAAGGGCTAATTCACTCCCAACGAAGACAAGATCTGCTTTTTGCTTGTACTGGGTCTCTCTG
GTTAGACCAGATCTGAGCCTGGGAGCTCTCTGGCTAACTAGGGAACCCACTGCTTAAGCCTCAATAAAG
CTTGCCTTGAGTGCTTCAAGTAGTGTGTGCCCGTCTGTTGTGTGACTCTGGTAACTAGAGATCCCTCAGA
CCCTTTTAGTCAGTGTGGAAAATCTCTAGCAGTAGTAGTTCATGTCATCTTATTATTCAGTATTTATAACTT
GCAAAGAAATGAATATCAGAGAGTGAGAGGAACTTGTTTATTGCAGCTTATAATGGTTACAAATAAAGC
AATAGCATCAC (SEQ ID NO:62)

### Aminoacid sequence:

MDYKDDDDKSPGGSESGYYDRMDYEDDRLRDGERCRDDSFFGETSHNYHKFDSEYERMGRGSGSALAPY
IPEDPKKKRKVD* (SEQ ID NO:63)

**MATR3 210-255 WITHOUT PROTAC** to generate BFP lentivirus

### CMV enhancer promoter

### Flag

### MATR3 210-255

### NLS

### BFP

AAATTTCACAAATAAAGCATTTTTTTCACTGCATTCTAGTTGTGGTTTGTCCAAACTCATCAATGTATCTTA
TCATGTCTGGCTCTAGCTATCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTCCAGAAGTAGTGAGGAGG
CTTTTTTGGAGGCCTAGGCTTTTGCGTCGAGACGTACCCAATTCGCCCTATAGTGAGTCGTATTACGCGC
GCTCACTGGCCGTCGTTTTACAACGTCGTGACTGGGAAAACCCTGGCGTTACCCAACTTAATCGCCTTGC
AGCACATCCCCCTTTCGCCAGCTGGCGTAATAGCGAAGAGGCCCGCACCGATCGCCCTTCCCAACAGTT
GCGCAGCCTGAATGGCGAATGGCGCGACGCGCCCTGTAGCGGCGCATTAAGCGCGGCGGGTGTGGTG
GTTACGCGCAGCGTGACCGCTACACTTGCCAGCGCCCTAGCGCCCGCTCCTTTCGCTTTCTTCCCTTCCTT
TCTCGCCACGTTCGCCGGCTTTCCCCGTCAAGCTCTAAATCGGGGGCTCCCTTTAGGGTTCCGATTTAGT
GCTTTACGGCACCTCGACCCCAAAAAACTTGATTAGGGTGATGGTTCACGTAGTGGGCCATCGCCCTGA
TAGACGGTTTTTCGCCCTTTGACGTTGGAGTCCACGTTCTTTAATAGTGGACTCTTGTTCCAAACTGGAAC
AACACTCAACCCTATCTCGGTCTATTCTTTTGATTTATAAGGGATTTTGCCGATTTCGGCCTATTGGTTAA
AAAATGAGCTGATTTAACAAAAATTTAACGCGAATTTTAACAAAATATTAACGTTTACAATTTCCCAGGT

GGCACTTTTCGGGGAAATGTGCGCGGAACCCCTATTTGTTTATTTTTCTAAATACATTCAAATATGTATCC
GCTCATGAGACAATAACCCTGATAAATGCTTCAATAATATTGAAAAAGGAAGAGTATGAGTATTCAACA
TTTCCGTGTCGCCCTTATTCCCTTTTTTGCGGCATTTTGCCTTCCTGTTTTTGCTCACCCAGAAACGCTGGT
GAAAGTAAAAGATGCTGAAGATCAGTTGGGTGCACGAGTGGGTTACATCGAACTGGATCTCAACAGCG
GTAAGATCCTTGAGAGTTTTCGCCCCGAAGAACGTTTTCCAATGATGAGCACTTTTAAAGTTCTGCTATG
TGGCGCGGTATTATCCCGTATTGACGCCGGGCAAGAGCAACTCGGTCGCCGCATACACTATTCTCAGAA
TGACTTGGTTGAGTACTCACCAGTCACAGAAAAGCATCTTACGGATGGCATGACAGTAAGAGAATTATG
CAGTGCTGCCATAACCATGAGTGATAACACTGCGGCCAACTTACTTCTGACAACGATCGGAGGACCGAA
GGAGCTAACCGCTTTTTTGCACAACATGGGGGATCATGTAACTCGCCTTGATCGTTGGGAACCGGAGCT
GAATGAAGCCATACCAAACGACGAGCGTGACACCACGATGCCTGTAGCAATGGCAACAACGTTGCGCA
AACTATTAACTGGCGAACTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCTGATAAATCTGGAGCCG
GTGAGCGTGGGTCTCGCGGTATCATTGCAGCACTGGGGCCAGATGGTAAGCCCTCCCGTATCGTAGTTA
TCTACACGACGGGGAGTCAGGCAACTATGGATGAACGAAATAGACAGATCGCTGAGATAGGTGCCTCA
CTGATTAAGCATTGGTAACTGTCAGACCAAGTTTACTCATATATACTTTAGATTGATTTAAAACTTCATTT
TTAATTTAAAAGGATCTAGGTGAAGATCCTTTTTGATAATCTCATGACCAAAATCCCTTAACGTGAGTTTT
CGTTCCACTGAGCGTCAGACCCCGTAGAAAAGATCAAAGGATCTTCTTGAGATCCTTTTTTTCTGCGCGT
AATCTGCTGCTTGCAAACAAAAAAACCACCGCTACCAGCGGTGGTTTGTTTGCCGGATCAAGAGCTACC
AACTCTTTTTCCGAAGGTAACTGGCTTCAGCAGAGCGCAGATACCAAATACTGTCCTTCTAGTGTAGCCG
TAGTTAGGCCACCACTTCAAGAACTCTGTAGCACCGCCTACATACCTCGCTCTGCTAATCCTGTTACCAGT
GGCTGCTGCCAGTGGCGATAAGTCGTGTCTTACCGGGTTGGACTCAAGACGATAGTTACCGGATAAGG
CGCAGCGGTCGGGCTGAACGGGGGGTTCGTGCACACAGCCCAGCTTGGAGCGAACGACCTACACCGAA
CTGAGATACCTACAGCGTGAGCTATGAGAAAGCGCCACGCTTCCCGAAGGGAGAAAGGCGGACAGGTA
TCCGGTAAGCGGCAGGGTCGGAACAGGAGAGCGCACGAGGGAGCTTCCAGGGGGAAACGCCTGGTAT
CTTTATAGTCCTGTCGGGTTTCGCCACCTCTGACTTGAGCGTCGATTTTTGTGATGCTCGTCAGGGGGGC
GGAGCCTATGGAAAAACGCCAGCAACGCGGCCTTTTTACGGTTCCTGGCCTTTTGCTGGCCTTTTGCTCA
CATGTTCTTTCCTGCGTTATCCCCTGATTCTGTGGATAACCGTATTACCGCCTTTGAGTGAGCTGATACCG
CTCGCCGCAGCCGAACGACCGAGCGCAGCGAGTCAGTGAGCGAGGAAGCGGAAGAGCGCCCAATACG
CAAACCGCCTCTCCCCGCGCGTTGGCCGATTCATTAATGCAGCTGGCACGACAGGTTTCCCGACTGGAA
AGCGGGCAGTGAGCGCAACGCAATTAATGTGAGTTAGCTCACTCATTAGGCACCCCAGGCTTTACACTT
TATGCTTCCGGCTCGTATGTTGTGTGGAATTGTGAGCGGATAACAATTTCACACAGGAAACAGCTATGA
CCATGATTACGCCAAGCGCGCAATTAACCCTCACTAAAGGGAACAAAAGCTGGAGCTGCAAGCTTGGCC
ATTGCATACGTTGTATCCATATCATAATATGTACATTTATATTGGCTCATGTCCAACATTACCGCCATGTTg
acattgattattgactagttattaatagtaatcaattacggggtcattagttcatagcccatatatggagttccgcgttacataactta
cggtaaatggcccgcctggctgaccgcccaacgacccccgcccattgacgtcaataatgacgtatgttcccatagtaacgccaatag
ggactttccattgacgtcaatgggtggagtatttacggtaaactgcccacttggcagtacatcaagtgtatcatatgccaagtacgc
cccctattgacgtcaatgacggtaaatggcccgcctggcattatgcccagtacatgaccttatgggactttcctacttggcagtacatc
tacgtattagtcatcgctattaccatggtgatgcggttttggcagtacatcaatgggcgtggatagcggtttgactcacggggatttc
caagtctccaccccattgacgtcaatgggagtttgttttggcaccaaaatcaacgggactttccaaaatgtcgtaacaactccgcccc
attgacgcaaatgggcggtaggcgtgtacggtgggaggtctatataagcagagctCGTTTAGTGAACCGGGGTCTCTCT
GGTTAGACCAGATCTGAGCCTGGGAGCTCTCTGGCTAACTAGGGAACCCACTGCTTAAGCCTCAATAAA
GCTTGCCTTGAGTGCTTCAAGTAGTGTGTGCCCGTCTGTTGTGTGACTCTGGTAACTAGAGATCCCTCAG
ACCCTTTTAGTCAGTGTGGAAAATCTCTAGCAGTGGCGCCCGAACAGGGACCTGAAAGCGAAAGGGAA
ACCAGAGCTCTCTCGACGCAGGACTCGGCTTGCTGAAGCGCGCACGGCAAGAGGCGAGGGGCGGCGA
CTGGTGAGTACGCCAAAAATTTTGACTAGCGGAGGCTAGAAGGAGAGAGATGGGTGCGAGAGCGTCA
GTATTAAGCGGGGGAGAATTAGATCGCGATGGGAAAAAATTCGGTTAAGGCCAGGGGGAAAGAAAAA
ATATAAATTAAAACATATAGTATGGGCAAGCAGGGAGCTAGAACGATTCGCAGTTAATCCTGGCCTGTT

AGAAACATCAGAAGGCTGTAGACAAATACTGGGACAGCTACAACCATCCCTTCAGACAGGATCAGAAG
AACTTAGATCATTATATAATACAGTAGCAACCCTCTATTGTGTGCATCAAAGGATAGAGATAAAAGACAC
CAAGGAAGCTTTAGACAAGATAGAGGAAGAGCAAAACAAAAGTAAGACCACCGCACAGCAAGCGGCC
GCTGATCTTCAGACCTGGAGGAGGAGATATGAGGGACAATTGGAGAAGTGAATTATATAAATATAAAG
TAGTAAAAATTGAACCATTAGGAGTAGCACCCACCAAGGCAAAGAGAAGAGTGGTGCAGAGAGAAAA
AAGAGCAGTGGGAATAGGAGCTTTGTTCCTTGGGTTCTTGGGAGCAGCAGGAAGCACTATGGGCGCAG
CCTCAATGACGCTGACGGTACAGGCCAGACAATTATTGTCTGGTATAGTGCAGCAGCAGAACAATTTGC
TGAGGGCTATTGAGGCGCAACAGCATCTGTTGCAACTCACAGTCTGGGGCATCAAGCAGCTCCAGGCAA
GAATCCTGGCTGTGGAAAGATACCTAAAGGATCAACAGCTCCTGGGGATTTGGGGTTGCTCTGGAAAAC
TCATTTGCACCACTGCTGTGCCTTGGAATGCTAGTTGGAGTAATAAATCTCTGGAACAGATTGGAATCAC
ACGACCTGGATGGAGTGGGACAGAGAAATTAACAATTACACAAGCTTAATACACTCCTTAATTGAAGAA
TCGCAAAACCAGCAAGAAAAGAATGAACAAGAATTATTGGAATTAGATAAATGGGCAAGTTTGTGGAA
TTGGTTTAACATAACAAATTGGCTGTGGTATATAAAATTATTCATAATGATAGTAGGAGGCTTGGTAGGT
TTAAGAATAGTTTTTGCTGTACTTTCTATAGTGAATAGAGTTAGGCAGGGATATTCACCATTATCGTTTCA
GACCCACCTCCCAACCCCGAGGGGACCCGACAGGCCCGAAGGAATAGAAGAAGAAGGTGGAGAGAGA
GACAGAGACAGATCCATTCGATTAGTGAACGGATCTCGACGGTATCGGTTAACTTTTAAAAGAAAAGGG
GGGATTGGGGGGTACAGTGCAGGGGAAAGAATAGTAGACATAATAGCAACAGACATACAAACTAAAG
AATTACAAAAACAAATTACAAAAATTCAAAATTTTATCGATCACGAGACTAGCCTCGAGAAGCTTGATTA
TATACTAGTATGAAACCCCACCTGTAGGTTTGGCAAGCTAGCTGCAGTAACGCCATTTTGCAAGGCATG
GAAAAATACCAAACCAAGAATAGAGAAGTTCAGATCAAGGGCGGGTACATGAAAATAGCTAACGTTGG
GCCAAACAGGATATCTGCGGTGAGCAGTTTCGGCCCCGGCCCGGGGCCAAGAACAGATGGTCACCGCA
GTTTCGGCCCCGGCCCGAGGCCAAGAACAGATGGTCCCCAGATATGGCCCAACCCTCAGCAGTTTCTTA
AGACCCATCAGATGTTTCCAGGCTCCCCCAAGGACCTGAAATGACCCTGCGCCTTATTTGAATTAACCAA
TCAGCCTGCTTCTCGCTTCTGTTCGCGCGCTTCTGCTTCCCGAGCTCTATAAAAGAGCTCACAACCCCTCA
CTCGGCTTAATTAAAAGCTTTATATAATCAGCATCACAAGTTTGTACAAAAAAGCAGGCTCCATGGATTA
CAAGGATGACGACGATAAGAGCCCGGGCGGATCCGAATCTGGTTATTATGACAGAATGGATTATGAA
GATGACAGATTAAGAGATGGAGAAAGGTGTAGGGATGATTCTTTTTTTGGTGAGACCTCGCATAACT
ATCATAAATTTGACAGTGAGTATGAGAGAATGGGACGTGGAAGCGGAAGCCCAAAAAAGAAGAGAA
AGGTAGACTAAGACCCAGCTTTCTTGTACAAAGTGGTGATGCTCTAGAATGCATGCATGCCGTACGATC
GCCGCCTCAGGCCTGAGTTCGAAGTATACGTCGACAATCAACCTCTGGATTACAAAATTTGTGAAAGATT
GACTGGTATTCTTAACTATGTTGCTCCTTTTACGCTATGTGGATACGCTGCTTTAATGCCTTTGTATCATGC
TATTGCTTCCCGTATGGCTTTCATTTTCTCCTCCTTGTATAAATCCTGGTTGCTGTCTCTTTATGAGGAGTT
GTGGCCCGTTGTCAGGCAACGTGGCGTGGTGTGCACTGTGTTTGCTGACGCAACCCCCACTGGTTGGGG
CATTGCCACCACCTGTCAGCTCCTTTCCGGGACTTTCGCTTTCCCCCTCCCTATTGCCACGGCGGAACTCA
TCGCCGCCTGCCTTGCCCGCTGCTGGACAGGGGCTCGGCTGTTGGGCACTGACAATTCCGTGGTGTTGT
CGGGGAAGCTGACGTCCTTTCCATGGCTGCTCGCCTGTGTTGCCACCTGGATTCTGCGCGGGACGTCTTC
TGCTACGTCCCTTCGGCCTCAATCAGCGGACTTCCTTCCCGCGGCCTGCTGCCGGCTCTGCGGCCTCTTCC
GCGTCTTCGCCTTCGCCCTCAGACGAGTCGGATCTCCCTTTGGGCCGCCTCCCCGCCTGGAATTCCTGCA
GCCCGGGGGATCAAGGATCTGCGATCGCTCCGGTGCCCGTCAGTGGGCAGAGCGCACATCGCCCACAG
TCCCCGAGAAGTTGGGGGGAGGGGTCGGCAATTGAACGGGTGCCTAGAGAAGGTGGCGCGGGGTAAA
CTGGGAAAGTGATGTCGTGTACTGGCTCCGCCTTTTTCCCGAGGGTGGGGGAGAACCGTATATAAGTGC
AGTAGTCGCCGTGAACGTTCTTTTTCGCAACGGGTTTGCCGCCAGAACACAGCTGAAGCTTCGAGGGGC
TCGCATCTCTCCTTCACGCGCCCGCCGCCCTACCTGAGGCCGCCATCCACGCCGGTTGAGTCGCGTTCTG
CCGCCTCCCGCCTGTGGTGCCTCCTGAACTGCGTCCGCCGTCTAGGTAAGTTTAAAGCTCAGGTCGAGAC
CGGGCCTTTGTCCGGCGCTCCCTTGGAGCCTACCTAGACTCAGCCGGCTCTCCACGCTTTGCCTGACCCT
GCTTGCTCAACTCTACGTCTTTGTTTCGTTTTCTGTTCTGCGCCGTTACAGATCCAAGCTGTGACCGGCGC
CTACGATCCACCGGTCGCCACCATGAGCGAGCTGATTAAGGAGAACATGCACATGAAGCTGTACATGGA

GGGCACCGTGGACAACCATCACTTCAAGTGCACATCCGAGGGCGAAGGCAAGCCCTACGAGGGCACCC
AGACCATGAGAATCAAGGTGGTCGAGGGCGGCCCTCTCCCCTTCGCCTTCGACATCCTGGCTACTAGCTT
CCTCTACGGCAGCAAGACCTTCATCAACCACACCCAGGGCATCCCCGACTTCTTCAAGCAGTCCTTCCCT
GAGGGCTTCACATGGGAGAGAGTCACCACATACGAAGACGGGGGCGTGCTGACCGCTACCCAGGACAC
CAGCCTCCAGGACGGCTGCCTCATCTACAACGTCAAGATCAGAGGGGTGAACTTCACATCCAACGGCCC
TGTGATGCAGAAGAAAACACTCGGCTGGGAGGCCTTCACCGAGACGCTGTACCCCGCTGACGGCGGCC
TGGAAGGCAGAAACGACATGGCCCTGAAGCTCGTGGGCGGGAGCCATCTGATCGCAAACATCAAGACC
ACATATAGATCCAAGAAACCCGCTAAGAACCTCAAGATGCCTGGCGTCTACTATGTGGACTACAGACTG
GAAAGAATCAAGGAGGCCAACAACGAGACCTACGTCGAGCAGCACGAGGTGGCAGTGGCCAGATACT
GCGACCTCCCTAGCAAACTGGGGCACAAGCTTAATTAAAGCGGCCGCGTCGAGAGATGGGGGAGGCTA
ACTGAGCTAGCTTAGTCATGCACCGGGCTGCAGGAATTCGAGCTCGGTACCTTTAAGACCAATGACTTA
CAAGGCAGCTGTAGATCTTAGCCACTTTTTAAAAGAAAAGGGGGGACTGGAAGGGCTAATTCACTCCCA
ACGAAGACAAGATCTGCTTTTTGCTTGTACTGGGTCTCTCTGGTTAGACCAGATCTGAGCCTGGGAGCTC
TCTGGCTAACTAGGGAACCCACTGCTTAAGCCTCAATAAAGCTTGCCTTGAGTGCTTCAAGTAGTGTGTG
CCCGTCTGTTGTGTGACTCTGGTAACTAGAGATCCCTCAGACCCTTTTAGTCAGTGTGGAAAATCTCTAG
CAGTAGTAGTTCATGTCATCTTATTATTCAGTATTTATAACTTGCAAAGAAATGAATATCAGAGAGTGAG
AGGAACTTGTTTATTGCAGCTTATAATGGTTACAAATAAAGCAATAGCATCAC...3' (SEQ ID NO:64)

### Aminoacid sequence:

MDYKDDDDKSPGGSESGYYDRMDYEDDRLRDGERCRDDSFFGETSHNYHKFDSEYERMGRGSGSPKKK
RKVD* (SEQ ID NO:65)

### EXAMPLE

### MATERIALS AND METHODS

### Ethics Statement

All procedures involving human samples were approved by IRCCS San Raffaele Scientific Institute Ethical Committee. All animals were handled in accordance with good animal practice and abiding by the Italian and European legislation. All animal procedures were approved by the local IACUC Committee and the Ministry of Health. The use of human PDX cells was regulated by an MTA and approved by the local Ethical Committee.

### Constructs and cloning procedures

FLAG MATR3 C-terminal truncation mutants 2-288, 2-322 (SEQ ID NO: 30) and 2-798 (SEQ ID NO:31) were generated though mutagenic PCR by the introduction of termination codons into the expression vector pCMV-Tag2B N-terminal FLAG MATR3 full-length (Addgene #32880), using the QuickChange Lightning site-directed mutagenesis kit (Agilent Technologies).

The PCR cycling parameters are reported in Table 1.

**Table 1. Thermocycling conditions used for the mutagenic PCR**

| **Segment** | **Cycles** | **Temperature** | **Time** |
|---|---|---|---|
| 1 | 1 | 95°C | 2 minutes |
| 2 | 18 | 95°C | 20 seconds |
| | | 60°C | 10 seconds |
| | | 68°C | 30 seconds/kb of plasmid length* |
| 3 | 1 | 68°C | 5 minutes |

| | | | |
|---|---|---|---|
| * For example, a 5-kb plasmid requires 2.5 minutes per cycle at 68°C. | | | |

Primers used for replacing specific amino acids with the termination codon are listed in Table 2.

**Table 2. List of primers used for cloning**

| **Construct** | | **Primer sequence** | **SEQ ID NO:** |
|---|---|---|---|
| FLAG MATR3₍₂₋₂₈₈₎ | Fw | CATGGACTCTTACCGAAGTA ATATCCCCATCTGTGCTCT | 66 |
| | Rv | AGAGCACAGATGGGGATAT TACTTCGGTAAGAGTCCATG | 67 |
| FLAG MATR3₍₂₋₃₂₂₎ | Fw | CGTCGATGCCAGCTTCTTTA AGAAATCTACCCAGAATGG | 68 |
| | Rv | CCATTCTGGGTAGATTTCTT AAAGAAGCTGGCATCGACG | 69 |
| FLAG MATR3₍₂₋₇₉₈₎ | Fw | GACTATGTGATACCTTAAAC AGGGTTTTACTGTAAGCTG | 70 |
| | Rv | CAGCTTACAGTAAAACCCTG TTTAAGGTATCACATAGTC | 71 |
| FLAG MATR3_{(289-end)} | Fw | AAAGGATCCTATCCCCATCT GTGCTCTATATGTG | 72 |
| | Rv | AAACTCGAGTTAAGTTTCCT TCTTCTG | 73 |
| DUX4 full-length (attB) | Fw | GGGGACAAGTTTGTACAAA AAAGCAGGCTCCGCCCTCCC GACACCCTCGGAC | 74 |
| | Rv | GGGGACCACTTTGTACAA GAAAGCTGGGTCTAAAGCTC CTCCAGCAGAGCC | 75 |
| DUX4 dbd (attB) | Fw | GGGGACAAGTTTGTACAAA AAAGCAGGCTCCGCCCTCCC GACACCCTCGGAC | 76 |
| | Rv | GGGGACCACTTTGTACAAGA AAGCTGGGTCTAGACCTGCG CGGGCGCCC | 77 |

FLAG MATR3 289-end were cloned into a pCMV-Tag2B vector (Addgene), previously XhoI and BamHI (New England BioLabs) digested and dephosphorylated (Antarctic Phosphatase; New England Biolabs). The coding sequence of interest was amplified by PCR (GoTaq flexi DNA polymerase; Promega) employing MATR3 full-length as template and 5'-end overhang primers containing restriction enzyme sites listed in Table 2. FLAG-MATR3₍₁₅₁₋₂₈₇₎ with 3x NLS was cloned into the mammalian expression vector pCMV-Flag2B (Addgene) that contains 3xNLS after a BamHI restriction site. The vector was digested with BamHI (New England BioLabs) at 37°C for 1 hour and then dephosphorylated (30 min at 37°C and 5 min at 70°C to inactivate the enzyme) using Antarctic Phosphatase (New England BioLabs). The coding sequence of interest was amplified by PCR (Expand High FidelityPLUS PCR System dNTPack, Roche) employing MATR3 full-length as template and 5'-end overhang primers containing BamHI restriction sites. The PCR product was digested at 37°C for 1 hour employing BamHI restriction enzymes (New England BioLabs) and purified using QIAquick PCR purification kit (QIAGEN). Ligation reaction was performed at 25°C for 3 hours using T4 DNA ligase enzyme (Promega). EGFP-MATR3₍₂₁₀₋₂₅₅₎ with 1x NLS was cloned with a linker of 5 glycines into the lentiviral vector pFUGW-EGFP (Addgene). The vector was digested with BsrGI (New England BioLabs) at 37°C for 1 hour and then dephosphorylated (30 min at 37°C and 5 min at 70°C to inactivate the enzyme) using Antarctic Phosphatase (New England BioLabs). The coding sequence of interest was amplified by PCR (Phusion^{®} High-Fidelity DNA Polymerase) employing pET-CPP-MATR3₍₂₁₀₋₂₅₅₎ without PROTAC (synthetized by Genscript) as template and 5'-end overhang primers containing BsrGI restriction sites. The PCR product was digested at 37°C for 1 hour employing BsrGI restriction enzymes (New England BioLabs) and purified using QIAquick PCR purification kit (QIAGEN). Ligation reaction was performed at 4°C overnight using T4 DNA ligase enzyme (Promega). In both cases 5 µl of the ligation product was used to transform TOP10 bacteria using heat shock method (45 seconds at 42°C). Transformed cells were plated on LB agar plates supplemented with antibiotics and grown at 37°C overnight. Colonies derived from the transformation of the ligation products were screened by colony-PCR (GoTaq DNA polymerase; Promega). Positive colonies were verified by sequencing. DUX4 full-length and dbd vectors were generated through the Gateway Technology, employing as DNA template pCS2-mkgDUX4 vector (Addgene #21156) as DNA template, the primers listed in Table 2 and plasmid pcDNA FRT/TO strep-HA as destination vector, kindly provided by Dr. Giulio Superti-Furga (CeMM Research Center for Molecular Medicine of the Austrian Academy of Sciences, 1090 Vienna, Austria). MATR3 constructs for expression in bacterial cells were cloned in pGEX-2tk vector digested with BamHI HF and EcoRI HF (New England BioLabs) for MATR3₍₂₋₁₅₁₎, MATR3₍₁₅₁₋₂₈₇₎ and CPP-MATR3₍₂₁₀₋₂₅₅₎ with and without PROTAC. MATR3₍₂₋₁₅₁₎, MATR3₍₁₅₁₋₂₈₇₎ inserts were amplified by PCR (GoTaq flexi DNA polymerase; Promega) using pCMV FLAG-*MATR3* vector as template and 5'-end overhang primers containing the restriction enzyme sites. CPP-MATR3₍₂₁₀₋₂₅₅₎ with and without PROTAC inserts were amplified by PCR using pET-CPP-MATR3₍₂₁₀₋₂₅₅₎ with and without PROTAC (synthetized by Genscript). In both cases, the ligation reaction was performed overnight at 4°C using DNA T4 ligase enzyme. 5 µl of the ligation product were used to transform TOP10 bacteria using heat shock method (45 seconds at 42°C). Transformed cells were plated on LB agar plates and grown at 37°C overnight. Colonies derived from the transformation of the ligation products were screened by colony-PCR (GoTaq DNA polymerase; Promega). Positive colonies obtained from the transformation were screened by sequencing. Human MATR3 fragment (residues 210-255) for NMR was cloned into pETM11-SUMO3 vector (EMBL) using pGEX-2TK-GST-MATR3₍₁₅₁₋₂₈₇₎ vector as template and 5'-end overhang primers containing the restriction enzyme sites. DUX4 inserts for expression in bacterial cells were amplified by PCR (GoTaq flexi DNA polymerase; Promega) employing pTO-STREP HA DUX4 vector as template and 5'-end overhang primers containing the restriction enzyme sites. DUX4 dbd, HD1 and HD2 inserts were cloned into the bacteria expression vector pET-GB1, which contain the GB1 peptide to improve the protein solubility and 6xHis as tag (J Biomol NMR. 2010 Jan;46(1):23-31). The vector was digested with NcoI and XhoI (New England BioLabs) and purified using QIAquick PCR purification kit (QIAGEN). 6xHis-DUX4 dbd terminal truncation mutant 1- 93 (that corresponds to DUX4 HD1 + linker) was generated though mutagenic PCR by the introduction of a termination codon into the expression vector pET-GB1 DUX4 dbd using the QuickChange Lightning site-directed mutagenesis kit (Agilent Technologies). This kit allows rapid and efficient in vitro mutagenesis using an Ultra high-fidelity DNA polymerase for mutagenic primer-directed replication of both plasmid strands. The procedure needs a supercoiled double stranded DNA vector with an insert of interest and two oligonucleotide primers both containing the desired mutation that anneal to the same sequence on opposite strands of the plasmid.

Primers used for replacing the Gly94 of DUX4 dbd with the termination codon (TAA) are:
- Forward: 5' GCCCGCCAGAAtaaCGGCGAAAGCGG 3' (SEQ ID NO:27)
- Reverse: 5' CCGCTTTCGCCGttaTTCTGGCGGGC 3'(SEQ ID NO:28)
The PCR cycling parameters are reported in Table 1.

Plasmids were transformed into XL-10 gold ultra-competent cells (Agilent Technologies) provided by the kit, by heat shock at 42°C for 45 seconds with 1-10 ng of purified plasmid and plated on kanamycin plates. Colonies obtained from the transformation were screened by sequencing. MATR3₍₂₋₂₈₈₎ constructs for lentiviral expression in FSHD and B-ALL cells include FUGW EGFP-MATR3₍₂₋₂₈₈₎ (obtained by Genscript), and pLBC2-BS-RFCA-BCVIII_FlagMATR3 FL and FlagMATR3₍₂₋₂₈₈₎, cloned using a gateway system into a bicistronic vector carrying a BFP reporter.

### Protein expression and purification

6×His-DUX4 fragments (DUX4-dbd, DUX4 HD1, DUX4 HD2, DUX4 HD1+linker), GST and GST-MATR3 fragments (MATR3₍₂₋₂₈₈₎, MATR3₍₂₋₁₅₁₎, MATR3₍₁₅₁₋₂₈₇₎, CPP-MATR3₍₂₁₀₋₂₅₅₎ with and without PROTAC) were expressed in Rosetta2(DE3) pLys *E.coli* (Novagen) for protein production. Bacteria were transformed by heat shock at 42°C for 45 sec with ~50 ng of plasmid and plated on kanamycin, for 6xHis-DUX4 fragments, or ampicillin plates, for GST and GST-MATR3 fragments. Bacteria from one colony were grown in LB medium supplemented with antibiotics at 37°C until they reached an OD₆₀₀ₙₘ= 0.6-0.7. The induction was made with 1 mM IPTG (Biosciences) for 3 hours at 37°C for GST-MATR3 and GST or 20 hours at 18°C for 6×His-DUX4 dbd. Bacterial pellets were re-suspended in Lysis Buffer 1 [PBS; 5 mM 2-mercaptoethanol, plus 1 tablet of cOmplete^{™}, EDTA-free Protease Inhibitor Cocktail (Roche)] or Lysis Buffer 2 [50 mM NaH₂PO₄, 1M NaCl pH 8.0, plus 1 tablet of cOmplete^{™}, EDTA-free Protease Inhibitor Cocktail (Roche)] for GST- and His-tagged proteins, respectively. Then bacteria were centrifuged at 6000 g at 4°C for 15 minutes. Bacteria were sonicated using Bandelin-sonoplus HD3100 (probe MS73) sonicator [10 min 30 sec on and 30 sec off 80% amplitude], incubated by gentle rotation for 15 minutes at 4°C after adding Triton X-100 (1%; Sigma), and centrifuged at 15000 rpm at 4°C for 20 minutes. The cleared lysates were incubated 1 hour at 4°C with Glutathione-Agarose beads (Sigma) or His-Select Nickel Affinity gel beads (Sigma), for GST- and His-tagged proteins, respectively. Beads were washed with Lysis Buffer 1 or Lysis Buffer 2 plus 10 mM imidazole (Fluka), for GST- and His-tagged proteins respectively. Proteins were eluted with elution solution 1 [20mM glutathione, 100mM Tris-HCl pH 8.0, 120mM NaCl] for GST-tagged protein and with elution solution 2 [50 mM NaH₂PO₄, 1M NaCl, 250 mM imidazole (pH 8.0)] for His-tagged proteins. Proteins were dialyzed overnight at 4°C in Slide-A-Lyzer Dialysis Cassettes (Thermo scientific) in Lysis Buffer. The purification steps and the obtained proteins were analyzed by Coomassie Blue staining loading samples on polyacrylamide gels. After the running, the gel was fixed by boiling 3 times in MilliQ water and then the gel was stained with Coomassie solution and incubate in gentle shaking for 15 minutes. The de-staining of the gel was made adding MilliQ water. Purified proteins were quantified at the Nanodrop and on gel using Bovine Serum Albumin (BSA) as protein standard (NEB).

### Protein expression and purification for NMR

The MATR3₍₂₁₀₋₂₅₅₎ fragment was expressed as fusion protein with N-terminal His₍₆₎SUMO3 tag in BL21 (DE3) Rosetta. Protein expression was induced at OD₆₀₀=0.8 with at 37°C for 3 hours with 1mM of IPTG in LB medium or in minimal medium containing ¹⁵NH₄Cl (3g/l) with or without ¹³C-D-glucose (4g/l). The bacterial cells were collected at 8000 rpm for 10 minutes and resuspended in 20 ml of lysis buffer (150 mM NaCl, 20 mM TRIS pH 8.0, 1 mM TCEP, 0.2% (v/v) NP40, 1mg/ml lysozyme, 2 µg/ml DNAseI, 20 µg/ml RNAseI). The fusion protein was extracted by sonication (5 cycle of 2 minutes 1s ON and 1s OFF with a KE76 tip at 60% of amplitude) and centrifuged at 18000g for 1 hour to collect the soluble fraction. His₍₆₎SUMO3-MATR3₍₂₁₀₋₂₅₅₎ fragment was purified from the soluble fraction using HisTrap HP affinity column (GE Healthcare) with a linear gradient of imidazole (Buffer A: 150 mM NaCl, 20 mM TRIS pH 8.0, 20mM imidazole and 1mM TCEP. Buffer B: 150 mM NaCl, 20 mM TRIS pH 8.0, 1 M imidazole and 1mM TCEP) by AKTA purify machine (GE Healthcare). The eluted fusion protein was digested with homemade SENP2 protease (purification protocol described in Methods Mol Biol. 2009; 497: 225-239) at 4°C for 6 hours, followed by affinity chromatography on Ni-NTA column (5ml of Ni²⁺ beads, Qiagen) to remove the tag. The protein solution was dialyzed against water to remove the salt and then lyophilized for 48 hours. The lyophilized MATR3₍₂₁₀₋₂₅₅₎ fragment was resuspended in 1ml of water to perform a size-exclusion chromatography on Superdex 75 prep grade column (GE Healthcare) in a buffer C (20 mM NaH₂PO₄/Na₂HPO₄ pH 6.3, 150 mM NaCl, 1mM TCEP). This construct was used for NMR binding assays. The DUX4 DNA binding domain (DUX4 dbd, residues 15-155) for NMR was cloned into pETM11-SUMO3 vector (EMBL) employing pETGB1-6xHis-DUX4 dbd (residues 1-159) vector as template and 5'-end overhang primers containing the restriction enzyme sites. The construct was expressed with His₍₆₎SUMO3 tag in *E. coli* BL21 (DE3) Rosetta competent cells after overnight induction with 1mM of isopropyl thio-β-D-galactoside (IPTG) at 18°C in LB medium. The bacterial cells were collected by centrifugation at 8000 rpm for 10 minutes and resuspended in 20ml per culture liter of lysis buffer (1 M NaCl, 20 mM TRIS pH 8.0, 1 mM TCEP, 0.2% (v/v) NP40, 1mg/ml lysozyme, 2 µg/ml DNAseI, 20 µg/ml RNAseI, 1 EDTA free protease tablet). The His₍₆₎SUMO3- DUX4 dbd protein was extracted by sonication (5 cycle of 2 minutes 1s ON and 1s OFF with a KE76 tip at 60% of amplitude) and centrifuged at 18000g for 30 minutes to collect the soluble faction. The tagged protein domain was then purified using a Ni-NTA column (Qiagen). In detail, 5 ml of Ni²⁺ beads were equilibrated with 5 column volumes (CV) of buffer 1 (150 mM NaCl, 20 mM TRIS pH 8.0, 10 mM imidazole, 1 mM TCEP) and mixed with the protein soluble faction. The Ni²⁺ beads were then washed with 5CV of buffer 2 (150 mM NaCl, 20 mM TRIS pH 8.0, 20 mM imidazole, 1 mM TCEP), and buffer 3 (0.3 M NaCl, 20 mM TRIS pH 8.0, 30 mM imidazole and 1 mM TCEP). His₍₆₎SUMO3- DUX4 dbd was eluted washing the Ni-NTA column with 3-4 CV of elution buffer (300 mM NaCl, 20 mM TRIS pH 8.0, 0.5 M imidazole and 1 mM TCEP). The eluted protein was cleaved overnight with homemade SENP2 protease and subsequently purified by affinity chromatography on 5ml of Ni²⁺ beads (QIAGEN) to remove the protease and His₍₆₎₋SUMO3 tag from the protein solution. The digested protein was concentrated using VivaSpin 5kDa cut-off (Sartorius) to reach the final volume of 5 ml. Size-exclusion chromatography was performed on a Hiload 16/60 Superdex 75 column (GE Healthcare) in a buffer containing 20 mM NaH₂PO₄/Na₂HPO₄ pH 6.3, 600 mM NaCl, 5 mM DTT. The final yield was ~10mg/L. This construct was used for NMR binding assays.

### MATR3₍₂₁₀₋₂₅₅₎ NMR spectroscopy and resonance assignment

NMR experiments were performed at 298K on a Bruker Avance 600 MHz equipped with inverse triple-resonance cryoprobe and pulsed field gradients (Bruker, Karlsruhe, Germany). Typical sample concentration was 0.1-0.2 mM. Data were processed using or Topspin 3.2 (Bruker) and analyzed with CCPNmr Analysis 2.4 (ref). The ¹H, ¹³C, ¹⁵N chemical shifts of the backbone atoms of MATR3₍₂₁₀₋₂₅₅₎ fragment were obtained from HNCA, CBCA(CO)NH, CBCANH, HNCO (acquired in NUS mode) and CON experiments.

### NMR binding assays

Protein concentrations were determined by UV spectroscopy using predicted extinction coefficients (ε) for MATR3₍₂₁₀₋₂₅₅₎ fragment (ε = 7450 M-1cm-1), DUX4 dbd (ε = 6990 M-1cm-1). In order to minimize dilution effect and maximize complex formation, the ¹⁵N/¹³C labelled MATR3₍₂₁₀₋₂₅₅₎ fragment was mixed with unlabeled DUX4 dbd to reach different stochiometric ratios (i.e. 1:0.5, 1:0.75, 1:1, 1:2) in a typical NMR buffer (20 mM NaH₂PO₄/Na₂HPO₄ pH 6.3, 150 mM NaCl, 1 mM DTT) and then overnight stored at 4°C. For each titration point a different sample was prepared. On each sample a 2D water-flip-back ¹⁵N-edited HSQC spectrum was acquired with 512 (100) complex points, 55 ms (60 ms) acquisition times, apodized by 60_{°} shifted squared (sine) window functions and zero filled to 1024 (512) points for 1H (15N). Assignment of each titration spectra in the presence of DUX4 dbd was made by following individual cross-peaks through the titration series. For each residue the weighted average of the ¹H and ¹⁵N chemical shift perturbation (CSP) was calculated as CSP = [(Δ²HN + Δ²N/25)/2]^{1/2}.

We also performed NMR binding experiment based on ¹³C direct detection. 2D-CON experiments were performed on free ¹⁵N/¹³C labelled MATR3₍₂₁₀₋₂₅₅₎ fragment and in complex with unlabeled DUX4 dbd (1:075). For each residue we measured the ¹³C chemical shift perturbation (CSP = |Δ(δ 13C)|) and the signal intensity reduction in ¹³C-¹⁵N CON spectra (I = 1-[(I₀/I)/Iᵣₐₜᵢₒₘₐₓ]), where I₀ and I are the single peak intensities of free and bound MATR3₍₂₁₀₋₂₅₅₎ and Iᵣₐₜᵢₒₘₐₓ is the maximum ratio value between I₀/I (used as normalization factor).

### GST pull-down assay

To test the interaction between GST-MATR3 fragments and 6xHis-DUX4 fragments, 40 pmol of GST only, GST-MATR3₍₂₋₂₈₈₎, GST-MATR3₍₂₋₁₅₁₎ or GST-MATR3₍₁₅₁₋₂₈₇₎ were immobilized on Glutathione-Sepharose beads (Healthcare) incubating 1 hour on rotation at 4°C in IPP100 buffer (10 mM Tris-HCl pH 8.00, 100 mM NaCl, 0.1% NP-40) supplemented with 0,1 mM PMSF. Next, 40 pmol of 6xHis-DUX4 dbd, 6xHis-DUX4 HD1, 6xHis-DUX4 HD2 or 6xHis-DUX4 HD1+linker was added incubating on rotation for 2 hours at 4°C. Beads were washed 4 times with IPP100 buffer, boiled in 50 µl of Laemmli Sample Buffer (Bio-Rad), centrifuged and the eluted proteins were collected. Samples were stored at -20°C. Pull-down samples were boiled at 95°C for 5 minutes and bounded fractions (10%) were loaded on two identical 12% or 15% SDS-Page acrylamide gel.

### His pull-down assay

To test the interaction between 6xHis-DUX4 dbd and MATR3 synthetic peptides (prepared by CASLO; Lot No.: P230321-01-01, P230321-01-02, Lot No.: P230321-01-03), 40 pmol of 6xHis-DUX4 dbd were immobilized on His-Select Nickel Affinity gel beads (Sigma) incubating 1 hour on rotation at 4°C in IPP100 buffer (10 mM Tris-HCl pH 8.00, 100 mM NaCl, 0.1% NP-40) supplemented with 0,1 mM PMSF. Next, 4 pmol of MATR3 synthetic peptides were added incubating on rotation for 2 hours at 4°C. MATR3 peptides were incubated with the beads as negative control. Beads were washed 4 times with IPP100 buffer, boiled in 50 µl of Laemmli Sample Buffer (Bio-Rad), centrifuged and the eluted proteins were collected. Samples were stored at -20°C. Pull-down samples were boiled at 95°C for 5 minutes and bounded fractions (10%) were loaded on 15% SDS-Page acrylamide gel. The immunoblotting was performed following the procedure reported above but the wet transfer was performed at 200mA for 1.5 hours (instead of 2 hours) at 4°C. To test the interaction between DUX4 dbd and 6xHis-SUM03-MATR3₍₂₁₀₋₂₅₅₎, 40 pmol of 6xHis-SUMO3-MATR3₍₂₁₀₋₂₅₅₎ were immobilized on His-Select Nickel Affinity gel beads (Sigma) incubating 1 hour on rotation at 4°C in IPP100 buffer (10 mM Tris-HCl pH 8.00, 100 mM NaCl, 0.1% NP-40) supplemented with 0,1 mM PMSF. Next, 40 pmol of DUX4₍₁₅₋₁₅₅₎ without tag were added incubating on rotation for 2 hours at 4°C. DUX4₍₁₅₋₁₅₅₎ alone was incubated with the beads as negative control. Beads were washed 4 times with IPP100 buffer, boiled in 50 µl of Laemmli Sample Buffer (Bio-Rad), centrifuged and the eluted proteins were collected. Samples were stored at -20°C. Pull-down samples were boiled at 95°C for 5 minutes and bounded fractions (10%) were loaded on two identical 15% SDS-Page acrylamide gel. The immunoblotting was performed following the procedure reported below.

### Immunoblotting

Electrophoresis was performed at 120V at RT in 1X Tris-Glycine SDS running buffer pH 8.3 [25mM Trizma (Sigma-Aldrich); 192mM Glycine (Sigma-Aldrich); 0,1% SDS (National Diagnostics)] prepared with milliQ water. To transfer proteins to nitrocellulose blotting membrane (GE Healthcare Life Sciences), a wet transfer method was used. The transfer was performed at 200mA for 2 hours at 4°C using 1X Tris-Glycine transfer buffer pH 8.3 [25mM Trizma (Sigma-Aldrich); 192mM Glycine (Sigma-Aldrich)] plus 20% methanol (Sigma-Aldrich). For hybridization, the nitrocellulose filters were saturated with 5% skim milk (Sigma- Aldrich) in D-PBS 0,1% Tween-20 (National Diagnostics) for 1 hour at RT. Incubation with the primary antibody was performed in 5% skim milk D-PBS 0,1% Tween-20, rocking overnight at 4°C. Filters were then washed 3 times with D-PBS 0,1% Tween-20 solution at RT. Secondary antibody conjugated to horseradish peroxidase was used at 1: 10.000 in 5% skim milk D-PBS 0,1% Tween-20 for 1 hour at room temperature. Filters were washed again as previously described.

For detection, filters were incubated for 5 minutes at RT with horseradish peroxidase (HRP) chemiluminescent substrate (SuperSignal West Pico Chemiluminescent Substrate or SuperSignal West Dura Chemiluminescent; Thermo Fisher Scientific).

Information for antibodies is provided in Table 3.

| **Primary antibody** | **Brand** | **Code** | **Dilution for WB** | **Specie** |
|---|---|---|---|---|
| α-6xHis | Clontech | 631212 | 1:5000 | Mouse |
| α-GST | Pharmacia Biotech | 27-4577-01 | 1:25000 | Goat |
| α-HA.11 | BioLegend | 901514 | 1:1000 | Mouse |
| α-FLAG M2 | Sigma | 1002600763 | 1:1000 | Mouse |
| α-DUX4 dbd | Abcam | ab220987 | 1:1000 | Rabbit |
| α-MATR3 | Thermo Fisher Scientific | PA5-57720 | 1:1000 | Rabbit |
| α-Tubulin | Sigma | T9026 | 1: 10000 | Mouse |
| Streptavidin-Peroxidase Polymer | Sigma | S 2438 | 1:5000 | |

**Table 3. Primary and secondary antibodies employed for immunoblotting**

| **Secondary antibody** | **Brand** | **Code** | **Dilution for WB** |
|---|---|---|---|
| α-Mouse IgG (H+L) HRP | Jackson Immunoresearch | 715-035-150 | 1:10000 |
| α-Goat IgG (H+L) HRP | Jackson Immunoresearch | 705-035-147 | 1:10000 |
| α-Rabbit IgG (H+L) HRP | Jackson Immunoresearch | 711-035-152 | 1:10000 |

### Cell culture

HEK293 (human embryonic kidney cells) Flip-In and HEK293T cells were grown in DMEM high glucose medium with L-Glutamine and Sodium Pyruvate (EuroClone) supplemented with 10% Fetal Bovine Serum (FBS, Thermo Fisher Scientific) and 1% penicillin/streptomycin (Pen/Strep, Thermo Fisher Scientific). FSHD and control primary human myoblasts were kindly provided by Dr. Rabi Tawil, the Richard Fields Center for FSHD Research biobank, Department of Neurology, University of Rochester, NY, USA (https://www.urmc.rochester.edu/neurology/fields-center.aspx). Myoblasts were grown in F10 medium (Sigma-Aldrich), supplemented with 20% FBS, 1% Pen-Strep, 10 ng/ml bFGF (Tebu-bio), and 1 µM dexamethasone (Sigma-Aldrich). To induce differentiation, myoblasts were plated at a confluence of 50,000 cells/cm2 and 24h after seeding, growth medium was replaced by DMEM:F12 (1: 1, Sigma-Aldrich) supplemented with 20% knockout serum replacer (KOSR, Thermo Fisher Scientific), 3.151 g/L glucose, 10 mM MEM non-essential amino acids (Thermo Fisher Scientific), 100 mM sodium pyruvate (Thermo Fisher Scientific). Differentiation was carried out for 96h. For freezing of adherent lines, cells were washed with D-PBS (EuroClone), detached with 1X trypsin (EuroClone), harvested, and centrifuged at 1100 rpm for 5 minutes. Cell pellets were resuspended in FBS and transferred to cryovials. 10% DMSO (Dimethyl Sulfoxide; Sigma-Aldrich) was added as cryoprotector. Cryovials were frozen into isopropanol filled cryoboxes, stored at -80°C and then long-term stored in liquid nitrogen. Human lymphoid leukemia cell lines, NALM6 and REH, were purchased from DMSZ and grown in RPMI-1640, 10-20% FBS, 2 mM L-Glutamine, 1 % Pen/Strep. Human DUX4-r patient derived xenograft (PDX) cells were obtained by Prof Jeremias lab (German Research Center for Environmental Health, Munich) and used for in vivo xeno-transplantation experiments. Short term culture of PDX cells was performed in RPMI-1640, 20 % FBS, 2 mM L-Glutamine, 1% Pen/Strep, 0.2% Gentamicin, 1%NA-pyruvate, 1x Insulin-Transferrin-Selenium, α-Thioglycerol. Cells were frozen in FBS-10% DMSO and stored in liquid nitrogen.

### Generation of DUX4 Flp-In T-REx 293 Cell Line

DUX4 coding sequence was cloned in frame into a pCDNA5/FRT/STREP-HA backbone using the Gateway gene cloning strategy. The plasmid was then co-transfected together with the pOG44 Flp-Recombinase Expression vector (ThermoFisher) into Flp-In T-REx 293 cells (Thermo Fisher Scientific) to generate a STREP-HA-DUX4-inducible cell line, according to the vendor protocol. Cells were then selected using both Blasticidin (Thermo Fisher Scientific) and Hygromycin B (Thermo Fisher Scientific) and resistant clones expanded and used for the experiments. STREP-HA DUX4 Flp-In T-REx 293 cells were growth in DMEM 10% Tetracycline-Free FBS (GIBCO) supplemented with 1% Pen/Strep (Thermo Fisher Scientific) and STREP-HA DUX4 expression was induced upon doxycycline (MERCK) administration. The parental Flp-In T-REx 293 cells were grown in parallel and used as negative control.

### Transfection of siRNA and plasmids

Transfection of siRNA was performed by using Lipofectamine 3000 Transfection Reagent (Thermo Fisher Scientific), following the manufacturer's instructions. For FSHD muscle cells, siRNAs were delivered 24h after induction of differentiation. Medium was replaced the day after transfection and myotubes were harvested 96h after induction of differentiation. Plasmids were delivered by using Lipofectamine LTX Reagent with PLUS Reagent (Thermo Fisher Scientific), following the manufacturer's instructions. When transfection of both siRNA and plasmid was required, HEK293 cells were reverse transfected with siRNA by using Lipofectamine 3000 Transfection Reagent and the day after they were transfected with DUX4 construct by using Lipofectamine LTX Reagent with PLUS Reagent, following manufacturer's instructions. Cells were harvested 48h after the last transfection.

### Lentiviral production and transduction of FSHD muscle cells

For MATR3 overexpression experiments in FSHD muscle cells, lentiviral particles were produced in HEK293T cells. Briefly, 4×10⁶ HEK293T cells were seeded in 10 cm dish plate and the day after transfected with 6.5 µg of lentiviral vectors carrying either GFP alone (pFUGW:GFP, a kind gift from Shanahan CM lab) or MATR3 cDNA fused to GFP (pFUGW:GFP-MATR3), 6 µg of pCMV-dR8.91 plasmid and 0.65 µg of pCMV-VSV-G plasmid. Three virus collections were performed for each construct. Viral preparation was concentrated of 100-fold by ultra-centrifuging the suspension at 20,000 rpm for 2h at 4°C and then resuspended in 250 µl of Opti-MEM Reduced Serum Medium (Thermo Fisher Scientific) and stored at -80°C. FSHD muscle cells in 12 wells plates, 24h after induction of differentiation, were transduced with 65 µl of concentrated virus in differentiation medium containing 8 µg/ml polybrene and harvested 72h after infection.

### ALL lines and PDX transduction

Lentiviral production was carried out in HEK293T packaging cells using the calcium phosphate method as previously described (Hum Mol Genet. 2015 Mar 1;24(5):1256-66). NALM6 (DUX4-r +ve) and REH (DUX4-r -ve) B-ALL cells were seeded at 10⁶cells/ml and transduced by directly adding the vectors in the culture medium at MOI 10 with FUGW GFP or FUGW GFP-MATR3 or GFP-MATR3₂₋₂₈₈ lentiviral vectors (LVs), where the GFP is fused to the N-terminal of the GOI; or with pLBC2-BS-RFCA-BCVIII bicistronic/BFP LVs. GFP/BFP expression levels in the transduced samples were analyzed by flow cytometry using a FACS CantoII (BD) or Cytoflex S (Beckman Coulter) after a minimum of 48h. For proliferation assays the cells were seeded at 2×10⁵cells/ml in complete medium and cell number was assessed by trypan blue cell counting at the indicated time points. For NALM6 xenograft transplantation the BFP+ve cells were purified by FACS sorting prior injection into NSG immunocompromised animals. Human patient 811 PDX cells (Nat Commun. 2021 Sep 27;12(1):5655) were transduced by 2 rounds of spin-infection (45 minutes, 1800rpm at 32°C) using freshly concentrated high titer/high MOI FUGW LVs. For the xenograft transplantation experiment the cells were infused into NSG immunocompromised animals after 24h from the infection by tail vein injection.

### Xenograft transplantations

For the NALM6 xenograft 10⁵ BFP+ve cells were infused into 8-10 weeks NSG animals (Charles River Laboratories Italia, SRL) by tail vein injection and leukemia progression was monitored through peripheral blood bleedings. The mice were sacrificed at 4 weeks when showing signs of sickness. The single cell suspension from the spleen of the mice was processed with Red blood cell (RBC) lysis buffer (Invitrogen) and used for flow cytometry staining of human CD371. For PDX transplantations 10⁵ GFP+ve cells were purified through FACS sorting and infused in 8-10 weeks NSG animals. The engraftment and expansion of the human cells into the animals was monitored by tail vein bleedings. Unless otherwise stated, mice were sacrificed when reaching a threshold of 70% engraftment in the PB or when showing signs of sickness. Bone marrow, spleens and PB were processed to obtain a single cell suspension and lysed with Red blood cell (RBC) lysis buffer (Invitrogen). Expression of the GFP reporter and human markers distribution was detected by flow cytometry.

### RNA extraction, reverse transcription, and quantitative real-time PCR

Total RNA was extracted from HEK293 cells, healthy or FSHD myotubes and B-ALL cells using PureLink RNA Mini Kit (Thermo Fisher Scientific)) or the NucleoSpin RNA XS, Micro kit for RNA purification (Macherey Nagel), following the manufacturers' instructions. Briefly, cells were lysed in Lysis buffer supplemented with 2-mercaptoethanol or TCEP and homogenized by passing the lysate 5-10 times through a 21-gauge syringe needle or through homogenizing cartridges (Macherey Nagel). After adding one volume of 70% ethanol, lysate was loaded onto the spin cartridge provided by the kit, washed, treated with DNAseI (PureLink DNase Set, Thermo Fisher Scientific; or rDNase, Macherey Nagel) and eluted in RNAse-free water. cDNA synthesis was performed using SuperScript III First-Strand Synthesis System (Thermo Fisher Scientific), following the manufacturer's instructions. Quantitative real-time PCR (qPCR) was performed with SYBR GreenER qPCR SuperMix Universal (Thermo Fisher Scientific) using CFX96 or CFX384 Real-Time PCR Detection System (Bio-Rad). Primers used for RT-qPCR are listed in Table 4. Relative quantification was performed using the ΔΔCt method.

**Table 4. List of primers used for RT-qPCR**

| **Target gene** | | **Sequence** | **SEQ ID NO:** |
|---|---|---|---|
| GAPDH | Fw | TCAAGAAGGTGGTGAAGCAGG | 23 |
| | Rv | ACCAGGAAATGAGCTTGACAAA | 26 |
| MATR3 | Fw | ATCAATGGAGCAAGTCACAGTC | 78 |
| | Rv | TGCAACATGAATGGATCACCC | 79 |
| ILF2 | Fw | CTCAGACTCTCGTCCGAATCC | 80 |
| | Rv | CAGAAGCAAGATAGCTGGCATC | 81 |
| PRKDC | Fw | GAGAAGGCGGCTTACCTGAG | 82 |
| | Rv | CGAAGGCCCGCTTTAAGAGA | 83 |
| RUVBL1 | Fw | GGCATGTGGCGTCATAGTAGA | 84 |
| | Rv | CACGGAGTTAGCTCTGTGACT | 85 |
| C1QBP | Fw | CGTGTGCTGGGCTCCTC | 86 |
| | Rv | AAAGCTTTGTCTCCGTCGGT | 87 |
| CDC23 | Fw | CTGCGAGTACCTCCATGGTC | 88 |
| | Rv | AGAGAGAAAGCCAACTCCGC | 89 |
| CDC27 | Fw | TGCTGACGTGTTTCTTGTCC | 90 |
| | Rv | TTGCACTGCCTTTCATTCTG | 91 |
| SMARCC2 | Fw | CCGTGACCCAGTTCGACAAC | 92 |
| | Rv | CGGCAGTTTAGTGAGCGGT | 93 |
| ANAPC7 | Fw | GCTTTTCGAGTCAGTGCTGC | 94 |
| | Rv | GGGGAGAATAACTCAGGGTTG | 95 |
| SLC25A5 | Fw | TTGATTTTGCCCGTACCCGT | 96 |
| | Rv | GGATCCGGAAGCATTCCCTT | 97 |
| DUX4 (overexpressed) | Fw | GCGCAACCTCTCCTAGAAAC | 98 |
| | Rv | AGCAGAGCCCGGTATTCTTC | 99 |
| DUX4 (endogenous) | Fw | CCCAGGTACCAGCAGACC | 100 |
| | Rv | TCCAGGAGATGTAACTCTAATCCA | 101 |
| MBD3L2 | Fw | GCGTTCACCTCTTTTCCAAG | 102 |
| | Rv | GCCATGTGGATTTCTCGTTT | 103 |
| RFPL2 | Fw | CCCACATCAAGGAACTGGAG | 104 |
| | Rv | TGTTGGCATCCAAGGTCATA | 105 |
| TRIM43 | Fw | ACCCATCACTGGACTGGTGT | 106 |
| | Rv | CACATCCTCAAAGAGCCTGA | 107 |
| TRIM48 | Fw | GGAGCTGTGTTTTGGTGACCT | 108 |
| | Rv | GTAGTTCATGCAGATGGGGCA | 109 |
| LEUTX | Fw | GGGAAACTGGCTTCAAAGCTA | 110 |
| | Rv | TGATGGCCGTGTCTGCATT | 111 |
| ZSCAN4 | Fw | TGGAAATCAAGTGGCAAAAA | 112 |
| | Rv | CTGCATGTGGACGTGGAC | 113 |
| DYSTROPHIN | Fw | AGCAAGAGCACAACAATTTGG | 114 |
| | Rv | CCCTGTTCGTCCCGTATCA | 115 |
| DUX4-r | Fw | gctgCTGTGGGGTTTCC | 116 |
| | Rv | AAGGCACCCCAGTGCCC | 117 |
| CLEC12A | Fw | CAGCTCTGTTAACTCACTCATCTT | 118 |
| | Rv | GAGCTGGAGGTGCTTTTTCCC | 119 |
| CD34 | Fw | GCAATGCTTCAACGAGAC | 120 |
| | Rv | TGTCCTTCTTAAACTCCGC | 121 |
| ERG e6alt-e7* | Fw | ggaagttttcagacaaacggattt | 122 |
| *from Nat Genet. 2016 Dec;48(12): 1481-1489 | Rv | accggtccaggctgatctc | 123 |
| ITGA6 | Fw | AGCTGTGCTTGCTCTACCTG | 124 |
| | Rv | GAGCAACAGCCGCTTGTCC | 125 |
| EGFP- MATR3₍₂₋₂₈₈₎ | Fw | tcgccgaccactaccagcagaa | 126 |
| | Rv | CTGGAATGACTTGGAgtacagct | 127 |
| FLAG-MATR3 | Fw | GGATGACGACGATAAGAGCC | 128 |
| | Rv | TAGCAGACAGACCAAAGCTGG | 129 |

### Total protein extraction

Cells were scraped in D-PBS and pelleted at 1100 rpm for 5 minutes at RT. Cell pellets were re-suspended in IP buffer to lysate the membranes [50 mM Tris-HCl pH 7,5; 150 mM NaCl; 1% NP-40; 5 mM EDTA; 5 mM EGTA; protease inhibitors]. After an incubation of 10 minutes on ice, cells were centrifuged at full speed for 10 minutes at 4°C. The total protein concentration of the supernatant was determined using Bradford reagent (Bio-Rad) and read at the spectrophotometer. 4X Laemmli buffer was added to the samples. Samples were boiled at 95°C for 5 minutes and equal amounts of total proteins were loaded on SDS-Page acrylamide gels. The immunoblotting was performed following the procedure reported above.

### Strep-Tactin pull-down assays

HEK293T cells were plated on 10cm cell culture plates and at 90% confluence they were transfected with pTO STREP-HA vector empty or carrying DUX4 full-length or DUX4 dbd using PolyFect Transfection Reagent (QIAGEN). 24 hours after transfection, cells were harvested and nuclear extracts were prepared by lysing the cell membrane with buffer N (300mM sucrose; 10mM Hepes pH 7.9; 10 mM KCl; 0,1mM EDTA; 0,1mM EGTA; 0,1mM DTT; 0,75mM spermidine; 0,15mM spermine; 0,1% NP-40 substitute; protease inhibitors) followed by extraction of nuclear proteins using buffer C420 (20mM Hepes pH 7.9, 420mM NaCl, 25% glycerol, 1mM EDTA, 1mM EGTA, 0.1 mM DTT, protease inhibitors). Nuclear extracts were cleared by ultracentrifugation at 100000 g, 4°C for 1 hour. The pull-down was performed using 600µg of nuclear proteins, adding 2 volumes of HEPES buffer (20mM Hepes pH 8; 50mM NaF; protease inhibitors) and TNN buffer [50mM Hepes pH 8.0; 150mM NaCl; 5mM EDTA; 0.5% NP-40 substitute; 50mM NaF; protease inhibitors] to reduce the NaCl concentration. Nuclear extracts were incubated for 1h at 4°C with Avidin, Benzonase (Sigma Aldrich) and RNase A (Thermo Fisher) and precleared with Protein G sepharose beads (GE Healthcare Life Sciences) for 1h at 4°C with rotation. Protein complexes were obtained by incubation of nuclear extracts with 40µl of Strep-Tactin sepharose beads (IBA Lifesciences) overnight at 4°C in gentle rotation. After 3 washes with IP-buffer (50mM Tris-HCl pH 7.5, 150mM NaCl, 1% NP-40, 1mM EDTA, 0.5mM EGTA), proteins were specifically eluted adding 2,5mM D-Biotin (Sigma-Aldrich). Input (10% or 0,5%) and bound fractions (10%) of the pull down were analyzed by immunoblotting.

### Myotube morphology analysis

For myotube morphology analysis, cells were fixed in 4% PFA, permeabilized with 1% TritonX-100 in PBS and immunostained with mouse MF20 antibody (Developmental Studies Hybridoma Bank; dilution 1:2) followed by Alexa Fluor 488 goat anti-mouse (Molecular Probes; dilution 1:500) and Hoechst (1 mg/ml, Sigma-Aldrich; dilution 1:2.000). Cells were imaged using fluorescence microscope (Observer.Z1, Zeiss). Fusion index analysis was performed with ImageJ software by counting the number of nuclei included or not into myotubes (myosin positive syncytia containing at least 3 nuclei). Three independent differentiation experiments were performed and 5 fields per well were analyzed.

### Cell viability and apoptotic assay

Cell viability in HEK293 and STREP-HA DUX4 Flp-In T-REx 293 cells was measured using the CellTiter-Glo luminescent assay (Promega), based on quantitation of ATP, following the manufacturer's instructions. Apoptosis was measured through Caspase-Glo 3/7 luminescent assay (Promega), based on quantification of caspase-3 and -7 activity, following the manufacturer's instructions. Briefly, 100µl of CellTiter-Glo or Caspase-Glo 3/7 Reagent respectively was added to 100µl of cell suspension in a white 96-well plate. The plate was incubated for 1 hour at room temperature in the dark and then luminescence was quantified by Wallac 1420 multilabel Victor3 microplate reader (Perkin Elmer). These assays were performed in STREP-HA DUX4 Flp-In T-REx 293 cells 24h after doxycycline administration, and in HEK293 cells 48h after transfection. Apoptotic levels in primary muscle cells from FSHD patients or healthy individuals transduced with lentiviral vectors carrying BFP-MATR3 or BFP-MATR3 2-288 or BFP only as control (as described above) were determined using the IncuCyte live imaging system (Essen BioScience). Briefly, 24 h post-transduction, differentiation medium was replaced with fresh medium containing 1ul/ml of CASP3/7 assay GREEN reagent (catalog #4440, Essen BioScience) and the 12-well plate was placed into the IncuCyte Live-Cell Analysis System for acquisition of green fluorescence as follows: 36 scans/well every 3 h for 72 h. Signal was measured and analyzed using the IncuCyte software.

### Reporter assay

The reporter assay allows to measure the specific ability of a transcription factor to bind its consensus sequence and to activate the transcription of target genes. 300.000 HEK293 cells/well or 700.000 cells/well were cultured in 12-well plates or in 6-well plate respectively in triplicate. At 90% confluence, cells were co-transfected with different combinations of plasmid using 4 µl of Lipofectamine LTX and Plus reagent (ThermoFisher Scientific). Specifically, in case of DUX4 reporter assay, cells were transfected with 0,4 µg of reporter (pLenti with DUX4 binding site and a minimal promoter controlling the expression of Turbo GFP, previously described in Hum Mol Genet. 2015 Oct 15;24(20):5901-14), 0,8 µg of pTO STREP-HA vector carrying DUX4 full-length, 1,2 µg of FLAG pCMV vector, empty or carrying MATR3 constructs. 24 hours post-transfection the medium was changed. 48 hours after transfection, cells were visualized at the microscope. For the reporter assay of DUX4-r transcriptional ability, the GFP reporter was transfected in combination with a pTO STREP-HA DUX4-r expressing vector and FLAG pCMV MATR3 constructs or EV in a ratio of 0.25:2:2. The cells were assayed after 24 hours for activation of the GFP-reporter by fluorescence microscopy, using Zeiss Observer./1 and the AxioVision software, and by flow cytometry, using the FACS Canto II (BD) and the FlowJo software (BD). The same cells were harvested and washed prior RNA purification which was performed using the PureLink RNA Mini Kit (Invitrogen).

### Flow cytometry

For detection of fluorescent reporters, cells were collected, pelleted to eliminate the media, washed in PBS, and resuspended in PBS prior acquisition. For the surface staining protocols, cells were harvested and centrifuged at 300xg for 5-10 min at 4 °C and washed once in ice cold PBS-2% FBS 2mM EDTA. The stainings were performed adding 50µl of staining cocktail per tube up to 5×10⁶, and incubating the samples for 20 minutes in the dark at 4 °C. Samples were washed once prior acquisition at the FACS CantoII (BD) or Cytoflex S (Beckman Coulter). Cells Sorting was performed using BD FACSAria (BD biosciences) or BD ASTRIOS (BD biosciences). When required, compensation was performed using unstained cells and single stained cells, or unstained cells and single stained UltraComp eBeads (eBioscience). Data analysis was performed with the FlowJo (BD) software. For the analysis of all flow cytometry data, the populations of interest were gated through Forward Scatter Area (FSC-A) versus Side Scatter Area (SSC-A) and doublets cells were excluded by gating through FSC-A versus FSC-Height (FSC-H). A minimum of 10000 events were recorded gating on live single cells for each sample. The same gating strategy was applied consistently to all samples. For the reporter assay, the median fluorescence intensity (MFI) was annotated for GFP+ve cell gates. Antibodies used for the immunophenotyping of B-ALL cells include: APC anti-human CD371 (CLEC12A) Antibody (50C1) BioLegend # 353606; PE anti-human CD38 Antibody (HB-7) BioLegend # 356604; PE-Cyanine7 anti-human CD45 Monoclonal Antibody (30-F11), eBioscience # 25-0451-82.

### Spheroid Assay

REH B-ALL cells carrying a doxycycline inducible DUX4-r fusion were transduced with MATR3₂₋₂₈₈ or BFP control vectors. After 48h from transduction, the cells were seeded at 10⁵cells/ml in RPMI 5%FBS medium in presence of 1µg/ml Doxycycline to induce transgene expression and assessed for spheroid formation. Aggregation in spheroid structures was measured by live imaging using the Incucyte system. Average phase area values were normalized to time 0.

### RNA-sequencing

NGS libraries were generated with the mRNA Stranded TruSeq protocol. Sequencing was performed on an Illumina Nova-Seq 6000 (Illumina, San Diego, CA), obtaining an average of 50 million pair-end reads, 100nt long per sample. Reads were trimmed using Trimmomatic, version 0.39, to remove adapters and exclude low-quality reads from the analysis. The remaining reads were then aligned to the reference genome GRCh38, GENCODE v31, using STAR aligner, version 2.5.3a. FeatureCounts (v 1.6.4) software was used to assign reads to the corresponding genes. Only genes with a CPM (Counts per million) value higher than 1 in at least four samples were retained. Gene expression read counts were exported and analyzed in the R environment (v. 4.0.5) to identify differentially expressed genes (DEGs) using the DESeq Bioconductor library (Genome Biol. 2014;15(12):550). To control differences due to library preparation batches, we included this variable in the DGE design (~condition+batch). P-values were adjusted using a threshold for false discovery rate (FDR) ≤ 0.05 (https://doi.org/10.1111/j.2517-6161.1995.tb02031.x). Using the 500 most variable genes in terms of RPKM (counts per million reads normalized on library sizes and gene lengths), we perform Principal Component Analysis (prcomp function in R) and clustering analysis via heatmap (CRAN package pheatmap - v.1.0.12). Volcano plot analysis was performed in R (using ggplot2 R library (v3.3.5)), labelling the 20 upregulated and downregulated genes with the most significant adjusted pvalue, and heatmaps of differential expressed genes were plotted with the pheatmap R library. The lists of genes induced or repressed by more than two-fold by iDUX4, vDUX4 and enDUX4 proposed by Jagannathan et al. (in supplementary data Jagannathan-Suppl-Table4.xlsx, Hum Mol Genet. 2016 Oct 15;25(20):4419-4431) were compared to the modulated genes of this study, filtered for absolute values of |log2 FC| > 1. Annotation differences between this dataset and our annotation (GENCODE v31 basic annotation) were corrected by updating the gene names from Jagannathan et al. to their most recent gene alias in GENCODE v31. This conversion was performed using the Ensembl IDs, which remain comparable across different annotation versions. To test the statistical significance of the overlap between gene lists, we used the Bioconductor package GeneOverlap (v3.15) using the number of expressed features in our experiment as genome size. List intersections were drawn with the CRAN package "venn" (v1.11).

### Statistical analysis

All statistical analyses were performed using GraphPad Prism 8.0 (GraphPad Software, San Diego, USA). Statistical significance was calculated by Student's t-test or One-way Anova on at least three independent experiments. P-value: *p < 0.05; **p < 0.01; ***p < 0.001; ****p < 0.0001. Details of each dataset are provided in the corresponding figure legend.

### RESULTS

### MATR3 in FSHD

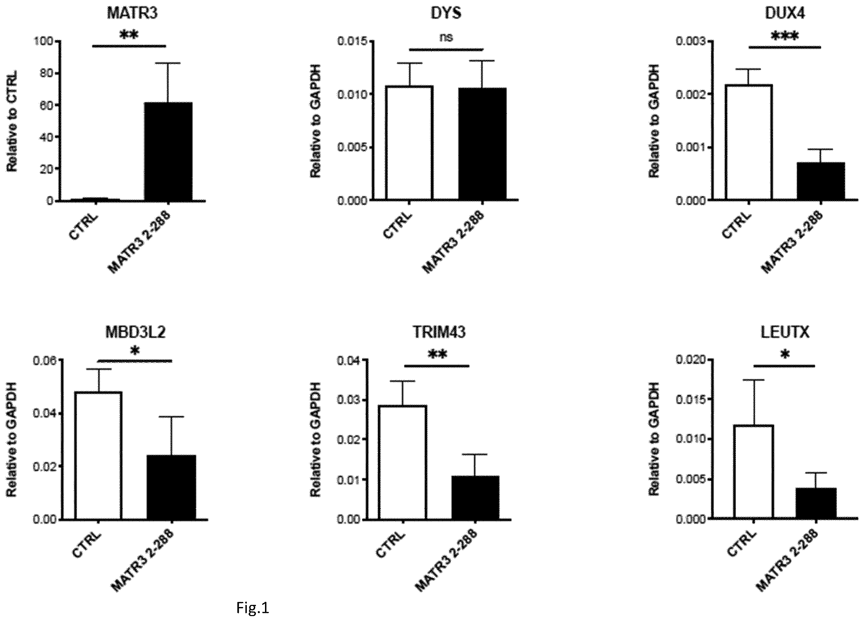

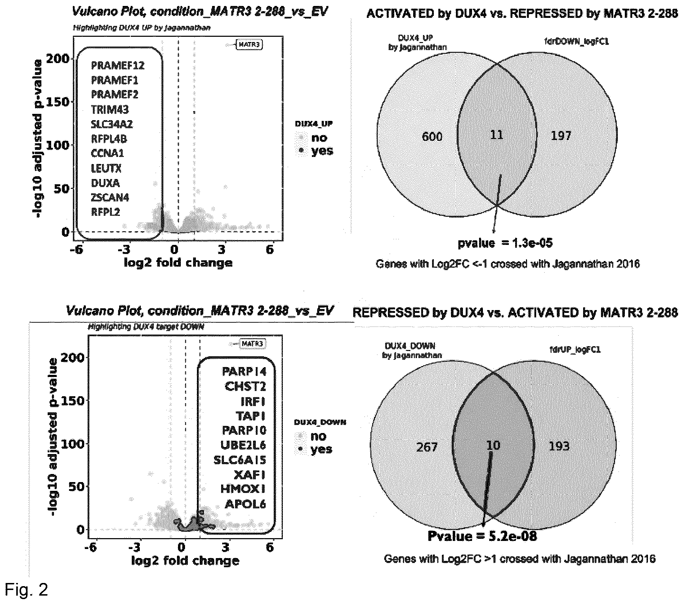

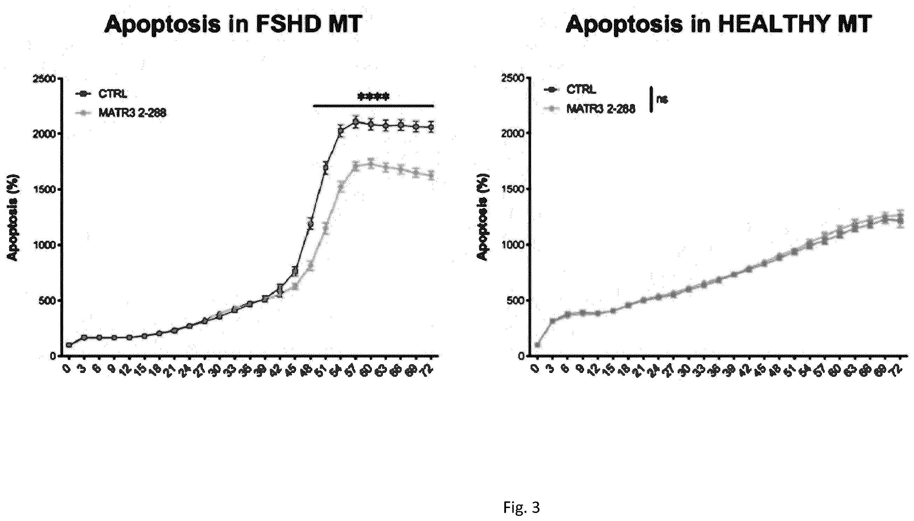

MATR3₂₋₂₈₈ was lentivirally delivered onto primary FSHD muscle cells and it was sufficient to significantly inhibit the expression of DUX4 and its targets (Figure 1). These results were extended genome-wide by using RNA sequencing (Figure 2). MATR3₂₋₂₈₈ delivery causes a significant rescue of FSHD muscle cell viability without instead affecting primary muscle cells derived from healthy donors supporting the safety of MATR3 targeting (Figure 3).

### MATR3 in DUX4-r leukemia

Since the dbd of wt DUX4 is maintained in all DUX4-r variants, the interaction with MATR3 should also be conserved. Indeed, using co-immunoprecipitation inventors demonstrated the ability of MATR3 to interact with a DUX4-r variant derived from ALL patients (Figure 4).

To test if MATR3₂₋₂₈₈ inhibits DUX4-r transcriptional activity, inventors used a reporter system where DUX4-r binding sites control GFP expression. As shown in Figure 5, inventors found that MATR3₂₋₂₈₈ is sufficient to inhibit transcriptional activation by DUX4-r. To test in vitro MATR3 ability to inhibit DUX4-r activity, inventors used NALM6 ALL cells which endogenously express DUX4-r and depend on it for their proliferation. NALM6 cells were transduced with lentiviral vectors expressing a BFP alone or BFP plus MATR3₂₋₂₈₈. MATR3₂₋₂₈₈ was sufficient to downregulate expression of DUX4-r targets (Figure 6). MATR3₂₋₂₈₈ also significantly reduced tumor spheroids formation promoted by ectopic DUX4-r overexpression in REH B-ALL cells (Figure 6). Importantly, MATR3₂₋₂₈₈ significantly inhibited DUX4-r transcriptional activity also in vivo (Figure 7). Moreover, both MATR3 and MATR3₂₋₂₈₈ significantly reduce NALM6 cell proliferation (Figure 8). Importantly, MATR3₂₋₂₈₈ does not affect proliferation of DUX4-r negative REH ALL cells (Figure 8), supporting the specificity of inventors' findings. To investigate the antileukemic potential of MATR3₂₋₂₈₈ in vivo, inventors used DUX4-r patient PDX cells previously shown to be dependent on DUX4-r, which inventors transduced with a GFP-MATR3₂₋₂₈₈ fusion or GFP alone, as control. Inventors found that MATR3₂₋₂₈₈ inhibits DUX4-r leukemia growth causing a significant delay in engraftment and expansion in peripheral blood (PB), compared to GFP alone (Figure 9). This delayed growth rate was reflected in significantly lower bone marrow (BM) white blood cell count (WBCC) and lower total % of GFP+ cells in PB, BM and spleen of the GFP-MATR3₂₋₂₈₈ cohort at sacrifice, compared to GFP alone (Figure 10).

### MATR3 and FSHD and leukemia

To map the portion of MATR3 involved in the inhibition of DUX4, the activity of full-length MATR3 was compared to that of various MATR3 deletion mutants. Intriguingly, a 137 amino acids (aa) N-terminal fragment (MATR3₁₅₁₋₂₈₇ (SEQ ID NO:11)) devoid of any known MATR3 functional domain is sufficient to inhibit expression of DUX4 targets and DUX4-induced cells death (Figure 11). Importantly, pull-down experiments using purified, recombinant proteins demonstrated that MATR3₁₅₁₋₂₈₇ directly binds to DUX4 dbd (Figure 11). To begin mapping the minimal region of MATR3 sufficient to bind DUX4 dbd, 3 synthetic peptides of 60 or 61 aa were used in pulldown experiments with recombinant DUX4 dbd. As shown in Figure 12, peptides 2 and 3 both interacted with DUX4 dbd.

Inventors expressed and purified a recombinant MATR3 fragment MATR3₂₁₀₋₂₅₅. Figure 13 shown that MATR3₂₁₀₋₂₅₅ is sufficient to directly bind to DUX4. Importantly, MATR3₂₁₀₋₂₅₅ is also sufficient to inhibit DUX4 toxicity (Figure 14) and DUX4-r transcriptional activity (Figure 15). To independently confirm the interaction between MATR3 and DUX4 and obtain structural details, inventors performed nuclear magnetic resonance (NMR). Using NMR spectroscopy, inventors first assigned the coordinates of each MATR3₂₁₀₋₂₅₅ amino acid (Figure 16) and obtained an initial characterization of its structure (Figure 17). Next, by NMR inventors confirmed the interaction between DUX4 dbd and MATR3₂₁₀₋₂₅₅ (Figure 18). Moreover, the combination of NMR spectroscopy and chemical shift perturbations allowed to highlight the most important MATR3 amino acids involved in DUX4 binding and locate them on the MATR3₂₁₀₋₂₅₅ structure (Figures 19-21). To determine the DUX4 region involved in MATR3 binding, inventor performed NMR spectroscopy using labeled DUX4 dbd and unlabeled MATR3₂₁₀₋₂₅₅. The DUX4 residues found to be significantly affected in the binding with MATR3 were then visualized on the crystallographic structure of DUX4 dbd in complex with DNA (Cell Rep. 2018 Dec 11;25(11):2955-2962.e3). Considering the residues most affected in terms of CSP and intensity reduction, the MATR3 binding site on DUX4 structure can be identified. The MATR3 binding site is located at the interface between the two DUX4 homeodomains. The α3 of each homeodomain is involved in the binding, thus suggesting that the residues on these two secondary structural elements involved in the DNA binding are also affected in the MATR3 interaction (Figures 22-24). Based on this, inventors asked if MATR3 could compete with DNA for DUX4 binding. Intriguingly, NMR shows that MATR3₂₁₀₋₂₅₅ and DNA compete for DUX4 binding (Figure 25). In summary, the present results show that MATR3 is a natural inhibitor of wt DUX4 in FSHD and rearranged DUX4-r in leukemia. MATR3 and its fragments bind directly to DUX4 DNA-binding domain and blocks activation of DUX4 or DUX4-r targets. In so doing, MATR3 blocks toxicity of wt DUX4 in FSHD and leukemogenic activity of DUX4-r in ALL.

## Claims

1. A peptide or variants thereof wherein the peptide essentially consists of:
- an amino acid sequence comprised in the sequence from aa. 1 to aa. 288 of matrin 3 (MATR3) (SEQ ID NO:1), or
- a variant of an amino acid sequence comprised in the sequence from aa. 1 to aa. 288 of matrin 3 (MATR3),
with the proviso that the peptide does not consist of the amino acid sequence from aa.1 to aa. 287 of matrin 3 (SEQ ID NO:17),
and preferably wherein the amino acid sequence is comprised in the sequence from aa. 1 to aa. 287 or from aa 2 to aa. 288 (SEQ ID NO:18) or from aa. 2 to aa. 287 (SEQ ID NO:19)

2. The peptide or variants thereof according to claim 1, wherein said amino acid sequence is long at least about 45, 50, 55, 60 or 61 amino acids, preferably 46 amino acids.

3. The peptide or variants thereof according to any one of previous claims wherein the amino acid sequence essentially consists of, comprises, or is comprised in the sequence:
- from aa. 210 to aa. 255 of MATR3 (SEQ ID NO:5), or
- from aa. 188 to aa. 248 of MATR3 (SEQ ID NO:3), or
- from aa. 228 to aa. 287 of MATR3 (SEQ ID NO:4), or
- from aa. 209 to aa. 288 of MATR3 (SEQ ID NO:7), or
- from aa. 210 to aa. 288 of MATR3 (SEQ ID NO:8), or
- from aa. 209 to aa. 287 of MATR3 (SEQ ID NO:12), or
- from aa. 210 to aa. 287 of MATR3 (SEQ ID NO:13), or

4. The peptide or variants thereof according to any one of previous claims wherein the amino acid sequence essentially consists of, comprises, or is comprised in the sequence from aa. 188 to aa. 288 of MATR3 (SEQ ID NO:6) or from aa. 151 to aa. 287 of MATR3 (SEQ ID NO:11) or from 188 to aa. 287 of MATR3 (SEQ ID NO:14) or from aa. 149 to aa. 287 of MATR3 (SEQ ID NO:15) or from aa. 151 to aa. 288 of MATR3 (SEQ ID NO:16).

5. The peptide or variants thereof according to any one of previous claims wherein the peptide essentially consists of the amino acid sequence:
- from aa. 210 to aa. 255 of MATR3 (SEQ ID NO:5), or
- from aa. 188 to aa. 248 of MATR3 (SEQ ID NO:3), or
- from aa. 228 to aa. 287 of MATR3 (SEQ ID NO:4), or
- from aa. 151 to aa. 287 **of MATR3** (SEQ ID NO:11), or
- from aa. 1 to aa. 288 of MATR3 (SEQ ID NO:1) or
- from aa. 2 to aa. 288 of MATR3 (SEQ ID NO:18).

6. The peptide or variants thereof according to any one of previous claims wherein the peptide or variants directly bind to DUX4 DNA-binding domain and/or inhibit DUX4 and rearranged DUX4 (DUX4-r) and/or block toxicity of wt DUX4 and leukemogenic activity of DUX4-r and/or compete for DUX4 binding.

7. A targeted protein degradation/degrader (TPD) compound or a nanoparticle comprising at least one peptide or variant thereof according to any one of the previous claims or a peptide essentially consisting of or comprising the sequence from aa. 1 to aa. 287 of MATR3.

8. An isolated nucleic acid encoding the peptide or variant thereof of any one of claims 1-6 or the TPD compound of claim 7.

9. A vector or expression vector comprising an isolated nucleic acid according to claim 8, preferably the vector or expression vector comprises said nucleic acid and a promoter operatively linked thereto or wherein said nucleic acid is operably linked to regulatory sequences capable of directing expression of said nucleic acid encoding said peptide in a host, preferably wherein said expression vector is a viral vector, preferably an AVV vector or a lentivirus vector, and/or wherein the promoter is a muscle-specific and/or a B-cell specific promoter.

10. A host cell comprising and/or expressing a peptide or variant thereof of any of claims 1-6, or a TPD of claim 7, or an isolated nucleic acid of claim 8, or a vector or expression vector of claim 9, preferably the cell is transformed and/or is an eukaryotic cell selected from the group consisting of a mammalian cell, an insect cell, a plant cell, a yeast cell and a protozoa cell, preferably the cell is a bacterial cell.

11. A cell transduced with the vector of claim 9, preferably said cell is an HSPC.

12. A pharmaceutical composition comprising the peptide or variant thereof of any one of claims 1-6 or a peptide essentially consisting of or comprising the sequence from aa. 1 to aa. 287 of MATR3 or the TPD compound or the nanoparticle of claim 7, or the isolated nucleic acid of claim 8, or the vector or the expression vector of claim 9, or the cell of claim 10 or 11 and at least one pharmaceutically acceptable carrier, preferably further comprises a therapeutic agent.

13. The peptide or variants thereof of any one of claims 1-6, or a peptide essentially consisting of or comprising the sequence from aa. 1 to aa. 287 of MATR3, the TPD or the nanoparticle of claim 7, the nucleic acid of claim 8, the vector or expression vector of claim 9, the cell of claim 10, the transduced cell of claim 11 or the pharmaceutical composition of claim 12 for medical use,
preferably for use in the treatment of a condition associated with an aberrant expression and/or function of at least one DUX4 protein and/or of at least one rearranged DUX4 protein preferably the condition associated with aberrant expression and/or function of DUX4 protein and/or of rearranged DUX4 protein is selected from the group consisting of: muscular dystrophy or cancer,
more preferably the cancer is acute lymphoblastic leukemia, or the muscular dystrophy is facioscapulohumeral muscular dystrophy (FSHD).

14. The peptide or variants thereof of any one of claims 1-6, or a peptide essentially consisting of or comprising the sequence from aa. 1 to aa. 287 of MATR3, the TPD or the nanoparticle of claim 7, the nucleic acid of claim 8, the vector or expression vector of claim 9, the cell of claim 10, the transduced cell of claim 11 or the pharmaceutical composition of claim 12 for use in the treatment of acute lymphoblastic leukemia or facioscapulohumeral muscular dystrophy (FSHD).

15. The transduced cell of claim 11 for use in the treatment of acute lymphoblastic leukemia or the viral vector of claim 9 for use in the treatment of facioscapulohumeral muscular dystrophy (FSHD).
